# EUROPEAN PATENT SPECIFICATION

(11) **EP 2 625 166 B1**
(45) Date of publication and mention of the grant of the patent: **24.09.2014**
(21) Application number: 11764738.8
(22) Date of filing: 04.10.2011
(51) Int. Cl.: C07D 209/86, C07D 409/06, C07D 405/12, C07D 409/12, G03F 7/031, C08F 2/46

(54) **OXIME ESTER DERIVATIVES OF BENZOCARBAZOLE COMPOUNDS AND THEIR USE AS PHOTOINITIATORS IN PHOTOPOLYMERIZABLE COMPOSITIONS**
OXIMESTER-DERIVATE VON BENZOCARBAZOL-VERBINDUNGEN UND DEREN VERWENDUNG ALS PHOTOINITIATOREN IN PHOTOPOLYMERISIERBARE ZUSAMMENSETZUNGEN
DÉRIVÉS ESTER D'OXIME DE COMPOSÉS BENZOCARBAZOLE ET LEUR UTILISATION COMME PHOTOINITIATEURS DANS DES COMPOSITIONS PHOTOPOLYMÉRISABLES

(30) Priority: 20.04.2011 US 201161477184 P; 20.04.2011 EP 11163190; 05.10.2010 EP 10186535; 05.10.2010 US 389734 P
(43) Date of publication of application: 14.08.2013
(73) Proprietor: BASF SE, 67056 Ludwigshafen (DE)
(72) Inventor: NISHIMAE, Yuichi, Ibaraki-Shi 567-0887 Osaka (JP); KURA, Hisatoshi, Takarazuka-shi HYOGO 665-0013 (JP); KUNIMOTO, Kazuhiko, Kawanishi 666-0145 (JP); YAMAGAMI, Ryuhei, Osaka-City, 531-0013 Osaka (JP); TANAKA, Keita, 663-8183 Hyogo (JP)
(74) Representative: Bernhardt, Wolfgang Willy-Hans
(86) International application number: PCT/EP2011/067303
(87) International publication number: WO 2012/045736

(56) References cited:
- EP-A1- 2 141 206
- WO-A1-02/100903
- WO-A1-2008/138724

## Description

The invention pertains to new oxime ester compounds based on specific carbazole derivatives and their use as photoinitiators in photopolymerizable compositions.

Oxime ester compounds having carbazole moieties are known in the art as photoinitiators. For example EP2105443, WO 2008/138724 and EP2141206 disclose corresponding compounds. The extremely broad generic scope of JP 2006-36750-A (corresponding to WO2005/080337) also encompasses compounds with a carbazole moiety and inter alia condensed rings. However, the examples only show ring formation to the carbazole moiety including the oxime group. In WO 2008/138732 also different carbazole-group containing photoinitiator compounds are described. Also here the broad generic scope covers a ring formation of substituents at the carbazole group, while no corresponging examples are disclosed.

In photopolymerization technology there still exists a need for highly reactive, easy to prepare and easy to handle photoinitiators. For example, in color filter resist applications, highly pigmented resists are required for the high color quality property. With the increase of the pigment content, the curing of color resists becomes more difficult. Hence, photoinitiators having a high sensitivity are required. In addition, also such new photoinitiators must meet the high requirements of the industry regarding properties like, for example, easy handling, high solubility, thermal stability and storage stability.

It now has been found, that selected compounds are in particular suitable as photoinitiators.
Thus, subject of the invention are compounds of the formula I 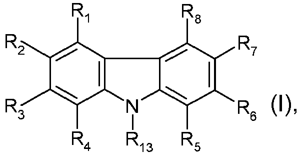 wherein
R₁, R₂, R₃, R₄, R₅, R₆, R₇ and R₈ independently of each other are hydrogen, C₁-C₂₀alkyl, 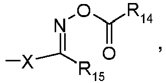 COR₁₆, OR₁₇, halogen, NO₂, or 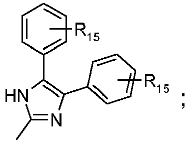 or R₁ and R₂, R₂ and R₃, R₃ and R₄, R₅ and R₆, R₆ and R₇, or R₇ and R₈ independently of each other are C₂-C₁₀alkenyl which is substituted by 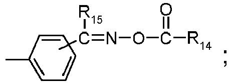 or R₁ and R₂, R₂ and R₃, R₃ and R₄, R₅ and R₆, R₆ and R₇, or R₇ and R₈ independently of each other together are -(CH₂)ₚ-Y-(CH₂)_{q}-;
or R₁ and R₂, R₂ and R₃, R₃ and R₄, R₅ and R₆, R₆ and R₇, or R₇ and R₈ independently
of each other together are 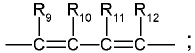 ***provided that*** at least one pair of R₁ and R₂, R₂ and R₃, R₃ and R₄, R₅ and R₆, R₆ and
R₇, or R₇ and R₈ is 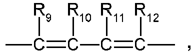 **R₉, R₁₀, R₁₁** and **R₁₂** independently of each other are hydrogen, C₁-C₂₀alkyl which is unsubstituted or substituted by one or more halogen, phenyl, CN, OH, SH, C₁-C₄-alkoxy, (CO)OH or by (CO)O(C₁-C₄alkyl);
or R₉, R₁₀, R₁₁, and R₁₂ independently of each other are unsubstituted phenyl or phenyl substituted by one or more C₁-C₆alkyl, halogen, CN, OR₁₇, SR₁₈ or by NR₁₉R₂₀;
or R₉, R₁₀, R₁₁, and R₁₂ independently of each other are halogen, CN, OR₁₇, SR₁₈, SOR₁₈, SO₂R₁₈ or NR₁₉R₂₀, wherein the substituents OR₁₇, SR₁₈ or NR₁₉R₂₀ optionally form 5- or 6-membered rings *via* the radicals R₁₇, R₁₈, R₁₉ and/or R₂₀ with one of the carbon atoms of the naphthyl ring;
or R₉, R₁₀, R₁₁ and R₁₂ independently of each other are 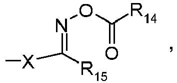 COR₁₆ or NO₂;
**Y** is O, S, NR₂₆ or a direct bond;
**p** is an integer 0,1, 2 or 3;
**q** is an integer 1, 2 or 3;
**X** is CO or a direct bond;
**R₁₃** is C₁-C₂₀alkyl which is unsubstituted or substituted by one or more halogen, R₁₇, COOR₁₇, OR₁₇, SR₁₈, CONR₁₉R₂₀, NR₁₉R₂₀, PO(OCₖH₂ₖ₊₁)₂, or by 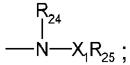 or R₁₃ is C₂-C₂₀alkyl which is interrupted by one or more O, S, SO, SO₂, NR₂₆ or CO,
or is C₂-C₁₂alkenyl which is uninterrupted or is interrupted by one or more O, CO or NMR₂₆,
wherein the interrupted C₂-C₂₀alkyl and the uninterrupted or interrupted C₂-C₁₂alkenyl are unsubstituted or are substituted by one or more halogen;
or R₁₃ is C₄-C₈cycloalkenyl, C₂-C₁₂alkinyl, or C₃-C₁₀cycloalkyl which is uninterrupted or is interrupted by one or more O, S, CO or NR₂₆;
or R₁₃ is phenyl or naphthyl each of which is unsubstituted or substituted by one or more OR₁₇, SR₁₈, NR₁₉R₂₀, 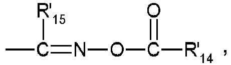 COR₁₆, CN, NO₂, halogen, C₁-C₂₀alkyl, C₁-C₄haloalkyl, C₂-C₂₀alkyl which is interrupted by one or more O, S, CO or NMR₂₆, or each of which is substituted by C₃-C₁₀cycloalkyl or by C₃-C₁₀cycloalkyl which is interrupted by one or more O, S, CO, or NR₂₆;
**k** is an integer 1-10;
**R₁₄** is hydrogen, C₃-C₈cycloalkyl, C₂-C₅alkenyl, C₁-C₂₀alkoxy or C₁-C₂₀alkyl which is unsubstituted or substituted by one or more halogen, phenyl, C₁-C₂₀alkylphenyl or CN; or R₁₄ is phenyl, or naphthyl, each of which is unsubstituted or substituted by one or more C₁-C₆alkyl, C₁-C₄haloalkyl, halogen, CN, OR₁₇, SR₁₈ and/or NR₁₉R₂₀;
or R₁₄ is C₃-C₂₀heteroaryl, C₁-C₈alkoxy, benzyloxy or phenoxy, which benzyloxy and phenoxy are unsubstituted or substituted by one or more C₁-C₆alkyl, C₁-C₄haloalkyl and/or halogen;
**R₁₅** is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, PO(OCₖH₂ₖ₊₁)₂, SO-C₁-C₁₀alkyl, SO₂-C₁-C₁₀alkyl, by C₂-C₂₀alkyl which is interrupted by one or more O, S or NR₂₆, or each of which is substituted by C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂₀heteroaryloxycarbonyl, OR₁₇, SR₁₈ or NR₁₉R₂₀;
or R₁₅ is hydrogen, C₂-C₁₂alkenyl, C₃-C₈cycloalkyl which is uninterrupted or is interrupted by one or more O, CO or NR₂₆;
or R₁₅ is C₁-C₂₀alkyl which is unsubstituted or substituted by one or more halogen, OR₁₇, SR₁₈, C₃-C₈cycloalkyl, C₃-C₂₀heteroaryl, C₆-C₂₀aryloxycarbonyl, C₃-C₂₀heteroaryloxycarbonyl, NR₁₉R₂₀, COOR₁₇, CONR₁₉R₂₀, PO(OCₖH₂ₖ₊₁)₂, 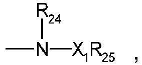 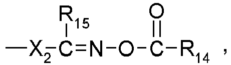 phenyl, or the C₁-C₂₀alkyl is substituted by phenyl which is substituted by halogen, C₁-C₂₀alkyl, C₁-C₄haloalkyl, OR₁₇, SR₁₈, or NR₁₉R₂₀;
or R₁₅ is C₂-C₂₀alkyl which is interrupted by one or more O, SO or SO₂, and which inter-rupted C₂-C₂₀alkyl is unsubstituted or substituted by one or more halogen, OR₁₇, COOR₁₇, CONR₁₉R₂₀, phenyl or by phenyl which is substituted by OR₁₇, SR₁₈ or NR₁₉R₂₀;
or R₁₅ is C₂-C₂₀alkanoyl or benzoyl which is unsubstituted or substituted by one or more C₁-C₆alkyl, halogen, phenyl, OR₁₇, SR₁₈ or NR₁₉R₂₀;
or R₁₅ is naphthoyl which is unsubstituted or is substituted by one or more OR₁₇ or is C₃-C₁₄heteroarylcarbonyl;
or R₁₅ is C₂-C₁₂alkoxycarbonyl which is uninterrupted or is interrupted by one or more O and which interrupted or uninterrupted C₂-C₁₂alkoxycarbonyl is unsubstituted or substituted by one or more hydroxyl groups;
or R₁₅ is phenoxycarbonyl which is unsubstituted or substituted by one or more C₁-C₆alkyl, halogen, C₁-C₄haloalkyl, phenyl, OR₁₇, SR₁₈ or NR₁₉R₂₀;
or R₁₅ is CN, CONR₁₉R₂₀, NO₂, C₁-C₄haloalkyl, S(O)ₘ-C₁-C₆alkyl; S(O)ₘ-phenyl which is unsubstituted or substituted by C₁-C₁₂alkyl or SO₂-C₁-C₆alkyl;
or R₁₅ is SO₂O-phenyl which is unsubstituted or substituted by C₁-C₁₂alkyl; or is diphenyl phosphinoyl or di-(C₁-C₄alkoxy)-phosphinoyl;
m is 1 or 2;
**R'₁₄** has one of the meanings as given for R₁₄;
**R'₁₅** has one of the meanings as given for R₁₅;
**X₁** is O, S, SO or SO₂;
**X₂** is O, CO, S or a direct bond;
**R₁₆** is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, or by C₁-C₂₀alkyl which is interrupted by one or more O, S, or NR₂₆, or each of which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂₀heteroaryloxycarbonyl, OR₁₇, SR₁₈ or NR₁₉R₂₀;
or R₁₆ is hydrogen, C₁-C₂₀alkyl which is unsubstituted or substituted by one or more halogen, phenyl, OH, SH, CN, C₃-C₆alkenoxy, OCH₂CH₂CN, OCH₂CH₂(CO)O(C₁-C₄alkyl), O(CO)-(C₁-C₄alkyl), O(CO)-phenyl, (CO)OH, or (CO)O(C₁-C₄alkyl);
or R₁₆ is C₂-C₁₂alkyl which is interrupted by one or more O, S or NR₂₆;
or R₁₆ is (CH₂CH₂O)ₙ₊₁H, (CH₂CH₂O)ₙ(CO)-(C₁-C₈alkyl), C₂-C₁₂alkenyl or C₃-C₈cycloalkyl;
or R₁₆ is phenyl substituted by SR₁₈ wherein the radical R₁₈ denotes a direct bond to the phenyl or naphthyl ring of the carbazole moiety to which the COR₁₆ group is attached;
**n** is 1-20;
**R₁₇** is hydrogen, phenyl-C₁-C₃alkyl, C₁-C₂₀alkyl which is unsubstituted or substituted by one or more halogen, OH, SH, CN, C₃-C₆alkenoxy, OCH₂CH₂CN, OCH₂CH₂(CO)O(C₁-C₄alkyl), O(CO)-(C₁-C₄alkyl), O(CO)-(C₂-C₄)alkenyl, O(CO)-phenyl, (CO)OH, (CO)O(C₁-C₄alkyl), C₃-C₂₀cycloalkyl, SO₂-(C₁-C₄haloalkyl), O(C₁-C₄haloalkyl) or by C₃-C₂₀cycloalkyl which is interrupted by one or more O;
or R₁₇ is C₂-C₂₀alkyl which is interrupted by one or more O, S or NR₂₆;
or R₁₇ is (CH₂CH₂O)ₙ₊₁H, (CH₂CH₂O)ₙ(CO)-(C₁-C₈alkyl), C₁-C₈alkanoyl, C₂-C₁₂alkenyl, C₃-C₆alkenoyl, or C₃-C₂₀cycloalkyl which is uninterrupted or interrupted by one or more O, S, CO, or NR₂₆;
or R₁₇ is C₁-C₈alkyl-C₃-C₁₀cycloalkyl which is uninterrupted or interrupted by one or more O;
or R₁₇ is benzoyl which is unsubstituted or substituted by one or more C₁-C₆alkyl, halogen, OH or C₁-C₃alkoxy;
or R₁₇ is phenyl, naphthyl or C₃-C₂₀heteroaryl, each of which is unsubstituted or substituted by one or more halogen, OH, C₁-C₁₂alkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenyl-amino or 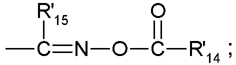 or R₁₇ forms a direct bond to one of the carbon atoms of the phenyl or naphthyl ring on which the group 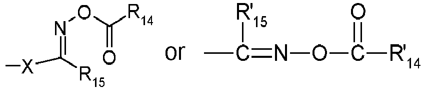 is located;
**R₁₆** is hydrogen, C₂-C₁₂alkenyl, C₃-C₂₀cycloalkyl, or phenyl-C₁-C₃alkyl, wherein the C₂-C₁₂alkenyl, C₃-C₂₀cycloalkyl, or phenyl-C₁-C₃alkyl is uninterrupted or interrupted by one or more O, S, CO, NR₂₆ or COOR₁₇;
or R₁₈ is C₁-C₂₀alkyl which is unsubstituted or is substituted by one or more OH, SH, CN, C₃-C₆alkenoxy, OCH₂CH₂CN, OCH₂CH₂(CO)O(C₁-C₄alkyl), O(CO)-(C₂-C₄)alkenyl, O(CO)-(C₁-C₄alkyl), O(CO)-phenyl or (CO)OR₁₇;
or R₁₈ is C₂-C₂₀alkyl which is interrupted by one or more O, S, CO, NR₂₆ or COOR₁₇;
or R₁₈ is (CH₂CH₂O)ₙH, (CH₂CH₂O)ₙ(CO)-(C₁-C₈alkyl), C₂-C₈alkanoyl or C₃-C₆alkenoyl;
or R₁₈ is benzoyl which is unsubstituted or substituted by one or more C₁-C₆alkyl, halogen, OH, C₁-C₄alkoxy or C₁-C₄alkylsulfanyl;
or R₁₈ is phenyl, naphthyl or C₃-C₂₀heteroaryl, each of which is unsubstituted or substituted by one or more halogen, C₁-C₁₂alkyl, C₁-C₄haloalkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenylamino, (CO)O(C₁-C₈alkyl), (CO)-C₁-C₈alkyl, (CO)N(C₁-C₈alkyl)₂ or 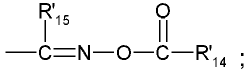 **R₁₉** and **R₂₀** independently of each other are hydrogen, C₁-C₂₀alkyl, C₂-C₄hydroxyalkyl, C₂-C₁₀alkoxyalkyl, C₂-C₅alkenyl, C₃-C₂₀cycloalkyl, phenyl-C₁-C₃alkyl, C₁-C₈alkanoyl, C₁-C₈alkanoyloxy, C₃-C₁₂alkenoyl SO₂-(C₁-C₄haloalkyl), or benzoyl;
or R₁₉ and R₂₀ are phenyl, naphthyl or C₃-C₂₀heteroaryl, each of which is unsubstituted or substituted by one or more halogen, C₁-C₄haloalkyl, C₁-C₂₀alkoxy, C₁-C₁₂alkyl, benzoyl or C₁-C₁₂alkoxy;
or R₁₉ and R₂₀ together with the N-atom to which they are attached form a 5- or 6-membered saturated or unsaturated ring which is uninterrupted or is interrupted by O, S or NR₁₇, and which 5- or 6-membered saturated or unsaturated ring is unsubstituted or substituted by one or more C₁-C₂₀alkyl, C₁-C₂₀alkoxy, =O, OR₁₇, SR₁₈, NR₂₁R₂₂, (CO)R₂₃, NO₂, halogen, C₁-C₄-haloalkyl, CN, phenyl, 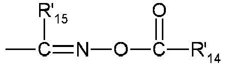 or by C₃-C₂₀cyclalkyl which is uninterrupted or is interrupted by one or more O, S, CO or NR₁₇; or R₁₉ and R₂₀ together with the N-atom to which they are attached form a heteroaromatic ring system which is unsubstituted or substituted by one or more C₁-C₂₀alkyl, C₁-C₄haloalkyl, C₁-C₂₀alkoxy, =O, OR₁₇, SR₁₈, NR₂₁R₂₂, (CO)R₂₃, 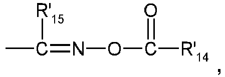 halogen, NO₂, CN, phenyl or by C₃-C₂₀cycloalkyl which is uninterrupted or is interrupted by one or more O, S, CO or NR₁₇;
**R₂₁** and **R₂₂** independently of each other are hydrogen, C₁-C₂₀alkyl, C₁-C₄haloalkyl, C₃-C₁₀cycloalkyl or phenyl;
or R₂₁ and R₂₂ together with N-atom to which they are attached form a 5- or 6-membered saturated or unsaturated ring, which is uninterrupted or is interrupted by O, S or NMR₂₆, and which 5- or 6-membered saturated or unsaturated ring is not condensed or to which 5- or 6-membered saturated or unsaturated ring a benzene ring is condensed;
**R₂₃** is hydrogen, OH, C₁-C₂₀alkyl, C₁-C₄haloalkyl, C₂-C₂₀alkyl which is interrupted by one or more O, CO or NR₂₆, C₃-C₂₀cycloalkyl which is uninterrupted or is interrupted by O, S, CO or NMR₂₆, or R₂₃ is phenyl, naphthyl, phenyl-C₁-C₄alkyl, OR₁₇, SR₁₈ or NR₂₁R₂₂;
**R₂₄** is (CO)OR₁₇, CONR₁₉R₂₀, (CO)R₁₇; or R₂₄ has one of the meanings given for R₁₉ and R₂₀;
**R₂₅** is COOR₁₇, CONR₁₉R₂₀, (CO)R₁₇; or R₂₅ has one of the meanings given for R₁₇;
**R₂₆** is hydrogen, C₁-C₂₀alkyl, C₁-C₄haloalkyl, C₂-C₂₀alkyl which is interrupted by one or more O or CO, or is phenyl-C₁-C₄alkyl, C₃-C₈cycloalkyl which is uninterrupted or is interrupted by one or more O or CO, or is (CO)R₁₉ or is phenyl which is unsubstituted or substituted by one or more C₁-C₂₀alkyl, halogen, C₁-C₄haloalkyl, OR₁₇, SR₁₈, NR₁₉R₂₀ or 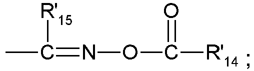 ***provided that*** at least one group 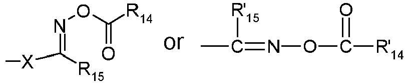 is present in the molecule.

The compounds of the formula I are characterized in that they comprise one or more annelated unsaturated ring(s) at the carbazole moiety. In other words, at least one pair of R₁ and R₂, R₂ and R₃, R₃ and R₄, R₅ and R₆, R₆ and R₇, or R₇ and R₈ is

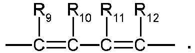

*C₁-C₂₀alkyl* is linear or branched and is, for example, C₁-C₁₈-, C₁-C₁₄-, C₁-C₁₂-, C₁-C₈-, C₁-C₆- or C₁-C₄alkyl or C₄-C₁₂- or C₄-C₈alkyl. Examples are methyl, ethyl, propyl, isopropyl, n-butyl, sec-butyl, isobutyl, tert-butyl, pentyl, hexyl, heptyl, 2,4,4-trimethylpentyl, 2-ethylhexyl, octyl, nonyl, decyl, dodecyl, tetradecyl, pentadecyl, hexadecyl, octadecyl and icosyl. C₁-C₆alkyl has the same meanings as given above for C₁-C₂₀alkyl up to the corresponding number of C-atoms.
Unsubstituted or substituted *C₁-C₂₀alkyl containing one or more C-C multiple bonds,* refers to alkenyl as explained below.
*C₁-C₄haloalkyl* is C₁-C₄alkyl as defined above substituted by halogen as defined below. The alkyl radical is for example mono- or poly-halogenated, up to the exchange of all H-atoms by halogen. It is for example CₙHₓHal_{y}, wherein x+y = 2n+1 and Hal is halogen, preferably F. Specific examples are chloromethyl, trichloromethyl, trifluoromethyl or 2-bromopropyl, especially trifluoromethyl or trichloromethyl.
*C₂-C₄hydroxyalkyl* means C₂-C₄alkyl, which substituted by one or two O-atoms. The alkyl radical is linear or branched. Examples are 2-hydroxyethyl, 1-hydroxyethyl, 1-hydroxypropyl, 2-hydroxypropyl, 3-hydroxypropyl, 1-hydroxybutyl, 4-hydroxybutyl, 2-hydroxybutyl, 3-hydroxybutyl, 2,3-dihydroxypropyl, or 2,4-dihydroxybutyl. *C₂-C₁₀alkoxyalkyl* is C₂-C₁₀alkyl, which is interrupted by one O-atom. C₂-C₁₀alkyl has the same meanings as given above for C₁-C₂₀alkyl up to the corresponding number of C-atoms. Examples are methoxymethyl, methoxyethyl, methoxypropyl, ethoxymethyl, ethoxyethyl, ethoxypropyl, porpoxymethyl, prpopxyethyl, propoxypropyl.
*C₂-C₂₀alkyl which is interrupted by one or more O*, *S, NR₂₆ or CO* is for example interrupted 1-9, 1-5, 1-3 or once or twice by O S, NR₂₆ or CO. If more than one interrupting radicals are present they are of the same kind or different. Two O-atoms are separated by at least one methylene group, preferably at least two methylene groups, namely ethylene. The alkyl groups are linear or branched. For example the following structural units will occur, -CH₂-CH₂-O-CH₂CH₃, -[CH₂CH₂O]_{y}-CH₃, wherein y = 1-9, -(CH₂-CH₂O)₇-CH₂CH₃, -CH₂-CH(CH₃)-O-CH₂-CH₂CH₃, -CH₂-CH(CH₃)-O-CH₂-CH₃, -CH₂-CH₂-S-CH₂CH₃, -CH₂-CH(CH₃)-NR₂₆-CH₂-CH₃, -CH₂-CH₂-COO-CH₂CH₃ or -CH₂-CH(CH₃)-OCO-CH₂-CH₂CH₃.
*C₃-C₁₀cycloalkyl,* C₃-C₁₀cycloalkyl and C₃-C₈Cycloalkyl in the context of the present application is to be understood as alkyl which at least comprises one ring. It is for example cyclopropyl, cyclobutyl, cyclopentyl, cyclohexyl, cyclooctyl, especially cyclopentyl and cyclohexyl. C₃-C₁₀cycloalkyl in the context of the present invention is also meant to cover bicyclic rings, in other words a bridged ring, such as for example 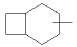 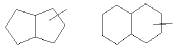 and corresponding rings. Further examples are structures like 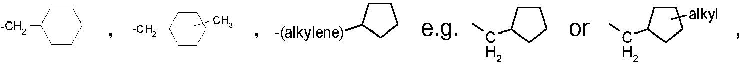 as well as bridged or fused ring systems, e.g. 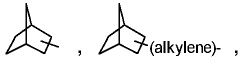 etc. are also meant to be
covered by the term. *C₃-C₂₀cycloalkyl which is interrupted by O*, *S, CO, NR₂₆* has the meanings given above, wherein at least one CH₂-group of the alkyl is exchanged by either O, S, CO or
NR₂₆. Examples are structures like 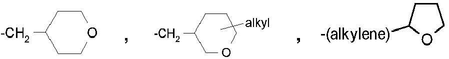 e.g. 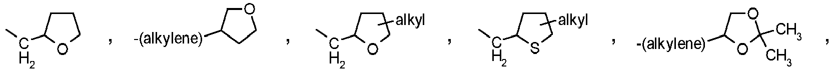 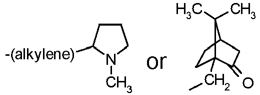 etc..
*C₁-C₈alkyl-C₃-C₁₀cycloalkyl* is C₃-C₁₀cycloalkyl, as defined above which is substituted by one or more alkyl groups with up to 8 carbon atoms. Examples are 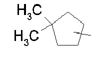 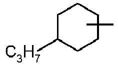 etc.
*C₁-C₈alkyl-C₃-C₁₀cycloalkyl which is interrupted by one or more O* is O-interrupted C₃-C₁₀cycloalkyl as described above which is substituted by one or more alkyl groups with up to 8 carbon atoms. Examples are 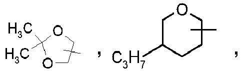 etc.
*C₁-C₁₂alkoxy* is C₁-C₁₂alkyl, which is substituted by one-O-atom. C₁-C₁₂alkyl has the same meanings as given above for C₁-C₂₀alkyl up to the corresponding number of C-atoms. C₁-C₄alkoxy is linear or branched, for example, methoxy, ethoxy, propoxy, iso-propoxy, n-butyloxy, sec-butyloxy, isobutyloxy, tert-butyloxy. C₁-C₈alkoxy and C₁-C₄-alkoxy have the same meanings as abopve up to the corresponding numbers of C-atoms.
*C₁-C₁₂alkylsulfanyl* is C₁-C₁₂alkyl, which is substituted by one-S-atom. C₁-C₁₂alkyl has the same meanings as given above for C₁-C₂₀alkyl up to the corresponding number of C-atoms. C₁-C₄alkylsulfanyl is linear or branched, for example, methylsulfanyl, ethylsul-fanyl, propylsulfanyl, isopropylsulfanyl, n-butylsulfanyl, sec-butylsulfanyl, isobutylsul-fanyl, tert-butylsulfanyl.
*Phenyl-C₁-C₃alkyl* is for example benzyl, phenylethyl, α-methylbenzyl or α,α-dimethylbenzyl, especially benzyl.
*Phenyl-C₁-C₃alkoxyl* is for example benzyloxy, phenylethyloxy, α-methylbenzyloxy or α,α-dimethylbenzyloxy, especially benzyloxy.
*C₂-C₁₂alkenyl* radicals are mono- or polyunsaturated and are for example C₂-C₁₀-, C₂-C₈-, C₂-C₅-alkenyl e.g. vinyl, allyl, methallyl, 1,1-dimethylallyl, 1-butenyl, 3-butenyl, 2-butenyl, 1,3-pentadienyl, 5-hexenyl, 7-octenyl or dodecenyl, especially allyl. C₂-C₅alkenyl radicals have the same meanings as given above for C₂-C₁₂alkenyl radicals up to the corresponding number of C-atoms.
*C₂-C₁₂alkenyl which is interrupted by one or more O*, *CO or NR₂₆* is for example interrupted 1-9, 1-5, 1-3 or once or twice by O S, NR₂₆ or CO. If more than one interrupting radicals are present they are of the same kind or different. Two O-atoms are separated by at least one methylene group, preferably at least two methylene groups, namely ethylene. The alkenyl groups are linear or branched and are defined as above. For example following structural units can be formed -CH=CH-O-CH₂CH₃, -CH=CH-O-CH=CH₂ etc..
*C₄-C₈cycloalkenyl* has one or more double bonds and is for example C₄-C₆-cycloalkenyl or C₆-C₈-cycloalkenyl. Examples are cyclobutenyl, cyclopentenyl, cyclohexenyl or cyclooctenyl, especially cyclopentenyl and cyclohexenyl, preferably cyclohexenyl.
*C₃-C₆alkenoxy* is mono or polyunsaturated and has one of the meanings given for alkenyl above with the attached oxy group up to the corresponding number of C-atoms. Examples are allyloxy, methallyloxy, butenyloxy, pentenoxy, 1,3-pentadienyloxy, 5-hexenyloxy.
*C₂-C₁₂alkinyl* is mono or polyunsaturated, linear or branched and is for example C₂-C₈-, C₂-C₆- or C₂-C₄alkinyl. Examples are ethinyl, propinyl, butinyl, 1-butinyl, 3-butinyl, 2-butinyl, pentinyl hexinyl, 2-hexinyl, 5-hexinyl, octinyl, etc..
*C₂-C₂₀alkanoyl* is linear or branched and is, for example,C₂-C₁₈-, C₂-C₁₄-, C₂-C₁₂-, C₂-C₈-, C₂-C₆- or C₂-C₄alkanoyl or C₄-C₁₂- or C₄-C₈alkanoyl. Examples are acetyl, propionyl, butanoyl, isobutanoyl, pentanoyl, hexanoyl, heptanoyl, octanoyl, nonanoyl, decanoyl, dodecanoyl, tetradecanoyl, pentadecanoyl, hexadecanoyl, octadecanoyl, icosanoyl, preferably acetyl. C₁-C₈alkanoyl has the same meanings as given above for C₂-C₂₀alkanoyl up to the corresponding number of C-atoms.
*C₂-C₁₂alkoxycarbonyl* is a linear or branched and is, for example, methoxycarbonyl, ethoxycarbonyl, propoxycarbonyl, n-butyloxycarbonyl, isobutyloxycarbonyl, 1,1-dimethylpropoxycarbonyl, pentyloxycarbonyl, hexyloxycarbonyl, heptyloxycarbonyl, octyloxycarbonyl, nonyloxycarbonyl, decyloxycarbonyl or dodecyloxycarbonyl, especially methoxycarbonyl, ethoxycarbonyl, propoxycarbonyl, n-butyloxycarbonyl or isobutyloxycarbonyl, preferably methoxycarbonyl.
*C₂-C₁₂alkoxycarbonyl which is interrupted by one or more O* is linear or branched. Two O-atoms are separated by at least two methylene groups, namely ethylene. Said interrupted alkoxycarbonyl is unsubstituted or substituted by one or more hydroxyl groups. *C₆-C₂₀aryloxycarbonyl* is for example phenyloxycabonyl [= phenyl-O-(CO)-], naphty-loxycarbonyl, anthryloxycarbonyl etc.
*C₅-C₂₀heteroaryloxycarbonyl* is C₅-C₂₀heteroaryl-O-CO-.
*C₃-C₁₀cycloalkylcarbonyl* is C₃-C₁₀cyclalkyl-CO-, wherein the cycloalkyl has one of the meanings indicated above up to the corresponding number of C-atoms. *C₃-C₁₀cycloalkylcarbonyl* which is interrupted by one or more O, S, CO, NR₂₆, refers to interrupted cycloalkyl-CO-, where the interrupted cycloalkyl is defined as described above.
*C₃-C₁₀cycloalkyloxycarbonyl* is C₃-C₁₀cycloalkyl-0-(CO)-, wherein the cycloalkyl has one of the meanings indicated above up to the corresponding number of C-atoms. *C₃-C₁₀cycloalkyloxycarbonyl* which is interrupted by one or more O, S, CO, NR₂₆ refers to interrupted cycloalkyl-O-(CO)-, where the interrupted cycloalkyl is defined as described above.
*C₁-C₂₀alkylphenyl* refers to phenyl which is substituted by one or more alkyl groups, where the sum of the C atoms is up to 20.
*C₆-C₂₀aryl* is for example phenyl, naphthyl, anthryl, phenanthryl, pyrene, chrysene, naphthacene, triphenylene etc., in particular phenyl or naphthyl, preferably phenyl. *Naphthyl* is 1-naphthyl or 2-naphthyl.
In the context of the present invention *C₃-C₂₀heteroaryl* is meant to comprise either one ring or a multiple ring system, e.g. a fused ring-system. Examples are thienyl, benzo[b]thienyl, naphtho[2,3-b]thienyl, thianthrenyl, furyl, dibenzofuryl, chromenyl, xanthenyl, thioxanthyl, phenoxathiinyl, pyrrolyl, imidazolyl, pyrazolyl, pyrazinyl, pyrimidinyl, pyridazinyl, indolizinyl, isoindolyl, indolyl, indazolyl, purinyl, quinolizinyl, isoquinolyl, quinolyl, phthalazinyl, naphthyridinyl, quinoxalinyl, quinazolinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl, isoxazolyl, furazanyl, phenoxazinyl, 7-phenanthryl, anthraquinone-2-yl (= 9,10-dioxo-9,10-dihydroanthracen-2-yl), 3-benzo[b]thienyl, 5-benzo[b]thienyl, 2-benzo[b]thienyl, 4-dibenzofuryl, 4,7-dibenzofuryl, 4-methyl-7-dibenzofuryl, 2-xanthenyl, 8-methyl-2-xanthenyl, 3-xanthenyl, 2-phenoxyathiinyl, 2,7-phenoxathiinyl, 2-pyrrolyl, 3-pyrrolyl, 5-methyl-3-pyrrolyl, 2-imidazolyl, 4-imidazolyl, 5-imidazolyl, 2-methyl-4-imidazolyl, 2-ethyl-4-imidazolyl, 2-ethyl-5-imidazolyl, 1*H*-tetrazol-5-yl, 3-pyrazolyl, 1-methyl-3-pyrazolyl, 1-propyl-4-pyrazolyl, 2-pyrazinyl, 5,6-dimethyl-2-pyrazinyl, 2-indolizinyl, 2-methyl-3-isoindolyl, 2-methyl-1-isoindolyl, 1-methyl-2-indolyl, 1-methyl-3-indolyl, 1,5-dimethyl-2-indolyl, 1-methyl-3-indazolyl, 2,7-dimethyl-8-purinyl, 2-methoxy-7-methyl-8-purinyl, 2-quinolizinyl, 3-isoquinolyl, 6-isoquinolyl, 7-isoquinolyl, 3-methoxy-6-isoquinolyl, 2-quinolyl, 6-quinolyl, 7-quinolyl, 2-methoxy-3-quinolyl, 2-methoxy-6-quinolyl, 6-phthalazinyl, 7-phthalazinyl, 1-methoxy-6-phthalazinyl, 1,4-dimethoxy-6-phthalazinyl, 1,8-naphthyridin-2-yl, 2-quinoxalinyl, 6-quinoxalinyl, 2,3-dimethyl-6-quinoxalinyl, 2,3-dimethoxy-6-quinoxalinyl, 2-quinazolinyl, 7-quinazolinyl, 2-dimethylamino-6-quinazolinyl, 3-cinnolinyl, 6-cinnolinyl, 7-cinnolinyl, 3-methoxy-7-cinnolinyl, 2-pteridinyl, 6-pteridinyl, 7-pteridinyl, 6,7-dimethoxy-2-pteridinyl, 2-carbazolyl, 3-carbazolyl, 9-methyl-2-carbazolyl, 9-methyl-3-carbazolyl, β-carbolin-3-yl, 1-methyl-β-carbolin-3-yl, 1-methyl-β-carbolin-6-yl, 3-phenanthridinyl, 2-acridinyl, 3-acridinyl, 2-perimidinyl, 1-methyl-5-perimidinyl, 5-phenanthrolinyl, 6-phenanthrolinyl, 1-phenazinyl, 2-phenazinyl, 3-isothiazolyl, 4-isothiazolyl, 5-isothiazolyl, 2-phenothiazinyl, 3-phenothiazinyl, 10-methyl-3-phenothiazinyl, 3-isoxazolyl, 4-isoxazolyl, 5-isoxazolyl, 4-methyl-3-furazanyl, 2-phenoxazinyl, 10-methyl-2-phenoxazinyl, etc..
*C₃-C₂₀heteroaryl* in particular is thienyl, benzo[b]thienyl, thianthrenyl, thioxanthyl, 1-methyl-2-indolyl or 1-methyl-3-indolyl; especially thienyl.
*C₄-C₂₀heteroarylcarbonyl* are C₃-C₂₀heteroaryl groups as defined above linked to the rest of the molecule via a CO group.
*Substituted aryl* radicals phenyl, naphthyl, C₆-C₂₀aryl or C₅-C₂₀heteroaryl are substituted 1 to 7, 1 to 6 or 1 to 4 times respectively, in particular one, two or three times. It is evident that a defined aryl radical cannot have more substituents than free positions at the aryl ring.
Substituents on the phenyl ring are preferably in positions 4 or in 3,4-, 3,4,5-, 2,6-, 2,4-or 2,4,6-configuration on the phenyl ring.
*Interrupted radicals* which are interrupted once or more times are for example interrupted 1-19, 1-15, 1-12, 1-9, 1-7, 1-5, 1-4, 1-3 or once or twice (it is evident, that the number of interrupting atoms depends on the number of C-atoms to be interrupted). *Substituted radicals,* which are substituted once or more times have for example 1-7, 1-5, 1-4, 1-3 or one or two identical or different substituents.
A radical substituted by *one or more* defined substituents is meant to have either one substitutent or more substituents of identical or different definitions as given.
*Halogen* is fluorine, chlorine, bromine and iodine, especially fluorine, chlorine and bromine, preferably fluorine and chlorine
If R₁ and R₂, R₂ and R₃, R₃ and R₄, or R₅ and R₆, R₆ and R₇, R₇ and R₈ independently of each other together are 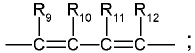 for example the following structures (Ia)-I(i) are formed: 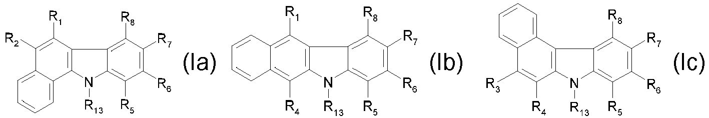 or for example also structures like (Id)-(Ih): 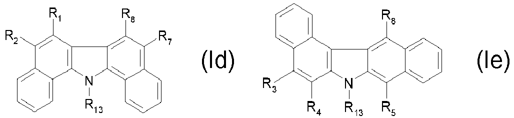 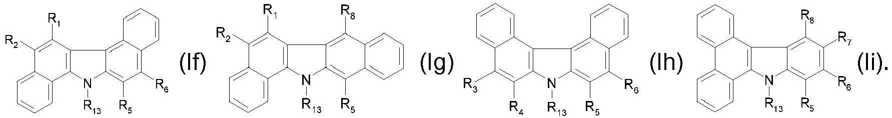 Preferred are structures (Ia).
Characterizing for the compounds of the formula I is, that at least one phenyl ring is condensed to the carbazole moiety to form a "naphthyl" ring. That is one of the above structures is given in formula I.

If R₁ and R₂, R₂ and R₃, R₃ and R₄, R₅ and R₆, R₆ and R₇, or R₇ and R₈ independently of each other together are -(CH₂)ₚ-Y-(CH₂)_{q}-, for example structures like 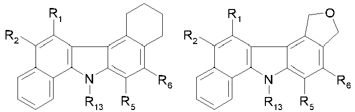 etc. are formed.
If the substituents OR₁₇, SR₁₈, SOR₁₈, SO₂R₁₈ or NR₁₉R₂₀, on a phenyl or naphthyl ring form 5- or 6-membered rings *via* the radicals R₁₇, R₁₈, R₁₉ and/or R₂₀ with one of the carbon atoms of the naphthyl ring, structures comprising three or more rings (inclusive the naphthyl ring) are obtained. Examples are 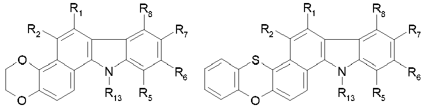 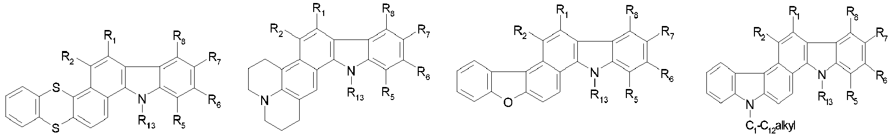 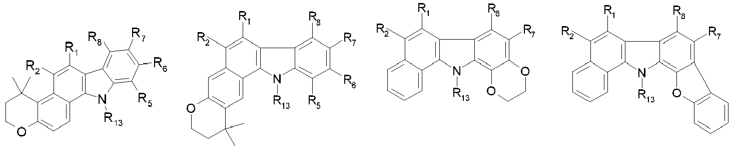 etc.. If R₁₇ forms a direct bond to one of the carbon atoms of the phenyl or naphthyl ring on which the group 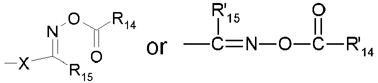 is located, for example structutures like 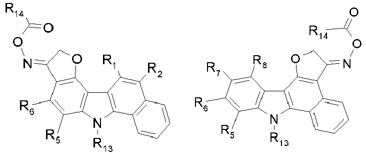 etc. are formed.

If R₁₆ is phenyl substituted by SR₁₈ wherein the radical R₁₈ denotes a direct bond to the phenyl or naphthyl ring of the carbazole moiety to which the COR₁₆ group is attached for example structures like 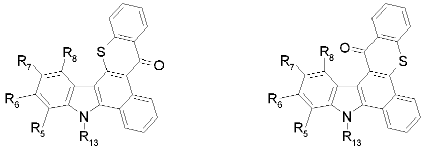 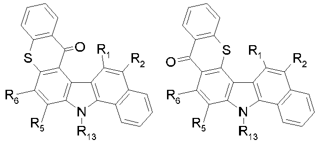 etc. are formed. That is, if R₁₆ is phenyl substituted by SR₁₈ wherein the radical R₁₈ denotes a direct bond to the phenyl or naphthyl ring of the carbazole moiety to which the COR₁₆ group is attached, a thioxanthyl moiety together with one of the phenyl rings or the naphthyl ring of the carbazoile moiety is formed.

If R₁₉ and R₂₀ together with the N-atom to which they are attached form a 5- or 6-membered saturated or unsaturated ring which optionally is interrupted by O, S or NR₁₇, saturated or unsaturated rings are formed, for example aziridine, pyrrole, thiazole, pyrrolidine, oxazole, pyridine, 1,3-diazine, 1,2-diazine, piperidine or morpholine. Preferably, if R₁₉ and R₂₀ together with the N-atom to which they are attached form a 5-or 6-membered saturated or unsaturated ring which optionally is interrupted by O, S or NR₁₇, 5- or 6-membered saturated rings which are not interrupted or which are interrupted by O or NR₁₇, in particular by O, are formed.
If R₂₁ and R₂₂ together with N-atom to which they are attached form a 5- or 6-membered saturated or unsaturated ring, which optionally, is interrupted by O, S or NR₂₆, and to which saturated or unsaturated ring optionally a benzene ring is condensed, saturated or unsaturated rings are formed, for example aziridine, pyrrole, thiazole, pyrrolidine, oxazole, pyridine, 1,3-diazine, 1,2-diazine, piperidine or morpholine, or corresponding annelated rings, e.g. 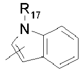 etc..

If R₁₉ and R₂₀ together with the N-atom to which they are attached form a heteroaromatic ring system, said ring system is meant to comprise more than one ring, e.g. two or three rings, as well as one or more than one heteroatoms, from the same kind or different ones. Suitable heteroatoms are for example, N, S, O or P, in particular N, S or O. Examples are, carbazole, indole, isoindole, indazole, purine, isoquinoline, quinoline, carboline, phenothiazine etc..

The terms *"and*/*or"* or *"or*/*and"* in the present context are meant to express that not only one of the defined alternatives (substituents) may be present, but also several of the defined alternatives (substituents) together, namely mixtures of different alternatives (substituents).
The term *"at least"* is meant to define one or more than one, for example one or two or three, preferably one or two.
The term *"optionally substituted"* means, that the radical to which it refers is either unsubstituted or substituted.
The term *"optionally interrupted"* means, that the radical to which it refers is either not interrupted or is interrupted.
Throughout this specification and the claims which follow, unless the context requires otherwise, the word *"comprise",* or variations such as *"comprises"* or *"comprising",* will be understood to imply the inclusion of a stated integer or step or group of integers or steps but not the exclusion of any other integer or step or group of integers or steps. The term *"(meth)acrylate"* in the context of the present application is meant to refer to the acrylate as well as to the corresponding methacrylate.
The *preferences* indicated in the text for the compounds according to the present invention in the context of this invention are intended to refer to all categories of the claims, that is to the claims directed to compositions, use, process, color filter etc. as well.

Oxime esters of formula I are prepared by methods described in the literature, for example by reaction of the corresponding oximes with an acyl halide, in particular a chloride, or an anhydride in an inert solvent such as for example *t*-butyl methyl ether, tetra-hydrofurane (THF) or dimethylformamide in the presence of a base, for example triethylamine or pyridine, or in a basic solvent such as pyridine. As example in the following the preparation of compounds of the formula la, wherein R₇ is the oxime ester group and X is a direct bond is described [the reactions for the compounds (Ib)-(Ih) are performed starting from the appropriate oxime]: 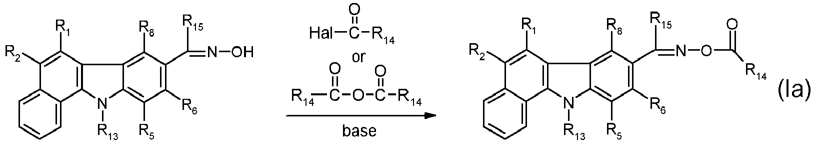 R₁, R₂, R₅, R₆, R₈, R₁₃, R₁₄ and R₁₅ are as defined above, Hal means a halogen atom, in particular Cl.
R₁₄ preferably is methyl.
Such reactions are well known to those skilled in the art, and are generally carried out at temperatures of -15 to +50°C, preferably 0 to 25°C.

When X is CO, the corresponding oxime is synthesized by the nitrosation of the methylene group with an alkyl nitrite such as for example methyl nitrite, ethyl nitrite, isopropyl nitrite, butyl nitrite or isoamyl nitrite. Then, the esterification is carried out under the same condition as mentioned above:

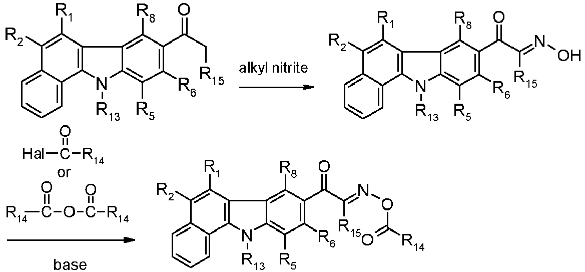

Subject of the invention therefore also is a process for the preparation of a compound of the formula I as defined above by reacting the corresponding oxime compound with an acyl halide of the formula II or an anhydride of the formula III 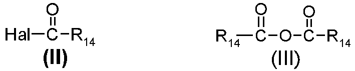 wherein **Hal** is a halogen, in particular Cl, and **R₁₄** is as defined above, in the presence of a base or a mixture of bases.

The oximes required as starting materials can be obtained by a variety of methods described in standard chemistry textbooks (for instance in J. March, Advanced Organic Chemistry, 4th Edition, Wiley Interscience, 1992), or in specialized monographs, for example, S.R. Sandler & W. Karo, Organic functional group preparations, Vol. 3, Academic Press.
One of the most convenient methods is, for example, the reaction of aldehydes or ketones with hydroxylamine or its salt in polar solvents like dimethylacetamide (DMA), aqueous DMA, ethanol or aqueous ethanol. In that case, a base such as sodium acetate or pyridine is added to control the pH of the reaction mixture. It is well known that the rate of the reaction is pH-dependent, and the base can be added at the beginning or continuously during the reaction. Basic solvents such as pyridine can also be used as base and/or solvent or cosolvent. The reaction temperature is generally from room temperature to the refluxing temperature of the mixture, usually about 20-120°C.

The corresponding ketone intermediates are for example prepared by the methods described in the literature, for example in standard chemistry textbooks (for instance in J. March, Advanced Organic Chemistry, 4th Edition, Wiley Interscience, 1992). In addition, successive Friedel-Crafts reaction is effective for synthesis of the intermediates. Such reactions are well known to those skilled in the art.

Another convenient synthesis of oximes is the nitrosation of "active" methylene groups with nitrous acid or an alkyl nitrite. Both alkaline conditions, as described for example in Organic Syntheses coll. Vol. VI (J. Wiley & Sons, New York, 1988), pp 199 and 840, and acidic conditions, as described, for example, in Organic Synthesis coll. vol V, pp 32 and 373, coll. vol. III, pp 191 and 513, coll. vol.II, pp. 202, 204 and 363, are suitable for the preparation of the oximes used as starting materials in the invention. Nitrous acid is usually generated from sodium nitrite. The alkyl nitrite can be for example methyl nitrite, ethyl nitrite, isopropyl nitrite, butyl nitrite, or isoamyl nitrite.

Another embodiment of the invention are the free oxime compounds of the formula (IA) 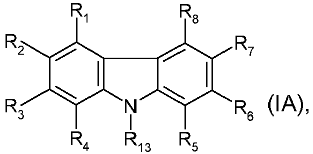 wherein
R₁, R₂, R₃, R₄, R₅, R₆, R₇ and R₈ independently of each other are hydrogen, C₁-C₂₀alkyl, 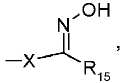 COR₁₆, OR₁₇, halogen, NO₂, or 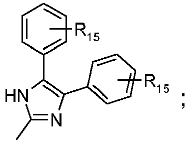 or R₁ and R₂, R₂ and R₃, R₃ and R₄, R₅ and R₆, R₆ and R₇, or R₇ and R₈ independently
of each other are C₂-C₁₀alkenyl which is substituted by 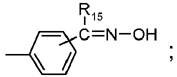 or R₁ and R₂, R₂ and R₃, R₃ and R₄, R₅ and R₆, R₆ and R₇, or R₇ and R₈ independently of each other together are -(CH₂)ₚ-Y-(CH₂)_{q}-;
or R₁ and R₂, R₂ and R₃, R₃ and R₄, R₅ and R₆, R₆ and R₇, or R₇ and R₈ independently
of each other together are 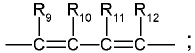 ***provided that*** at least one pair of R₁ and R₂, R₂ and R₃, R₃ and R₄, R₅ and R₆, R₆ and R₇, or R₇ and R₈ is 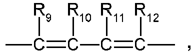 **R₉, R₁₀, R₁₁** and **R₁₂** independently of each other are hydrogen, C₁-C₂₀alkyl which is unsubstituted or substituted by one or more halogen, phenyl, CN, OH, SH, C₁-C₄-alkoxy, (CO)OH or by (CO)O(C₁-C₄alkyl);
or R₉, R₁₀, R₁₁, and R₁₂ independently of each other are unsubstituted phenyl or phenyl substituted by one or more C₁-C₆alkyl, halogen, CN, OR₁₇, SR₁₈ or by NR₁₉R₂₀;
or R₉, R₁₀, R₁₁, and R₁₂ independently of each other are halogen, CN, OR₁₇, SR₁₈, SOR₁₈, SO₂R₁₈ or NR₁₉R₂₀, wherein the substituents OR₁₇, SR₁₈ or NR₁₉R₂₀ optionally form 5- or 6-membered rings *via* the radicals R₁₇, R₁₈, R₁₉ and/or R₂₀ with one of the carbon atoms of the naphthyl ring;
or R₉, R₁₀, R₁₁ and R₁₂ independently of each other are 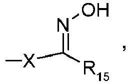 COR₁₆ or NO₂;
- **Y**: is O, S, NR₂₆ or a direct bond;
- **p**: is an integer 0,1, 2 or 3;
- **q**: is an integer 1, 2 or 3;
- **X**: is CO or a direct bond;

**R**₁₃ is C₁-C₂₀alkyl which is unsubstituted or substituted by one or more halogen, R₁₇, COOR₁₇, OR₁₇, SR₁₈, CONR₁₉R₂₀, NR₁₉R₂₀, PO(OCₖH₂ₖ₊₁)₂, or by 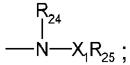 or R₁₃ is C₂-C₂₀alkyl which is interrupted by one or more O, S, SO, SO₂, NR₂₆ or CO,
or is C₂-C₁₂alkenyl which is uninterrupted or interrupted by one or more O, CO or NR₂₆, wherein the interrupted C₂-C₂₀alkyl and the uninterrupted or interrupted C₂-C₁₂alkenyl are unsubstituted or are substituted by one or more halogen;
or R₁₃ is C₄-C₈cycloalkenyl, C₂-C₁₂alkinyl, or C₃-C₁₀cycloalkyl which is uninterrupted or interrupted by one or more O, S, CO or NR₂₆;
or R₁₃ is phenyl or naphthyl each of which is unsubstituted or substituted by one or more OR₁₇, SR₁₈, NR₁₉R₂₀, 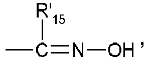 COR₁₆, CN, NO₂, halogen, C₁-C₂₀alkyl, C₁-C₄haloalkyl, C₂-C₂₀alkyl which is interrupted by one or more O, S, CO or NR₂₆, or each of which is substituted by C₃-C₁₀cycloalkyl or by C₃-C₁₀cycloalkyl which is interrupted by one or more O, S, CO, or NR₂₆;
k is an integer 1-10;
R₁₅ is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, PO(OCₖH₂ₖ₊₁)₂, SO-C₁-C₁₀alkyl, SO₂-C₁-C₁₀alkyl, by C₂-C₂₀alkyl which is interrupted by one or more O, S or NR₂₆, or each of which is substituted by C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂₀heteroaryloxycarbonyl, OR₁₇, SR₁₈ or NR₁₉R₂₀;
or R₁₅ is hydrogen, C₂-C₁₂alkenyl, C₃-C₈cycloalkyl which is uninterrupted or is interrupted by one or more O, CO or NR₂₆;
or R₁₅ is C₁-C₂₀alkyl which is unsubstituted or substituted by one or more halogen, OR₁₇, SR₁₈, C₃-C₈cycloalkyl, C₃-C₂₀heteroaryl, C₆-C₂₀aryloxycarbonyl, C₃-C₂₀hetero-aryloxycarbonyl, NR₁₉R₂₀, COOR₁₇, CONR₁₉R₂₀, PO(OCₖH₂ₖ₊₁)₂, 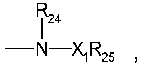 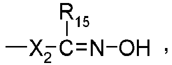 phenyl, or the C₁-C₂₀alkyl is substituted by phenyl which is substituted by halogen, C₁-C₂₀alkyl, C₁-C₄haloalkyl, OR₁₇, SR₁₈, or NR₁₉R₂₀;
or R₁₅ is C₂-C₂₀alkyl which is interrupted by one or more O, SO or SO₂, and which inter-rupted C₂-C₂₀alkyl is unsubstituted or substituted by one or more halogen, OR₁₇, COOR₁₇, CONR₁₉R₂₀, phenyl or by phenyl which is substituted by OR₁₇, SR₁₈ or NR₁₉R₂₀;
or R₁₅ is C₂-C₂₀alkanoyl or benzoyl which is unsubstituted or substituted by one or more C₁-C₆alkyl, halogen, phenyl, OR₁₇, SR₁₈ or NR₁₉R₂₀;
or R₁₅ is naphthoyl which is unsubstituted or is substituted by one or more OR₁₇ or is C₃-C₁₄heteroarylcarbonyl;
or R₁₅ is C₂-C₁₂alkoxycarbonyl which is uninterrupted or is interrupted by one or more O and which interrupted or uninterrupted C₂-C₁₂alkoxycarbonyl is unsubstituted or substituted by one or more hydroxyl groups;
or R₁₅ is phenoxycarbonyl which is unsubstituted or substituted by one or more C₁-C₆alkyl, halogen, C₁-C₄haloalkyl, phenyl, OR₁₇, SR₁₈ or NR₁₉R₂₀;
or R₁₅ is CN, CONR₁₉R₂₀, NO₂, C₁-C₄haloalkyl, S(O)ₘ-C₁-C₆alkyl; S(O)ₘ-phenyl which is unsubstituted or substituted by C₁-C₁₂alkyl or SO₂-C₁-C₆alkyl;
or R₁₅ is SO₂O-pheny which is unsubstituted or substituted by C₁-C₁₂alkyl; or is diphenyl phosphinoyl or di-(C₁-C₄alkoxy)-phosphinoyl;
m is 1 or 2; R'₁₅ has one of the meanings as given for R₁₅;
X₁ is O, S, SO or SO₂;
X₂ is O, CO, S or a direct bond;
R₁₆ is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, or by C₁-C₂₀alkyl which is interrupted by one or more O, S, or NR₂₆, or each of which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂₀heteroaryloxycarbonyl, OR₁₇, SR₁₈ or NR₁₉R₂₀;
or R₁₆ is hydrogen, C₁-C₂₀alkyl which is unsubstituted or substituted by one or more halogen, phenyl, OH, SH, CN, C₃-C₆alkenoxy, OCH₂CH₂CN, OCH₂CH₂(CO)O(C₁-C₄alkyl), O(CO)-(C₁-C₄alkyl), O(CO)-phenyl, (CO)OH or (CO)O(C₁-C₄alkyl);
or R₁₆ is C₂-C₁₂alkyl which is interrupted by one or more O, S or NR₂₆;
or R₁₆ is (CH₂CH₂O)ₙ₊₁H, (CH₂CH₂O)ₙ(CO)-(C₁-C₈alkyl), C₂-C₁₂alkenyl or C₃-C₈cycloalkyl;
or R₁₆ is phenyl substituted by SR₁₈ wherein the radical R₁₈ denotes a direct bond to the phenyl or naphthyl ring of the carbazole moiety to which the COR₁₆ group is attached;
n is 1-20;
R₁₇ is hydrogen, phenyl-C₁-C₃alkyl, C₁-C₂₀alkyl which is unsubstituted or substituted by one or more halogen, OH, SH, CN, C₃-C₆alkenoxy, OCH₂CH₂CN, OCH₂CH₂(CO)O(C₁-C₄alkyl), O(CO)-(C₁-C₄alkyl), O(CO)-(C₂-C₄)alkenyl, O(CO)-phenyl, (CO)OH, (CO)O(C₁-C₄alkyl), SO₂-(C₁-C₄haloalkyl), O(C₁-C₄haloalkyl), C₃-C₂₀cycloalkyl, or by C₃-C₂₀cycloalkyl which is interrupted by one or more O;
or R₁₇ is C₂-C₂₀alkyl which is interrupted by one or more O, S or NR₂₆;
or R₁₇ is (CH₂CH₂O)ₙ₊₁H, (CH₂CH₂O)ₙ(CO)-(C₁-C₈alkyl), C₁-C₈alkanoyl, C₂-C₁₂alkenyl, C₃-C₆alkenoyl, or C₃-C₂₀cycloalkyl which is uninterrupted or interrupted by one or more O, S, CO or NR₂₆;
or R₁₇ is C₁-C₈alkyl-C₃-C₁₀cycloalkyl which is uninterrupted or interrupted by one or more O;
or R₁₇ is benzoyl which is unsubstituted or substituted by one or more C₁-C₆alkyl, halogen, OH or C₁-C₃alkoxy;
or R₁₇ is phenyl, naphthyl or C₃-C₂₀heteroaryl, each of which is unsubstituted or substituted by one or more halogen, OH, C₁-C₁₂alkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenyl-amino or 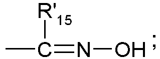 or R₁₇ forms a direct bond to one of the carbon atoms of the phenyl or naphthyl ring on which the group 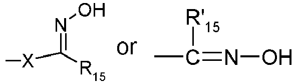 is located;
R₁₈ is hydrogen, C₂-C₁₂alkenyl, C₃-C₂₀cycloalkyl, or phenyl-C₁-C₃alkyl, wherein the C₂-C₁₂alkenyl, C₃-C₂₀cycloalkyl, or phenyl-C₁-C₃alkyl is uninterrupted or interrupted by one or more O, S, CO, NR₂₆ or COOR₁₇;
or R₁₈ is C₁-C₂₀alkyl which is unsubstituted or is substituted by one or more OH, SH, CN, C₃-C₆alkenoxy, OCH₂CH₂CN, OCH₂CH₂(CO)O(C₁-C₄alkyl), O(CO)-(C₂-C₄)alkenyl, O(CO)-(C₁-C₄alkyl), O(CO)-phenyl or (CO)OR₁₇;
or R₁₈ is C₂-C₂₀alkyl which is interrupted by one or more O, S, CO, NR₂₆ or COOR₁₇;
or R₁₈ is (CH₂CH₂O)ₙH, (CH₂CH₂O)ₙ(CO)-(C₁-C₈alkyl), C₂-C₈alkanoyl or C₃-C₆alkenoyl;
or R₁₈ is benzoyl which is unsubstituted or substituted by one or more C₁-C₆alkyl, halogen, OH, C₁-C₄alkoxy or C₁-C₄alkylsulfanyl;
or R₁₈ is phenyl, naphthyl or C₃-C₂₀heteroaryl, each of which is unsubstituted or substituted by one or more halogen, C₁-C₁₂alkyl, C₁-C₄haloalkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenylamino, (CO)O(C₁-C₈alkyl), (CO)-C₁-C₈alkyl, (CO)N(C₁-C₈alkyl)₂ or 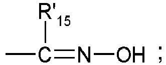 **R₁₉** and **R₂₀** independently of each other are hydrogen, C₁-C₂₀alkyl, C₂-C₄hydroxyalkyl, C₂-C₁₀alkoxyalkyl, C₂-C₅alkenyl, C₃-C₂₀cycloalkyl, phenyl-C₁-C₃alkyl, SO₂-(C₁-C₄haloalkyl), C₁-C₈alkanoyl, C₁-C₈alkanoyloxy, C₃-C₁₂alkenoyl or benzoyl;
or R₁₉ and R₂₀ are phenyl, naphthyl or C₃-C₂₀heteroaryl, each of which is unsubstituted or substituted by one or more halogen, C₁-C₄haloalkyl, C₁-C₂₀alkoxy, C₁-C₁₂alkyl, benzoyl or C₁-C₁₂alkoxy;
or R₁₉ and R₂₀ together with the N-atom to which they are attached form a 5- or 6-membered saturated or unsaturated ring which is uninterrupted or is interrupted by O, S or NR₁₇, and which 5- or 6-membered saturated or unsaturated ring is unsubstituted or substituted by one or more C₁-C₂₀alkyl, C₁-C₂₀alkoxy, =O, OR₁₇, SR₁₈, NR₂₁R₂₂, (CO)R₂₃, NO₂, halogen, C₁-C₄-haloalkyl, CN, phenyl, 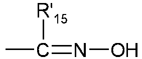 or by C₃-C₂₀cyclalkyl which is uninterrupted or is interrupted by one or more O, S, CO or NR₁₇; or R₁₉ and R₂₀ together with the N-atom to which they are attached form a heteroaromatic ring system which is unsubstituted or substituted by one or more C₁-C₂₀alkyl, C₁-C₄haloalkyl, C₁-C₂₀alkoxy, =O, OR₁₇, SR₁₈, NR₂₁R₂₂, (CO)R₂₃, 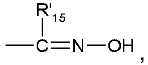 halogen, NO₂, CN, phenyl or by C₃-C₂₀cycloalkyl which is uninterrupted or is interrupted by one or more O, S, CO or NR₁₇;
**R₂₁** and **R₂₂** independently of each other are hydrogen, C₁-C₂₀alkyl, C₁-C₄haloalkyl, C₃-C₁₀cycloalkyl or phenyl;
or R₂₁ and R₂₂ together with N-atom to which they are attached form a 5- or 6-membered saturated or unsaturated ring, which is uninterrupted or is interrupted by O, S or NR₂₆, and which 5- or 6-membered saturated or unsaturated ring is not condensed or to which 5- or 6-membered saturated or unsaturated ring a benzene ring is condensed;
**R₂₃** is hydrogen, OH, C₁-C₂₀alkyl, C₁-C₄haloalkyl, C₂-C₂₀alkyl which is interrupted by one or more O, CO or NR₂₆, C₃-C₂₀cycloalkyl which is uninterrupted or is interrupted by O, S, CO or NR₂₆,
or R₂₃ is phenyl, naphthyl, phenyl-C₁-C₄alkyl, OR₁₇, SR₁₈ or NR₂₁R₂₂;
R₂₄ is (CO)OR₁₇, CONR₁₉R₂₀, (CO)R₁₇; or R₂₄ has one of the meanings given for R₁₉ and R₂₀;
R₂₅ is COOR₁₇, CONR₁₉R₂₀, (CO)R₁₇; or R₂₅ has one of the meanings given for R₁₇;
R₂₆ is hydrogen, C₁-C₂₀alkyl, C₁-C₄haloalkyl, C₂-C₂₀alkyl which is interrupted by one or more O or CO, or is phenyl-C₁-C₄alkyl, C₃-C₈cycloalkyl which is uninterrupted or is interrupted by one or more O or CO, or is (CO)R₁₉ or is phenyl which is unsubstituted or substituted by one or more C₁-C₂₀alkyl, halogen, C₁-C₄haloalkyl, OR₁₇, SR₁₈, NR₁₉R₂₀ or 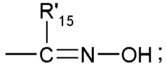 ***provided that* at least one group** 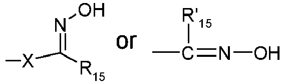 **is present in the molecule.**

The preferences for the radicals defined for the compounds of the formula (IA) correspond to the ones as given for the compounds of the formula (I) as given below, except that each defined oxime ester group, as for example 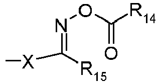 is exchanged by the corresponding free oxime radical

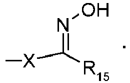

Every oxime ester group can exist in two configurations, (Z) or (E). It is possible to separate the isomers by conventional methods, but it is also possible to use the iso-meric mixture as such as photoinitiating species. Therefore, the invention also relates to mixtures of configurational isomers of compounds of the formula I.

Preferred are compounds of the formula I as defined above, wherein
**R₁, R₂, R₃, R₄, R₅, R₆, R₇** and **R₈** independently of each other are hydrogen, C₁-C₂₀alkyl, 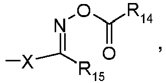 COR₁₆ or NO₂,
or R₁ and R₂, R₂ and R₃, R₃ and R₄, R₅ and R₆, R₆ and R₇ or R₇ and R₈ independently of each other together are 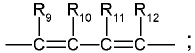 ***provided that*** at least one pair of R₁ and R₂, R₂ and R₃, R₃ and R₄, R₅ and R₆, R₆ and R₇, or R₇ and R₈ is 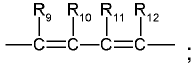 **X** is CO or a direct bond;
**R₁₃** is C₁-C₂₀alkyl which is unsubstituted or substituted by one or more halogen, OR₁₇, SR₁₈, COOR₁₇, CONR₁₉R₂₀ or by PO(OCₖH₂ₖ₊₁)₂;
or R₁₃ is C₂-C₂₀alkyl which is interrupted by one or more O, S, NR₂₆ or CO;
or R₁₃ is phenyl or naphthyl both of which are unsubstituted or substituted by one or more 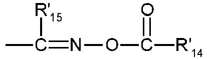 or COR₁₆;
**R₁₄** is C₁-C₂₀alkyl, phenyl or C₁-C₈alkoxy;
**R₁₅** is phenyl, naphthyl, C₃-C₂₀heteroaryl each of which is unsubstituted or substituted by one or more phenyl, halogen, C₁-C₄haloalkyl, OR₁₇, SR₁₈, or C₂-C₂₀alkyl which is interrupted by one or more O or S, or each of which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₄-C₂₀heteroaryloxycarbonyl, OR₁₇, SR₁₈, NR₁₉R₂₀ or PO(OCₖH₂ₖ₊₁)₂;
or R₁₅ is C₁-C₂₀alkyl which is unsubstituted or substituted by one or more OR₁₇, SR₁₈, C₃-C₈cycloalkyl, C₃-C₂₀heteroaryl, NR₁₉R₂₀, COOR₁₇, CONR₁₉R₂₀ or PO(OCₖH₂ₖ₊₁)₂;
**R'₁₄** has one of the meanings as given for R₁₄;
**R'₁₅** has one of the meanings as given for R₁₅;
**R₁₆** is phenyl which is unsubstituted or substituted by one or more OR₁₇, SR₁₈, NR₁₉R₂₀, or by C₂-C₂₀alkyl which is interrupted by one or more O, S, or NR₂₆,
or R₁₈ is phenyl which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₄-C₂₀heteroaryloxycarbonyl, OR₁₇, SR₁₈ or NR₁₉R₂₀;
or R₁₆ is C₁-C₂₀alkyl which is unsubstituted or substituted by halogen, phenyl, OH, SH, CN, C₃-C₆alkenoxy, OCH₂CH₂(CO)O(C₁-C₄alkyl), O(CO)-(C₁-C₄alkyl), or (CO)O(C₁-C₄alkyl);
**R₁₇** is C₁-C₂₀alkyl which is unsubstituted or substituted by one or more halogen, OCH₂CH₂(CO)O(C₁-C₄alkyl), O(C₁-C₄alkyl), (CO)O(C₁-C₄alkyl), C₃-C₂₀cycloalkyl, or by C₃-C₂₀cycloalkyl which is interrupted by one or more O; or
R₁₇ is C₂-C₂₀alkyl which is interrupted by one or more O;
**R₁₈** is methyl substituted by (CO)OR₁₇;
**R₁₉** and **R₂₀** independently of one another are hydrogen, phenyl, C₁-C₂₀alkyl, C₁-C₈alkanoyl or C₁-C₈alkanoyloxy;
or R₁₉ and R₂₀ together with the N-atom to which they are attached form a heteroaromatic ring system which is unsubstituded or substituted by 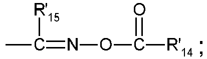 ***provided that*** at least one group 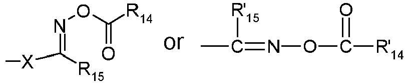 is present in the molecule.

Emphasis has to be laid on compounds of the formula I as defined above, wherein **R₁, R₂**, **R₅**, **R₆**, **R₇** and **R₈** independently of each other are hydrogen, 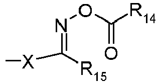 COR₁₆, or NO₂,
R₃ and R₄, together are 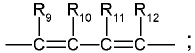 **R₉, R₁₀, R₁₁** and **R₁₂** are hydrogen
**X** is a direct bond;
**R₁₃** is C₁-C₂₀alkyl;
**R₁₄** is C₁-C₂₀alkyl;
**R₁₅** is C₁-C₂₀alkyl or phenyl which is substituted by one or more OR₁₇ or C₁-C₂₀alkyl; **R₁₆** is phenyl which is substituted by one or more C₁-C₂₀alkyl or OR₁₇; and
**R₁₇** is C₁-C₂₀alkyl which is unsubstitued or substituted by one or more halogen or is C₂-C₂₀alkyl which is interrupted by one or more O. ***provided** that* at least one group 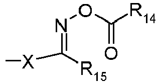 is present in the molecule.

Subject of the invention further are compounds of the formula (I) as defined above, wherein
**R₁**, **R₂**, **R₃**, **R₄**, **R₅**, **R₆**, **R₇** and **R₈** independently of each other are hydrogen,
or R₁ and R₂, R₃ and R₄, or R₅ and R₆, independently of each other together
are 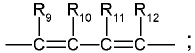 ***provided** that* at least one pair of R₁ and R₂, R₃ and R₄ or R₅ and R₆
is 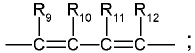 or R₂ is 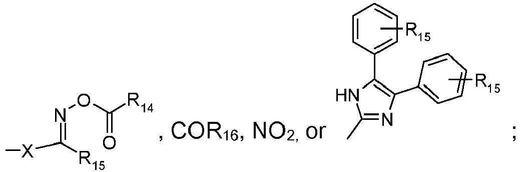 or R₇ is 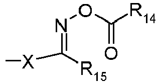 or COR₁₆;
**R₉, R₁₁** and **R₁₂** are hydrogen;
**R₁₀** is hydrogen, OR₁₇ or COR₁₆;
**X** is CO or a direct bond;
**R₁₃** is C₁-C₂₀alkyl which is unsubstituted or substituted by one or more halogen, R₁₇, OR₁₇, SR₁₈, or by PO(OCₖH₂ₖ₊₁)₂;
or R₁₃ is C₂-C₂₀alkyl which is interrupted by one or more O;
or R₁₃ is phenyl;
**k** is an integer 2;
**R₁₄** is C₁-C₂₀alkyl or thienyl;
**R₁₅** is phenyl or naphthyl each of which is unsubstituted or substituted by one or more OR₁₇ or C₁-C₂₀alkyl, or R₁₅ is thienyl, hydrogen, C₁-C₂₀alkyl which is unsubstituted or substituted by one or more OR₁₇, SR₁₈, C₃-C₈cycloalkyl, NR₁₉R₂₀ or by COOR₁₇;
or R₁₅ is C₂-C₂₀alkyl which is interrupted by SO₂;
**R₁₆** is phenyl or naphthyl each of which is unsubstituted or substituted by one or more OR₁₇, SR₁₈, NR₁₉R₂₀, or by C₁-C₂₀alkyl;
or R₁₆ is thienyl;
**R₁₇** is hydrogen, C₁-C₈alkanoyl, C₁-C₂₀alkyl which is unsubstituted or substituted by one or more halogen, O(CO)-(C₁-C₄alkyl), O(CO)-(C₂-C₄)alkenyl or by C₃-C₂₀cycloalkyl which is interrupted by one or more O;
or R₁₇ is C₂-C₂₀alkyl which is interrupted by one or more O;
**R₁₈** is C₃-C₂₀cycloalkyl, C₁-C₂₀alkyl which is unsubstituted or is substituted by one or more OH, O(CO)-(C₂-C₄)alkenyl or (CO)OR₁₇;
or R₁₈ is phenyl which is unsubstituted or substituted by one or more halogen;
**R₁₉** and **R₂₀** independently of each other are C₁-C₈alkanoyl or C₁-C₈alkanoyloxy;
or R₁₉ and R₂₀ together with the N-atom to which they are attached form a 5- or 6-membered saturated ring which is interrupted by O; ***provided that*** at least one group 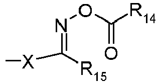 is present in the molecule.

Examples of the compounds according to the invention are the compounds of the formula (Ia)-(Ig) as defined above. Interesting are compounds of the formula (Ia), (Ib), (Ic), especially of the formula (Ia) or (Ic), or of the formula (Ia), (Ic) or (Id), in particular of the formula (Ia).

**R₁, R₂, R₃**, **R₄**, **R₅**, **R₆**, **R₇** and **R₈** for example independently of each other are hydrogen, 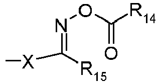 or COR₁₆, or R₁ and R₂, R₂ and R₃, R₃ and R₄, or R₅ and R₆, R₆ and R₇, R₇ and R₈ independently of each other together are 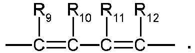 For example R₃ and R₄ or R₁ and R₂ together are 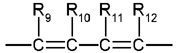 or R₃ and R₄ and R₅ and R₆ together are 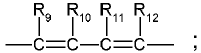 in particular R₃ and R₄ together are 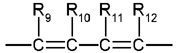 R₁, R₅, R₆ and R₈ for example are hydrogen. R₇ in particular is hydrogen, 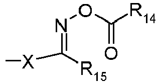 or COR₁₆. Or R₇ is 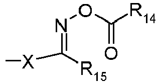 or COR₁₆, in particular 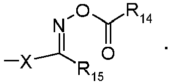 R₂ especially is 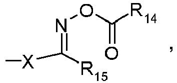 COR₁₆ or 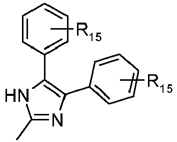 or R₂ together with R₁ is 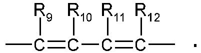 In particular R₂ is COR₁₆.
**X** preferably is a direct bond.

**R₉, R₁₀, R₁₁** and **R₁₂** for example independently of each other are hydrogen, C₁-C₂₀alkyl, unsubstituted phenyl or phenyl substituted by one or more C₁-C₆alkyl, halogen, OR₁₇ or by SR₁₈; or R₉, R₁₀, R₁₁, and R₁₂ independently of each other are halogen, OR₁₇, SR₁₈ or NR₁₉R₂₀, wherein the substituents OR₁₇, SR₁₈ or NR₁₉R₂₀ optionally form 5- or 6-membered rings *via* the radicals R₁₇, R₁₈, R₁₉ and/or R₂₀ with one of the carbon atoms of the naphthyl ring; or R₉, R₁₀, R₁₁ and R₁₂ independently of each other are 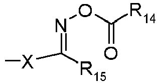 or COR₁₆.

Especially R₉, R₁₀, R₁₁ and R₁₂ for example independently of each other are hydrogen, C₁-C₂₀alkyl, unsubstituted phenyl or phenyl substituted by one or more C₁-C₆alkyl, halogen, OR₁₇ or by SR₁₈; or R₉, R₁₀, R₁₁, and R₁₂ independently of each other are halogen, OR₁₇, SR₁₈ or NR₁₉R₂₀; or R₉, R₁₀, R₁₁ and R₁₂ independently of each other are 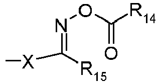 or COR₁₆.

R₉, R₁₀, R₁₁ and R₁₂ for example independently of each other are hydrogen, C₁-C₂₀alkyl, unsubstituted phenyl or phenyl substituted by one or more C₁-C₆alkyl; or R₉, R₁₀, R₁₁ and R₁₂ independently of each other are 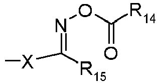 or COR₁₆.

In another embodiment R₉, R₁₀, R₁₁ and R₁₂ for example independently of each other are hydrogen, C₁-C₂₀alkyl, unsubstituted phenyl or phenyl substituted by one or more C₁-C₆alkyl, halogen, OR₁₇ or by SR₁₈; or R₉, R₁₀, R₁₁, and R₁₂ independently of each other are halogen, OR₁₇, SR₁₈ or NR₁₉R₂₀, wherein the substituents OR₁₇, SR₁₈ or NR₁₉R₂₀ optionally form 5- or 6-membered rings *via* the radicals R₁₇, R₁₈, R₁₉ and/or R₂₀ with one of the carbon atoms of the naphthyl ring.

Further R₉, R₁₀, R₁₁ and R₁₂ for example independently of each other are hydrogen, C₁-C₂₀alkyl, unsubstituted phenyl or phenyl substituted by one or more C₁-C₆alkyl, halogen, OR₁₇ or by SR₁₈, or R₉, R₁₀, R₁₁, and R₁₂ independently of each other are halogen, OR₁₇, SR₁₈, NR₁₉R₂₀ or COR₁₆.

Or R₉, R₁₀, R₁₁ and R₁₂ for example independently of each other are hydrogen, C₁-C₂₀alkyl, unsubstituted phenyl or phenyl substituted by one or more C₁-C₆alkyl, halogen, OR₁₇ or by SR₁₈; or R₉, R₁₀, R₁₁, and R₁₂ independently of each other are halogen, OR₁₇, COR₁₆ or NR₁₉R₂₀.

Preferably R₉, R₁₁ and R₁₂ are hydrogen and R₁₀ is hydrogen, OR₁₇ or COR₁₆.

**R₁₃** is for example C₁-C₂₀alkyl which is unsubstituted or substituted by one or more halogen, COOR₁₇ or by CONR₁₉R₂₀;
or R₁₃ is C₂-C₂₀alkyl which is interrupted by one or more O, S, SO, SO₂, NR₂₆ or CO,
or is C₂-C₁₂alkenyl which optionally is interrupted by one or more O, CO or NR₂₆,
or R₁₃ is C₃-C₁₀cycloalkyl which optionally is interrupted by one or more O, S, CO, NR₂₆, or R₁₃ is phenyl or naphthyl both of which are unsubstituted or substituted by one or more OR₁₇, SR₁₈, NR₁₉R₂₀, 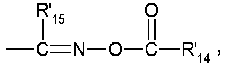 COR₁₆, NO₂, halogen, C₁-C₂₀alkyl, C₁-C₄haloalkyl, C₂-C₂₀alkyl which is interrupted by one or more O; or is C₁-C₂₀alkyl which is unsubstituted or substituted by one or more halogen, R₁₇, COOR₁₇, OR₁₇, SR₁₈, CONR₁₉R₂₀ or by PO(OCₖH₂ₖ₊₁)₂; or is C₂-C₂₀alkyl which is interrupted by one or more O.

Further R₁₃ for example is C₁-C₂₀alkyl which is unsubstituted or substituted by one or more halogen, R₁₇, COOR₁₇, OR₁₇, SR₁₈, CONR₁₉R₂₀ or by PO(OCₖH₂ₖ₊₁)₂, or is C₂-C₂₀alkyl which is interrupted by one or more O, or is C₂-C₁₂alkenyl, C₃-C₁₀cycloalkyl, or R₁₃ is phenyl or naphthyl both of which are unsubstituted or substituted by one or more OR₁₇, SR₁₈, NR₁₉R₂₀, 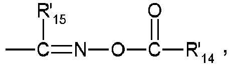 COR₁₆, NO₂, halogen, C₁-C₂₀alkyl, C₁-C₄haloalkyl, C₂-C₂₀alkyl which is interrupted by one or more O.

In another embodiment R₁₃ for example is C₁-C₂₀alkyl which is unsubstituted or substituted by one or more halogen, R₁₇, OR₁₇, SR₁₈, or by PO(OCₖH₂ₖ₊₁)₂, or is C₂-C₂₀alkyl which is interrupted by one or more O, or is C₂-C₁₂alkenyl, C₃-C₁₀cycloalkyl, phenyl or naphthyl.

Or R₁₃ for example is C₁-C₂₀alkyl which is unsubstituted or substituted by one or more halogen, R₁₇, OR₁₇, SR₁₈, or by PO(OCₖH₂ₖ₊₁)₂, or is C₂-C₂₀alkyl which is interrupted by one or more O, or is phenyl, C₂-C₁₂alkenyl, or C₃-C₁₀cycloalkyl.

Or R₁₃ for example is C₁-C₂₀alkyl, phenyl, C₂-C₁₂alkenyl, or C₃-C₁₀cycloalkyl.

Or R₁₃ for example is C₁-C₂₀alkyl, C₂-C₁₂alkenyl, or C₃-C₁₀cycloalkyl.

Preferably R₁₃ is C₁-C₂₀alkyl, in particular C₁-C₈alkyl, for example 2-ethylhexyl.

**R₁₄** is for example hydrogen, C₃-C₈cycloalkyl, C₂-C₅alkenyl, C₁-C₂₀alkoxy or C₁-C₂₀alkyl which is unsubstituted or substituted by one or more halogen or phenyl;
or R₁₄ is phenyl or naphthyl, both of which are unsubstituted or substituted by one or more C₁-C₆alkyl, C₁-C₄haloalkyl, halogen, OR₁₇, SR₁₈ and/or NR₁₉R₂₀;
or R₁₄ is C₃-C₅heteroaryl, for example thienyl, or is C₁-C₈alkoxy, benzyloxy or phenoxy.

Or R₁₄ is for example C₁-C₂₀alkyl which is unsubstituted or substituted by one or more halogen or phenyl;
or R₁₄ is C₃-C₅heteroaryl, for example thienyl, or is phenyl which is unsubstituted or substituted by one or more C₁-C₆alkyl, C₁-C₄haloalkyl, halogen, OR₁₇, SR₁₈ and/or NR₁₉R₂₀;
or R₁₄ is C₁-C₈alkoxy, benzyloxy or phenoxy.

In another embodiment R₁₄ denotes C₁-C₂₀alkyl which is unsubstituted or substituted by phenyl;
or R₁₄ is phenyl which is unsubstituted or substituted by one or more C₁-C₆alkyl.

Preferably R₁₄ is C₁-C₂₀alkyl, C₃-C₅heteroaryl, for example thienyl, or is phenyl, in particular C₁-C₂₀alkyl or thienyl, especially C₁-C₈alkyl.

**R₁₅** for example is C₆-C₂₀aryl or C₅-C₂₀heteroaryl each of which is unsubstituted or substituted by one or more phenyl, halogen, C₁-C₄haloalkyl, CN, NO₂, OR₁₇, SR₁₈, NR₁₉R₂₀, C₁-C₂₀alkyl;
or R₁₅ is hydrogen, C₃-C₈cycloalkyl which optionally is interrupted by one or more O, CO, or NR₂₆;
or R₁₅ is C₁-C₂₀alkyl which is unsubstituted or substituted by one or more halogen, OR₁₇, C₃-C₈cycloalkyl, C₅-C₂₀heteroaryl, C₈-C₂₀phenoxycarbonyl, C₅-C₂₀heteroaryloxy-carbonyl, NR₁₉R₂₀, COOR₁₇, CONR₁₉R₂₀, PO(OCₖH₂ₖ₊₁)₂, 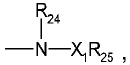 phenyl or by
phenyl which is substituted by halogen, C₁-C₂₀alkyl, C₁-C₄haloalkyl, OR₁₇ or NR₁₉R₂₀, wherein the unsubstituted or substituted C₁-C₂₀alkyl;
or R₁₅ is C₂-C₂₀alkyl which is interrupted by one or more O, S or SO₂,
or R₁₅ is C₂-C₂₀alkanoyl, benzoyl, C₂-C₁₂alkoxycarbonyl, phenoxycarbonyl, CONR₁₉R₂₀, NO₂ or C₁-C₄haloalkyl.

Further, R₁₅ is for example hydrogen, C₆-C₂₀aryl, in particular phenyl or naphthyl each of which is unsubstituted or substituted by C₁-C₁₂alkyl, or is C₃-C₅heteroaryl, for example thienyl, or is C₃-C₈cycloalkyl, C₁-C₂₀alkyl which is unsubstituted or is substituted by one or moreOR₁₇, SR₁₇, C₃-C₈-cycloalkyl, NR₁₉R₂₀ or COOR₁₇;
or R₁₅ is C₂-C₂₀alkyl which is interrupted by one or more O or SO₂.

Interesting are compounds of the formula I wherein R₁₅ for example is hydrogen, phenyl, naphthyl each of which are unsubstituted or substituted by C₁-C₈alkyl, or is thienyl, C₁-C₂₀alkyl which is unsubstituted or is substituted by one or more OR₁₇, SR₁₇, C₃-C₈-cycloalkyl, NR₁₉R₂₀ or COOR₁₇;
or R₁₅ is C₂-C₂₀alkyl which is interrupted by one or more O or SO₂.

In particular R₁₅ for example is C₃-C₈cycloalkyl or C₁-C₂₀alkyl, in particular C₁-C₂₀alkyl, especially C₁-C₁₂alkyl.

Preferences for **R'₁₄** and **R'₁₅** are as given above for R₁₄ and R₁₅, respectively.

**X₁** is for example O, S or SO; e.g. O or S, in particular O.

**R**₁₆ is for example C₆-C₂₀aryl, in particular phenyl or naphthyl, especially phenyl, or C₅-C₂₀heteroaryl, in particular thienyl, each of which is unsubstituted or substituted by one or more phenyl, halogen, C₁-C₄haloalkyl, CN, NO₂, OR₁₇, SR₁₈, NR₁₉R₂₀, or by C₁-C₂₀alkyl which is interrupted by one or more O, or each of which is substituted by one or more C₁-C₂₀alkyl which is unsubstituted or substituted by one or more halogen, COOR₁₇, CONR₁₉R₂₀, phenyl, C₃-C₈cycloalkyl, C₅-C₂₀heteroaryl, C₆-C₂₀aryloxycarbonyl, C₅-C₂₀heteroaryloxycarbonyl, OR₁₇, SR₁₈ or NR₁₉R₂₀;
or R₁₆ is hydrogen, C₁-C₂₀alkyl which is unsubstituted or substituted by one or more halogen, phenyl, OH, SH, C₃-C₆alkenoxy, OCH₂CH₂(CO)O(C₁-C₄alkyl), O(CO)-(C₁-C₄alkyl), O(CO)-phenyl, (CO)OH or (CO)O(C₁-C₄alkyl);
or R₁₆ is C₂-C₁₂alkyl which is interrupted by one or more O, or is (CH₂CH₂O)ₙ₊₁H, (CH₂CH₂O)ₙ(CO)-(C₁-C₈alkyl), C₂-C₁₂alkenyl or C₃-C₈cycloalkyl, and n is 1-20, for example 1-12 or 1-8, in particular 1 or 2.

The more R₁₆ for example is phenyl or naphthyl, especially phenyl, thienyl or carbazole each of which is unsubstituted or substituted by one or more phenyl, halogen, C₁-C₄haloalkyl, OR₁₇, SR₁₈, NR₁₉R₂₀ or by C₁-C₂₀alkyl;
or R₁₆ is C₁-C₂₀alkyl which is unsubstituted or substituted by one or more halogen, phenyl, OH, SH, C₃-C₆alkenoxy, OCH₂CH₂(CO)O(C₁-C₄alkyl), O(CO)-(C₁-C₄alkyl), O(CO)-phenyl, (CO)OH or (CO)O(C₁-C₄alkyl);
or R₁₆ is C₂-C₁₂alkyl which is interrupted by one or more O, or is (CH₂CH₂O)ₙ₊₁H, (CH₂CH₂O)ₙ(CO)-(C₁-C₈alkyl), C₂-C₁₂alkenyl or C₃-C₈cycloalkyl, and n is 1-20, for example 1-12 or 1-8, in particular 1 or 2.

Further R₁₆ for example is phenyl or naphthyl, especially phenyl, each of which is unsubstituted or substituted by one or more phenyl, halogen, C₁-C₄haloalkyl, OR₁₇, SR₁₈, NR₁₉R₂₀ or by C₁-C₂₀alkyl; or R₁₆ is C₃-C₅heteroaryl, especially thienyl.

In particular R₁₆ for example is is phenyl which is unsubstituted or substituted by one or more OR₁₇, SR₁₈, NR₁₉R₂₀ or by C₁-C₂₀alkyl, or R₁₆ is thienyl.

Preferably R₁₆ for example is phenyl or naphthyl each of which is unsubstituted or substituted by one or more C₁-C₂₀alkyl.

Epecially R₁₆ is phenyl which is substituted by one or more or C₁-C₂₀alkyl.

**R₁₇** for example is hydrogen, phenyl-C₁-C₃alkyl, C₁-C₂₀alkyl which is unsubstituted or substituted by one or more halogen, OH, OCH₂CH₂(CO)O(C₁-C₄alkyl), O(CO)-(C₁-C₄alkyl), O(CO)-(C₂-C₄)alkenyl, O(CO)-phenyl, (CO)OH, (CO)O(C₁-C₄alkyl), C₃-C₂₀cycloalkyl, or by C₃-C₂₀cycloalkyl which is interrupted by one or more O;
or R₁₇ is C₂-C₂₀alkyl which is interrupted by one or more O; is (CH₂CH₂O)ₙ₊₁H, (CH₂CH₂O)ₙ(CO)-(C₁-C₈alkyl), C₁-C₈alkanoyl, C₂-C₁₂alkenyl, C₃-C₆alkenoyl, or C₃-C₂₀cycloalkyl which optionally is interrupted by one or more O;
or R₁₇ is benzoyl which is unsubstituted or substituted by one or more C₁-C₆alkyl, halogen, OH or C₁-C₃alkoxy;
or R₁₇ is phenyl, naphthyl or C₅-C₂₀heteroaryl, each of which is unsubstituted or substituted by one or more halogen, OH, C₁-C₁₂alkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenyl-amino or

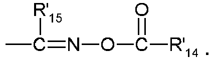

In another embodiment R₁₇ for example is hydrogen, phenyl-C₁-C₃alkyl, C₁-C₂₀alkyl which is unsubstituted or substituted by one or more halogen, O(CO)-(C₁-C₄alkyl), O(CO)-(C₂-C₄alkenyl) or by C₂-C₂₀alkyl which is interrupted by one or more O; or is C₁-C₈alkanoyl, C₂-C₁₂alkenyl, C₃-C₆alkenoyl, C₂-C₂₀alkyl which is interrupted by one or more O, C₃-C₂₀cycloalkyl which optionally is interrupted by one or more O; or is benzoyl which is unsubstituted or substituted by one or more C₁-C₆alkyl, halogen, OH or C₁-C₃alkoxy; or is phenyl or naphthyl each of which is unsubstituted or substituted by one or more halogen, C₁-C₁₂alkyl or C₁-C₁₂alkoxy.

R₁₇ for example also is hydrogen, phenyl-C₁-C₃alkyl, C₁-C₈alkanoyl, C₁-C₂₀alkyl which is unsubstituted or is substituted by one or more halogen, C₃-C₂₀cycloalkyl, O(CO)-(C₁-C₄alkyl), O(CO)-(C₂-C₄alkenyl) or by C₂-C₂₀alkyl which is interrupted by one or more O, or R₁₇ is C₂-C₂₀alkyl which is interrupted by one or more O.

In particular R₁₇ is hydrogen, C₁-C₈alkanoyl, C₁-C₂₀alkyl which is unsubstituted or substituted by one or more O(CO)-(C₁-C₄alkyl), O(CO)-(C₂-C₄alkenyl) or by C₂-C₂₀alkyl which is interrupted by one or more O, or R₁₇ is C₂-C₂₀alkyl which is interrupted by one or more O.

**R₁₆** for example is C₃-C₂₀cycloalkyl which is uninterrupted or interrupted by one or more O;
or R₁₈ is C₁-C₂₀alkyl which is unsubstituted or is substituted by one or more OH, O(CO)-(C₂-C₄)alkenyl, O(CO)-(C₁-C₄alkyl) or (CO)OR₁₇;
or R₁₈ is C₂-C₂₀alkyl which is interrupted by one or more O, S, CO, NR₂₆ or COOR₁₇;
or R₁₈ is C₂-C₈alkanoyl or C₃-C₆alkenoyl, benzoyl;
or R₁₈ is phenyl, naphthyl or C₃-C₂₀heteroaryl, each of which is unsubstituted or substituted by one or more halogen, C₁-C₁₂alkyl, C₁-C₄haloalkyl, C₁-C₁₂alkoxy or NO₂.

In another embocdiment R₁₈ is for example C₃-C₂₀cycloalkyl, C₁-C₂₀alkyl which is unsubstituted or is substituted by one or more OH, O(CO)-(C₂-C₄)alkenyl, O(CO)-(C₁-C₄alkyl) or (CO)OR₁₇;
or R₁₈ is phenyl or naphthyl each of which is unsubstituted or substituted by one or more halogen or C₁-C₁₂alkyl, in particular by halogen.

R₁₈ for example is C₁-C₂₀alkyl, C₂-C₁₂alkenyl, C₃-C₂₀cycloalkyl, phenyl-C₁-C₃alkyl, C₂-C₈alkanoyl, benzoyl, phenyl or naphthyl.

For example R₁₈ is C₁-C₂₀alkyl which is substituted by one or more OH, O(CO)-(C₂-C₄)alkenyl, O(CO)-(C₁-C₄alkyl) or (CO)OR₁₇, or R₁₈ is phenyl which is substituted by one or more halogen.

Preferably R₁₈ is C₁-C₈alkyl which is substituted as defined above.

**R₁₉** and **R₂₀** for example independently of each other are hydrogen, C₁-C₂₀alkyl, C₂-C₄hydroxyalkyl, C₃-C₂₀cycloalkyl, phenyl-C₁-C₃alkyl, phenyl or naphthyl, C₁-C₈alkanoyl, C₁-C₈alkanoyloxy, C₃-C₁₂alkenoyl or benzoyl;
or R₁₉ and R₂₀ together with the N-atom to which they are attached form a 5- or 6-membered saturated or unsaturated ring which optionally is interrupted by O, S or NR₁₇; or R₁₉ and R₂₀ together with the N-atom to which they are attached form a heteroaromatic ring system which is unsubstituted or substituted by one or more C₁-C₂₀alkyl, C₁-C₄haloalkyl, or

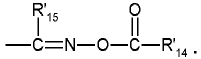

Furthermore R₁₉ and R₂₀ for example independently of each other are hydrogen, C₁-C₂₀alkyl, C₂-C₄hydroxyalkyl, C₃-C₂₀cycloalkyl, phenyl-C₁-C₃alkyl, C₁-C₈alkanoyl, C₁-C₈alkanoyloxy, C₃-C₁₂alkenoyl or benzoyl;
or R₁₉ and R₂₀ together with the N-atom to which they are attached form a 5- or 6-membered saturated ring which optionally is interrupted by O or NR₁₇; or R₁₉ and R₂₀ together with the N-atom to which they are attached form a carbazole ring.

R₁₉ and R₂₀ for example independently of each other are hydrogen, C₁-C₂₀alkyl, C₂-C₄hydroxyalkyl, C₃-C₂₀cycloalkyl, phenyl-C₁-C₃alkyl, C₁-C₈alkanoyl, C₁-C₈alkanoyloxy, C₃-C₁₂alkenoyl or benzoyl;
or R₁₉ and R₂₀ together with the N-atom to which they are attached form a 5- or 6-membered saturated ring which optionally is interrupted by O or NR₁₇.

Preferably R₁₉ and R₂₀ independently of each other are C₁-C₈alkanoyl, C₁-C₈alkanoyloxy; or R₁₉ and R₂₀ together with the N-atom to which they are attached form a morpholino ring.

**R₂₁** and **R₂₂** for example independently of each other are hydrogen, C₁-C₂₀alkyl, C₁-C₄haloalkyl, C₃-C₁₀cycloalkyl or phenyl;
or R₂₁ and R₂₂ together with N-atom to which they are attached form a morpholino ring. Especially R₂₁ and R₂₂ independently of each other are hydrogen or C₁-C₂₀alkyl.

**R₂₃** is for example hydrogen, OH, phenyl or C₁-C₂₀alkyl. In particular R₂₃ is hydrogen, OH or C₁-C₄alkyl.

The preferences for **R₂₄** are as given for R₁₉ and R₂₀.
The preferences for **R₂₅** are as given for R₁₇.

**R₂₆** for example is hydrogen, C₁-C₂₀alkyl, C₁-C₄haloalkyl, C₂-C₂₀alkyl which is interrupted by one or more O or CO, or is phenyl-C₁-C₄alkyl, C₃-C₈cycloalkyl which optionally is interrupted by one or more O or CO, or is (CO)R₁₉ or phenyl which is unsubstituted or substituted by one or more C₁-C₂₀alkyl, halogen, C₁-C₄haloalkyl, OR₁₇, SR₁₈, NR₁₉R₂₀.
Or R₂₆ for example denotes hydrogen, C₁-C₂₀alkyl, C₁-C₄haloalkyl, is phenyl-C₁-C₄alkyl, C₃-C₈cycloalkyl, (CO)R₁₉ or phenyl which is unsubstituted or substituted by one or more C₁-C₂₀alkyl.
Further R₂₆ for example is hydrogen or C₁-C₂₀alkyl, in particular C₁.C₄alkyl.

Examples of the compounds of the formula I of the present invention are

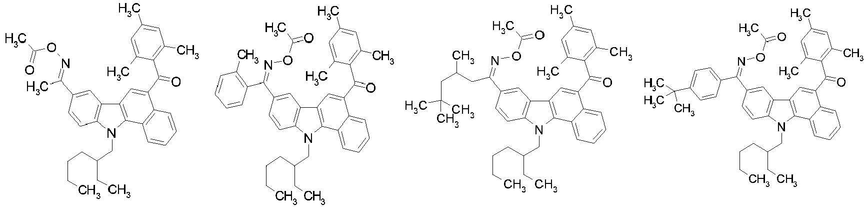

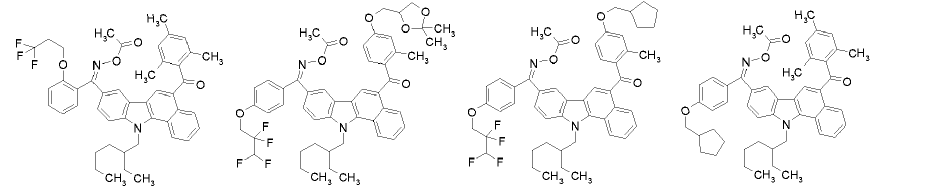

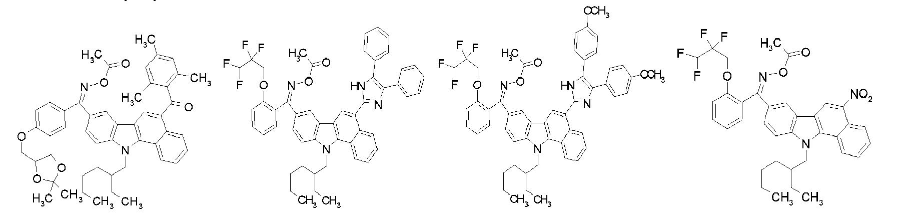

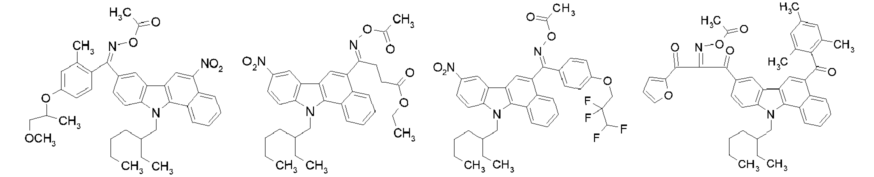

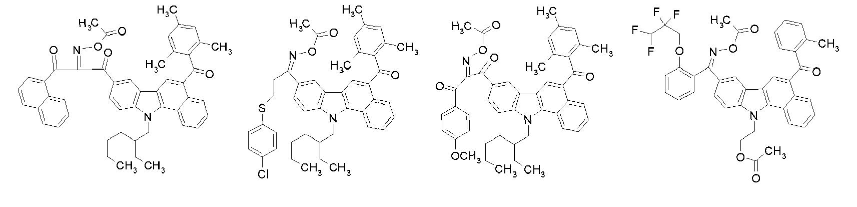

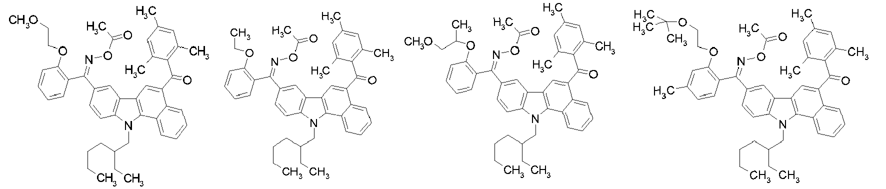

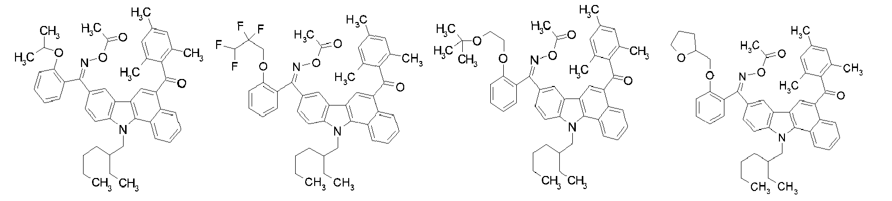

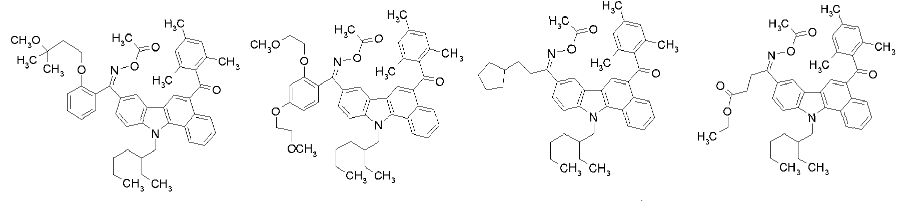

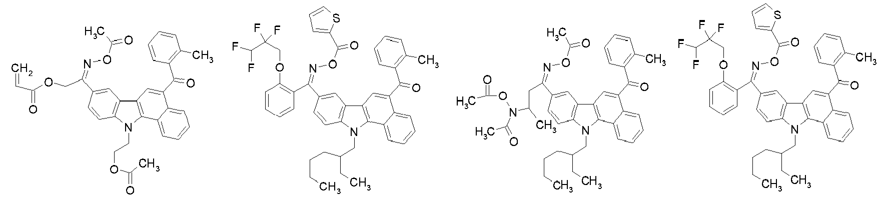

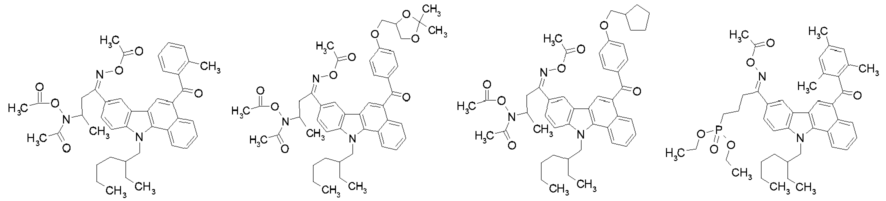

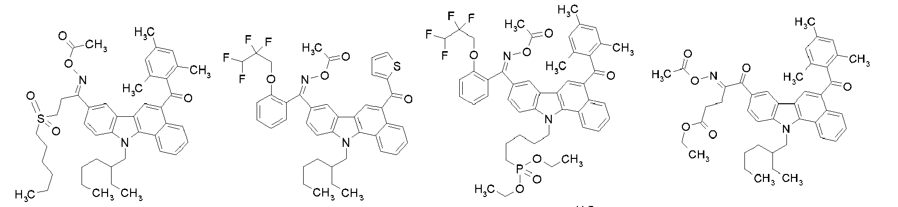

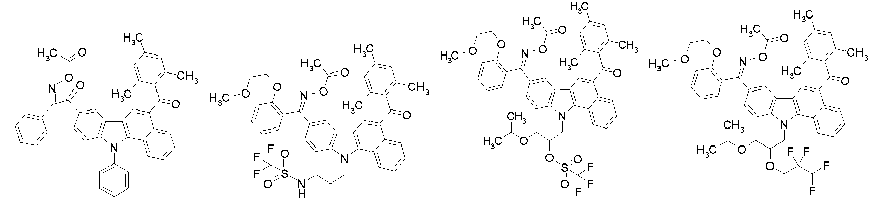

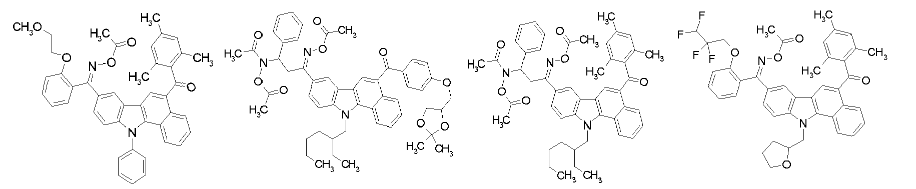

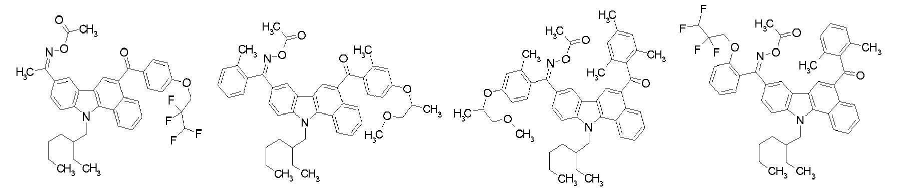

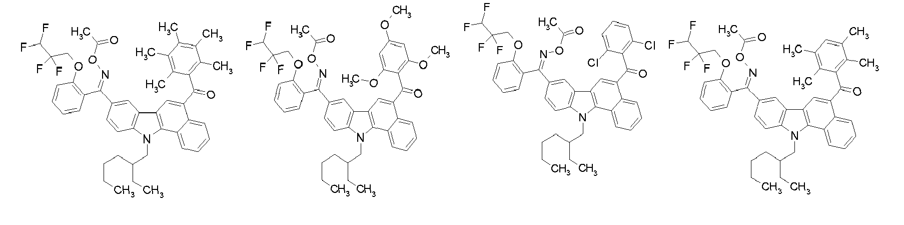

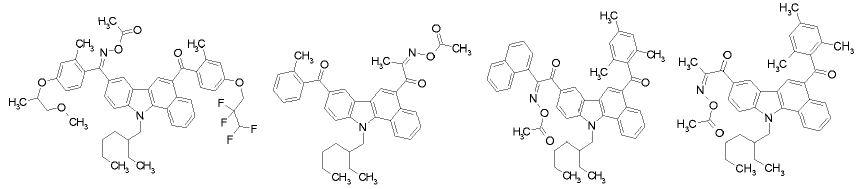

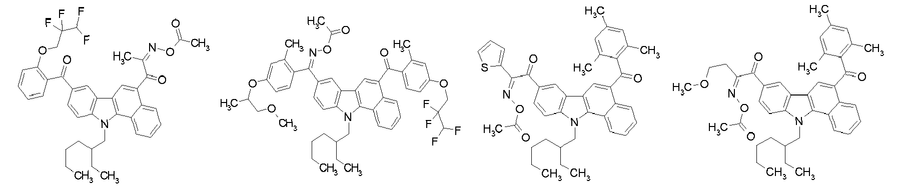

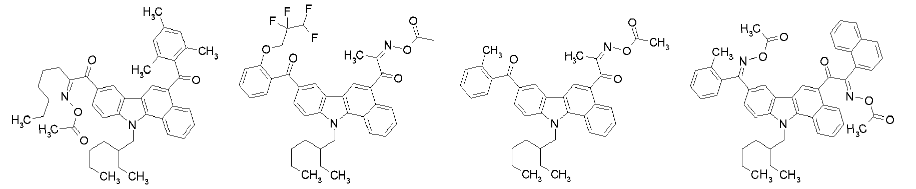

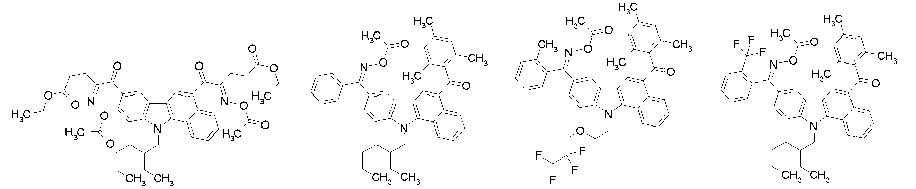

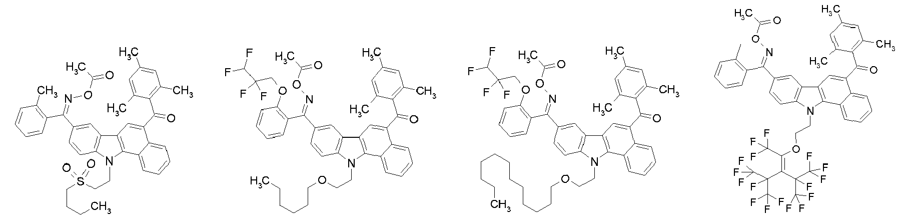

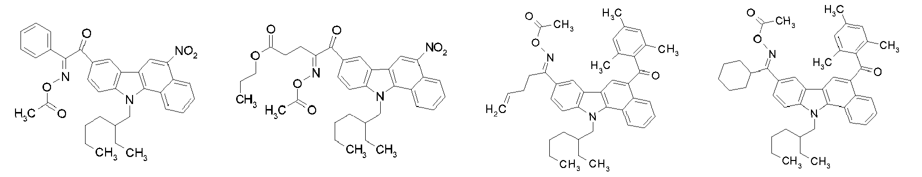

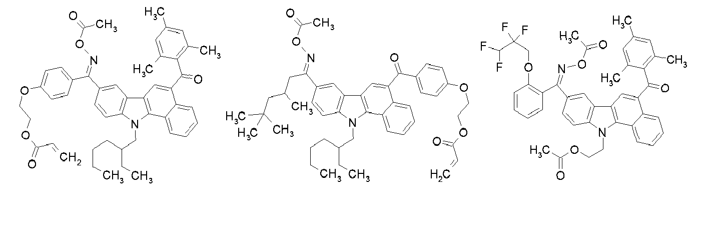

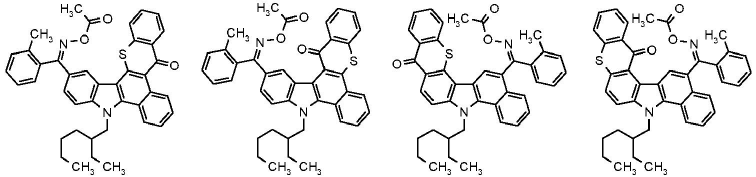

The compounds of the formula I are suitable as radical photoinitiators.
Accordingly, subject of the invention is the use of a compound of the formula (I) as defined above for the photopolymerization of a composition comprising at least one ethylenically unsaturated photopolymerizable compound.

Another subject of the present invention therefore is a photopolymerizable composition comprising
(a) at least one ethylenically unsaturated photopolymerizable compound and
(b) as photoinitiator, at least one compound of the formula I as defined above.

The composition may comprise additionally to the photoinitiator (b) at least one further photoinitiator (c), and/or other additives (d).
The composition may comprise additionally to the photoinitiator (b) at least one further photoinitiator (c) or other additives (d) or both, at least one further photoinitiator (c) and other additives (d).

The unsaturated compounds (a) may include one or more olefinic double bonds. They may be of low (monomeric) or high (oligomeric) molecular mass. Examples of monomers containing a double bond are alkyl, hydroxyalkyl, cycloalkyl (which optionally interrupted by O) or amino acrylates, or alkyl, hydroxyalkyl, cycloalkyl (which optionally interrupted by O) or amino methacrylates, for example methyl, ethyl, butyl, 2-ethylhexyl or 2-hydroxyethyl acrylate, tetrahydrofurfuryl acrylate, isobornyl acrylate, methyl methacrylate, cyclohexyl methacrylate or ethyl methacrylate. Silicone acrylates are also advantageous. Other examples are acrylonitrile, acrylamide, methacrylamide, N-substituted (meth)acrylamides, vinyl esters such as vinyl acetate, vinyl ethers such as isobutyl vinyl ether, styrene, alkyl- and halostyrenes, N-vinylpyrrolidone, vinyl chloride or vinylidene chloride.
Examples of monomers containing two or more double bonds are the diacrylates of ethylene glycol, propylene glycol, neopentyl glycol, hexamethylene glycol or of bisphenol A, and 4,4'-bis(2-acryl-oyloxyethoxy)diphenylpropane, trimethylolpropane triacrylate, pentaerythritol triacrylate or tetraacrylate, vinyl acrylate, divinylbenzene, divinyl succinate, diallyl phthalate, triallyl phosphate, triallyl isocyanurate or tris(2-acryloylethyl) isocyanurate.
Examples of polyunsaturated compounds of relatively high molecular mass (oligomers) are acrylated epoxy resins, polyesters containing acrylate-, vinyl ether- or epoxy-groups, and also polyurethanes and polyethers. Further examples of unsaturated oligomers are unsaturated polyester resins, which are usually prepared from maleic acid, phthalic acid and one or more diols and have molecular weights of from about 500 to 3000. In addition it is also possible to employ vinyl ether monomers and oligomers, and also maleate-terminated oligomers with polyester, polyurethane, polyether, polyvinyl ether and epoxy main chains. Of particular suitability are combinations of oligomers which carry vinyl ether groups and of polymers as described in WO 90/01512. However, copolymers of vinyl ether and maleic acid-functionalized monomers are also suitable. Unsaturated oligomers of this kind can also be referred to as prepolymers.

Particularly suitable examples are esters of ethylenically unsaturated carboxylic acids and polyols or polyepoxides, and polymers having ethylenically unsaturated groups in the chain or in side groups, for example unsaturated polyesters, polyamides and polyurethanes and copolymers thereof, polymers and copolymers containing (meth)acrylic groups in side chains, and also mixtures of one or more such polymers.
Examples of unsaturated carboxylic acids are acrylic acid, methacrylic acid, crotonic acid, itaconic acid, cinnamic acid, and unsaturated fatty acids such as linolenic acid or oleic acid. Acrylic and methacrylic acid are preferred.
Suitable polyols are aromatic and, in particular, aliphatic and cycloaliphatic polyols. Examples of aromatic polyols are hydroquinone, 4,4'-dihydroxydiphenyl, 2,2-di(4-hydroxyphenyl)propane, and also novolaks and resols. Examples of polyepoxides are those based on the abovementioned polyols, especially the aromatic polyols, and epichlorohydrin. Other suitable polyols are polymers and copolymers containing hydroxyl groups in the polymer chain or in side groups, examples being polyvinyl alcohol and copolymers thereof or polyhydroxyalkyl methacrylates or copolymers thereof. Further polyols which are suitable are oligoesters having hydroxyl end groups.
Examples of aliphatic and cycloaliphatic polyols are alkylenediols having preferably 2 to 12 C atoms, such as ethylene glycol, 1,2- or 1,3-propanediol, 1,2-, 1,3- or 1,4-butanediol, pentanediol, hexanediol, octanediol, dodecanediol, diethylene glycol, triethylene glcyol, polyethylene glycols having molecular weights of preferably from 200 to 1500, 1,3-cyclopentanediol, 1,2-, 1,3- or 1,4-cyclohexanediol, 1,4-dihydroxymethylcyclo-hexane, glycerol, tris(β-hydroxyethyl)amine, trimethylolethane, trimethylolpropane, pentaerythritol, dipentaerythritol and sorbitol.

The polyols may be partially or completely esterified with one carboxylic acid or with different unsaturated carboxylic acids, and in partial esters the free hydroxyl groups may be modified, for example etherified or esterified with other carboxylic acids. Examples of esters are:
trimethylolpropane triacrylate, trimethylolethane triacrylate, trimethylolpropane trimethacrylate, trimethylolethane trimethacrylate, tetramethylene glycol dimethacrylate, triethylene glycol dimethacrylate, tetraethylene glycol diacrylate, pentaerythritol diacrylate, pentaerythritol triacrylate, pentaerythritol tetraacrylate, dipentaerythritol diacrylate, dipentaerythritol triacrylate, dipentaerythritol tetraacrylate, dipentaerythritol pentaacrylate, dipentaerythritol hexaacrylate, tripentaerythritol octaacrylate, pentaerythritol dimethacrylate, pentaerythritol trimethacrylate, dipentaerythritol dimethacrylate, dipentae-rythritol tetramethacrylate, tripentaerythritol octamethacrylate, pentaerythritol diitaconate, dipentaerythritol tris-itaconate, dipentaerythritol pentaitaconate, dipentaerythritol hexaitaconate, ethylene glycol diacrylate, 1,3-butanediol diacrylate, 1,3-butanediol dimethacrylate, 1,4-butanediol diitaconate, sorbitol triacrylate, sorbitol tetraacrylate, pentaerythritol-modified triacrylate, sorbitol tetra methacrylate, sorbitol pentaacrylate, sorbitol hexaacrylate, oligoester acrylates and methacrylates, glycerol diacrylate and triacrylate, 1,4-cyclohexane diacrylate, bisacrylates and bismethacrylates of polyethylene glycol with a molecular weight of from 200 to 1500, or mixtures thereof.

Also suitable as components (a) are the amides of identical or different, unsaturated carboxylic acids with aromatic, cycloaliphatic and aliphatic polyamines having preferably 2 to 6, especially 2 to 4, amino groups. Examples of such polyamines are ethylenediamine, 1,2- or 1,3-propylenediamine, 1,2-, 1,3- or 1,4-butylenediamine, 1,5-pentylenediamine, 1,6-hexylenediamine, octylenediamine, dodecylenediamine, 1,4-diaminocyclohexane, isophoronediamine, phenylenediamine, bisphenylenediamine, di-ß-aminoethyl ether, diethylenetriamine, triethylenetetramine, di(ß-aminoethoxy)- or di(ß-aminopropoxy)ethane. Other suitable polyamines are polymers and copolymers, preferably with additional amino groups in the side chain, and oligoamides having amino end groups. Examples of such unsaturated amides are methylenebisacrylamide, 1,6-hexamethylenebisacrylamide, diethylenetriaminetrismethacrylamide, bis(methacryl-amidopropoxy)ethane, ß-methacrylamidoethyl methacrylate and N[(ß-hydroxyethoxy)-ethyl]acrylamide.

Suitable unsaturated polyesters and polyamides are derived, for example, from maleic acid and from diols or diamines. Some of the maleic acid can be replaced by other dicarboxylic acids. They can be used together with ethylenically unsaturated comonomers, for example styrene. The polyesters and polyamides may also be derived from dicarboxylic acids and from ethylenically unsaturated diols or diamines, especially from those with relatively long chains of, for example 6 to 20 C atoms. Examples of polyurethanes are those composed of saturated or unsaturated diisocyanates and of unsaturated or, respectively, saturated diols.
Polymers with (meth)acrylate groups in the side chain are likewise known. They may, for example, be reaction products of epoxy resins based on novolaks with (meth)acrylic acid, or may be homo- or copolymers of vinyl alcohol or hydroxyalkyl derivatives thereof which are esterified with (meth)acrylic acid, or may be homo- and copolymers of (meth)acrylates which are esterified with hydroxyalkyl (meth)acrylates.
Other suitable polymers with acrylate or methacrylate groups in the side chains are, for example, solvent soluble or alkaline soluble polyimide precursors, for example poly(amic acid ester) compounds, having the photopolymerizable side groups either attached to the backbone or to the ester groups in the molecule, i.e. according to EP 624826. Such oligomers or polymers can be formulated with the new photoinitiators and optionally reactive diluents, like polyfunctional (meth)acrylates in order to prepare highly sensitive polyimide precursor resists.

The photopolymerizable compounds can be used alone or in any desired mixtures. It is preferred to use mixtures of polyol (meth)acrylates.

Examples of the component (a) are also polymers or oligomers having at least two ethylenically unsaturated groups and at least one carboxyl function within the molecule structure, such as a resin obtained by the reaction of a saturated or unsaturated polybasic acid anhydride with a product of the reaction of an epoxy compound and an unsaturated monocarboxylic acid, for example, photosensitive compounds as described in JP 6-1638 and JP 10301276 and commercial products such as EB9696, UCB Chemicals; KAYARAD TCR1025, Nippon Kayaku Co.,LTD., or an addition product formed between a carboxyl group-containing resin and an unsaturated compound having an α,β-unsaturated double bond and an epoxy group (for example, ACA200M, Daicel Industries, Ltd.).

As diluent, a mono- or multi-functional ethylenically unsaturated compound, or mixtures of several of said compounds, can be included in the above composition up to 70 % by weight based on the solid portion of the composition.

Subject of the invention also is a photopolymerizable composition as described above, wherein the component (a) is a resin obtained by the reaction of a saturated or unsaturated polybasic acid anhydride with a product of the reaction of an epoxy resin and an unsaturated monocarboxylic acid.
Such components are for example described in JP06-1938, JP08-278629, JP08-278630, JP10-301276, JP2001-40022, JP10-221843, JP11-231523, JP2002-206014-A or JP2006-53569-A, the disclosure of which hereby is incorpoarted by reference.
The unsaturated compounds (a) can also be used as a mixture with nonphotopolymerizable, film-forming components. These may, for example, be physically drying polymers or solutions thereof in organic solvents, for instance nitrocellulose or cellulose acetobutyrate. They may also, however, be chemically and/or thermally curable (heat-curable) resins, examples being polyisocyanates, polyepoxides and melamine resins, as well as polyimide precursors. The use of heat-curable resins at the same time is important for use in systems known as hybrid systems, which in a first stage are photopolymerized and in a second stage are crosslinked by means of thermal aftertreatment.

The invention also provides compositions comprising as component (a) at least one ethylenically unsaturated photopolymerizable compound which is emulsified or dissolved in water.

Many variants of such radiation-curable aqueous prepolymer dispersions are commercially available. A prepolymer dispersion is understood as being a dispersion of water and at least one prepolymer dispersed therein. The concentration of water in these systems is, for example, from 5 to 80% by weight, in particular from 30 to 60% by weight. The concentration of the radiation-curable prepolymer or prepolymer mixture is, for example, from 95 to 20% by weight, in particular from 70 to 40% by weight. In these compositions the sum of the percentages given for water and prepolymer is in each case 100, with auxiliaries and additives being added in varying quantities depending on the intended use.
The radiation-curable, film-forming prepolymers which are dispersed in water and are often also dissolved are aqueous prepolymer dispersions of mono- or polyfunctional, ethylenically unsaturated prepolymers which are known per se, can be initiated by free radicals and have for example a content of from 0.01 to 1.0 mol of polymerizable double bonds per 100 g of prepolymer and an average molecular weight of, for example, at least 400, in particular from 500 to 10'000. Prepolymers with higher molecular weights, however, may also be considered depending on the intended application. Use is made, for example, of polyesters containing polymerizable C-C double bonds and having an acid number of not more than 10, of polyethers containing polymerizable C-C double bonds, of hydroxyl-containing reaction products of a polyepoxide, containing at least two epoxide groups per molecule, with at least one α,ß-ethylenically unsaturated carboxylic acid, of polyurethane (meth)acrylates and of acrylic copolymers which contain α,ß-ethylenically unsaturated acrylic radicals, as are described in EP 12339. Mixtures of these prepolymers can likewise be used. Also suitable are the polymerizable prepolymers described in EP 33896, which are thioether adducts of polymerizable prepolymers having an average molecular weight of at least 600, a carboxyl group content of from 0.2 to 15% and a content of from 0.01 to 0.8 mol of polymerizable C-C double bonds per 100 g of prepolymer. Other suitable aqueous dispersions, based on specific alkyl (meth)acrylate polymers, are described in EP 41125, and suitable waterdispersible, radiation-curable prepolymers of urethane acrylates can be found in DE 2936039. Further additives which may be included in these radiation-curable aqueous prepolymer dispersions are dispersion auxiliaries, emulsifiers, antioxidants, e.g. 2,2-thiobis(4-methyl-6t-butylphenol) or 2,6-di-t-butylphenol, light stabilizers, dyes, pigments, fillers, such as glass or alumina, for example talc, gypsum, silicic acid, rutile, carbon black, zinc oxide, iron oxides, reaction accelerators, levelling agents, lubricants, wetting agents, thickeners, flatting agents, antifoams and other auxiliaries customary in paint technology. Suitable dispersion auxiliaries are water-soluble organic compounds which are of high molecular mass and contain polar groups, examples being polyvinyl alcohols, polyvinylpyrrolidone or cellulose ethers. Emulsifiers which can be used are nonionic emulsifiers and, if desired, ionic emulsifiers as well.

It is of course also possible to add other known photoinitiators (c), for example mixtures with camphor quinone; benzophenone, benzophenone derivatives, such as for example given below as sensitzers; ketal compounds, as for example benzildimethylketal (Irgacure® 651); acetophenone, acetophenone derivatives, for example α-hydroxycycloalkyl phenyl ketones or α-hydroxyalkyl phenyl ketones, such as for example 2-hydroxy-2-methyl-1-phenyl-propanone (Darocur® 1173), 1-hydroxycyclohexyl-phenyl-ketone (Irgacure® 184), 1-(4-dodecylbenzoyl)-1-hydroxy-1-methyl-ethane, 1-(4-isopropylbenzoyl)-1-hydroxy-1-methyl-ethane, 1-[4-(2-hydroxyethoxy)-phenyl]-2-hydroxy-2-methyl-1-propan-1-o n e (Irgacure^{®}2959); 2-hydroxy-1-{4-[4-(2-hydroxy-2-methyl-propionyl)-benzyl]-phenyl}-2-methyl-propan-1-one (Irgacure®127); 2-hydroxy-1-{4-[4-(2-hydroxy-2-methyl-propionyl)-phenoxy]-phenyl}-2-methyl-propan-1-o n e ; 1-[4-(benzoyl-phenyl-thia)phenyl]-2-methyl-2-(p-tolylsulfonyl)-propane-1-one (Esacure® 1001 M); oligomeric α-hydroxyalkyl phenyl ketones e.g. Esacure®ONE, Esacure®KIP 100; dialkoxyacetophenones, α-aminoacetophenones, e.g. (4-methylthiobenzoyl)-1-methyl-1-morpholinoethane (Irgacure® 907), (4-morpholinobenzoyl)-1-benzyl-1-dimethylaminopropane (Irgacure® 369), (4-morpholinobenzoyl)-1-(4-methylbenzyl)-1-dimethylaminopropane (Irgacure® 379), (4-(2-hydroxyethyl)aminobenzoyl)-1-benzyl-1-dimethylaminopropane), (3,4-dimethoxybenzoyl)-1-benzyl-1-dimethyl-aminopropane; 4-aroyl-1,3-dioxolanes, benzoin alkyl ethers, phenylglyoxalic esters and derivatives thereof, e.g. methyl α-oxo benzeneacetate, oxo-phenyl-acetic acid 2-(2-hydroxy-ethoxy)-ethyl ester, dimeric phenylglyoxalic esters, e.g. oxo-phenyl-acetic acid 1-methyl-2-[2-(2-oxo-2-phenyl-acetoxy)-propoxy]-ethyl ester (Irgacure® 754); other oximeesters, e.g. 1,2-octanedione 1-[4-(phenylthio)phenyl]-2-(O-benzoyloxime) (Irgacure® OXE01), ethanone 1-[9-ethyl-6-(2-methylbenzoyl)-9H-carbazol-3-yl]-1-(O-acetyloxime) (Irgacure® OXE02), 9H-thioxanthene-2-carboxaldehyde 9-oxo-2-(O-acetyl-oxime), peresters, e,g. benzophenone tetracarboxylic peresters as described for example in EP 126541, monoacyl phosphine oxides, e.g. (2,4,6-trimethylbenzoyl)diphenylphosphine oxide(Darocur® TPO), ethyl (2,4,6 trimethylbenzoyl phenyl) phosphinic acid ester; bisacylphosphine oxides, e.g. bis(2,6-dimethoxy-benzoyl)-(2,4,4-trimethyl-pentyl)phosphine oxide, bis(2,4,6-trimethylbenzoyl)-phenylphosphine oxide (Irgacure® 819), bis(2,4,6-trimethylbenzoyl)-2,4-dipentoxyphenylphosphine oxide, trisacylphosphine oxides, halomethyltriazines, e.g. 2-[2-(4-methoxy-phenyl)-vinyl]-4,6-bis-trichloromethyl-[1,3,5]triazine, 2-(4-methoxy-phenyl)-4,6-bis-trichloromethyl-[1,3,5]-triazene, 2-(3,4-dimethoxy-phenyl)-4,6-bis-trichloromethyl-[1,3,5]triazine, 2-methyl-4,6-bis-trichloromethyl-[1,3,5]triazine, hexaarylbisimidazole / coinitiators systems, e.g. ortho-chlorohexaphenyl-bisimidazole combined with 2-mercaptobenzthiazole, ferrocenium compounds, or titanocenes, e.g. bis(cyclopentadienyl)-bis(2,6-difluoro-3-pyrryl-phenyl)titanium (Irgacure®784). Further, borate compounds can be used as coinitiators. The Darocur® and Irgacure® compounds are available from BASF SE; Esacure is available from Lamberti SPA.
Where the novel photoinitiator systems are employed in hybrid systems, use is made, in addition to the novel free-radical hardeners, of cationic photoinitiators, of peroxide compounds, such as benzoyl peroxide (other suitable peroxides are described in US 4950581 column 19, lines 17-25), of aromatic sulfonium-, phosphonium- or iodonium salts as described for example in US4950581, column 18, line 60 to column 19, line 10 or cyclopentadienylarene-iron(II) complex salts, for example (η⁶-iso-propylbenzene)(η⁵-cyclopentadienyl)iron(II) hexafluorophosphate, as well as oxime sulfonic acid esters, as are, for example described in EP780729. Also pyridinium and (iso)quinolinium salts as described e.g. in EP497531 and EP441232 may be used in combination with the new photoinitiators.
The new photoinitiators, either alone or in mixtures with other known photoinitiators and sensitizers, can be used also in the form of a dispersion or emulsion in water or aqueous solutions. In case the compounds are used in emulsions or dispersions conveniently customary dispersants or emulsifiers are added to prepare a stable emulsion or dispersion. Corresponding suitable additives are known to the person skilled in the art.
The photopolymerizable compositions generally comprise 0.05 to 25 % by weight, preferably 0.01 to 10 % by weight, in particular 0.01 to 5 % by weight of the photoinitiator, based on the solid composition. The amount refers to the sum of all photoinitiators added, if mixtures of initiators are employed. Accordingly, the amount either refers to the photoinitiator (b) or the photoinitiators (b) +(c).

In addition to the photoinitiator the photopolymerizable mixtures may include various additives (d). Examples of these are thermal inhibitors, which are intended to prevent premature polymerization, examples being hydroquinone, hydroquinone derivatives, p-methoxyphenol, ß-naphthol or sterically hindered phenols, such as 2,6-di-tert-butyl-p-cresol In order to increase the stability on storage in the dark it is possible, for example, to use copper compounds, such as copper naphthenate, stearate or octoate, phosphorus compounds, for example triphenylphosphine, tributylphosphine, triethyl phosphite, triphenyl phosphite or tribenzyl phosphite, quaternary ammonium compounds, for example tetramethylammonium chloride or trimethylbenzylammonium chloride, or hydroxylamine derivatives, for example N-diethylhydroxylamine. To exclude atmospheric oxygen during the polymerization it is possible to add paraffin or similar wax-like substances which, being of inadequate solubility in the polymer, migrate to the surface in the beginning of polymerization and form a transparent surface layer which prevents the ingress of air. It is also possible to apply an oxygen-impermeable layer on top of the coating, for example poly(vinylalcohol-co-vinylacetate). Light stabilizers which can be added in a small quantity are UV absorbers, for example those of the hydroxyphenylbenzotriazole, hydroxyphenyl-benzophenone, oxalamide or hydroxyphenyl-s-triazine type. These compounds can be used individually or in mixtures, with or without sterically hindered amines (HALS). Examples of such UV absorbers and light stabilisers are disclosed in WO 04/074328, page 12, line 9 to page14, line 23, said disclosure hereby is incorporated by reference.

To accelerate the photopolymerization it is possible to add amines as component (d), for example triethanolamine, N-methyldiethanolamine, ethyl-p-dimethylaminobenzoate, 2-(dimethylamino)ethyl benzoate, 2-ethylhexyl-p-dimethylaminobenzoate, octyl-para-N,N-dimethylaminobenzoate, N-(2-hydroxyethyl)-N-methyl-para-toluidine or Michler's ketone. The action of the amines can be intensified by the addition of aromatic ketones of the benzophenone type. Examples of amines which can be used as oxygen scavengers are substituted N,N-dialkylanilines, as are described in EP339841. Other accelerators, coinitiators and autoxidizers are thiols, thioethers, disulfides, phosphonium salts, phosphine oxides or phosphines, as described, for example, in EP438123, in GB2180358 and in JP Kokai Hei 6-68309.
It is further possible to add chain transfer agents which are customary in the art to the compositions according to the invention as component (d). Examples are mercaptans, amines and benzothiazol.
Photopolymerization can also be accelerated by adding further photosensitizers or coinitiators (as component (d)) which shift or broaden the spectral sensitivity. These are, in particular, aromatic compounds, for example benzophenone and derivatives thereof, thioxanthone and derivatives thereof, anthraquinone and derivatives thereof, coumarin and phenothiazine and derivatives thereof, and also 3-(aroylmethyl-ene)thiazolines, rhodanine, camphorquinone, but also eosine, rhodamine, erythrosine, xanthene, thioxanthene, acridine, e.g. 9-phenylacridine, 1,7-bis(9-acridinyl)heptane, 1,5-bis(9-acridinyl)pentane, cyanine and merocyanine dyes. Examples of suitable sensitizer compounds (d) are disclosed in WO 06/008251, page 36, line 30 to page 38, line 8, the disclosure of which is hereby incorporated by reference.

A photopolymerizable composition, comprising as further additive (d) a photosensitizer compound selected from the group consisting of benzophenone and its derivatives, thioxanthone and its derivatives, anthraquinone and its derivatives, or coumarine and its derivatives is preferred.
Examples of benzophenone derivatives, thioxanthone derivatives, anthraquinone deriatives and coumarin derivatives are benzophenone, 4-phenyl benzophenone, 4-methoxy benzophenone, 4,4'-dimethoxy benzophenone, 4,4'-dimethyl benzophenone, 4,4'-dichlorobenzophenone 4,4'-bis(dimethylamino)benzophenone, 4,4'-bis(diethylamino)benzophenone, 4,4'-bis(methylethylamino)benzophenone, 4,4'-bis(p-isopropylphenoxy)benzophenone, 4-methyl benzophenone, 2,4,6-trimethylbenzophenone, 4-(4-methylthiophenyl)-benzophenone, 3,3'-dimethyl-4-methoxy benzophenone, methyl-2-benzoylbenzoate, 4-(2-hydroxyethylthio)-benzophenone, 4-(4-tolylthio)benzophenone, 1-[4-(4-benzoyl-phenylsulfanyl)-phenyl]-2-methyl-2-(toluene-4-sulfonyl)-propan-1-one, 4-benzoyl-N,N,N-trimethylbenzenemethanaminium chloride, 2-hydroxy-3-(4-benzoyl-phenoxy)-N,N,N-trimethyl-1-propanaminium chloride monohydrate, 4-(13-acryloyl-1,4,7,10,13-pentaoxatridecyl)-benzophenone, 4-benzoyl-N,N-dimethyl-N-[2-(1-oxo-2-propenyl)oxy]ethyl-benzenemethanaminium chloride; thioxanthone, 2-isopropylthioxan-thone, 2-chlorothioxanthone, 1-chloro-4-propoxythioxanthone, 2-dodecylthioxanthone, 2,4-diethylthioxanthone, 2,4-dimethylthioxanthone, 1-methoxycarbonylthioxanthone, 2-ethoxycarbonylthioxanthone, 3-(2-methoxyethoxycarbonyl)-thioxanthone, 4-butoxy-arbnylthioxanthone, 3-butoxycarbonyl-7-methylthioxanthone, 1-cyano-3chlorothioxanthone, 1-ethoxycarbonyl-3-chlorothioxanthone, 1-ethoxycarbonyl-3-ethoxythioxanthne, 1-ethoxycarbonyl-3-aminothioxanthone, 1-ethoxycarbonyl-3-phenylsulfurylthioxanthone, 3,4-di-[2-(2-methoxyethoxy)ethoxycarbonyl]-thioxanthone, 1,3-dimethyl-2-hydroxy-9Hthioxanthen-9-one 2-ethylhexylether, 1-ethoxycarbonyl-3-(1-methyl-1-morpholinothyl)-thioxanthone, 2-methyl-6-dimethoxymethyl-thioxanthone, 2-methyl-6-(1,1-dimethxyenzyl)-thioxanthone, 2-morpholinomethylthioxanthone, 2-methyl-6-morpholinoethylhioxanthone, N-allylthioxanthone-3,4-dicarboximide, N-octylthioxanthone-3,4-dicarbximide, N-(1,1,3,3-tetramethylbutyl)-thioxanthone-3,4-dicarboximide, 1-phenoxythioanhone, 6-ethoxycarbonyl-2methoxythioxanthone, 6-ethoxycarbonyl-2-methylthioxanhone, thioxanthone-2-carboxylic acid polyethyleneglycol ester, 2-hydroxy-3-(3,4-dimethyl-9-oxo-9H-thioxanthon-2-yloxy)-N,N,N-trimethyl-1-propanaminium chloride, couarin 1, Coumarin 2, Coumarin 6, Coumarin 7, Coumarin 30, Coumarin 102, Coumarin 106, Coumarin 138, Coumarin 152, Coumarin 153, Coumarin 307, Coumarin 314, Coumarin 314T, Coumarin 334, Coumarin 337, Coumarin 500, 3-benzoyl coumarin, 3-benzoyl-7-methoxycoumarin, 3-benzoyl-5,7-dimethoxycoumarin, 3-benzoyl-5,7-dipropoxycoumarin, 3-benzoyl-6,8-dichlorocoumarin, 3-benzoyl-6-chloro-coumarin, 3,3'-carbonyl-bis[5,7-di(propoxy)coumarin], 3,3'-carbonyl-bis(7-methoxycoumarin), 3,3'-carbonyl-bis(7-diethylamino-coumarin), 3-isobutyroylcoumarin, 3-benzoyl-5,7-dimethoxy-coumarin, 3-benzoyl-5,7-diethoxy-coumarin, 3-benzoyl-5,7-dibutoxycoumarin, 3-benzoyl-5,7-di(methoxyethoxy)-coumarin, 3-benzoyl-5,7-di(allyloxy)coumarin, 3-benzoyl-7-dimethylaminocoumarin, 3-benzoyl-7-diethylaminocoumarin, 3-isobutyroyl-7-dimethylaminocoumarin, 5,7-dimethoxy-3-(1-naphthoyl)-coumarin, 5,7-diethoxy-3-(1-naphthoyl)-coumarin, 3-benzoylbenzo[f]coumarin, 7-diethylamino-3-thienoylcoumarin, 3-(4-cyanobenzoyl)-5,7-dimethoxycoumarin, 3-(4-cyanobenzoyl)-5,7-dipropoxycoumarin, 7-dimethylamino-3-phenylcoumarin, 7-diethylamino-3-phenylcoumarin, the coumarin derivatives disclosed in JP 09-179299-A and JP 09-325209-A, for example 7-[{4-chloro-6-(diethylamino)-S-triazine-2-yl}amino]-3-phenylcoumarin, 9,10-anthraquinone and anthracene.
The curing process can be assisted by adding photosensitizers, in particular, in compositions which are pigmented (for example with titanium dioxide), and also by adding a component which under thermal conditions forms free radicals, for example an azo compound such as 2,2'-azobis(4-methoxy-2,4-dimethylvaleronitrile), a triazene, diazo sulfide, pentazadiene or a peroxy compound, for instance a hydroperoxide or peroxycarbonate, for example t-butyl hydroperoxide, as described for example in EP245639. The compositions according to the invention may comprise as further additive (d) a photoreducable dye, e.g., xanthene-, benzoxanthene-, benzothioxanthene, thiazine-, pyronine-, porphyrine- or acridine dyes, and/or trihalogenmethyl compounds which can be cleaved by irradiation. Similar compositions are for example described in EP445624.
Further additives known in the art may be added as component (d), as for example flow improvers, adhesion promoters, such as vinyltrimethoxysilane, vinyltriethoxysilane vinyltris(2-methoxyethoxy)silane, N-(2-aminoethyl)-3-aminopropylmethyldimethoxysilane, N-(2-aminoethyl)3-aminopropyltrimethoxysilane, 3-aminopropyltriethoxysilane, 3-glycid-oxypropyltrimethoxysilane, 3-glycidoxypropylmethyldimethoxysilane, 2-(3,4-epoxycyclohexyl)ethyltrimethoxysilane, 3-chloropropylmethyldimethoxysilane, 3-chloropropyltrimethoxysilane, 3-methacryloxypropyltrimethoxysilane and 3-mercaptopropyltrimethoxysilane. Surfactants, optical brighteners, pigments, dyes, wetting agents, levelling assistants, dispersants, aggregation preventers, antioxidants or fillers are further examples for additives (d).
In order to cure thick and pigmented coatings it is appropriate to add glass microspheres or pulverized glass fibres, as described for example in US 5013768.
Further suitable components (d) are, as already mentionend above, surfactants and dispersants and other components, in particular to support the application of pigments or colorants in the formulation.
It is preferred to apply a surface treatment to the pigments in order to make the pigment easy to disperse and to stabilize the resultant pigment dispersion. The surface treatment reagents are, for example, surfactants, polymeric dispersants, general texture improving agents, pigment derivatives and mixtures thereof. It is especially preferred when the colorant composition according to the invention comprises at least one polymeric dispersant and/or at least pigment derivative.
Suitable surfactants include anionic surfactants such as alkylbenzene- or alkylnahthalene-sulfonates, alkylsulfosuccinates or naphthalene formaldehyde sulfonates; cationic surfactants including, for example, quaternary salts such as benzyl tributyl ammonium chloride; or nonionic or amphoteric surfactants such as polyoxyethylene surfactants and alkyl- or amidopropyl betaines, respectively.
Illustrative examples of the surfactant include polyoxyethylene alkyl ethers such as polyoxyethylene lauryl ether, polyoxyethylene stearyl ether and polyoxyethylene oleyl ether; polyoxyethylene alkylphenyl ethers such as polyoxyethylene octylphenyl ether and polyoxyethylene nonylphenyl ether; polyethylene glycol diesters such as polyethylene glycol dilaurate and polyethylene glycol distearate; sorbitan fatty acid esters; fatty acid modified polyesters; tertiary amine modified polyurethanes; polyethyleneimines; those available under the trade names of KP (a product of Shin-Etsu Chemical Co., Ltd), Polyflow (a product of KYOEISHA CHEMICAL Co., Ltd), F-Top (a product of Tochem Products Co., Ltd), MEGAFAC (a product of Dainippon Ink & Chemicals, Inc.), Fluorad (a product of Sumitomo 3M Ltd), Asahi Guard and Surflon (products of Asahi Glass Co., Ltd); and the like.
These surfactants may be used alone or in admixture of two or more.
The surfactant is generally used in an amount of 50 parts or less by weight, preferably 0 to 30 parts by weight, based on 100 parts by weight of the colorant composition.

Polymeric dispersants include high molecular weight polymers with pigment affinic groups. Examples are: statistical co-polymers comprised from, for instance, styrene derivatives, (meth)acrylates and (meth)acrylamides, and such statistical co-polymers modified by post modification; block co-polymers and/or comb polymers comprised from, for instance, styrene derivatives, (meth)acrylates and (meth)acrylamides, and such block co-polymers and/or comb polymers modified by post modification; polyethylenimines, which for instance is crafted with polyesters; polyamines, which for instance is crafted with polyesters; and many kinds of (modified) polyurethanes. Polymeric dispersants may also be employed. Suitable polymeric dispersants are, for example, BYK's DISPERBYK® 101, 115, 130, 140, 160, 161, 162, 163, 164, 166, 168, 169, 170, 171, 180, 182, 2000, 2001, 2009, 2020, 2025, 2050, 2070, 2090, 2091, 2095, 2096, 2150, 2163, 2164, BASFs EFKA® 4008, 4009, 4010, 4015, 4020, 4046, 4047, 4050, 4055, 4060, 4061, 4080, 4300, 4310, 4320, 4330, 4340, 4400, 4401, 4402, 4403, 4406, 4500, 4510, 4520, 4530, 4540, 4550, 4560, 4570, 4580, 4585, 4590, 4800, Ajinomoto Fine Techno's Ajisper PB®711, 821, 822, 823, 824, 827, 881, Lubrizol's SOLSPERSE® 1320, 13940, 17000, 20000, 21000, 24000, 26000, 27000, 28000, 31845, 32000, 32500, 32550, 32600, 33500, 34750, 36000, 36600, 37500, 38500, 39000, 41000, 41090, 44000 ,53095, Kawaken Fine Chemicals' Hinoact KF-1300M, T-6000, T-8000, T-8000E, T-9050, Kusumoto Chemicals' Disparlon PW-36, DA-325, DA-375, DA-7301, Kyoheisha Chemical's Flowlen DOPA-15B, DOPA-15BHFS, DOPA-17HF, DOPA-22, DOPA-33, G-600, G-700, G-820, G-900, NC-500, KDG-2400 and combinations thereof.
It is preferred to use EFKA® 4046, 4047, 4060, 4061, 4300, 4310, 4320, 4330, 4340, 4585, DISPERBYK® 161, 162, 163, 164, 165, 166, 168, 169, 170, 2000, 2001, 2020, 2050, 2090, 2091, 2095, 2096, 2105, 2150, 2163, 2164, PB®711, 821, 822, 823, 824, 827, 881, SOLSPERSE® 24000, 31845, 32500, 32550, 32600, 33500, 34750, 36000, 36600, 37500, 39000, 41090, 44000, 53095, Hinoact T-6000, T-8000E, Disparlon PW-36, DA-7301, and combinations thereof as dispersant.

Suitable texture improving agents are, for example, fatty acids such as stearic acid or behenic acid, and fatty amines such as laurylamine and stearylamine. In addition, fatty alcohles or ethoxylated fatty alcohles polyols such as aliphatic 1,2-diols or epoxidized soy bean oil, waxes, resin acids and resin acid salts may be used for this purpose.

Suitable pigment dispersants are, for example, copper phthalocyanine derivatives such as BASF's EFKA® 6745, Lubrizol's SOLSPERSE® 5000, 12000, BYK's SYNERGIST 2100 and azo derivatives such as EFKA® 6750, SOLSPERSE® 22000 and SYNERGIST 2105.
The above mentioned dispersants and surfactants for pigments are for example employed in compositions of the present invention which are used as resist formulations, in particular in color filter formulations.

Subject of the invention also is a photopolymerizable composition as described above as further additive (d) comprising a dispersant or a mixture of dispersants as well as a photopolymerizable composition as described above as further additive (d) comprising a pigment or a mixture of pigments.

The choice of additive(s) (d) is made depending on the field of application and on properties required for this field. The additives described above are customary in the art and accordingly are added in amounts which are usual in the respective application.

Binders (e) as well can be added to the novel compositions. This is particularly expedient when the photopolymerizable compounds are liquid or viscous substances. The quantity of binder may, for example, be 2-98 %, preferably 5-95 % and especially 20-90 %, by weight relative to the overall solids content. The choice of binder is made depending on the field of application and on properties required for this field, such as the capacity for development in aqueous and organic solvent systems, adhesion to substrates and sensitivity to oxygen.
Examples of suitable binders are polymers having a molecular weight of about 2'000 to 2'000'000, preferably 3'000 to 1'000'000. Examples of alkali developable binders are acrylic polymer having carboxylic acid function as a pendant group, such as conventionally known copolymers obtained by copolymerizing an ethylenic unsaturated carboxylic acid such as (meth)acrylic acid, 2-carboxyethyl (meth)acrylic acid, 2-carboxypropyl (meth)acrylic acid itaconic acid, crotonic acid, maleic acid, fumaric acid and ω-carboxypolycaprolactone mono(meth)acrylate, with one or more monomers selected from esters of (meth)acrylic acid, such as methyl (meth)acrylate, ethyl (meth)acrylate, propyl (meth)acrylate, butyl (meth)acrylate, benzyl (meth)acrylate, 2-ethylhexyl (meth)acrylate, hydroxyethyl (meth)acrylate, hydroxypropyl (meth)acrylate, glycerol mono(meth)acrylate, tricyclo[5.2.1.0^{2,6}]decan-8-yl (meth)acrylate, glycidyl (meth)acrylate, 2-methylglycidyl (meth)acrylate, 3,4-epoxybutyl (meth)acrylate, 6,7-epoxyheptyl (meth)acrylate; vinyl aromatic compounds, such as styrene, α-methylstyrene, vinyltoluene, p-chlorostyrene, vinylbenzyl glycidyl ether; amide type unsaturated compounds, (meth)acrylamide diacetone acrylamide, N-methylolacrylamide, N-butoxymethacrylamide; and polyolefin type compounds, such as butadiene, isoprene, chloroprene and the like; methacrylonitrile, methyl isopropenyl ketone, mono-2-[(meth)acryloyloxy]ethyl succinate, N-phenylmaleimide, maleic anhydride, vinyl acetate, vinyl propionate, vinyl pivalate, polystyrene macromonomer, or polymethyl (meth)acrylate macromonomer. Examples of copolymers are copolymers of acrylates and methacrylates with acrylic acid or methacrylic acid and with styrene or substituted styrene, phenolic resins, for example novolak, (poly)hydroxystyrene, and copolymers of hydroxystyrene with alkyl acrylates, acrylic acid and/or methacrylic acid. Preferable examples of copolymers are copolymers of methyl methacrylate/methacrylic acid, copolymers of benzyl methacrylate/methacrylic acid, copolymers of methyl methacrylate/ethyl acrylate/methacrylic acid, copolymers of benzyl methacrylate/methacrylic acid/styrene, copolymers of benzyl methacrylate/methacrylic acid/hydroxyethyl methacrylate, copolymers of methyl methacrylate/butyl methacrylate/ methacrylic acid/styrene, copolymers of methyl methacrylate/benzyl methacrylate/methacrylic acid/hydroxyphenyl methacrylate. Examples of solvent developable binder polymers are poly(alkyl methacrylates), poly(alkyl acrylates), poly(benzylmethacrylate-cohydroxyethylmethacrylate-co-methacrylic acid), poly(benzylmethacrylate-co-methacrylic acid); cellulose esters and cellulose ethers, such as cellulose acetate, cellulose acetobutyrate, methylcellulose, ethylcellulose; polyvinylbutyral, polyvinylformal, cyclized rubber, polyethers such as polyethylene oxide, polypropylene oxide and polytetrahydrofuran; polystyrene, polycarbonate, polyurethane, chlorinated polyolefins, polyvinyl chloride, vinyl chloride/vinylidene copolymers, copolymers of vinylidene chloride with acrylonitrile, methyl methacrylate and vinyl acetate, polyvinyl acetate, copoly(ethylene/vinyl acetate), polymers such as polycaprolactam and poly(hexamethylene adipamide), and polyesters such as poly(ethylene glycol terephtalate) and poly(hexamethylene glycol succinate) and polyimide binder resins.
The polyimide binder resin in the present invention can either be a solvent soluble polyimide or a polyimide precursor, for example, a poly(amic acid).

Preferred is a photopolymerizable composition, comprising as binder polymer (e), a copolymer of methacrylate and methacrylic acid.
Interesting further are polymeric binder components as described e.g. in JP 10-171119-A, in particular for use in color filters.
Subject of the invention further is a photopolymerizable composition as described above, wherein the component (a) is a resin obtained by the reaction of a saturated or unsaturated polybasic acid anhydride with a product of the reaction of an epoxy resin and an unsaturated monocarboxylic acid.

The photopolymerizable compositions can be used for various purposes, for example as printing ink, e.g. screen printing inks, inks for offset- or flexo printing, as a clear finish, as a white or colored finish, for example for wood or metal, as powder coating, as a coating material, inter alia for paper, wood, metal or plastic, as a daylight-curable coating for the marking of buildings and roadmarking, for photographic reproduction techniques, for holographic recording materials, for image recording techniques or to produce printing plates which can be developed with organic solvents or with aqueous alkalis, for producing masks for screen printing, as dental filling compositions, as adhesives, as pressure-sensitive adhesives, as laminating resins, as etch resists, solder resists, electroplating resists, or permanent resists, both liquid and dry films, as photostructurable dielectric, for printed circuit boards and electronic circuits, as resists to manufacture color filters for a variety of display applications or to generate structures in the manufacturing process of plasma-display panels and electroluminescence displays, (as for example described in US5853446, EP863534, JP 09-244230-A, JP10-62980-A, JP08-171863-A, US5840465, EP855731, JP05-271576-A, JP 05-67405-A) for the production of holographic data storage (HDS) material, for the production of optical switches, optical lattices (interference lattice), light circuits, for producing three-dimensional articles by mass curing (UV curing in transparent moulds) or by the stereolithography technique, as is described, for example, in US4575330, to produce composite materials (for example styrenic polyesters, which may, if desired, contain glass fibres and/or other fibres and other auxiliaries) and other thick-layered compositions, for coating or sealing electronic components and integrated circuits, or as coatings for optical fibres, or for producing optical lenses, e.g. contact lenses or Fresnel lenses. The compositions according to the invention are further suitable for the production of medical equipment, auxiliaries or implants. Further, the compositions according to the invention are suitable for the preparation of gels with thermotropic properties, as for example described in DE19700064 and EP678534.

The novel photoinitiators may additionally be employed as initiators for emulsion polymerizations, pearl polymerizations or suspension polymerizations, as polymerization initiators for fixing ordered states of liquid-crystalline monomers and oligomers, or as initiators for fixing dyes on organic materials.

In coating materials, use is frequently made of mixtures of a prepolymer with polyunsaturated monomers, which may additionally include a monounsaturated monomer as well. It is the prepolymer here which primarily dictates the properties of the coating film, and by varying it the skilled worker is able to influence the properties of the cured film. The polyunsaturated monomer functions as a crosslinking agent which renders the film insoluble. The monounsaturated monomer functions as a reactive diluent, which is used to reduce the viscosity without the need to employ a solvent.
Unsaturated polyester resins are usually used in two-component systems together with a monounsaturated monomer, preferably with styrene. For photoresists, specific one-component systems are often used, for example polymaleimides, polychalcones or polyimides, as described in DE 2308830.

The novel photoinitiators can also be used for the polymerization of radiation-curable powder coatings. The powder coatings can be based on solid resins and monomers containing reactive double bonds, for example maleates, vinyl ethers, acrylates, acrylamides and mixtures thereof. A free-radically UV-curable powder coating can be formulated by mixing unsaturated polyester resins with solid acrylamides (for example methyl methylacrylamidoglycolate) and a novel free-radical photoinitiator, such formulations being as described, for example, in the paper "Radiation Curing of Powder Coating", Conference Proceedings, Radtech Europe 1993 by M. Wittig and Th. Gohmann. The powder coatings can also contain binders, as are described, for example, in DE 4228514 and in EP 636669. Free-radically UV-curable powder coatings can also be formulated by mixing unsaturated polyester resins with solid acrylates, methacrylates or vinyl ethers and with a novel photoinitiator (or photoinitiator mixture). The powder coatings may also comprise binders as are described, for example, in DE 4228514 and in EP 636669. The UV-curable powder coatings may additionally comprise white or coloured pigments. For example, preferably rutiletitanium dioxide can be employed in concentrations of up to 50% by weight in order to give a cured powder coating of good hiding power. The procedure normally comprises electrostatic or tribostatic spraying of the powder onto the substrate, for example metal or wood, melting of the powder by heating, and, after a smooth film has formed, radiation-curing of the coating with ultraviolet and/or visible light, using for example medium-pressure mercury lamps, metal halide lamps or xenon lamps. A particular advantage of the radiation-curable powder coatings over their heat-curable counterparts is that the flow time after melting the powder particles can be delayed in order to ensure the formation of a smooth, high-gloss coating. In contrast to heat-curable systems, radiation-curable powder coatings can be formulated to melt at lower temperatures without the unwanted effect of shortening their lifetime. For this reason, they are also suitable as coatings for heat-sensitive substrates, for example wood or plastics. In addition to the novel photoinitiator systems, the powder coating formulations may also include UV absorbers. Appropriate examples are listed above in sections 1.-8.

The novel photocurable compositions are suitable, for example, as coating materials for substrates of all kinds, for example wood, textiles, paper, ceramics, glass, plastics such as polyesters, polyethylene terephthalate, polyolefins or cellulose acetate, especially in the form of films, and also metals such as Al, Cu, Ni, Fe, Zn, Mg or Co and GaAs, Si or SiO₂ to which it is intended to apply a protective layer or, by means of imagewise exposure, to generate an image.

The novel radiation-sensitive compositions further find application as negative resists, having a very high sensitivity to light and being able to be developed in an aqueous alkaline medium without swelling. They are suitable for the production of printing forms for relief printing, planographic printing, photogravure or of screen printing forms, for the production of relief copies, for example for the production of texts in braille, for the production of stamps, for use in chemical milling or as a microresist in the production of integrated circuits. The compositions further may be used as photopatternable dielectric layer or coating, encapsulating material and isolating coating in the production of computer chips, printed boards and other electric or electronic components. The possible layer supports, and the processing conditions of the coating substrates, are just as varied.

The novel composition also relates to a photosensitive thermosetting resin composition and a method of forming a solder resist pattern by the use thereof, and more particularly relates to a novel photosensitive thermosetting resin composition useful as materials for the production of printed circuit boards, the precision fabrication of metallic articles, the etching of glass and stone articles, the relief of plastic articles, and the preparation of printing plates and particularly useful as a solder resist for printed circuit boards and to a method of forming a solder resist pattern by the steps of exposing a layer of the resin composition selectively to an actinic ray through a photomask having a pattern and developing the unexposed part of the layer.

The solder resist is a substance which is used during the soldering of a given part to a printed circuit board for the purpose of preventing molten solder from adhering to irrelevant portions and protecting circuits. It is, therefore, required to possess such properties as high adhesion, insulation resistance, resistance to soldering temperature, resistance to solvents, resistance to alkalis, resistance to acids, and resistance to plating.

Because the photocurable compositions according to the invention have a good thermal stability and are sufficiently resistant to inhibition by oxygen, they are particularly suitable for the production of color filters or color mosaic systems, such as described, for example, in EP 320 264. Color filters usually are employed in the manufacturing of flat panel displays such as LCD's, PDP(plasma panel display), EL (electroluminessence) display, and projection systems, image sensors, CCD (charge coupled device), and CMOS (complementary metal oxide semiconductor) sensors for scanner, digital camera and video camera.

The color filters usually are prepared by forming red, green and blue pixels and a black matrix on a glass substrate. In these processes photocurable compositions according to the invention can be employed. A particularly preferred method of use comprises adding of the coloring matters, dyes and pigments of red, green and blue colors to the light-sensitive resin composition of the present invention, coating of the substrate with the composition, drying of the coating with a short heat treatment, patternwise exposure of the coating to actinic radiation and subsequent development of the pattern in an aqueous alkaline developer solution and optionally a heat treatment. Thus, by subsequently applying a red, green and blue pigmented coating, in any desired order, on top of each other with this process a color filter layer with red, green and blue color pixels can be produced.

The development is carried out by washing out the areas which were not polymerized with a suitable alkali developing solution. This process is repeated to form the image having plural colors.

In the light-sensitive resin composition of the present invention, with a process in which at least one or more picture elements are formed on a transparent substrate and then an exposure is given from a side of the transparent substrate, on which the above picture elements are not formed, the above picture elements can be utilized as a light-shielding mask. In this case, for example, in the case where an overall exposure is given, a position adjustment of a mask gets unnecessary and a concern on a position slippage thereof is removed. And, it is possible to cure all of the part on which the above picture elements are not formed. Further, in this case, it is possible as well to develop and remove a part of the portion on which the above picture elements are not formed by using partially a light-shielding mask.

Since in either case, no gap is formed between the picture elements which are formed formerly and those which are formed later, the composition of the present invention is suitable for, for example, a forming material for a color filter. To be concrete, the coloring matters, dyes and pigments of red, green and blue colors are added to the light-sensitive resin composition of the present invention, and the processes for forming an image are repeated to form the picture elements of red, green and blue colors. Then, the light-sensitive resin composition to which, for example, the black coloring materials, dyes and pigments are added is provided on an overall face. An overall exposure (or a partial exposure via a light-shielding mask) can be provided thereon to form the picture elements of a black color all over the spaces (or all but a partial region of the light-shielding mask) between the picture elements of red, green and blue colors.

In addition to a process in which the light-sensitive resin composition is coated on a substrate and dried, the light-sensitive resin composition of the present invention can be used as well for a layer transfer material. That is, the light-sensitive resin composition is layer-wise provided directly on a temporary support, preferably on a polyethylene terephthalate film, or on a polyethylene terephthalate film on which an oxygen-shielding layer and a peeling layer or the peeling layer and the oxygen-shielding layer are provided. Usually, a removable cover sheet made of a synthetic resin is laminated thereon for a protection in handling. Further, there can be applied as well a layer structure in which an alkali soluble thermoplastic resin layer and an intermediate layer are provided on a temporary support and further a light-sensitive resin composition layer is provided thereon (JP 5-173320-A).

The above cover sheet is removed in use and the light-sensitive resin composition layer is laminated on a permanent support. Subsequently, peeling is carried out between those layer and a temporary support when an oxygen-shielding layer and a peeling layer are provided, between the peeling layer and the oxygen-shielding layer when the peeling layer and the oxygen-shielding layer are provided, and between the temporary support and the light-sensitive resin composition layer when either the peeling layer or the oxygen-shielding layer is not provided, and the temporary support is removed.

A metal support, glass, ceramics, and a synthetic resin film can be used as a support for a color filter. Glass and a synthetic resin film which is transparent and have an excellent dimension stability is particularly preferred.

The thickness of the light-sensitive resin composition layer is usually 0.1 to 50 micrometers, in particular 0.5 to 5 micrometers.

A diluted aqueous solution of an alkaline substance can be used as a developing solution for the light-sensitive resin composition of the present invention if the composition contains alkali soluble resin or alkali soluble monomers or oligomers, and further a developer solution prepared by adding a small amount of a water-miscible organic solvent thereto is included as well.

Examples of suitable alkaline materials include alkali metal hydroxides (for example, sodium hydroxide and potassium hydroxide), alkali metal carbonates (for example, sodium carbonate and potassium carbonate), alkali metal bicarbonates (for example, sodium bicarbonate and potassium bicarbonate), alkali metal silicates (for example, sodium silicate and potassium silicate), alkali metal metasilicates (for example, sodium metasilicate and potassium metasilicate), triethanolamine, diethanolamine, monoethanolamine, morpholine, tetraalkylammonium hydroxides (for example, tetramethylammonium hydroxide), or trisodium phosphate. The concetration of the alkaline substance is 0.01 to 30 weight %, and pH is preferably 8 to 14.

Suitable organic solvents which are miscible with water include methanol, ethanol, 2-propanol, 1-propanol, butanol, diacetone alcohol, ethylene glycol monomethyl ether, ethylene glycol monoethyl ether, ethylene glycol mono-n-butyl ether, diethyleneglycol dimethyl ether, propyleneglycol monomethyl ether acetate, ethyl-3-ethoxypropionate, methyl-3-methoxypropionate, n-butyl acetate, benzyl alcohol, acetone, methyl ethyl ketone, cyclopentanone, cyclohexanone, 2-heptanone, 2-pentanone, epsilon-caprolactone, gamma-butylolactone, dimethylformamide, dimethylacetoamide, hexamethylphosphoramide, ethyl lactate, methyl lactate, epsilon-caprolactam, and N-methyl-pyrrolidinone. The concentration of the organic solvent which is miscible with water is 0.1 to 30 weight %.

Further, a publicly known surface active agent can be added. The concentration of the surface active agent is preferably 0.001 to 10 weight %.

The light sensitive resin composition of the present invention can also be developed with organic solvents, including blends of two or more solvents, not containing alkaline compounds. Suitable solvents include methanol, ethanol, 2-propanol, 1-propanol, butanol, diacetone alcohol, ethylene glycol monomethyl ether, ethylene glycol monoethyl ether, ethylene glycol mono-n-butyl ether, diethyleneglycol dimethyl ether, propyleneglycol monomethyl ether acetate, ethyl-3-ethoxypropionate, methyl-3-methoxypropionate, n-butyl acetate, benzyl alcohol, acetone, methyl ethyl ketone, cyclopentanone, cyclohexanone, 2-heptanone, 2-pentanone, epsilon-caprolactone, gamma-butylolactone, dimethylformamide, dimethylacetamide, hexamethylphosphoramide, ethyl lactate, methyl lactate, epsilon-caprolactam, and N-methylpyrrolidinone. Optionally, water can be added to these solvents up to a level at which still a clear solution is obtained and at which sufficient solubility of the unexposed areas of the light sensitive composition is maintained.

The developer solution can be used in all forms known to the person skilled in the art, for example in form of a bath solution, puddle, or a spraying solution. In order to remove the non-cured portion of the light-sensitive resin composition layer, there can be combined the methods such as rubbing with a rotary brush and rubbing with a wet sponge. Usually, the temperature of the developing solution is preferably at and around room temperature to 40°C. The developing time is changeable according to the specific kind of the light-sensitive resin composition, the alkalinity and temperature of the developing solution, and the kind and concentration of the organic solvent in the case where it is added. Usually, it is 10 seconds to 2 minutes. It is possible to put a rinsing step after the development processing.

A final heat treatment is preferably carried out after the development processing. Accordingly, a support having a layer which is photopolymerized by exposing (hereinafter referred to as a photocured layer) is heated in an electric furnace and a drier, or the photocured layer is irradiated with an infrared lamp or heated on a hot plate. The heating temperature and time depend on the composition used and the thickness of the formed layer. In general, heating is preferably applied at about 120°C to about 250°C, for about 5 to about 60 minutes.

The pigment which can be comprised in the composition according to the present invention, including a pigmented color filter resist composition, is preferably a processed pigment, for example a powdery or pasty product prepared by finely dispersing a pigment into at least one resin selected from the group consisting of acrylic resin, vinyl chloride-vinyl acetate copolymer, maleic acid resin and ethyl cellulose resin.
The red pigment comprises, for example, an anthraquinone type pigment alone, a diketopyrolopyrole type pigment alone, a mixture of them or a mixture consisting of at least one of them and a disazo type yellow pigment or an isoindoline type yellow pigment, in particular C. I. Pigment Red 177 alone, C. I. Pigment Red 254 alone, a mixture of C. I. Pigment Red 177 and C. I. Pigment Red 254 or a mixture consisting of at least one member of C. I. Pigment Red 177 and C. I. Pigment Red 254, and C. I. Pigment Yellow 83 or C. I. Pigment Yellow 139 ("C.I." refers to the Color Index, known to the person skilled in the art and publicly available).
Further suitable examples for the pigment are C.I. Pigment Red 9, 97, 105, 122, 123, 144, 149, 168, 176, 179, 180, 185, 202, 207, 209, 214, 222, 242, 244, 255, 264, 272 and C.I. Pigment Yellow 12, 13, 14, 17, 20, 24, 31, 53, 55, 93, 95, 109, 110, 128, 129, 138, 139, 150, 153, 154,155, 166, 168, 185, 199, 213 and C.I. Pigment Orange 43. Examples of the dyes for red color are C. I. Solvent Red 25, 27, 30, 35, 49, 83, 89, 100, 122, 138, 149, 150, 160, 179, 218, 230, C. I. Direct Red 20, 37, 39, 44, and C. I. Acid Red 6, 8, 9, 13, 14, 18, 26, 27, 51, 52, 87, 88, 89, 92, 94, 97, 111, 114, 115, 134, 145, 151, 154, 180, 183, 184, 186, 198, C. I. Basic Red 12, 13, C. I. Disperse Red 5, 7, 13, 17 and 58. The Red dyes can be used in combination with yellow and/or orange dyes. The green pigment comprises for instance a halogenated phthalocyanine type pigment alone or its mixture with a disazo type yellow pigment, an quinophthalone type yellow pigment or a metal complex, in particular C. I. Pigment Green 7 alone, C. I. Pigment Green 36 alone, or a mixture consisting of at least one member of C. I. Pigment Green 7, C. I. Pigment Green 36 and C. I. Pigment Yellow 83, C. I. Pigment Yellow 138 or C. I. Pigment Yellow 150. Other suitable green pigments are C.I. Pigment Green 15, 25 and 37.
Examples for suitable green dyes are C. I. Acid Green 3, 9, 16, C. I. Basic Green 1 and 4.
Examples for suitable blue pigments are phthalocyanine type pigments, used either alone or in combination with an dioxazine type violet pigment, for instance, C. I. Pigment Blue 15:6 alone, a combination of C. I. Pigment Blue 15:6 and C. I. Pigment Violet 23. Further examples for blue pigments are such of C. I. Pigment Blue 15:3, 15:4, 16, 22, 28 and 60. Other suitable pigments are C. I. Pigment Violet 14,19, 23, 29, 32, 37, 177 and C. I. Orange 73.
Examples for suitable blue dyes are C. I. Solvent Blue 25, 49, 68, 78, 94, C. I. Direct Blue 25, 86, 90, 108, C. I. Acid Blue 1, 7, 9, 15, 103, 104, 158, 161, C. I. Basic Blue 1, 3, 9, 25, and C. I. Disperse Blue 198.
The pigment of the photopolymeric composition for black matrix preferably comprises at least one member selected from the group consisting of carbon black, titanium black and iron oxide. However, a mixture of other pigments which, in total, give the black appearance, can also be used. For example, also C. I. Pigment Black 1, 7 and 31 can be used alone or in combination.
Other examples of the dyes used for color filter are C. I. Solvent Yellow 2, 5, 14, 15, 16, 19, 21, 33, 56, 62, 77, 83, 93, 162, 104, 105, 114, 129, 130, 162, C. I. Disperse Yellow 3, 4, 7, 31, 54, 61, 201, C. I. Direct Yellow 1, 11, 12, 28, C. I. Acid Yellow 1, 3, 11, 17, 23, 38, 40, 42, 76, 98, C. I. Basic Yellow 1, C. I. Solvent Violet 13, 33, 45, 46, C. I. Disperse Violet 22, 24, 26, 28, C. I. Acid Violet 49, C. I. Basic Violet 2, 7, 10, C. I. Solvent Orange 1, 2, 5, 6, 37, 45, 62, 99, C. I. Acid Orange 1, 7, 8, 10, 20, 24, 28, 33, 56, 74, C. I. Direct Orange 1, C. I. Disperse Orange 5, C. I. Direct Brown 6, 58, 95, 101, 173, C. I. Acid Brown 14, C. I. Solvent Black 3, 5, 7, 27, 28, 29, 35, 45 and 46.

In some special cases of manufacturing color filters, complementary colors, yellow, magenta, cyan and optionally green, are used instead of red, green and blue. As yellow for this type of color filters, the abovementioned yellow pigments and dyes can be employed. Examples of the colorants suitable for magenta color are C. I. Pigment Red 122, 144, 146, 169, 177, C. I. Pigment Violet 19 and 23. Examples of cyan color are aluminum phthalocyanine pigments, titanium phthalocyanine pigments, cobalt phthalocyanine pigments, and tin phthalocyanine pigments.

For any color, combinations of more than two pigments can also be used. Especially suitable in color filter applications are powdery processed pigments prepared by finely dispersing the above mentioned pigments into a resin.

The concentration of the pigment in the total solid component (pigments of various colors and resin) is for example in the range of 5% to 80% by weight, in particular in the range of 20% to 45% by weight.

The pigments in the color filter resist composition have preferably a mean particle diameter smaller than the wavelength of visible light (400 nm to 700 nm). Particularly preferred is a mean pigment diameter of < 100 nm.

If necessary, the pigments may be stabilized in the photosensitive composition by pretreatment of the pigments with a dispersant to improve the dispersion stability of the pigment in the liquid formulation. Suitable additives are described above.

Preferably, the color filter resist composition according to the present invention contains additionally at least one addition polymerizable monomeric compound as component (a).
The ethylenically unsaturated compounds (a), also for the color filter resist applications are as described above
Suitable examples of esters as component (a) based on polyols are trimethylolpropane tri(meth)acrylate, trimethylolpropane tri(acryloyloxypropyl)ether, trimethylolethane tri(meth)acrylate, ethylene glycol di(meth)acrylate, diethylene glycol di(meth)acrylate, triethylene glycol di(meth)acrylate, tetraethylene glycol di(meth)acrylate, tetramethylene glycol di(meth)acrylate, neopentyl glycol di(meth)acrylate, pentaerythritol di(meth)-acrylate, pentaerythritol tri(meth)acrylate, pentaerythritol tetra(meth)acrylate, pentaerythritol tri(meth)acrylate monooxalate, dipentaerythritol di(meth)acrylate, dipentaerythritol tri(meth)acrylate, dipentaerythritol tetra(meth)acrylate, dipentaerythritol penta(meth)acrylate, dipentaerythritol hexa(meth)acrylate, dipentaerythritol penta-(meth)acrylate mono(2-hydroxyethyl) ether, tripentaerythritol octa(meth)acrylate, 1,3-butanediol di(meth)acrylate, 1,4-butanediol diitaconate, hexanediol di(meth)acrylate, 1,4-cyclohexanediol di(meth)acrylate, sorbitol tri(meth)acrylate, sorbitol tetra-(meth)acrylate, sorbitol penta(meth)acrylate, sorbitol hexa(meth)acrylate, oligoester (meth)acrylates, glycerol di(meth)acrylate and tri(meth)acrylate, di(meth)acrylates of polyethylene glycol with a molecular weight of from 200 to 1500, pentaerythritol diitaconate, dipentaerythritol trisitaconate, dipentaerythritol pentaitaconate, dipentaerythritol hexaitaconate, ethylene glycol diitaconate, propylene glycol diitaconate, 1,3-butanediol diitaconate, 1,4- butanediol diitaconate, tetramethylene glycol diitaconate, sorbitol tetraitaconate, ethylene glycol dicrotonate, tetramethylene glycol dicrotonate, pentaerythritol dicrotonate, ethylene glycol dimaleate, tiethylene glycol dimaleate, pentaerythritol dimaleate, sorbitol tetramaleate, or mixtures thereof.

Other examples are pentaerythritol and dipentaerythritol derivatives shown in the following formula (XII) and (XIII): 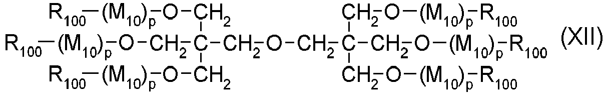 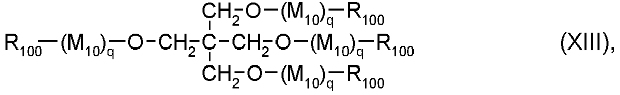 wherein
M₁₀ is -(CH₂CH₂O)- or-[CH₂CH(CH₃)O]-,
R₁₀₀ is -COCH=CH₂ or -COC(CH₃)=CH₂,
p is 0 to 6 (total of p: 3 - 24), and q is 0 to 6 (total of q: 2 - 16).

Examples of polyepoxides are those based on the abovementioned polyols and epichlorohydrin. Typical examples are bis(4-glycidyloxyphenyl)methane, 2,2-bis(4-glycidyl-oxyphenyl)propane, 2,2-bis(4-glycidyloxyphenyl)hexafluoropropane, 9,9-bis(4-glycidyl-oxyphenyl)fluorene, bis[4-(2-glycidyloxyethoxy)phenyl]methane, 2,2-bis[4-(2-glycidyl-oxyethoxy)phenyl]propane, 2,2-bis[4-(2-glycidyloxyethoxy)phenyl]hexafluoropropane, 9,9-bis[4-(2-glycidyloxyethoxy)phenyl]fluorene, bis[4-(2-glycidyloxypropoxy)phenyl]methane, 2,2-bis[4-(2-glycidyloxypropoxy)phenyl]propane, 2,2-bis[4-(2-glycidyloxypropoxy)phenyl]hexafluoropropane, 9,9-bis[4-(2-glycidyloxypropoxy)phenyl]fluorene, and glycidyl ethers of phenol and cresol novolacs.
Typical examples of component (a) based on polyepoxides are 2,2-bis[4-{(2-hydroxy-3-acryloxy)propoxy}phenyl]propane, 2,2-bis[4-{(2-hydroxy-3-acryloxy)propoxyethoxy}phenyl]propane, 9,9-bis[4-{(2-hydroxy-3-acryloxy)propoxy}phenyl]fluorene, 9,9-bis[4-{(2-hydroxy-3-acryloxy)propoxyethoxy}phenyl]fluorine, and reaction products of epoxy resins based on novolacs with (meth)acrylic acid.
Polyethers obtained from the reaction of the abovementioned polyols or polyepoxides with the unsaturated counpounds with a hydroxy group such as 2-hydroxyethyl (meth)acrylate, vinyl alcohol can also be used as component (a).
Other examples for suitable compoents (a) are unsaturated urethanes derived from a polyisocyanate and an unsaturated compound having a hydroxy group or from a polyisocyanate, a polyol and an unsaturated compound having a hydroxy group.
Other examples are polyesters, polyamides, or polyurethanes having ethylenically unsaturated groups in the chain. Suitable unsaturated polyesters and polyamides are also derived, for example, from maleic acid and diols or diamines. Some of the maleic acid can be replaced by other dicarboxylic acids. The polyesters and polyamides may also be derived from dicarboxylic acids and ethylenically unsaturated diols or diamines, especially from those with relatively long chains of, for example 6 to 20 C atoms. Examples of polyurethanes are those composed of saturated or unsaturated diisocyanates and of unsaturated or, respectively, saturated diols.
Other suitable polymers with acrylate or methacrylate groups in the side chains are, for example, solvent soluble or alkaline soluble polyimide precursors, for example poly(amic acid ester) compounds, having the photopolymerizable side groups either attached to the backbone or to the ester groups in the molecule, i.e. according to EP 624826. Such oligomers or polymers can be formulated optionally with reactive diluents, like polyfunctional (meth)acrylates in order to prepare highly sensitive polyimide precursor resists.
Further examples of the component (a) are also polymers or oligomers having at least one carboxyl function and at least two ethylenically unsaturated groups within the molecular structure, such as a resin obtained by the reaction of a saturated or unsaturated polybasic acid anhydride with a product of the reaction of phenol or cresol novolac epoxy resin and an unsaturated monocarboxylic acid, for example, commercial products such as EB9696, UCB Chemicals; KAYARAD TCR1025, Nippon Kayaku Co.,LTD. Examples of the polybasic acid anhydride are maleic anhydride, succinic anhydride, itaconic anhydride, phthalic anhydride, tetrahydrophthalic anhydride, hexahydrophthalic anhydride, methyltetrahydrophathalic anhydride, glutaric anhydride, glutaconic anhydride, citraconic anhydride, diglycolic anhydride, iminodiacetic anhydride, 1,1-cyclopentanediacetic anhydride, 3,3-dimethylglutaric anhydride, 3-ethyl-3-methylglutaric anhydride, 2-phenylglutaric anhydride, homophthalic anhydride, trimellitic anhydride, chlorendic anhydride, pyromellitic dianhydride, benzophenone tetracarboxylic acid dianhydride, biphenyl tetracarboxylic acid dianhydride, and biphenylether tetracarboxylic acid dianhydride.
Other examples are the products from the polycondensation reaction and/or addition reaction of the compound of formula (XIV) with one or more abovementioned polybasic acid anhydrides. 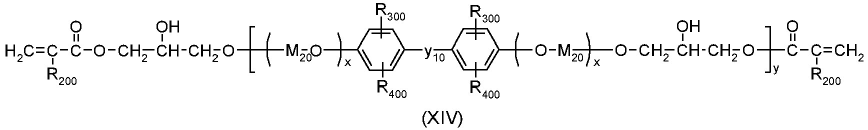 wherein Y₁₀ is 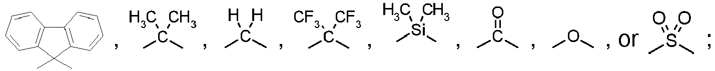 R₂₀₀ is hydrogen or methyl,
R₃₀₀ and R₄₀₀ independently of each other are hydrogen, methyl, Cl, or Br, M₂₀ is substituted or unsubstituted alkylene having 1 to 10 carbon atoms, x is 0 to 5, and y is 1 to 10. Examples of such compounds as component (a) are described in JP2002-206014A, JP2004-69754A, JP2004-302245A, JP2005-77451A, JP2005-316449A, JP2005-338328A and JP3754065B2.
Polymers or oligomers as abovementioned have for example a molecular weight of about 1'000 to 1'000'000, preferably 2'000 to 200'000 and an acid value of about 10 to 200 mg KOH/g, preferably 20 to 180 mg KOH/g.
A preferred photopolymerizable composition comprises as component (a) a compound having at least two ethylenically unsaturated bonds and at least one carboxylic acid group in the molecule, in particular a reaction product obtained by adding an epoxy group containing unsaturated compound to a part of the carboxyl groups of a carboxylic acid group containing polymer or a reaction product of the compound shown below with one or more polybasic acid anhydrides. Further preferred components (a) comprise a compound obtained from the reaction of a compound of the formula XIV with one or more polybasic acid anhydrides.
Further examples are reaction products obtained by adding an epoxy group containing unsaturated compound to a part of the carboxyl groups of a carboxylic acid group containing polymer. As the carboxylic acid containing polymer, the abovementioned binder polymers which are resulting from the reaction of an unsaturated carboxylic acid compound with one or more polymerizable compounds, for example, copolymers of (meth)acrylic acid, benzyl (meth)acrylate, styrene and 2-hydroxyethyl (meth)acrylate, copolymers of (meth)acrylic acid, styrene and α-methystyrene, copolymers of (meth)acrylic acid, N-phenylmaleimide, styrene and benzyl (meth)acrylate, copolymers of (meth)acrylic acid and styrene, copolymers of (meth)acrylic acid and benzyl (meth)acrylate, copolymers of tetrahydrofurfuryl (meth)acrylate, styrene and (meth)acrylic acid, and the like.
Examples of the unsaturated compounds having an epoxy group are given below in the formula (V-1) - (V-15); 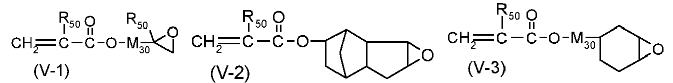 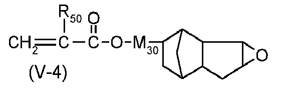 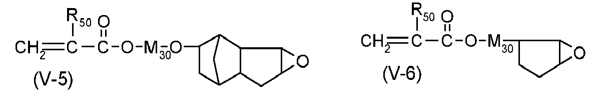 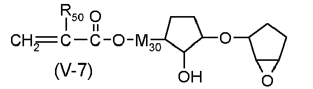 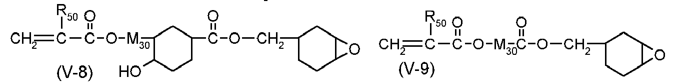 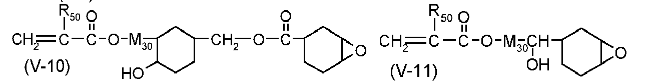 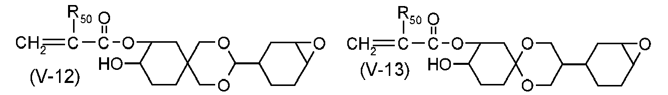 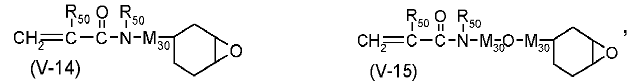 wherein R₅₀ is hydrogen or methyl group, M₃₀ is substituted or unsubstituted alkylene having 1 to 10 carbon atoms.

Among these compounds, compounds having alicyclic epoxy groups are particularly preferred, because these compounds have a high reactivity with carboxyl group-containing resins, accordingly the reaction time can be shortened. These compounds further do not cause gelation in the process of reaction and make it possible to carry out the reaction stably. On the other hand, glycidyl acrylate and glycidyl methacrylate are advantageous from the viewpoint of sensitivity and heat resistance because they have a low molecular weight and can give a high conversion of esterification. Concrete examples of the abovementioned compounds are, for example a reaction product of a copolymer of styrene, α-methyl styrene and acrylic acid or a copolymer of methyl methacrylate and acrylic acid with 3,4-epoxycyclohexylmethyl (meth)acrylate.

Unsaturated compounds having a hydroxy group such as 2-hydroxyethyl (meth)-acrylate and glycerol mono(meth)acrylate can be used instead of the above mentioned epoxy group containing unsaturated compounds as the reactant for carboxylic acid group containing polymers.
Other examples are half esters of anhydride containing polymers, for example reaction products of a copolymer of maleic anhydride and one or more other polymerizable compounds with (meth)acrylates having an alcoholic hydroxy group such as 2-hydroxyethyl (meth)acrylate or having an epoxy group for example such as the compounds described in the formula (V-1) - (V-15).
Reaction products of polymers having alcoholic hydroxy groups such as copolymers of 2-hydroxyethyl (meth)acrylate, (meth)acrylic acid, benzy methacylate and styrene, with (meth)acrylic acid or (meth)acryl chloride can also be used as component (a).
Other examples are reaction products of a polyester with terminal unsaturated groups, which is obtained from the reaction of a dibasic acid anhydride and a compound having at least two epoxy groups followed by further reaction with an unsaturated compound, with a polybasic acid anhydride.
Further examples are resins obtained by the reaction of a saturated or unsaturated polybasic acid anhydride with a reaction product obtained by adding epoxy group containing (meth)acrylic compound to all of the carboxyl groups of a carboxylic acid containing polymer as mentioned above.

The photopolymerizable compounds can be used alone or in any desired mixtures.

In a color filter resist composition the whole amount of the monomers contained in the photopolymerizable composition is preferably 5 to 80 % by weight, in particular 10 to 70 % by weight based on the whole solid contents of the composition, i.e. the amount of all components without the solvent(s).

As the binder used in the color filter resist composition, which is soluble in an alkaline aqueous solution and insoluble in water, for example, a homopolymer of a polymerizable compound having one or more acid groups and one or more polymerizable unsaturated bonds in the molecule, or a copolymer of two or more kinds thereof, and a copolymer of one or more polymerizable compounds having one or more unsaturated bonds copolymerizable with these compounds and containing no acid group, can be used. Such compounds can be obtained by copolymerizing one or more kinds of a low molecular compound having one or more acid groups and one or more polymerizable unsaturated bonds in the molecule with one or more polymerizable compounds having one or more unsaturated bonds copolymerizable with these compounds and containing no acid group. Examples of acids groups are, a -COOH group, a -SO₃H group, a -SO₂NHCO- group, a phenolic hydroxy group, a -SO₂NH- group, and a -CO-NH-CO-group. Among those, a high molecular compound having a -COOH group is particularly preferred.

Preferably, the organic polymer binder in the color filter resist composition comprises an alkali soluble copolymer comprising, as addition polymerizable monomer units, at least an unsaturated organic acid compound such as acrylic acid, methacrylic acid and the like. It is preferred to use as a further co-monomer for the polymer binder an unsaturated organic acid ester compound such as methyl acrylate, ethyl (meth)acrylate, benzyl (meth)acrylate, styrene and the like to balance properties such as alkaline solubility, adhesion rigidity, chemical resistance etc..
The organic polymer binder can either be a random co-polymer or a block-co-polymer, for example, such as described in US 5368976.

Examples of the polymerizable compounds having one or more -COOH groups and one or more polymerizable unsaturated bonds in a molecule are (meth)acrylic acid, 2-carboxyethyl (meth)acrylic acid, 2-carboxypropyl (meth)acrylic acid, crotonic acid, cinnamic acid, mono[2-(meth)acryloyloxyethyl] succinate, mono[2-(meth)acryloyloxyethyl] adipate, mono[2-(meth)acryloyloxyethyl] phthalate, mono[2-(meth)acryloyloxyethyl] hexahydrophthalate, mono[2-(meth)acryloyloxyethyl] maleate, mono[2-(meth)acryloyl-oxypropyl] succinate, mono[2-(meth)acryloyloxypropyl] adipate, mono[2-(meth)acryloyl-oxypropyl] phthalate, mono[2-(meth)acryloyloxypropyl] hexahydrophthalate, mono[2-(meth)acryloyloxypropyl] maleate, mono[2-(meth)acryloyloxybutyl] succinate, mono[2-(meth)acryloyloxybutyl] adipate, mono[2-(meth)acryloyloxybutyl] phthalate, mono[2-(meth)acryloyloxybutyl] hexahydrophthalate, mono[2-(meth)acryloyloxybutyl] maleate, 3-(alkylcarbamoyl)acrylic acid, α-chloroacrylic acid, maleic acid, monoesterified maleic acid, fumaric acid, itaconic acid, citraconic acid, mesaconic acid, maleic anhydride, and ω-carboxypolycaprolactone mono(meth)acrylate.
Vinylbenzenesulfonic acid and 2-(meth)acrylamide-2-methylpropanesulfonic acid are examples of the polymerizable compounds having one or more -SO₃H groups and one or more polymerizable unsaturated bonds.
N-methylsulfonyl (meth)acrylamide, N-ethylsulfonyl (meth)acrylamide, N-phenylsulfonyl (meth)acrylamide, and N-(p-methylphenylsulfonyl) (meth)acrylamide are examples of the polymerizable compounds having one or more -SO₂NHCO- groups and one or more polymerizable unsaturated bonds.
Examples of polymerizable compounds having one or more phenolic hydroxy groups and one or more polymerizable unsaturated bonds in a molecule include hydroxyphenyl (meth)acrylamide, dihydroxyphenyl (meth)acrylamide, hydroxyphenyl-carbonyl-oxyethyl (meth)acrylate, hydroxyphenyloxyethyl (meth)acrylate, hydroxyphenylthioethyl (meth)acrylate, dihydroxyphenylcarbonyloxyethyl (meth)acrylate, dihydroxyphenyloxy-ethyl (meth)acrylate, and dihydroxy-phenylthioethyl (meth)acrylate.

Examples of the polymerizable compounds having one or more -SO₂NH- groups and one or more polymerizable unsaturated bonds in the molecule include compounds represented by formula (a) or (b):

CH₂= CHA₁₀₀-Y₁₀₀-A₂₀₀-SO₂-NH-A₃ (a)

CH₂ = CHA₄₀₀-Y₂₀₀-A₅₀₀-NH-SO₂-A₆₀₀ (b)

wherein Y₁₀₀ and Y₂₀₀ each represents -COO-, -CONA₇₀₀-, or a single bond; A₁₀₀ and A₄₀₀ each represents H or CH₃; A₂₀₀ and A₅₀₀ each represents C₁-C₁₂alkylene optionally having a substituent, cycloalkylene, arylene, or aralkylene, or C₂-C₁₂alkylene into which an ether group and a thioether group are inserted, cycloalkylene, arylene, or aralkylene; A₃₀₀ and A₆₀₀ each represents H, C₁-C₁₂alkyl optionally having a substituent, a cycloalkyl group, an aryl group, or an aralkyl group; and A₇₀₀ represents H, C₁-C₁₂alkyl optionally having a substituent, a cycloalkyl group, an aryl group, or an aralkyl group.

The polymerizable compounds having one or more -CO-NH-CO- group and one or more polymerizable unsaturated bond include maleimide and N-acryloyl-acrylamide. These polymerizable compounds become the high molecular compounds comprising a -CO-NH-CO- group, in which a ring is formed together with a primary chain by polymerization. Further, a methacrylic acid derivative and an acrylic acid derivative each having a -CO-NH-CO- group can be used as well. Such methacrylic acid derivatives and the acrylic acid derivatives include, for example, a methacrylamide derivative such as N-acetylmethacrylamide, N-propionylmethacrylamide, N-butanoylmethacrylamide, N-pentanoylmethacrylamide, N-decanoylmethacrylamide, N-dodecanoylmethacrylamide, N-benzoylmethacrylamide, N-(p-methylbenzoyl)methacryl-amide, N-(p-chlorobenzoyl)methacrylamide, N-(naphthyl-carbonyl)methacrylamide, N-(phenylacetyl)-methacrylamide, and 4-methacryloylaminophthalimide, and an acrylamide derivative having the same substituent as these. These polymerizable compounds polymerize to be compounds having a -CO-NH-CO- group in a side chain.

Examples of polymerizable compounds having one or more polymerizable unsaturated bond and containing no acid group include a compound having a polymerizable unsaturated bond, selected from esters of (meth)acrylic acid, such as methyl (meth)acrylate, ethyl (meth)acrylate, propyl (meth)acrylate, butyl (meth)acrylate, tetrahydrofurfuryl (meth)acrylate, benzyl (meth)acrylate, 2-ethylhexyl (meth)acrylate, hydroxyethyl (meth)acrylate, hydroxypropyl (meth)acrylate, hydroxybutyl (meth)acrylate, glycerol mono(meth)acrylate, dihydroxypropyl (meth)acrylate, allyl (meth)acrylate, cyclohexyl (meth)acrylate, phenyl (meth)acrylate, methoxyphenyl (meth)acrylate, methoxyethyl (meth)acrylate, phenoxyethyl (meth)acrylate, methoxydiethyleneglycol (meth)acrylate, methoxytriethyleneglycol (meth)acrylate, methoxypropyl (meth)acrylate, methoxydipropyleneglycol (meth)acrylate, isobornyl meth(acrylate), dicyclopentadienyl (meth)acrylate, 2-hydroxy-3-phenoxypropyl (meth)acrylate, tricyclo[5.2.1.0^{2,6}]decan-8-yl (meth)acrylate, aminoethyl (meth)acrylate, N, N-dimethylaminoethyl (meth)acrylate, aminopropyl (meth)acrylate, N, N-dimethylaminopropyl (meth)acrylate, glycidyl (meth)-acrylate, 2-methylglycidyl (meth)acrylate, 3,4-epoxybutyl (meth)acrylate, 6,7-epoxy-heptyl (meth)acrylate; vinyl aromatic compounds, such as styrene, α-methylstyrene, vinyltoluene, p-chlorostyrene, polychlorostyrene, fluorostyrene, bromostyrene, ethoxymethyl styrene, methoxystyrene, 4-methoxy-3-methystyrene, dimethoxystyrene, vinylbenzyl methyl ether, vinylbenzyl glycidyl ether, indene, 1-methylindene; vinyl or allyl esters, such as vinyl acetate, vinyl propionate, vinyl butylate, vinyl pivalate, vinyl benzoate, vinyl trimethylacetate, vinyl diethylacetate, vinyl barate, vinyl caproate, vinyl chloroacetate, vinyl dichloroacetate, vinyl methoxyacetate, vinyl butoxyacetate, vinyl phenylacetate, vinyl acetate, vinyl acetoacetate, vinyl lactate, vinyl phenylbutylate, vinyl cyclohexylcarboxylate, vinyl salicylate, vinyl chlorobenzoate, vinyl tetrachlorobenzoate, vinyl naphthoate, allyl acetate, allyl propionate, allyl butylate, allyl pivalate, allyl benzoate, allyl caproate, allyl stearate, allyl acetoacetate, allyl lactate; vinyl or allyl ethers, such as vinyl methyl ether, vinyl ethyl ether, vinyl hexyl ether, vinyl octyl ether, vinyl ethylhexyl ether, vinyl methoxyethyl ether, vinyl ethoxyethyl ether, vinyl chloroethyl ether, vinyl hydroxyethyl ether, vinyl ethybutyl ether, vinyl hydroxyethoxyethyl ether, vinyl dimethylaminoethyl ether, vinyl diethylaminoethyl ether, vinyl butylaminoethyl ether, vinyl benzyl ether, vinyl tetrahydrofurfuryl ether, vinyl phenyl ether, vinyl tolyl ether, vinyl chlorophenyl ether, vinyl chloroethyl ether, vinyl dichlorophenyl ether, vinyl naphthyl ether, vinyl anthryl ether, allyl glycidyl ether; amide type unsaturated compounds, such as (meth)acrylamide, N, N-dimethyl (meth)acrylamide, N, N-diethyl (meth)acrylamide, N, N-dibutyl (meth)acrylamide, N, N-diethylhexyl (meth)acrylamide, N, N-dicyclohexyl (meth)acrylamide, N, N-diphenyl (meth)acrylamide, N-methyl-N-phenyl (meth)acrylamide, N-hydroxyethyl-N-methyl (meth)acrylamide, N-methyl (meth)-acrylamide, N-ethyl (meth)acrylamide, N-propyl (meth)acrylamide, N-butyl (meth)-acrylamide, N-hydroxyethyl (meth)acrylamide, N-heptyl (meth)acrylamide, N-octyl (meth)acrylamide, N-ethyhexyl (meth)acrylamide, N-hydroxyethyl (meth)acrylamide-cyclohexyl, N-benzyl (meth)acrylamide, N-phenyl (meth)acrylamide, N-tolyl (meth)-acrylamide, N-hydroxyphenyl (meth)acrylamide, N-naphthyl (meth)acrylamide, N-phenylsulfonyl (meth)acrylamide, N-methylphenylsulfonyl (meth)acrylamide and N-(meth)acryloylmorpholine, diacetone acrylamide, N-methylol acrylamide, N-butoxyacrylamide; polyolefin type compounds, such as butadiene, isoprene, chloroprene and the like; (meth)acrylonitrile, methyl isopropenyl ketone, maleimide, N-phenylmaleimide, N-methylphenylmaleimide, N-methoxyphenylmaleimide, N-cyclohexylmaleimide, N-alkylmaleimide, maleic anhydride, polystyrene macromonomer, polymethyl (meth)acrylate macromonomer, polybutyl (meth)acrylate macromonomer; crotonates, such as butyl crotonate, hexyl crotonate, glycerine monocrotonate; and itaconates, such as dimethyl itaconate, diethyl itaconate, dibutyl itaconate; and maleates or fumarates, such as dimethyl mareate, dibutyl fumarate.

Preferable examples of copolymers are copolymers of methyl (meth)acrylate and (meth)acrylic acid, copolymers of benzyl (meth)acrylate and (meth)acrylic acid, copolymers of methyl (meth)acrylate/, ethyl (meth)acrylate and (meth)acrylic acid, copolymers of benzyl (meth)acrylate, (meth)acrylic acid and styrene, copolymers of benzyl (meth)acrylate, (meth)acrylic acid and 2-hydroxyethyl (meth)acrylate, copolymers of methyl (meth)acrylate/, butyl (meth)acrylate, (meth)acrylic acid and styrene, copolymers of methyl (meth)acrylate, benzyl (meth)acrylate, (metha)crylic acid and hydroxyphenyl (meth)acrylate, copolymers of methyl (meth)acrylate, (meth)acrylic acid and polymethyl (meth)acrylate macromonomer, copolymers of benzyl (meth)acrylate, (meth)acrylic acid and polymethyl (meth)acrylate macromonomer, copolymers of tetrahydrofurfuryl (meth)acrylate, styrene and (meth)acrylic acid, copolymers of methyl (meth)acrylate, (meth)acrylic acid and polystyrene macromonomer, copolymers of benzyl (meth)acrylate, (meth)acrylic acid and polystyrene macromonomer, copolymers of benzyl (meth)acrylate, (meth)acrylic acid, 2-hydroxyethyl (meth)acrylate and polystyrene macromonomer, copolymers of benzyl (meth)acrylate, (meth)acrylic acid, 2-hydroxypropyl (meth)acrylate and polystyrene macromonomer, copolymers of benzyl (meth)acrylate, (meth)acrylic acid, 2-hydroxy-3-phenoxypropyl (meth)acrylate and polymethyl (meth)acrylate macromonomer, copolymers of methyl (meth)acrylate, (meth)acrylic acid, 2-hydroxyethyl (meth)acrylate and polystyrene macromonomer, copolymers of benzyl (metha)crylate, (meth)acrylic acid, 2-hydroxyethyl (meth)acrylate and polymethyl (meth)acrylate macromonomer, copolymers of N-phenylmaleimide, benzyl (meth)acrylate, (meth)acrylic acid and styrene, copolymers of benzyl (meth)acrylate, (meth)acrylic acid, N-phenylmaleimide, mono-[2-(meth)acryloyloxy-ethyl] succinate and styrene, copolymers of allyl (meth)acrylate, (meth)acrylic acid, N-phenylmaleimide, mono-[2-(meth)acryloyloxyethyl] succinate and styrene, copolymers of benzyl (meth)acrylate, (meth)acrylic acid, N-phenylmaleimide, glycerol mono(meth)-acrylate and styrene, copolymers of benzyl (meth)acrylate, ω-carboxypolycaprolactone mono(meth)acrylate, (meth)acrylic acid, N-phenylmaleimide, glycerol mono(meth)-acrylate and styrene, and copolymers of benzyl (meth)acrylate, (meth)acrylic acid, N-cyclohexylmaleimide and styrene.
There can be used as well hydroxystyrene homo- or co-polymers or a novolak type phenol resin, for example, poly(hydroxystyrene) and poly(hydroxystyrene-covinylcyclohexanol), a novolak resin, a cresol novolak resin, and a halogenated phenol novolak resin. More specifically, it includes, for example, the methacrylic acid copolymers, the acrylic acid copolymers, the itaconic acid copoymers, the crotonic acid copolymers, the maleic anhydride co-polymers, for example, with styrene as a co-monomer, and maleic acid copolymers, and partially esterified maleic acid copolymers each described in, for example, JP 59-44615-B4 (the term "JP-B4" as used herein refers to an examined Japanese patent publication), JP 54-34327-B4, JP 58-12577-B4, and JP 54-25957-B4, JP 59-53836-A, JP 59-71048-A, JP 60-159743-A, JP 60-258539-A, JP 1-152449-A, JP 2-199403-A, and JP 2-199404-A, and which copolymers can be further reacted with an amine, as e.g disclosed in US5650263; further, a cellulose derivative having a carboxyl group on a side chain can be used, and particularly preferred are copolymers of benzyl (meth)acrylate and (meth)acrylic acid and copolymers of benzyl (meth)acrylate, (meth)acrylic acid and other monomers, for example as described in US 4139391, JP 59-44615-B4, JP 60-159743-A and JP 60-258539-A.

With respect to those having carboxylic acid groups among the above organic binder polymers, it is possible to react some or all of the carboxylic acid groups with glycidyl(meth)acrylate or an epoxy(meth)acrylate to obtain photopolymerizable organic binder polymers for the purpose of improving the photosensitivity, coating film strength, the coating solvent and chemical resistance and the adhesion to the substrate. Examples are disclosed in, JP 50-34443-B4 and JP 50-34444-B4, US5153095, by T. Kudo et al. in J. Appl. Phys., Vol. 37 (1998), p. 3594-3603, US5677385, and US5650233.

The weight-average molecular weight of the binders is preferably 500 to 1'000'000, e.g. 3'000 to 1'000'000, more preferably 5'000 to 400'000.

These compounds may be used singly or as a mixture of two or more kinds. The content of the binder in the light-sensitive resin composition is preferably 10 to 95 weight %, more preferably 15 to 90 weight % based on the whole solid matters.

Further, in the color filter the total solid component of each color may contain an ionic impurity-scavenger, e.g. an organic compound having an epoxy group. The concentration of the ionic impurity scavenger in the total solid component generally is in the range from 0.1 % by weight to 10% by weight.
Examples of color filters, especially with respect to the above described combinations of pigments and ionic impurity scavenger are given in EP 320264. It is understood, that the photoinitiators according to the present invention, i.e. the compounds of the formula I in the color filter formulations described in EP 320264 can replace the triazine initiator compounds.

The compositions according to this invention can comprise additionally a crosslinking agent which is activated by an acid, for example as described in JP 10-221843A, and a compound which generates acid thermally or by actinic radiation and which activates a crosslinking reaction.
The compositions according to this invention can also comprise latent pigments which are transformed into finely dispersed pigments during the heat treatment of the latent pigment containing photosensitive pattern or coating. The heat treatment can be performed after exposure or after development of the latent pigment-containing photoimageable layer. Such latent pigments are soluble pigment precursors which can be transformed into insoluble pigments by means of chemical, thermal, photolytic or radiation induced methods as described, for example, in US5879855. This transformation of such latent pigments can be enhanced by adding a compound which generates acid at actinic exposure or by adding an acidic compound to the composition. Therefore, a color filter resist can also be prepared, which comprises a latent pigment in a composition according to this invention.

Examples for color filter resists, the composition of such resists and the processing conditions are given by T. Kudo et al., Jpn. J. Appl. Phys. Vol. 37 (1998) 3594; T. Kudo et al., J. Photopolym. Sci. Technol. Vol 9 (1996) 109; K. Kobayashi, Solid State Technol. Nov. 1992, p. S15-S18; US5368976; US5800952; US5882843; US5879855; US5866298; US5863678; JP 06-230212A; EP320264; JP 09-269410A; JP 10-221843A; JP01-090516A; JP 10-171119A, US5821016, US5847015, US5882843, US5719008, EP881541, or EP902327.
The photoinitiators of the present invention can be used in color filter resists, for example, such as those given as examples above, or can partially or fully replace the known photoinitiators in such resists. It is understood by a person skilled in the art that the use of the new photoinitiators of the present invention is not limited to the specific binder resins, crosslinkers and formulations of the color filter resist examples given hereinbefore but can be used in conjunction with any radically polymerizable component in combination with a dye or color pigment or latent pigment to form a photosensitive color filter ink or color filter resist.

Accordingly, subject of the invention also is a color filter prepared by providing red, green and blue (RGB) colour elements and, optionally a black matrix, all comprising a photosensitive resin and a pigment on a transparent substrate and providing a transparent electrode either on the surface of the substrate or on the surface of the color filter layer, wherein said photosensitive resin comprises a polyfunctional acrylate monomer, an organic polymer binder and a photopolymerization initiator of formula I or I' as described above or a photoinitiator mixture of a compound of the formula I or I'. The monomer and binder components, as well as suitable pigments are as described above. In the manufacture of color filters the transparent electrode layer can either be applied on the surface of the transparent substrate or can be provided on the surface of the red, green and blue picture elements and the black matrix. The transparent substrate is for example a glass substrate which can additionally have an electrode layer on its surface.
It is preferred to apply a black matrix between the color areas of different color in order to improve the contrast of a color filter.

The photosensitive compositions of the present invention, as already stated above, are also suitable for the preparation of the black matrix of color filters. Said black matrix composition for example comprises a photoinitiator compound of the formula I of the present invention,
an organic binder, in particular an organic binder, which is an epoxy acrylate resin having a carboxyl group,
a black coloring material,
a polymer dispersant, in particular a polymer dispersant containing a basic functional group.
The person skilled in the art is familiar with such formulations. Examples of suitable black matrix compositions and the components (other than the photoinitiator) as described above are given in JP Patent No. 3754065, the disclosure of which hereby is incorporated by reference.

Instead of forming a black matrix using a photosensitive composition and patterning the black photosensitive composition photolithographically by patternwise exposure (i.e. through a suitable mask) to form the black pattern separating the red green and blue coloured areas on the tranparent substrate it is alternatively possible to use an inorganic black matrix. Such inorganic black matrix can be formed from deposited (i.e. sputtered) metal (i.e. chromium) film on the transparent substrate by a suitable imaging process, for example utilizing photolithographic patterning by means of an etch resist, etching the inorganic layer in the areas not protected by the etch resist and then removing the remaining etch resist.

There are different methods known how and at which step in the color filter manufacturing process the black matrix can be applied. It can either be applied directly on the transparent substrate prior to formation of the red, green and blue (RGB) colour filter as already mentioned above, or it can be applied after the RGB colour filter is formed on the substrate.

In a different embodiment of a color filter for a liqid crystal display, according to US626796, the black matrix can also be applied on the substrate opposite to the RGB color filter element-carrying substrate, which is separated from the former by a liquid crystal layer.

If the transparent electrode layer is deposited after applying the RGB color filter elements and -optionally - the black matrix, an additional overcoat film as aprotective layer can be applied on the color filter layer prior to deposition of the electrode layer, for example, as described in US 5650263.

To form an overcoat layer of a color filter, photosensitive resin or thermosetting resin compositions are employed. The photosensitive composition of the present invention can also be used to form such overcoat layers, because a cured film of the composition is excellent in flatness, hardness, chemical and thermal resistance, transparency especially in a visible region, adhesion to a substrate, and suitability for forming a transparent conductive film, e.g., an ITO film, thereon. In the production of a protective layer, there has been a demand that unnecessary parts of the protective layer, for example on scribing lines for cutting the substrate and on bonding pads of solid image sensors should be removed from the substrate as described in JP57-42009A, JP1-130103A and JP1-134306A. In this regard, it is difficult to selectively form a protective layer with good precision using the above-mentioned thermosetting resins. The photosensitive composition, however, allows to easily remove the unnecessary parts of the protective layer by photolithography.

It is obvious to those skilled in the art, that the photosensitive compositions of the present invention can be used for generating red, green and blue color pixels and a black matrix, for the manufacture of a color filter, regardless of the above described differences in processing, regardless, of additional layers which can be applied and regardless of differences in the design of the color filter. The use of a composition according to the present invention to form colored elements shall not be regarded as limited by different designs and manufacturing processes of such color filters.

The photo-sensitive composition of the present invention can suitably be used for forming a color filter but will not be limited to this application. It is useful as well for a recording material, a resist material, a protective layer, a dielectric layer, in display applications and display elements, a paint, and a printing ink.

The photosensitive compositions according to the invention are also suitable for manufacturing interlayer insulating layers or dielectric layers in a liquid crystal display, and more particularly in a reflection type liquid crystal display including an active matrix type display having a thin film transistor (TFT) as a switching device, and a passive matrix type without a switching device.
In recent years, liquid crystal displays have, for example, been widely used for pockettype TV sets and terminal devices for communication by virtue of its small thickness and light weight. A reflection type liquid crystal display without necessity of using a back light is in particular in demand because it is ultra-thin and light-weight, and it can significantly reduce power consumption. However, even if a back light is removed out of a presently available transmission type color liquid crystal display and a light reflection plate is added to a lower surface of the display, it would cause a problem in that the efficiency of utilizing lights is low, and it is not possible to have practical brightness. As a solution to this problem, there have been suggested various reflection type liquid crystal displays for enhancing an efficiency of utilizing lights. For instance, a certain reflection type liquid crystal display is designed to include a pixel electrode having reflection function.
The reflection type liquid crystal display includes an insulating substrate and an opposing substrate spaced away from the insulating substrate. A space between the substrates is filled with liquid crystals. A gate electrode is formed on the insulating substrate, and both the gate electrode and the insulating substrate are covered with a gate insulating film. A semiconductor layer is then formed on the gate insulating film above the gate electrode. A source electrode and a drain electrode are also formed on the gate insulating film in contact with the semiconductor layer. The source electrode, the drain electrode, the semiconductor layer, and the gate electrode cooperate with one another to thereby constitute a bottom gate type TFT as a switching device.
An interlayer insulating film is formed covering the source electrode, the drain electrode, the semiconductor layer, and the gate insulating film therewith. A contact hole is formed throughout the interlayer insulating film on the drain electrode. A pixel electrode made of aluminum is formed on both the interlayer insulating film and an inner sidewall of the contact hole. The drain electrode of the TFT is eventually in contact with the pixel electrode through the interlayer insulating film. The interlayer insulating layer is generally designed to have a roughened surface by which the pixel electrode acts as a reflection plate which diffuses lights to get a wider angle for viewing (angle of visibility).
The reflection type liquid crystal display remarkably enhances an efficiency of using lights by virtue that the pixel electrode acts as a light reflection plate.
In the above-mentioned reflection type liquid crystal display, the interlayer insulating film is designed to have projections and recesses by photolithography. To form and control a fine shape of the projections and recesses in micrometer order for surface roughness and to form contact holes, photolithography methods using positive and negative photoresists are used. For these resists the compositions according to the invention are especially suitable.

The photosensitive compositions according to the invention can further be used for manufacturing spacers, which control a cell gap of the liquid crystal part in liquid crystal display panels. Since the properties of light transmitted or reflected through the liquid crystal layer in a liquid crystal display are dependent on the cell gap, the thickness accuracy and uniformity over the pixel array are critical parameters for the performance of the liquid crystal display unit. In a liquid crystal cell, the spacing between the substrates in the cell is maintained constant by sparsely distributing glass or polymer spheres about several micrometers in diameter as spacers between the substrates. The spacers are thus held between the substrates to maintain the distance between the substrates at a constant value. The distance is determined by the diameter of the spacers. The spacers assure the minimum spacing between the substrates; i.e., they prevent a decrease in distance between the substrates. However, they cannot prevent the substrates from being separated apart from each other, i.e. the increase in distance between the substrates. Additionally, this method of using spacer beads has problems of the uniformity in the diameter of spacer beads and difficulty in the even dispersion of spacer beads on the panel, as well as nonuniform orientation and decrease in brightness and/or optical aperture depending on the location of spacers on pixel array region. Liquid crystal displays having a large image display area have recently been attracting much attention. However, the increase in the area of a liquid crystal cell generally produces the distortion of the substrates constituting the cell. The layer structure of the liquid crystal tends to be destroyed due to the deformation of the substrate. Thus, even when spacers are used for maintaining the spacing between the substrates constant, a liquid crystal display having a large image display area is unfeasible because the display experiences disturbances. Instead of the above spacer sphere dispersion method, a method of forming columns in the cell gap as spacers has been proposed. In this method, columns of a resin are formed as spacers in the region between the pixel array region and the counter electrode to form a prescribed cell gap. Photosensitive materials having adhesive properties with photolithography are commonly used, for instance, in the manufacturing process of color filters. This method is advantageous compared with the conventional method using spacer beads in the points that location, number and height of the spacers may be controlled freely. In recent years, as the spread of the touch panel type liquid crystal displays such as mobile audio players and handheld game platforms, the mechanical stress to liquid crystal panel tends to grow. The demand for spacer that controls the cell gap to raise mechanical strength becomes strong thus the multi-spacer method is used. According to the multi-spacer method, when cell gap narrows by pressure from the outside, adding to main-spacer that controls the cell gap normally lower sub-spacer supports the cell gap against external stress. The multi-spacer can follow the contraction of liquid crystal at low temperature conditions by main-spacer and prevent to generate bubbles inside the liquid crystal. The multi-spacer which contains main-spacer and sub-spacer is formed in the same step using, for example, a halftone mask as described in JPA-2011065133. The photosensitive compositions according to the invention are eligible for manufacturing process using halftone mask.
In a color liquid crystal display panel, such spacers are formed in the nonimaging area under black matrix of color filter elements. Therefore, the spacers formed using photosensitive compositions do not decrease brightness and optical aperture. Photosensitive compositions for producing protective layer with spacers for color filters are disclosed in JP 2000-81701 A and dry film type photoresists for spacer materials are also disclosed in J P 11-174459A and J P 11-174464A. As described in the documents, the photosensitive compositions, liquid and dry film photoresists, are comprising at least an alkaline or acid soluble binder polymer, a radically polymerizable monomer, and a radical initiator. In some cases, thermally crosslinkable components such as epoxide and carboxylic acid may additionally be included.
The steps to form spacers using a photosensitive composition are as follows:
a photosensitive composition is applied to the substrate, for instance a color filter panel and after the substrate is prebaked, it is exposed to light through a mask. Then, the substrate is developed with a developer and patterned to form the desired spacers. When the composition contains some thermosetting components, usually a postbaking is carried out to thermally cure the composition.

The photocurable compositions according to the invention are suitable for producing spacers for liquid crystal displays (as described above) because of their high sensitivity.

The photosensitive compositions according to the invention are also suitable for manufacturing microlens arrays used in liquid crystal display panels, image sensors and the like.
Microlenses are microscopic passive optical components that fit on active optoelectronic devices such as detectors, displays, and light emitting devices (light-emitting diodes, transversal and vertical cavity lasers) to improve their optical input or output quality. The areas of applications are wide and cover areas such as telecommunications, information technology, audio-visual services, solar cells, detectors, solid-state light sources, and optical interconnects.
Present optical systems use a variety of techniques to obtain efficient coupling between microlenses and microoptical devices.
The microlens arrays are used for condensing illuminating light on the picture element regions of a nonluminescent display device, such as a liquid crystal display devices, to increase the brightness of the display, for condensing incident light or as a means for forming an image on the photoelectric conversion regions of a line image sensor used for example in facsimiles and the like to improve the sensitivity of these devices, and for forming an image to be printed on a photosensitive means used in liquid crystal printers or light emitting diode (LED) printers.
The most common application is their use to improve the efficiency of photodetector arrays of a solid-state image sensing device such as a charge coupled device (CCD). In a detector array, the collection of as much light as possible in each detector element or pixel is wanted. If a microlens is put on top of each pixel, the lens collects incoming light and focuses it onto an active area that is smaller than the size of the lens. According to the prior-art, microlens arrays can be produced by a variety of methods; for each of them compositions according to the present invention may be employed.
(1) A method for obtaining convex lenses wherein a pattern of the lenses in a planar configuration is drawn on a thermoplastic resin by a conventional photolithographic technique or the like, and then the thermoplastic resin is heated to a temperature above the softening point of the resin to have flowability, thereby causing a sag in the pattern edge (so called "reflowing") (see, e.g., JP 60-38989A, JP 60-165623A, JP 61-67003A, and JP 2000-39503A). In this method, when the thermoplastic resin used is photosensitive, a pattern of the lenses can be obtained by exposure of this resin to light.
(2) A method for forming a plastic or glass material by the use of a mold or a stamper. As lens material, a photocurable resin and a thermosetting resin can be used in this method (see, e.g., WO99/38035).
(3) A method for forming convex lenses on the basis of a phenomenon in which when a photosensitive resin is exposed to light in a desired pattern by the use of an aligner, unreacted monomers move from the unexposed regions to the exposed regions, resulting in a swell of the exposed regions (see, e.g., Journal of the Research Group in Microoptics Japanese Society of Applied Physics, Colloquium in Optics, Vol. 5, No. 2, pp. 118-123 (1987) and Vol. 6, No. 2, pp. 87-92(1988)).
   On the upper surface of a supporting substrate, a photosensitive resin layer is formed. Thereafter, with the use of a separate shading mask, the upper surface of the photo-sensitive resin layer is illuminated with light from a mercury lamp or the like, so that the photosensitive resin layer is exposed to the light. As a result, the exposed portions of the photosensitive resin layer swell into the shape of convex lenses to form the light condensing layer having a plurality of microlens.
(4) A method for obtaining convex lenses wherein a photosensitive resin is exposed to light by a proximity exposure technique in which a photomask is not brought into contact with the resin, to cause a blur at the pattern edge, so that the amount of photochemical reaction products is distributed depending upon the degree of blurring at the pattern edge (see, e.g., JP 61-153602A).
(5) A method for generating a lens effect wherein a photosensitive resin is exposed to light with a particular intensity distribution to form a distribution pattern of refractive index depending upon the light intensity (see, e.g., JP 60-72927A and JP 60-166946A). The photosensitive compositions according to the invention can be used in any one of the above-mentioned methods to form microlens arrays using photocurable resin compositions.

A particular class of techniques concentrates on forming microlenses in thermoplastic resins like photoresist. An example is published by Popovic et al. in the reference SPIE 898, pp.23-25 (1988). The technique, named reflow technique, comprises the steps of defining the lenses' footprint in a thermoplastic resin, e.g. by photolithography in a photosensitive resin like a photoresist, and subsequently heating this material above its reflow temperature. The surface tension draws the island of photoresist into a spherical cap with a volume equal to the original island before the reflow. This cap is a plano-convex microlens. Advantages of the technique are, amongst others, the simplicity, the reproducibility, and the possibility of integration directly on top of a light-emitting or light-detecting optoelectronic device.
In some cases, an overcoat layer is formed on the patterned lens units with a rectangular shape prior to reflowing to avoid a sagging of the island of the resin in the middle without reflow into a spherical cap in the reflow step. The overcoat acts as a permanent protective layer. The coating layer is also made of a photosensitive composition. Microlens arrays can also be fabricated by the use of a mold or a stamper as, for example, disclosed in EP0932256. A process of manufacturing the planar microlens array is as follows: a release agent is coated on a shaping surface of a stamper on which convex portions are densely arranged, and a photocurable synthetic resin material having a high refractive index is set on the shaping surface of the stamper. Next, the base glass plate is pushed onto the synthetic resin material, thereby spreading the synthetic resin material, and the synthetic resin material is cured by irradiating with ultraviolet radiation or by heating and is shaped to form the convex microlenses. Thereafter the stamper is peeled off. Then, a photocurable synthetic resin material having a low refractive index is additionally coated onto the convex microlenses as an adhesive layer and a glass substrate which is made into a cover glass plate is pushed onto the synthetic resin material, thereby spreading the same. The synthetic resin material is then cured and finally the planar microlens array is formed.
As disclosed in US5969867, a similar method using a mold is applied for the production of a prism sheet, which is used as a part of backlight units for color liquid crystal display panels to enhance the brightness. A prism sheet forming a prism row on one side is mounted on the light-emitting surface of the backlight. For fabricating a prism sheet, an active energy ray-curable composition is cast and spread in a lens mold which is made of metal, glass or resin and forms the lens shape of the prism row, etc., after which a transparent substrate sheet is placed onto it and active energy rays from an active energy ray-emitting source are irradiated through the sheet for curing. The prepared lens sheet is then released from the lens mold to obtain the lens sheet.
The active energy ray-curable composition used to form the lens section must have a variety of properties, including adhesion to the transparent substrate, and suitable optical characteristics.
Lenses at least with some photoresists in the prior art are not desirable for some applications since the optical transmittance in the blue end of the optical spectrum is poor. Because the photocurable compositions according to the invention have low yellowing properties, both thermally and photochemically, they are suitable for the production of microlens arrays as described above.

The novel radiation-sensitive compositions are also suitable for photo-lithographic steps used in the production process of plasma display panels (PDP), particularly for the imaging forming process of barrier rib, phosphor layer and electrodes.
The PDP is a planar display for displaying images and information by virtue of the emission of light by gas discharge. By the construction of panel and the method of operation, it is known in two types, i.e. DC (direct current) type and AC (alternating current) type.
By way of example, the principle of the DC type color PDP will be briefly explained. In the DC type color PDP, the space intervening between two transparent substrates (generally glass plates) is divided into numerous minute cells by latticed barrier ribs interposed between the transparent substrates. In the individual cells a discharge gas, such as He or Xe, is sealed. On the rear wall of each cell there is a phosphor layer which, on being excited by the ultraviolet light generated by the discharge of the discharge gas, emits visible light of three primary colors. On the inner faces of the two substrates, electrodes are disposed as opposed to each other across the relevant cells. Generally, the cathodes are formed of a film of transparent electroconductive material such as NESA glass. When a high voltage is applied between these electrodes formed on the fore wall and the rear wall, the discharge gas which is sealed in the cells induces plasma discharge and, by virtue of the ultraviolet light radiated consequently, incites the fluorescent elements of red, blue, and green colors to emit lights and effect the display of an image. In the full-color display system, three fluorescent elements severally of the three primary colors of red, blue, and green mentioned above jointly form one picture element.
The cells in the DC type PDP are divided by the component barrier ribs of a lattice, whereas those in the AC type PDP are divided by the barrier ribs which are arranged parallel to each other on the faces of the substrates. In either case, the cells are divided by barrier ribs. These barrier ribs are intended to confine the luminous discharge within a fixed area to preclude false discharge or cross talk between adjacent discharge cells and ensure ideal display.

The compositions according to the invention also find application for the production of one- or more-layered materials for the image recording or image reproduction (copies, reprography), which may be mono- or polychromatic. Furthermore the materials are suitable for color proofing systems. In this technology formulations containing microcapsules can be applied and for the image production the radiation curing can be followed by a thermal treatment. Such systems and technologies and their applications are for example disclosed in US5376459.

The compounds of the formula I are also suitable as photoinitiators in the holographic data storage application. Said photoinitiators generate radicals and initiate polymerization of monomer upon irradiation with blue laser radiation, suitable for holographic data storage. The wavelength range of the blue laser is 390-420 nm, preferably 400-410 nm and particularly 405 nm. Holographic storage systems (holographic recording media) are for example used to record and to retrieve a large amount of data with fast access time. The photoinitiators of the invention are for example in particular suitable for systems as described for example in WO 03/021358.
The holographic data storage system is preferably comprised of a matrix network of low-refractive index matrix precursors and high-refractive index photopolymerizable monomers.
The matrix precursor and photoactive monomer can be selected such that (a) the reaction by which the matrix precursor is polymerized during the cure is independent from the reaction by which the photoactive monomer will be polymerized during writing of a pattern, e.g. data, and (b) the matrix polymer and the polymer resulting from polymerization of the photoactive monomer (the photopolymer) are compatible with each other. The matrix is considered to be formed when the photorecording material, i.e. the matrix material plus the photoactive monomer, photoinitiator and/or additives, exhibits an elastic modulus of at least about 10⁵ Pa, generally about 10⁵ Pa to about 10⁹ Pa.
The media matrix is formed by in-situ polymerization which yields as cross-linked network in the presence of the photopolymerizable monomers which remain "dissolved" and unreacted. The matrix containing un-reacted, photopolymerizable monomers can also be formed by other means, for example by using a solid-resin matrix material in which the photoreactive, liquid monomer is homogeneously distributed. Then, monochromatic exposure generates the holographic pattern, which according to the light intensity distribution, polymerizes the photoreactive monomers in the solid pre-formed matrix. The unreacted monomers (where light intensity was at a minimum) diffuse through the matrix, producing a modulation of the refractive index that is determined by the difference between the refractive indices of the monomer and the matrix and by the relative volume fraction of the monomer. The thickness of the recording layer is in the range of several micrometers up to a thickness of one millimeter. Because of such thick holographic data storage layers it is required that the photoinitiator combines high photoreactivity with low absorbance, in order to render the layer transparent at the laser wavelength to assure that the extent of photopolymerization is as little as possible dependent on the exposure depth into the recording layer.
It was found that the photoinitiators of the present invention combine high reactivity with low absorbance at 405 nm and are suitable for this application. Dyes and sensitizers can also be added to the formulations. Suitable dyes and sensitizers for blue laser radiation are for example coumarines, xanthones, thioxanthones, see list above.
In particular relevant are thioxanthones, coumarins and benzophenones as mentioned under items 1., 2. and 3. in the list given above.
It was found that the photoinitiators allow photopolymerization of monomers in thick layers, such as required for holographic data storage, with high sensitivity and yield recording layers which are sensitive to blue laser radiation. The photoinitiators, when applied at a concentration of 2-8 wt% in the photosensitive layer of 20 micron thickness yield an absorbance of the layer which comprises the photoinitiator, of less than 0.4, preferably less than 0.2 at the laser wavelength.
The photoinitiators are in particular suitable for the preparation of optical articles (for example optical waveguides) or holographic recording media e.g. comprising a polymer and an organic photoinitiator as described above, having a maximum absorption at a UV wavelength in the range of 340-450 nm, wherein the refractive index contrast adjusted sensitivity is greater than 3x10⁻⁶Δn/(mJ/cm²). For example, the polymer is formed by polymerizing a material comprising component 1 and component 2, wherein component 1 comprises a NCO-terminated pre-polymer and component 2 comprises a polyol. Component 1 is, for example, diphenylmethane diisocyanate, toluene diisocyanate, hexamethylene diisocyanate, a derivative of hexamethylene diisocyanate, a methylenebiscyclohexylisocyanate, a derivative of methylenebiscyclohexylisocyanate. Component 2 is for example a polyol of propylene oxide. Preferably, the photoactive monomer is an acrylate monomer. In such media the shrinkage induced by writing is usually less than 0.25%.

Photocuring further is of great importance for printings, since the drying time of the ink is a critical factor for the production rate of graphic products, and should be in the order of fractions of seconds. UV-curable inks are particularly important for screen printing and offset inks.

As already mentioned above, the novel mixtures are highly suitable also for producing printing plates. This application uses, for example, mixtures of soluble linear polyamides or styrene/butadiene and/or styrene/isoprene rubber, polyacrylates or polymethyl methacrylates containing carboxyl groups, polyvinyl alcohols or urethane acrylates with photopolymerizable monomers, for example acrylamides and/or methacrylamides, or acrylates and/or methacrylates, and a photoinitiator. Films and plates of these systems (wet or dry) are exposed over the negative (or positive) of the printed original, and the uncured parts are subsequently washed out using an appropriate solvent or aqueous solutions.
Another field where photocuring is employed is the coating of metals, in the case, for example, of the coating of metal plates and tubes, cans or bottle caps, and the photocuring of polymer coatings, for example of floor or wall coverings based on PVC. Examples of the photocuring of paper coatings are the colourless varnishing of labels, record sleeves and book covers.

Also of interest is the use of the novel photoinitiators for curing shaped articles made from composite compositions. The composite compound consists of a self-supporting matrix material, for example a glass fibre fabric, or alternatively, for example, plant fibres [cf. K.-P. Mieck, T. Reussmann in Kunststoffe 85 (1995), 366-370], which is impregnated with the photocuring formulation. Shaped parts comprising composite compounds, when produced using the novel compounds, attain a high level of mechanical stability and resistance. The novel compounds can also be employed as photocuring agents in moulding, impregnating and coating compositions as are described, for example, in EP7086. Examples of such compositions are gel coat resins, which are subject to stringent requirements regarding curing activity and yellowing resistance, and fibre-reinforced mouldings, for example, light diffusing panels which are planar or have lengthwise or crosswise corrugation. Techniques for producing such mouldings, such as hand lay-up, spray lay-up, centrifugal casting or filament winding, are described, for example, by P.H. Selden in "Glasfaserverstärkte Kunststoffe", page 610, Springer Verlag Berlin-Heidelberg-New York 1967. Examples of articles which can be produced by these techniques are boats, fibre board or chipboard panels with a double-sided coating of glass fibre-reinforced plastic, pipes, containers, etc. Further examples of moulding, impregnating and coating compositions are UP resin gel coats for mouldings containing glass fibres (GRP), such as corrugated sheets and paper laminates. Paper laminates may be based on urea resins or melamine resins. Prior to production of the laminate, the gel coat is produced on a support (for example a film). The novel photocurable compositions can also be used for casting resins or for embedding articles, for example electronic components, etc..

The compositions and compounds according to the invention can be used for the production of holographies, waveguides, optical switches wherein advantage is taken of the development of a difference in the index of refraction between irradiated and unirradiated areas.

The use of photocurable compositions for imaging techniques and for the optical production of information carriers is also important. In such applications, as already described above, the layer (wet or dry) applied to the support is irradiated imagewise, e.g. through a photomask, with UV or visible light, and the unexposed areas of the layer are removed by treatment with a developer. Application of the photocurable layer to metal can also be carried out by electrodeposition. The exposed areas are polymeric through crosslinking and are therefore insoluble and remain on the support. Appropriate colouration produces visible images. Where the support is a metallized layer, the metal can, following exposure and development, be etched away at the unexposed areas or reinforced by electroplating. In this way it is possible to produce electronic circuits and photoresists. When used in image-forming materials the novel photoinitiators provide excellent performance in generating so called printout images, whereby a color change is induced due to irradiation. To form such printout images different dyes and/or their leuco form are used and examples for such print out image systems can be fount e.g. in WO96/41240, EP706091, EP511403, US3579339 and US4622286.

The novel photoinitiator is also suitable for a photopatternable composition for forming a dielectric layer of a multilayer layer circuit board produced by a sequential build-up process.

The invention, as described above, provides compositions, as well as a process, for producing pigmented and nonpigmented paints and varnishes, powder coatings, printing inks, printing plates, adhesives, pressure-sensitive adhesives, dental compositions, gel coats, photoresists for electronics, electroplating resist, etch resist, both liquid and dry films, solder resist, as resists to manufacture color filters for a variety of display applications, to generate structures in the manufacturing processes of plasma-display panels (e.g. barrier rib, phosphor layer, electrode), electroluminescence displays and LCD (e.g. interlayer insulating layer, spacers, microlens array), for holographic data storage (HDS), as composition for encapsulating electrical and electronic components, for producing magnetic recording materials, micromechanical parts, waveguides, optical switches, plating masks, etch masks, colour proofing systems, glass fibre cable coatings, screen printing stencils, for producing three-dimensional objects by means of stereolithography, and as image recording material, for holographic recordings, microelectronic circuits, decolorizing materials, decolorizing materials for image recording materials, for image recording materials using microcapsules, as a photoresist material used for forming dielectric layers in a sequential build-up layer of a printed circuit board; wherein the process comprises irradiating a composition as described abbove with electromagnetic radiation in the range from 150 to 600 nm, or with electron beam or with X-rays.

Substrates used for photographic information recordings include, for example, films of polyester, cellulose acetate or polymer-coated papers; substrates for offset printing formes are specially treated aluminium, substrates for producing printed circuits are copper-clad laminates, and substrates for producing integrated circuits are, for example, silicon wafers. The layer thickness of the photosensitive layer for photographic materials and offset printing forms is generally from about 0.5 µm to 10 µm, while for printed circuits it is from 0.1 µm to about 100 µm. Following the coating of the substrates, the solvent is removed, generally by drying, to leave a coat of the photoresist on the substrate.

Coating of the substrates can be carried out by applying to the substrate a liquid composition, a solution or a suspension. The choice of solvents and the concentration depend principally on the type of composition and on the coating technique. The solvent should be inert, i.e. it should not undergo a chemical reaction with the components and should be able to be removed again, after coating, in the course of drying. Examples of suitable solvents are ketones, ethers and esters, such as methyl ethyl ketone, isobutyl methyl ketone, cyclopentanone, cyclohexanone, N-methylpyrrolidone, dioxane, tetrahydrofuran, 2-methoxyethanol, 2-ethoxyethanol, 1-methoxy-2-propanol, 1,2-dimethoxyethane, ethyl acetate, n-butyl acetate, ethyl 3-ethoxypropionate, 2-methoxypropylacetate, methyl-3-methoxypropionate, 2-heptanone, 2-pentanone, and ethyl lactate.
The solution is applied uniformly to a substrate by means of known coating techniques, for example by spin coating, dip coating, knife coating, curtain coating, brushing, spraying, especially by electrostatic spraying, and reverse-roll coating, and also by means of electrophoretic deposition. It is also possible to apply the photosensitive layer to a temporary, flexible support and then to coat the final substrate, for example a copper-clad circuit board, or a glass substrate by transferring the layer via lamination.
The quantity applied (coat thickness) and the nature of the substrate (layer support) are dependent on the desired field of application. The range of coat thicknesses generally comprises values from about 0.1 µm to more than 100 µm, for example 0.1 µm to 1 cm, preferably 0.5 µm to 1000 µm.

Following the coating of the substrates, the solvent is removed, generally by drying, to leave an essentially dry resist film of the photoresist on the substrate.

The photosensitivity of the novel compositions can extend in general from about 150 nm to 600 nm, for example 190-600 nm, (UV-vis region). Suitable radiation is present, for example, in sunlight or light from artificial light sources. Consequently, a large number of very different types of light sources are employed. Both point sources and arrays ("lamp carpets") are suitable. Examples are carbon arc lamps, xenon arc lamps, low-, medium-, high- and super high- pressure mercury lamps, possibly with metal halide dopes (metal-halogen lamps), microwave-stimulated metal vapour lamps, excimer lamps, superactinic fluorescent tubes, fluorescent lamps, argon incandescent lamps, electronic flashlights, photographic flood lamps, light emitting diodes (LED), electron beams and X-rays. The distance between the lamp and the substrate to be exposed in accordance with the invention may vary depending on the intended application and the type and output of lamp, and may be, for example, from 2 cm to 150 cm. Laser light sources, for example excimer lasers, such as F₂ excimer lasers at 157 nm exposure, KrF excimer lasers for exposure at 248 nm and ArF excimer lasers for exposure at 193 nm are also suitable. Lasers in the visible region can also be employed.

The term "imagewise" exposure includes both, exposure through a photomask comprising a predetermined pattern, for example a slide, a chromium mask, a stencil mask or a reticle, as well as exposure by means of a laser or light beam, which for example is moved under computer control over the surface of the coated substrate and in this way produces an image. Suitable UV laser exposure systems for the purpose are, for example, provided by Etec and Orbotech (DP-100™ DIRECT IMAGING SYSTEM). Other examples of laser light sources are, for example excimer lasers, such as F₂ excimer lasers at 157 nm exposure, KrF excimer lasers for exposure at 248 nm and ArF excimer lasers for exposure at 193 nm. Further suitable are solid state UV lasers (e.g. Gemini from ManiaBarco, DI-2050 from PENTAX) and violet laser diodes with 405 nm output (DI-2080, DI-PDP from PENTAX). Lasers in the visible region can also be employed. And the computer-controlled irradiation can also be achieved by electron beams. It is also possible to use masks made of liquid crystals that can be addressed pixel by pixel to generate digital images, as is, for example, described by A. Bertsch, J.Y. Jezequel, J.C. Andre in Journal of Photochemistry and Photobiology A: Chemistry 1997, 107, p. 275-281 and by K.-P. Nicolay in Offset Printing 1997, 6, p. 34-37. Following the imagewise exposure of the material and prior to development, it may be advantageous to carry out thermal treatment for a short time. After the development a thermal post bake can be performed to harden the composition and to remove all traces of solvents. The temperatures employed are generally 50-250°C, preferably 80-220°C; the duration of the thermal treatment is in general between 0.25 and 60 minutes.

The photocurable composition may additionally be used in a process for producing printing plates or photoresists as is described, for example, in DE4013358. In such a process the composition is exposed for a short time to visible light with a wavelength of at least 400 nm, without a mask, prior to, simultaneously with or following imagewise irradiation.
After the exposure and, if implemented, thermal treatment, the unexposed areas of the photosensitive coating are removed with a developer in a manner known per se. As already mentioned, the novel compositions can be developed by aqueous alkalis or organic solvents. Particularly suitable aqueous-alkaline developer solutions are aqueous solutions of tetraalkylammonium hydroxides or of alkali metal silicates, phosphates, hydroxides and carbonates. Minor quantities of wetting agents and/or organic solvents may also be added, if desired, to these solutions. Examples of typical organic solvents, which may be added to the developer liquids in small quantities, are cyclohexanone, 2-ethoxyethanol, toluene, acetone and mixtures of such solvents. Depending on the substrate also solvents, e.g. organic solvents, can be used as developer, or, as mentioned above mixtures of aqueous alkalis with such solvents. Particularly useful solvents for solvent development include methanol, ethanol, 2-propanol, 1-propanol, butanol, diacetone alcohol, ethylene glycol monomethyl ether, ethylene glycol monoethyl ether, ethylene glycol mono-n-butyl ether, diethyleneglycol dimethyl ether, propyleneglycol monomethyl ether acetate, ethyl-3-ethoxypropionate, methyl-3-methoxypropionate, n-butyl acetate, benzyl alcohol, acetone, methyl ethyl ketone, cyclopentanone, cyclohexanone, 2-heptanone, 2-pentanone, epsilon-caprolactone, gamma-butylolactone, dimethylformamide, dimethylacetamide, hexamethylphosphoramide, ethyl lactate, methyl lactate, epsilon-caprolactam, and N-methylpyrrolidinone. Optionally, water can be added to these solvents up to a level at which still a clear solution is obtained and at which sufficient solubility of the unexposed areas of the light sensitive composition is maintained.

The invention therefore also provides a process for the photopolymerization of compounds containing ethylenically unsaturated double bonds, i.e. monomeric, oligomeric or polymeric compounds containing at least one ethylenically unsaturated double bond, which comprises adding to these compounds at least one photoinitiator of the formula I as described above and irradiating the resulting composition with electromagnetic radiation, in particular light of the wavelength 150 to 600 nm, in particular 190-600 nm, with electron beam, or with X-rays.
In other words, adding to these compounds compounds containing ethylenically unsaturated double bonds at least one photoinitiator of the formula I as described above and irradiating the resulting composition with electromagnetic radiation, in particular light of the wavelength 150 to 600 nm, in particular 190-600 nm, with electron beam, or with X-rays.

The invention further provides a coated substrate which is coated on at least one surface with a composition as described above, and describes a process for the photographic production of relief images, in which a coated substrate is subjected to imagewise exposure and then the unexposed portions are removed with a developer. Imagewise exposure may be effected by irradiating through a mask or by means of a laser or electron beam as already described above. Of particular advantage in this context is the laser beam exposure already mentioned above.

The compounds of the invention have a good thermal stability, low volatility, good storage stability and high solubility, and are also suitable for photopolymerisations in the presence of air (oxygen). Further, they cause only low yellowing in the compositions after photopolymerization.

The examples which follow illustrate the invention in more detail. Parts and percentages are, as in the remainder of the description and in the claims, by weight, unless stated otherwise. Where alkyl radicals having more than three carbon atoms are referred to in the following examples without any mention of specific isomers, the n-isomers are meant in each case.

### Example 1 Synthesis of 1-[11-(2-Ethylhexyl)-5-(2,4,6-trimethylbenzoyl)-11H-benzo[a]carbazol-8-yl]-ethanone oxime O-acetate

### 1.a 11-(2-Ethylhexyl)-11H-benzo[a]carbazole

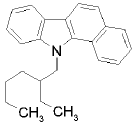

11 H-Benzo[a]carbazole can be prepared according to some procedures, for instance, the procedure described in SYNLETT, 2006, 7, 1021. The structure is confirmed by the ¹H-NMR spectrum (CDCl₃). δ [ppm]: 7.29 (td, 1 H), 7.44 (td, 1 H), 7.53 (td), 7.57-7.61 (m, 3H), 7.66 (d, 1H), 8.01 (d, 1H), 8.11-8.15 (m, 3H), 8.77 (br, 1H).
To 11 H-benzo[a]carbazole (0.70 g; 3.22 mmol) in DMF (3 ml) is added sodium hydride (0.19 g; 4.67 mmol) at 0°C, After stirring 1 h at 0°C, 1-bromo-2-ethylhexane (1.24 g; 6.44 mmol) is added at 0°C, and the mixture is stirred at room temperature overnight. The reaction mixture is poured into ice-water, and the crude product is extracted twice with AcOEt (acetic acid ethyl ester). The combined organic layer is washed with H₂O and brine, dried over MgSO₄, and concentrated and dried in vacuo to give a yellow liquid as a crude product (1.08 g). The product is used for the next reaction without further purification. The structure is confirmed by ¹H-NMR spectrum (CDCl₃). δ [ppm]:0.80-0.92 (m, 6H), 1.16-1.47 (m, 8H), 2.27 (m, 1H), 4.65-4.69 (m, 2H), 7.27 (td, 1 H), 7.44-7.59 (m, 4H), 7.66 (d, 1 H), 8.02 (d, 1 H), 8.14 (d, 1 H), 8.18 (d, 1 H), 8.54 (d, 1 H).
1.b 1-[11-(2-Ethylhexyl)-5-(2,4,6-trimethylbenzoyl)-11H-benzo[a]carbazol-8-yl}-ethanone

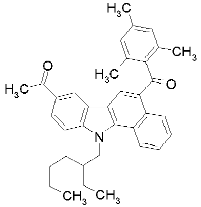

To 11-(2-ethylhexyl)-11H-benzo[a]carbazole (0.42 g; 1.27 mmol) in CH₂Cl₂ (20 mL) are added 2,4,6-trimethylbenzoyl chloride (0.23 g; 1.27 mmol) and AlCl₃ (0.17 g; 1.27 mmol) at 0°C. After stirring at room temperature for 2.5 hours, acetyl chloride (0.17 g; 1.31 mmol) and AlCl₃ (0.19; 1.40 mmol) are added at 0°C, and the mixture is stirred at room temperature for 3 hours. The reaction mixture is poured into ice-water, and the crude product is extracted twice with CH₂Cl₂. The combined organic layer is washed with water and brine, dried over MgSO₄, and concentrated to give a beige solid (0.45 g; 75%). The structure is confirmed by ¹H-NMR spectrum (CDCl₃). δ [ppm]: 0.82 (t, 3H), 0.90 (t, 3H), 1.18-1.45 (m, 6H), 2.20 (s, 6H), 2.29-2.30 (m, 1H), 2.41 (s, 3H), 2.72 (s, 3H), 4.74-4.77 (m, 2H), 6.98 (s, 2H), 7.58 (d, 1H), 7.70-7.79 (m, 2H), 8.10 (dd, 1H), 8.39 (s, 1 H), 8.60 (d, 1 H), 8.64 (d, 1 H), 9.53 (d, 1 H).
1.c 1-[11-(2-Ethylhexyl)-5-(2,4,6-trimethylbenzoyl)-11H-benzo[a]carbazol-8-yl}]-ethanone oxime O-acetate

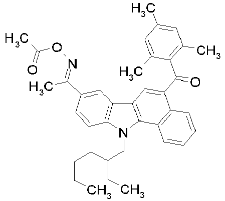

To 1-[11-(2-ethylhexyl)-5-(2,4,6-trimethylbenzoyl)-11H-benzo[a]carbazol-8-yl}-ethanone (0.49 g; 0.95 mmol) in toluene (5 mL) and N-methylpyrrolidone (NMP) (0.85 ml) warmed at 80°C are added sodium acetate (93.5 mg; 1.14 mmol) and hydroxylammonium chloride (78.9 mg; 1.14 mmol), and then, the mixture is stirred at 100°C overnight. After the reaction mixture is cooled at 0°C, triethylamine (0.145 g; 1.43 mmol) and acetyl chloride (0.11 g; 1.43 mmol) are added there, and then the mixture is stirred at room temperature for 2 hours. After the reaction is completed, the reaction mixture is added into water. Then the crude product is extracted with toluene. The organic layer is washed with water and brine, dried over MgSO₄, and concentrated to give a residue. This residue is washed with *tert*-butylmethylether to give a white solid (0.33g; 60%). The structure is confirmed by ¹H-NMR spectrum (CDCl₃). δ [ppm]: 0.82 (t, 3H), 0.89 (t, 3H), 1.19-1.39 (m, 8H), 2.26 (s, 6H), 2.27-2.30 (m, 1 H),2.29 (s, 3H), 2.39 (s, 3H), 2.50 (s, 3H), 4.72-4.76 (m, 2H), 6.96 (s, 2H), 7.16-7.18 (m, 1H), 7.24-7.27 (m,1H), 7.57 (d, 1 H), 7.69-7.77 (m, 2H), 7.87 (dd, 1 H), 8.31 (s, 1 H), 8.38 (s, 1 H), 8.64 (d, 1 H), 9.53 (d, 1 H)

### Examples 2-17:

The corresponding diketone intermediates of the compounds of examples 2-17 are generally prepared according to the procedure as disclosed in example 1.b. with the corresponding alkanoyl or aroyl chloride instead of acetyl chloride. Especially, the intermediates of examples 11-14 are prepared with 3-chloropropionyl chloride followed by reacting with the corresponding alkoxide or alkylthiolate after reaction with 2,4,6-trimethylbenzoyl chloride. Furthermore, in case of example 14, the sulfide is trans-formed into sulfone by oxidation with m-chloroperbonzoic acid. The intermediates of examples 15 and 16 are prepared with chloroacetyl chloride instead of 3-chloropropionyl chloride followed by reacting with the corresponding alkylthiolate or carboxylic acid. The diketone intermediate of example 17 is prepared with 2-butenoyl chloride. Although each oximation and the next esterification are conducted according to the procedure as disclosed in example 1.c and 1.d, oximation in examples 3-8 is conducted with pyridine instead of a mixed solvent of toluene and NMP at 100 °C. The compounds of examples 3-8 are obtained as an isomeric mixture, but the compounds of the other examples are obtained as a single component.

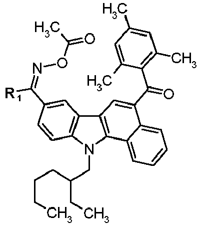

**Table 1:**

| Ex. | R₁ | ¹H-NMR spectrum (CDCl₃) δ[ppm] |
|---|---|---|
| 2 | 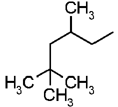 | 0.81-0.93 (m), 1.21-1.37 (m), 1.84-1.94 (m), 2.20 (s), 2.26 (s), 2.27-2.33 (m), 2.39 (s), 2.91-2.93 (m), 4.71-4.76 (m), 6.96 (s), 7.57 (d), 7.68 (s), 7.77 (m), 7.87 (d), 8.25 (s), 8.36 (s), 8.64 (d), 9.50 (d) |
| 3 | 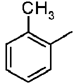 | 0.81 (t), 0.88 (t), 1.16-1.41 (m), 2.04 (s), 2.13 (s), 2.18 (s), 2.20 (s), 2.23-2.27 (m), 2.39 (s), 4.68-4.72 (m), 6.95 (s), 7.14 (d), 7.23-7.45 (m), 7.51 (d), 8.30 (s), 8.36 (s), 8.51 (s), 8.64 (d), 8.67 (d), 9.51 (d), 9.58 (d) |
| 4 | 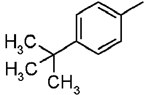 | 0.81-0.94 (m), 1.19 (s), 1.27 (s), 1.33 (s), 1.40 (s), 1.41-1.46 (m), 2.05 (s), 2.13 (s), 2.18 (s), 2.19 (s), 2.35 (s), 2.36 (s), 4.70-4.74 (m), 4.76-4.78 (m), 6.92 (s), 6.92 (s), 7.28 (d), 7.38-7.43 (m), 7.46-7.50 (m), 7.62 (d)7.70-7.77 (m), 7.94 (s), 8.28 (s), 8.33-8.34 (m), 8.35 (s), 8.63 (d), 8.66 (d), 5.21 (d), 9.56 (d) |
| 5 | 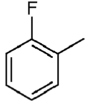 | 0.81 (t), 0.87 (t), 1.17-1.39 (m), 2.09 (s), 2.20 (s), 2.22-2.31 (m), 2.38 (s), 4.70-4.73 (m), 6.94 (s), 7.20-7.28 (m), 7.42-7.44 (m), 7.48-7.54 (m), 7.68-7.77 (m), 8.35 (s), 8.42 (s), 8.63 (d), 9.52 (dd) |
| | | ¹⁹F-NMR spectrum (CDCl₃) δ[ppm]; -110.97 |
| 6 | 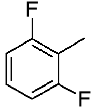 | 0.81 (t), 0.88 (t), 1.18-1.40 (m), 2.05 (s), 2.11 (s), 2.20 (s), 2.38 (s), 4.70-4.73 (m), 6.95 (s), 7.06 (t), 7.47-7.53 (m), 7.69-7.76 (m), 8.36 (s), 8.46 (d), 8.63 (d), 9.51 (dd) |
| | | ¹⁹F-NMR spectrum (CDCl₃) δ[ppm]; -109.73 |
| 7 | 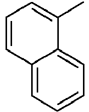 | 0.76-0.81 (m), 0.84-0.90 (m), 1.17-1.36 (m), 1.83 (s), 2.19 (s), 2.22-2.28 (m), 2.39 (s), 4.66-4.69 (m), 6.94 (s), 7.31-7.45 (m), 7.51-7.60 (m), 7.67-7.76 (m), 7.95-8.00 (m), 8.36 (s), 8.59-8.62 (m), 9.52 (dd) |
| 8 | 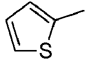 | 0.83 (t), 0.87-0.96 (m), 1.22-1.47 (m), 2.04 (s), 2.05 (s), 2.19 (s), 2.28-2.33 (m), 2.37 (d), 4.75-4.99 (m), 6.93 (s), 7.00-7.03 (m), 7.05 (dd), 7.08-7.10 (m), 7.23-7.25 (m), 7.48-7.49 (m), 7.50 (d), 7.59 (s), 7.63 (s), 7.65 (s), 7.70-7.79 (m), 7.96 (d), 8.21 (s), 8.29 (s), 8.66 (d), 9.56 (d), 9.59 (dd) |
| 9 | 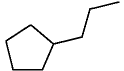 | 0.81 (t), 0.89 (t), 1.12-1.39 (m), 1.57-1.62 (m), 1.80-1.89 (m), 2.20 (s), 2.27-2.30 (m), 2.38 (s), 2.93-2.97 (m), 4.68-4.79 (m), 6.96 (s), 7.57 (d), 7.68-7.77 (m), 7.82 (d), 8.29 (s), 8.37 (s), 8.64 (d), 9.55 (d) |
| 10 | 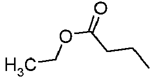 | 0.82 (t), 0.89 (t), 1.21-1.42 (m), 2.21 (s), 2.27-2.28 (m), 2.40 (s), 2.60 (t), 3.26 (t), 4.10 (q), 4.72-4.76 (m), 6.96 (s), 7.58 (d), 7.69-7.79 (m), 8.30 (s), 8.37 (s), 8.64 (d), 9.53 (d) |
| 11 | 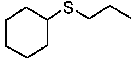 | 0.82 (t), 0.89 (t), 1.17-1.44 (m), 1.60-1.62 (m), 1.74-1.75 (m), 1.92-1.95 (m), 2.21 (s), 2.28-2.32 (m), 2.39 (s), 2.70-2.77 (m), 3.20-3.24 (m), 4.72-4.76 (m), 6.96 (s), 7.58 (d), 7.69-7.80 (m), 8.31 (s), 8.37 (s), 8.64 (d), 9.53 (d) |
| 12 | 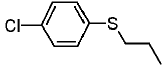 | 0.82 (t), 0.86-0.91 (m), 1.17-1.44 (m), 2.17 (s), 2.19 (s), 2.21 (s), 2.27-2.30 (m), 2.38 (s), 3.08-3.12 (m), 3.23-3.27 (m), 4.72-4.75 (m), 6.96 (s), 7.23-7.30 (m), 7.55 (d), 7.65-7.77 (m), 8.30 (s), 8.37 (s), 8.64 (d), 9.52 (dd) |
| 13 | 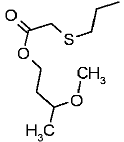 | 0.82 (t), 0.90 (t), 1.11 (d), 1.18-1.43 (m), 1.72-1.79 (m), 2.20 (s), 2.27-2.31 (m), 2.38 (s), 2.88-2.92 (m), 3.25 (d), 3.28-3.30 (m), 3.35-3.39 (m), 4.20 (t), 4.72-4.76 (m), 6.95 (s), 7.59 (d), 7.60-7.78 (m), 7.84 (dd), 8.35 (d), 8.41 (s), 8.65 (d), 9.54 (d) |
| 14 | 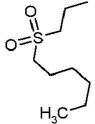 | 0.82-0.91 (m), 1.19-1.42 (m), 1.82 (m), 2.20 (s), 2.30 (s), 2.38 (s), 3.01 (t), 3.21 (t), 3.48 (t), 4.73 (m), 6.96 (s), 7.60 (d), 7.71-7.80 (m), 8.35 (s), 8.37 (s), 8.63 (d), 9.51 (s) |
| 15 | 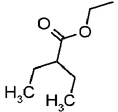 | 0.64 (t), 0.82 (t), 0.86-0.90 (m), 1.19-1.15 (m), 2.04-2.14 (m), 2.20 (s), 2.28 (s), 2.39 (s), 4.58-4.80 (m), 5.41 (s), 7.56 (s), 7.70-7.76 (m), 8.25 (d), 8.35 (s), 8.64 (d), 9.56 (dd) |
| 16 | 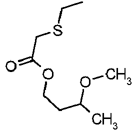 | 0.82 (t), 0.89 (t), 1.14 (d), 1.18-1.44 (m), 1.73-1.81 (m), 2.21 (s), 2.29 (s), 2.39 (s), 3.27 (d), 3.37-3.41 (m), 4.12 (s), 4.23 (t), 4.71-4.75 (m), 6.96 (s), 7.59 (d), 7.60-7.77 (m), 7.87 (dd), 8.38 (s), 8.62 (d), 9.52 (d) |
| 17 | 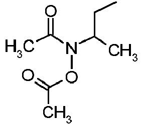 | 0.79-0.84 (m), 0.87-0.91 (m), 1.14-1.43 (m), 1.81 (s), 2.18 (s), 2.21 (s), 2.27-2.31 (m), 2.38 (s), 3.26-3.29 (m), 4.70-4.79 (m), 4.81-4.94 (m), 6.96 (s), 7.60 (d), 7.69-7.76 (m), 7.88 (d), 8.38-8.91 (m), 8.64 (d), 9.50 (d) |

### Example 18 Synthesis of {11-(2-Ethylhexyl)-8-[2-(2-methoxyethoxy)benzoyl]-11H-benzo[a]carbazole-5-yl}-(2,4,6-trimethylphenyl)-methanone oxime O-acetate

### 18.a [11-(2-Ethylhexyl)-8-(2-fluorobenzoyl)-11 H-benzo[a]carbazol-5-yl]-(2,4,6-trimethylphenyl)-methanone

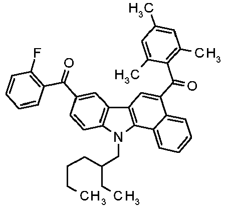

To 11-(2-ethylhexyl)-11H-benzo[a]carbazole (47.16 g; 143.0 mmol) in CH₂Cl₂ (400 mL) are added 2,4,6-trimethylbenzoyl chloride (27.45 g; 150.0 mmol) and AlCl₃ (20.00 g; 150.0 mmol) at 0°C. After stirring at room temperature for 2 hours, AlCl₃ (22.93 g; 172.0 mmol) is added at 0°C and 2-fluorobenzoyl chloride (23.78 g; 150.0 mmol) is added dropwise, and then the mixture is stirred at room temperature for 3 hours. The reaction mixture is poured into ice-water, and the crude product is extracted twice with CH₂Cl₂. The combined organic layer is washed with water and brine, dried over MgSO₄. After 230 ml of n-hexane is added there, CH₂Cl₂ was removed by concentration to give a beige solid. It is collected by filitration, and washed with n-hexane, and then it is dried to give a beige solid (81.81 g; 95.7%). The structure is confirmed by ¹H-NMR spectrum (CDCl₃). δ [ppm]: 0.84 (t), 0.89 (t), 1.18-1.42 (m), 2.20 (s), 2.28-2.31 (m), 2.40 (s), 4.72-4.76 (m), 6.96 (s), 7.19 (t), 7.29 (t), 7.52-7.60 (m), 7.70-7.84 (m), 8.38 (s), 8.63-8.66 (m), 9.56 (d); ¹⁹F-NMR spectrum (CDCl₃). δ [ppm]: -111.58

### 18.b Synthesis of [11-(2-Ethylhexyl)-5-(2,4,6-trimethylbenzoyl)-11H-benzo[a]-carbazole-8-yl}-[2-(2-methoxyethoxy)phenyl]-methanone oxime

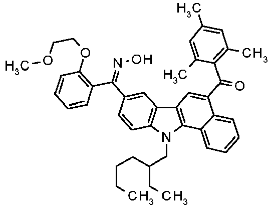

To 1 1-(2-Ethylhexyl)-8-(2-fluorobenzoyl)-11H-benzo[a]carbazole-5-yl-(2,4,6-trimethyl-phenyl)-methanone (5.98 g; 10.0 mmol) in pyridine (10 mL) are added 2-methoxyethanol (2.28 g; 30.00 mmol) and potassium *tert*-butoxide (1.68 g; 15.00 mmol) at room temeratature. The mixture is heated at 80 °C, and it is stirred for 3.5 hours. To the reaction mixture is added hydroxylammonium chloride (2.08 g; 30.00 mmol), and then the mixture is stirred at 100 °C overnight. After it is cooled at room temperature, the reaction mixture is poured into 200 ml of water. The precipitated solid is collected by filtration, and then it is washed with CH₃OH. The desired compound is obtained in 71 % yield (4.73 g) as a white solid. The structure is confirmed by ¹H-NMR spectrum (CDCl₃). δ [ppm]: 0.81 (t), 0.87 (t), 1.17-1.43 (m), 2.18 (s), 2.26-2.28 (m), 2.36 (s), 2.39 (s), 2.96 (s), 3.08 (t), 3.11 (t), 3.45 (t), 3.81 (t), 4.08 (t), 4.65-4.75 (m), 6.87 (d), 6.93 (s), 7.03-7.12 (m), 7.24-7.26 (m), 7.36-7.47 (m), 7.50-7.51 (m), 7.57 (br), 7.67-7.76 (m), 8.27 (d), 8.30 (d), 8.33 (s), 8.62 (d), 9.55 (dd), 9.59 (dd)

### 18.c Synthesis of [11-(2-Ethylhexyl)-5-(2,4,6-trimethylbenzoyl)-11H-benzo[a]carb-azole-8-yl}-[2-(2-methoxyethoxy)phenyl]-methanone oxime O-acetate

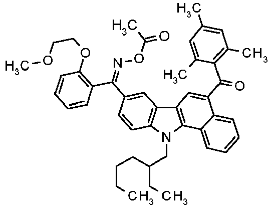

To (11-(2-Ethylhexyl)-8-{hydroxyimino-[2-(2-methoxyethoxy)phenyl]-methyl}-11H-benz-o[a]carbazole-5-yl)-(2,4,6-trimethylphenyl)-methanone (2.59 g; 3.87 mmol) in acetone (20 ml) are added triethylamine (0.78 g; 7.74 mmol) and acetyl chloride (0.61 g; 7.74 mmol) at 0 °C. The mixture is stirred for 2 hours. After the reaction is completed, the reaction mixture is added into water. Then the crude product is extracted with *tert-*butylmethyether. The organic layer is washed with water and brine, dried over MgSO₄, and concentrated to give a residue. This residue is recrystallized with *tert-*butylmethylether to give a white solid (2.00 g; 73%). The product is composed of an isomeric mixture, and the structure is confirmed by ¹H-NMR spectrum (CDCl₃). δ [ppm]: 0.81 (t), 0.87 (t), 1.16-1.43 (m), 2.06 (s), 2.10 (s), 2.18 (s), 2.19 (s), 2.27-2.32 (m), 2.38 (s), 2.94 (s), 3.08 (t), 3.14 (s), 3.22 (s), 3.45 (t), 3.82 (t), 4.05 (t), 4.69-4.74 (m), 6.89 (d), 6.94 (s), 7.05 (t), 7.14 (dd), 7.39-7.49 (m), 7.67-7.77 (m), 8.26 (s), 8.33 (s), 8.36 (s), 8.63 (d), 9.54 (dd), 9.59 (dd)

### Examples 19-41:

The corresponding diketone intermediates of the compounds of examples 19-41 are prepared with mono- or di-fluorinated benzoyl chloride. Then, the oximes and oxime esters are prepared according to the procedure as disclosed in examples 18b and 18c and the oxime esters are obtained as an isomeric mixture. The compounds and their properties are collected in the following table 2.

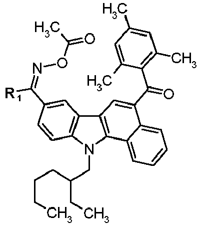

**Table 2:**

| Ex. | R₁ | ¹H-NMR spectrum (CDCl₃). δ [ppm] |
|---|---|---|
| 19 | 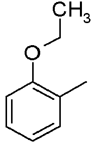 | 0.78-0.90 (m), 1.15-1.42 (m), 2.06 (s), 2.07 (s), 2.18 (s), 2.19 (s), 2.22-2.32 (m), 2.38 (s), 2.39 (s), 3.74 (q), 4.00 (q), 4.69-4.76 (m), 6.84 (d), 6.94 (s), 7.00-7.05 (m), 7.42-7.51 (m), 7.60 (d), 7.67-7.76 (m), 8.20 (s), 8.30 (s), 8.36 (s), 8.45 (s), 8.62 (d), 9.54 (d), 9.60 (d) |
| 20 | 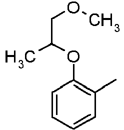 | 0.77-0.92 (m), 1.19-1.41 (m), 2.06 (s), 2.18 (s), 2.19 (s), 2.22-2.30 (m), 2.36 (s), 2.39 (s), 2.41 (s), 2.44 (s), 3.01 (d), 3.16-3.22 (m), 3.30-3.34 (m), 4.49-4.53 (m), 4.69-4.76 (m), 6.90 (d), 6.94 (s), 6.98-7.09 (m), 7.15 (dd), 7.38-7.46 (m), 7.51 (d), 7.69-7.76 (m), 8.15 (dd), 8.18 (s), 8.30 (s), 8.36 (s), 8.37 (s), 8.44 (s), 8.62 (d), 8.70 (d), 9.55 (d), 9.58 (d) |
| 21 | 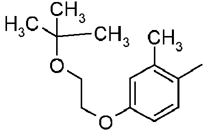 | 0.78-0.93(m), 1.17-1.54(m), 1.26(s), 2.05(s), 2.13(s), 2.19(s), 2.38(s), 3.73(t), 3.72(t), 4.09(t), 4.13(t), 4.68-4.73(m), 6.78(d), 6.83(d), 6.88(s), 6.95(s), 6.98(d), 7.31 (m), 7.43(d), 7.68(d), 7.69-7.74(m), 8.25(s), 8.29(s), 8.38(s), 8.52(s), 8.63(m), 9.50(d), 9.57(d) |
| 22 | 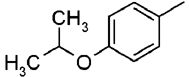 | 0.79-0.96(m), 1.17-1.45(m), 1.34(d), 1.40(d), 2.02(s), 2.14(s), 2.17(s), 2.19(s), 2.35(s), 2.36(s), 4.59-4.64(m), 4.72-4.82(m), 6.85(d), 6.93(m), 7.32(d), 7.41 (d), 7.48(d), 7.54(d), 7.61 (d), 7.68-7.76(m), 7.90(s), 8.27(s), 8.36(s), 8.37(s), 8.64(d), 8.67(d), 9.52(d), 9.56(d) |
| 23 | 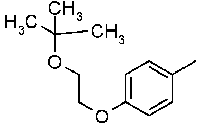 | 0.80-0.98(m), 1.23(s), 1.26(s), 1,20-1,53(m), 2.02(s), 2.13(s), 2,18(d), 2.36(d), 3.71 (t), 3,76(t), 4.10(t), 4.15(t), 6.89(d), 6.93(d), 6.99(d) 7.32(d) 7.38(d), 7.39(d), 7.46(d), 7.53(d), 7.60(d), 7.70-7.79(m), 7.89(s), 8.27(s), 8.36(s), 8.39(s), 8.63-8.67(m), 9.53(d), 9.53(m), 9.56(d) |
| 24 | 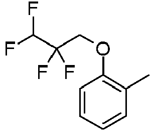 | 0.82 (t), 0.87 (t), 1.16-1.56 (m), 2.05 (s), 2.19 (s), 2.26-2.29 (m), 2.39 (s), 4.30-4.34 (t), 4.65-4.77 (m), 5.39-5.65 (tt), 6.95 (s), 7.01 (d), 7.15-7.20 (m), 7.39 (d), 7.46-7.53 (m), 7.70-7.77 (m), 8.35 (s), 8.42 (s), 8.63 (d), 9.51 (d) |
| | | ¹⁹F-NMR spectrum (CDCl₃) δ[ppm]; -140.66, -126.32 |
| 25 | 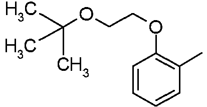 | 0.81 (t), 0.87 (t), 1.21-1.38 (m), 2.05 (s), 2.20 (s), 2.26-2.29 (m), 2.39 (s), 3.41 (t), 4.00 (t), 4.64-4.76 (m), 6.85 (s), 7.03-7.06 (m), 7.14 (d), 7.67-7.77 (m), 8.37 (s), 8.47 (s), 8.63 (d), 9.55 (d), |
| 26 | 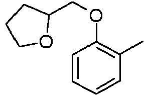 | 0.83 (t), 0.86 (t), 1.16-1.42 (m), 1.60-1.68 (m), 2.05 (s), 2.19 (s), 2.26-2.29 (m), 2.39 (s), 3.59-3.61 (m), 3.93-3.98 (m), 4.65-4.76 (m), 6.95 (s), 7.03-7.07 (m), 7.15 (dd), 7.39-7.47 (m), 7.68-7.76 (m), 8.36 (s), 8.45 (s), 8.63 (d), 9.54 (d), |
| 27 | 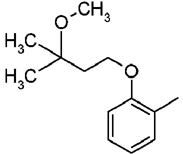 | 0.81 (t), 0.87 (t), 1.17-1.42 (m), 2.05 (s), 2.20 (s), 2.20-2.28 (m), 2.39 (s), 3.06 (s), 4.03 (t), 4.64-4.76 (m), 6.95 (s), 7.02-7.06 (m), 7.13 (d), 7.38 (d), 7.43-7.46 (m), 8.37 (s), 8.48 (s), 8.63 (d), 9.53 (d), |
| 28 | 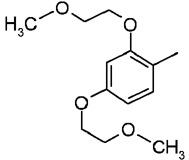 | 0.81 (t), 0.87 (t), 2.05 (s), 2.19 (s), 2.22-2.29 (m), 2.38 (s), 3.14 (s), 3.42 (t), 3.49 (s), 3.80 (t), 4.00 (t), 4.17 (t), 4.69-4.73 (m), 6.58 (d), 6.64 (s), 6.95 (s), 7.04 (d), 7.38 (d), 7.47 (d), 7.69-7.74 (m), 8.38 (s), 8.47 (s), 8.62 (d), 9.55 (d) |
| 29 | 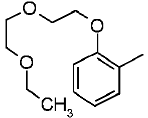 | 0.83 (t), 0.90 (t), 1.05 (t), 1.14-1.43 (m), 2.10 (s), 2.19 (s), 2.38 (s), 3.22-3.32 (m), 3.86 (t), 4.71-4.76 (m), 6.90 (d), 6.94 (s), 7.06 (td), 7.40-7.44 (m), 7.49 (s), 7.51 (s), 7.56 (t), 7.69-7.78 (m), 8.25 (d), 8.29 (d), 8.36 (s), 8.64 (d), 9.58 (dd) |
| 30 | 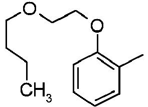 | 0.72 (t), 0.81 (t), 0.87 (t), 1.11-1.23 (m), 1.25-1.38 (m), 2.05 (s), 2.17 (s), 2.20 (s), 2.22-2.32 (m), 2.39 (s), 3.25 (t), 3.49 (t), 4.05 (t), 4.68-4.72 (m), 6.95 (s), 7.03-7.07 (m), 7.11 (dd), 7.38-7.47 (m), 7.67-7.77 (m), 8.37 (s), 8.47 (d), 8.63 (d), 9.54 (dd) |
| 31 | 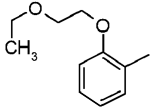 | 0.81 (t), 0.84 (t), 0.94 (t), 1.16-1.40 (m), 2.06 (s), 2.20 (s), 2.23-2.39 (m), 2.39 (s), 3.31 (q), 3.50 (t), 4.06 (t), 4.68-4.72 (m), 6.95 (s), 7.04-7.07 (m), 7.14 (dd), 7.38-7.47 (m), 7.67-7.77 (m), 8.37 (s), 8.46 (d), 8.63 (d), 9.54 (dd) |
| 32 | 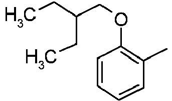 | 0.61 (t), 0.79-0.88 (t), 1.04-1.10 (m), 1.16-1.40 (m), 2.05 (s), 2.19 (s), 2.23-2.30 (m), 2.39 (s), 3.77 (br), 4.64-4.76 (m), 6.94 (s), 6.99-7.06 (m), 7.17 (dd), 7.37-7.46 (m), 7.67-7.76 (m), 8.36 (s), 8.43 (d), 8.63 (d), 9.55 (dd) |
| 33 | 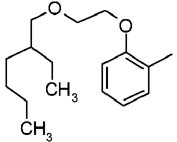 | 0.68 (t), 0.76-0.89 (m), 1.09-1.40 (m), 2.04 (s), 2.05 (s), 2.20 (s), 2.22-2.31 (m), 2.39 (s), 3.15 (dd), 3.48 (), 4.06 (t), 4.12 (q), 4.74-4.78 (m), 6.95 (s), 7.03-7.09 (m), 7.13 (dd), 7.38-7.47 (m), 7.67-7.76 (m), 8.37 (s), 8.47 (d), 8.63 (d), 9.54 (dd) |
| 34 | 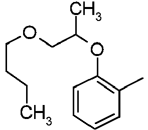 | 0.75-0.93 (m), 1.17-1.41 (m), 2.05 (s), 2.26-2.28 (m), 2.39 (s), 3.20-3.26 (m), 3.35-3.39 (m), 4.50 (q), 4.68-4.73 (m), 6.94 (s), 7.03 (td), 7.09 (d), 7.14 (d), 7.37-7.45 (m), 7.67-7.77 (m), 8.36 (s), 8.45 (d), 8.63 (d), 9.55 (dd) |
| 35 | 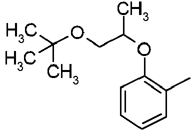 | 0.78-0.89 (m), 1.15-1.41 (m), 2.05 (s), 2.19 (s), 2.27-2.28 (m), 2.39 (s), 3.06-3.09 (m), 3.30 (br), 4.42 (q), 4.69-4.72 (m), 6.95 (s), 7.02 (t), 7.08-7.15 (m), 7.37-7.45 (m), 7.67-7.76 (m), 8.36 (s), 8.45 (d), 8.62 (d), 9.55 (dd) |
| 36 | 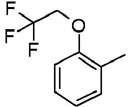 | 0.80 (t), 0.87 (t), 1.16-1.41 (m), 2.07 (s), 2.10 (s), 2.19 (s), 2.25-2.28 (m), 2.38 (s), 4.32 (q), 4.69-4.72 (m), 6.94 (s), 7.05 (d), 7.15 (s), 7.17 (s), 7.19-7.20 (m), 7.22 (d), 7.26-7.27 (m), 7.41-7.52 (m), 7.67-7.76 (m), 8.15 (d), 8.29 (s), 8.35 (s), 8.41 (s), 8.63 (d), 9.52 (dd), 9.57 (dd) |
| | | ¹⁹F-NMR spectrum (CDCl₃) δ[ppm]; -73.89 |
| 37 | 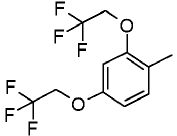 | 0.81 (t), 0.87 (t), 1.18-1.40 (m), 2.05 (s), 2.09 (s), 2.19 (s), 2.22-2.32 (m), 2.38 (s), 4.30 (q), 4.43 (q), 4.6-4.73 (m), 6.68 (s), 6.70 (d), 6.94 (s), 7.16 (d), 7.42-7.49 (m), 7.69-7.76 (m), 8.36 (s), 8.38 (s), 8.63 (d), 9.49 (d) |
| | | ¹⁹F-NMR spectrum (CDCl₃) δ[ppm]; -73.80, -73.78 |
| 38 | 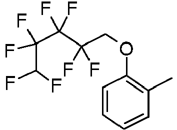 | 0.80 (t), 0.86 (t), 1.16-1.42 (m), 2.04 (s), 2.05 (s), 2.19 (s), 2.25-2.29 (m), 2.38 (t), 4.40-4.46 (m), 4.64-4.74 (m), 5.76-6.02 (m), 6.94 (s), 7.04 (d), 7.18-7.20 (m), 7.38 (dd), 7.45 (s), 7.47-7.53 (m), 7.67-7.76 (m), 8.36 (s), 8.42 (d), 8.62 (d), 9.52 (dd) |
| | | ¹⁹F-NMR spectrum (CDCl₃) δ[ppm]; 137.43, 130.40, 125.42, 119.68 |
| 39 | 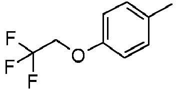 | 0.79-0.90 (m), 0.94 (t), 1.18-1.47 (m), 2.04 (s), 2.13 (s), 2.19 (d), 2.24-2.32 (m), 2.36 (d), 4.35-4.47 (m), 4.72-4.78 (m), 6.93 (d), 7.03 (d), 7.37-7.41 (m), 7.49 (d), 7.59-7.64 (m), 7.70-7.77 (m), 7.92 (s), 8.28 (s), 8.36-8.38 (m), 8.63-8.68 (m), 9.50-9.56 (m) |
| | | ¹⁹F-NMR spectrum (CDCl₃) δ[ppm]; -73.85 |
| 40 | 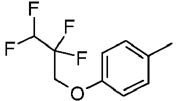 | 0.79-0.90 (m), 0.93-0.96 (m), 1.18-1.47 (m), 2.03 (s), 2.13 (s), 2.18 (s), 2.20 (s), 2.35 (s), 2.37 (s), 4.35-4.37 (m), 4.67-4.78 (m), 5.92-6.28 (m), 6.91-6.94 (m), 7.20 (d), 7.35-7.41 (m), 7.48 (d), 7.59-7.64 (m), 7.7-7.79 (m), 7.92 (s), 8.28 (s), 8.38 (s), 8.63-8.68 (m), 9.51 (d), 9.55 (d) |
| | | ¹⁹F-NMR spectrum (CDCl₃) δ[ppm]; -139.12, -124.80 |
| 41 | 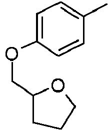 | 0.80 (t), 0.88 (t), 1.18-1.41 (m), 1.78-1.83 (m), 1.96-2.04 (m), 2.12 (s), 2.19 (s), 2.26-2.29 (m), 2.35 (s), 2.37 (s), 3.84 (q), 3.99 (q), 4.29-4.35 (m), 4.70-4.75 (m), 6.88-6.92 (m), 6.94 (s), 6.98 (dd), 7.31-7.36 (m), 7.47 (d), 7.55 (d), 7.67-7.76 (m), 7.89 (d), 8.27 (s), 8.37 (s), 8.41 (s), 8.63 (d), 9.52 (dd) |

### Examples 42-60:

The substituents on the N atom of the cmpounds of examples 42-52 are intoduced with the corresponding haloalkane or haloalkylether instead of 1-bromide-2-ethylhexane according to the procedure as disclosed in example 1.a. A substituent on the N atom of the compound of example 53 is introduced by reaction with 1-bromo-3-chloropropane followed by ethyl thioglycolate. A substituent on the N atom of the compound of example 54 is introduced by reaction with 1-bromo-6-chlorohexane followed by triethyl phosphite. Substituents on the N atom of the compounds of examples 55 and 56 are introduced by reaction with glycidyl isopropyl ehter followed by acetyl chloride. In case of the compound of example 57, 11-phenyl-11*H*-benzo[a]carbazole is prepared by replacment of phenylhydrazine with diphenylhydrazine according to the procedure as described in SYNLETT, 2006, 7, 1021 mentioned in example 1.a. A substituent on the N atom of the compound of example 58 is introduced with bromo ethanol. The following diketones, oxime, and oxime esters are prepared according to the procedure as disclosed in example 18. The compound of example 59 is prepared by reacting example 58 with acryloyl chloride. The compounds of examples 42-53 are obtained as an isomeric mixture, and the others are obtained as a single component. The compounds and their properties are collected in the following table 3.

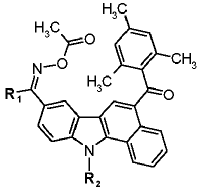

**Table 3:**

| Ex. | R1 | R2 | ¹H-NMR spectrum (CDCl₃). δ [ppm] |
|---|---|---|---|
| 42 | 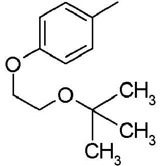 | 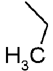 | 0.81 (t), 0.89 (t), 1.12-1.39 (m), 1.57-1.62 (m), 1.80-1.89 (m), 2.20 (s), 2.27-2.30 (m), 2.38 (s), 2.93-2.97 (m), 4.68-4.79 (m), 6.96 (s), 7.57 (d), 7.68-7.77 (m), 7.82 (d), 8.29 (s), 8.37 (s), 8.64 (d), 9.55 (d) |
| 43 | 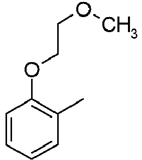 | 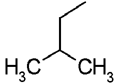 | 0.82 (t), 0.89 (t), 1.21-1.42 (m), 2.21 (s), 2.27-2.28 (m), 2.40 (s), 2.60 (t), 3.26 (t), 4.10 (q), 4.72-4.76 (m), 6.96 (s), 7.58 (d), 7.69-7.79 (m), 8.30 (s), 8.37 (s), 8.64 (d), 9.53 (d) |
| 44 | 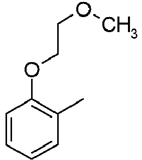 | 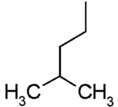 | 1.10-1.14 (m), 1.89-2.00 (m), 2.06 (s), 2.10 (s), 2.18 (s), 2.19 (s), 2.38 (s), 2.94 (s), 3.13-3.15 (m), 3.47 (t), 3.83 (t), 4.06 (t), 4.77-4.81 (m), 6.95 (s), 7.03-7.07 (m), 7.13 (d), 7.40-7.45 (m), 7.72-7.78 (m), 8.23 (s), 8.31 (s), 8.37 (s), 8.51 (s), 8.57 (d), 9.56 (d), 9.60 (d) |
| 45 | 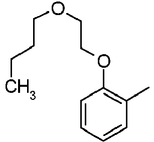 | 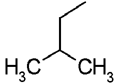 | 0.71 (t), 9.87-0.88 (m), 1.01 (d), 1.05 (d), 1.10-1.14 (m), 1.25-1.32 (m), 2.05 (s), 2.10 (s), 2.18 (s), 2.19 (s), 2.39 (s), 2.51-2.57 (m), 3.06 (t), 3.15 (t), 3.21-3.26 (m), 3.49 (t), 3.83 (t), 4.05 (t), 4.60-4.65 (m), 6.95 (s), 7.03-7.07 (m), 7.14 (dd), 7.38-7.47 (m), 7.51 (d), 7.69-7.76 (m), 8.25 (d), 8.31 (s), 8.37 (s), 8.47 (d), 8.55 (dd), 9.55 (dd), 9.60 (dd) |
| 46 | 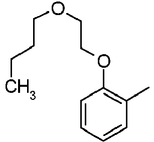 | 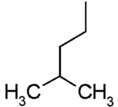 | 0.70 (t), 1.11 (t), 1.26-1.33 (m), 1.89-2.00 (m), 2.05 (s), 2.19 (s), 2.38 (s), 3.24 (t), 3.51 (t), 4.06 (t), 4.79 (t), 6.95 (s), 7.03-7.07 (m), 7.13 (dd), 7.38-7.47 (m), 7.70-7.78 (m), 8.38 (s), 8.52 (d), 8.57 (d), 9.56 (dd) |
| 47 | 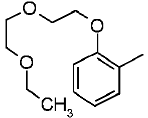 | 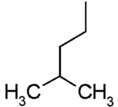 | 1.06 (t), 1.11 (t), 1.89-2.01 (m), 2.05 (s), 2.19 (s), 2.38 (s), 3.27-3.30 (m), 3.32 (d), 3.40-3.42 (m), 3.61 (t), 4.09 (t), 4.39 (t), 6.95 (t), 7.03-7.07 (m), 7.12 (dd), 7.38 (dd), 7.42-7.47 (m), 7.70-7.78 (m), 8.38 (s), 8.53 (s), 8.57 (dd), 9.56 (dd) |
| 48 | 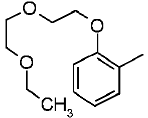 | 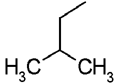 | 1.01 (t), 1.03-1.09 (m), 2.05 (s), 2,10 (s), 2.18 (s), 2.38 (s), 2.53-2.56 (m), 3.22 (s), 3.23-3.26 (m), 3.29-3.36 (m), 3.41-3.43 (m), 3.59 (t), 3.86 (t), 4.09 (t), 4.61 (d), 6.95 (s), 7.03-7.07 (m), 7.13 (dd), 7.39 (dd), 7.42-7.47 (m), 7.70-7.77 (m), 8.30 (s), 8.37 (s), 8.47 (d), 8.55 (dd), 9.53 (dd), 9.60 (dd) |
| 49 | 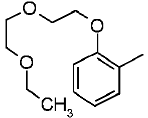 | 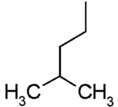 | 1.12 (d), 1.18-1.19 (m), 1.21-1.25 (m), 1.90-2.08 (m), 2.13 (s), 2.17 (s), 2.19 (s), 2.35 (s), 2.37 (s), 3.52-3.57 (m), 3.61-3.66 (m), 3.71-3.77 (m), 3.87 (t), 3.92 (t), 6.89 (d), 6.93 (d), 6.98 (dd), 7.31-7.36 (m), 7.42 (dd), 7.46 (d), 7.54 (dd), 7.61 (d), 7.70-7.79 (m), 7.89 (d), 8.27 (s), 8.38 (s), 8.44 (d), 8.57-8.62 (m), 9.55-9.60 (m) |
| 50 | 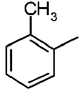 | 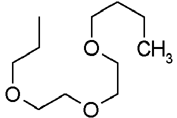 | 0.86 (t), 1.29 (m), 1.50 (m), 2.04 (s), 2.17 (s), 2.19 (s), 2.38 (s), 3.37 (t), 3.45 (t), 3.51 (t), 3.56 (t), 3.63 (t), 4.13 (t), 5.00 (t), 6.95 (s), 7.11 (d), 7.26-7.38 (m), 7.54 (d), 7.14-7.75 (m), 8.37 (s), 8.55 (s), 8.63 (d), 9.52 (d) |
| 51 | 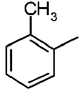 | 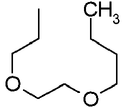 | 0.85 (t), 1.28 (m), 1.47 (m), 2.03 (s), 2.17 (s), 2.19 (s), 2.38 (s), 3.35 (t), 3.50 (t), 3.60 (t), 4.15 (t), 5.00 (t), 6.95 (s), 7.11 (d), 7.26-7.38 (m), 7.55 (d), 7.71-7.76 (m), 8.37 (s), 8.55 (s), 8.61 (d), 9.52 (d) |
| 52 | 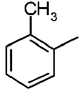 | 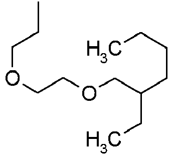 | 0.78 (t), 0.82-0.86 (m), 1.17-1.31 (m), 1.43-1.46 (m), 2.03 (s), 2.17 (s), 2.19 (s), 2.39 (s), 3.23-3.25 (m), 3.48-3.50 (m), 3.61-3.63 (m), 4.16 (t), 4.99 (t),6.95 (t), 7.12 (dd), 7.26-7.36 (m), 7.37-7.41 (m), 7.55 (d), 7.69-7.78 (m), 8.37 (s), 8.56 (s), 8.63 (dd), 9.53 (dd) |
| 53 | 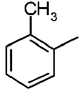 | 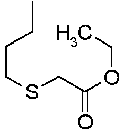 | 1.23 (t), 2.18 (s), 2.23 (s), 2.36 (m), 2.40 (s), 2.81 (t), 4.11 (q), 4.93 (t), 6.95 (s), 7.17 (d), 7.21-7.43 (m), 7.51 (d), 7.70-7.78 (m), 8.07(d), 8.37 (s), 8.57 (s), 8.61 (d), 9.52 (d) |
| 54 | 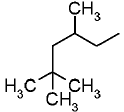 | 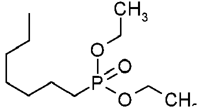 | 0.86 (s), 0.89 (d), 1.20-1.36 (m), 1.49-1.73 (m), 1.90 (m), 2.11 (m), 2.19 (s), 2.26 (s), 2.38 (s), 2.92 (d), 4.07 (m), 4.81 (t), 6.95 (s), 7.56 (d), 7.73-7.78 (m), 7.88 (d), 8.27 (s), 8.35 (s), 8.54 (d), 9.56 (d) |
| 55 | 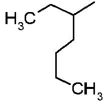 | 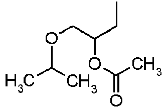 | 0.87-1.21 (m), 1.26-1.77 (m), 1.92 (s), 2.19 (s), 2.23 (s), 2.31 (s), 2.75 (m), 3.28 (m), 3.65-3.70 (m), 5.01-5.29 (m), 5.64 (m), 6.95 (s), 7.29 (d), 7.62 (d), 7.64-7.78 (m), 8.09 (s), 8.27 (s), 8.32 (s), 8.85-8.89 (m), 9.51-9.55 (m) |
| 56 | 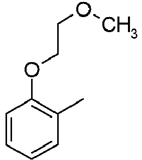 | 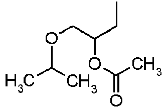 | 1.20-1.29 (m), 1.58 (s), 1.74 (s), 2.04 (s), 2.08 (s), 2.18 (s), 2,38 (s), 2.93 (s), 3.14 (m), 3.46 (t), 3.62-3.71 (m), 3.82 (t), 4.05 (t), 4.95-5.29 (m), 5.63 (m), 6.87 (d), 6.93 (s), 7.02-7.08 (m), 7.12 (d), 7.40-7.45 (m), 7.56-7.75 (m), 8.20 (s), 8.28 (s), 8.32 (s), 8.40 (s), 8.85-8.90 (m), 9.50-9.56 (m) |
| 57 | 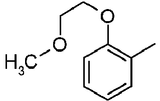 | 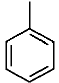 | 2.05 (s), 2.22 (s), 2.40 (s), 3.15 (s), 3.17 (s), 3.48 (t), 4.05 (t), 6.97-7.04 (m), 7.11 (dd), 7.23 (dd), 7.27-7.31 (m), 7.40-7.44 (m), 7.50-7.52 (m), 7.61-7.68 (m), 8.44 (s), 8.59 (d), 9.47 (d) |
| 58 | 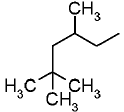 | 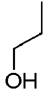 | 0.86 (s), 0.88 (d), 1.20-1.35 (m), 1.76 (m), 1.89 (m), 2.19 (s), 2.26 (s), 2.38 (s), 2.92 (d), 4.37 (t), 5.06 (t), 6.95 (s), 7.67-7.78 (m), 7.87 (d), 8.27 (s), 8.35 (s), 8.64 (d), 9.55 (d) |
| 59 | 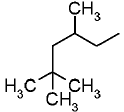 | 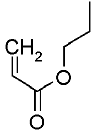 | 0.85 (s), 0.89 (d), 1.24-1.35 (m), 1.86 (m), 2.19 (s), 2.26 (s), 2.38 (s), 2.92 (d), 4.81 (t), 5.17 (t), 5.81 (d), 5.98-6.10 (m), 6.25 (d), 6.95 (s), 7.65 (d), 7.38-7.79 (m), 7.88 (d), 8.28 (s), 8.35 (s), 8.64 (d), 9.54 (d) |

### Examples 60-68:

In case of the compound of example 60, the corresponding diketone intermediate is prepared according to the procedure as disclosed in example 1.b with thienoyl choride and ethyl succinyl chloride instead of 2,4,6-trimethylbenzoyl choride and acetyl chloride. Then, the oxime and the oxime ester are prepared according to the procedure as disclosed in example 1.c. In case of the compounds of examples 61 and 62, the corresponding diketone intermediates are prepared according to the procedure as disclosed in example 18.a with 1-naphthoyl chloride or 2,6-dimethoxybenozyl chloride instead of 2,4,6-trimethylbenzoyl chloride, and in case of the compounds of examples 63-68, the corresponding diketone intermediates are prepared according to the procedure as disclosed in example 18.a with 4-fluorobenzoyl chloride instead of 2,4,6-trimethylbenzoyl chloride.Then, the oximes and the oxime esters are prepared according to the procedure as disclosed in examples 18.b and 18.c. The compounds of examples 61 and 62 are obtained as an isomeric mixture, and the others are obtained as a single component. The compounds and their properties are collected in the following table 4.

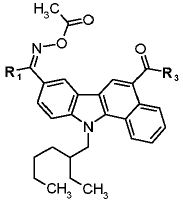

**Table 4:**

| Ex. | R₁ | R₃ | ¹H-NMR spectrum (CDCl₃). δ [ppm] |
|---|---|---|---|
| 60 | 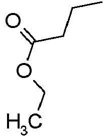 | 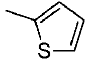 | 0.83-0.89 (m), 1.21-1.45 (m), 2.29 (m), 2.65 (t), 3.29 (t), 4.11 (q), 4.75 (m), 7.18 (t), 7.58-7.68 (m), 7.78 (d), 7.91 (d), 8.45 (s), 8.53-5.55 (m), 8.62 (d) |
| 61 | 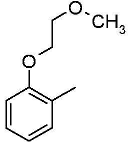 | 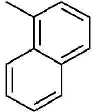 | 0.82-0.93 (m), 1.20-1.44 (m), 2.03 (s), 2.28-2.30 (m), 3.13 (s), 3.21 (s), 3.43 (t), 4.02 (t), 4.69-4.80 (m), 7.00-7.04 (m), 7.12 (dd), 7.40 (td), 7.45-7.50 (m), 7.53-7.60 (m), 7.62-7.71 (m), 7.96-7.98 (m), 8.04 (d), 8.32 (s), 8.07 (s), 8.37 (s), 8.51-8.54 (m), 8.65 (d), 8.95 (d) |
| 62 | 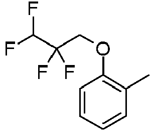 | 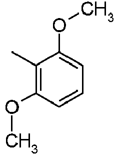 | 0.81 (t), 0.86 (t), 1.17-1.42 (m), 2.05 (s), 2.21-2.28 (m), 3.71 (s), 4.27-4.32 (m), 4.64-4.76 (m), 5.39-5.68 (m), 6.68 (d), 7.01 (d), 7.15-7.22 (m), 7.36-7.42 (m), 7.46-7.55 (m), 7.65-7.74 (m), 8.39 (d), 8.48 (s), 8.60 (d), 9.61 (dd) |
| | | | ¹⁹F-NMR spectrum (CDCl₃) δ[ppm]; -140.52, - 125.68 |
| 63 | 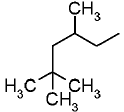 | 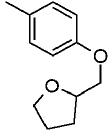 | 0.84-0.92 (m), 1.21-1.42 (m), 1.82 (m), 1.93-2.10 (m), 2.27 (m), 2.86-2.95 (m), 3.84 (q), 3.94 (q), 4.07 (d), 4.31 (m), 4.76 (m), 6.99 (d), 7.56 (t), 7.59 (d), 7.64 (t), 7.88 (d), 7.93 (d), 8.31 (s), 8.38 (s), 8.43 (d), 8.62 (d) |
| 64 | 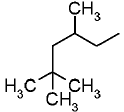 | 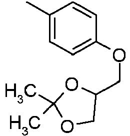 | 0.74-0.92 (m), 1.21-1.477 (m), 1.93 (m), 2.26 (m), 2.86-2.95 (m), 3.91-4.21 (m), 4.52 (m), 4.74 (m), 6.99 (d), 7.55-7.60 (m), 7.65 (t), 7.87 (d), 7.94 (d), 8.31 (s), 8.43 (s), 8.44 (d), 8.62 (d) |
| 65 | 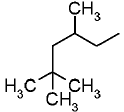 | 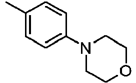 | 0.83-0.94 (m), 1.94 (m), 2.27 (m), 2.84-3.00 (m), 3.36 (t), 3.88 (t), 4.75 (m), 6.92 (d), 7.53-7.66 (m), 7.87 (d), 7.91 (d), 8.30 (s), 8.40-8.43 (m), 8.62 (d) |
| 66 | 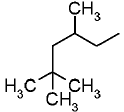 | 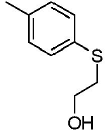 | 0.84-0.91 (m), 1.21-1.50 (m), 1.93 (m), 2.14 (t), 2.26 (m), 2.86-3.00 (m), 3.25 (t), 3.88 (q), 4.75 (m), 7.42 (d), 7.57-7.61 (m), 7.66 (t), 7.84-7.88 (m), 8.32 (s), 8.40 (s), 8.52 (d), 8.62 (d) |
| 67 | 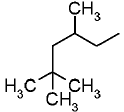 | 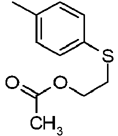 | 0.75-0.91 (m), 1.09-1.50 (m), 1.93 (m), 2.06 (s), 2.27 (m), 2.90-2.95 (m), 3.27 (t), 4.32 (t), 4.74 (m), 7.42 (d), 7.54-7.68 (m), 7.88 (d), 8.34 (s), 8.39 (s), 8.50 (d), 8.62 (d) |
| 68 | 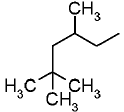 | 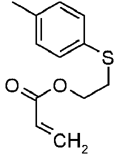 | 0.84-0.91 (m), 1.21-1.50 (m), 1.93 (m), 2.26 (m), 2.86-2.97 (m), 3.31 (t), 4.41 (t), 4.75 (m), 5.86 (d), 6.12 (m), 6.42 (d), 7.43 (d), 7.56-7.60 (m), 7.66 (t), 7.88 (d), 8.32 (s), 8.39 (s), 8.48 (d), 8.63 (d) |

### Example 69 Synthesis of [11-(2-Ethylhexyl)-3-methoxy-5-(2,4,6-trimethylbenzoyl)-11H-benzo[a]carbazole-8-yl]-o-tolyl-methanone oxime O-acetate

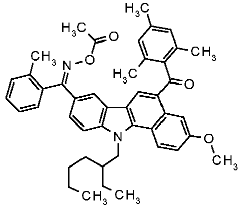

The intermediate, 11-(2-ethylhexyl)-3-methoxy-11 H-benzo[a]carbazole, is prepared according to the procedure disclosed in example 1.a with 6-methoxy-1-tetralone instead of α-tetralone. The next reactions are conducted according to the procedure of example 2. The product is composed of an isomeric mixture, and the structure is confirmed by ¹H-NMR spectrum (CDCl₃). δ [ppm]: 0.79 (t), 0.84-0.90 (m), 1.15-1.43 (m), 2.03 (s), 2.13 (s), 2.17 (s), 2.19 (s), 2.22-2.38 (m), 2.39 (s), 4.03 (s), 4.05 (s), 4.6-4.66 (m), 6.95 (s), 7.12 'd), 7.29-7.42 (m), 8.32 (s), 8.38 (s), 8.46 (s), 8.52 (d), 9.12 (d), 9.20 (d).

### Example 70 Synthesis of (13-(2-Ethylhexyl)-5-(2,4,6-trimethylbenzoyl)-13H-dibenzo[a,i]carbazole-8-yl]-[4-(2,2,3,3-tetrafluoropropoxy)-phenyl]-methanone oxime O-acetate

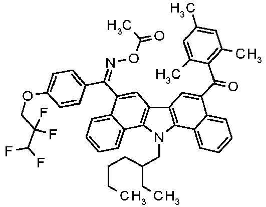

The intermediate, 13-(2-ethylhexyl)-13H-dibenzo[a,i]carbazole, is prepared according to the procedure disclosed in example 1.a with 1-naphthylhydrazine hydrochloride instead of phenylhydrazine. The next reactions are conducted according to the procedure as disclosed in example 18. The product is composed of an isomeric mixture, and the structure is confirmed by ¹H-NMR spectrum (CDCl₃). δ [ppm]: 0.53-0.61 (m), 0.87-0.90 (m), 1.24-1.32 (m), 1.79 (d), 2.04 (s), 2.20 (s), 2.21 (s), 2.27 (s), 2.36 (s), 2.37 (s), 4.31-4.37 (d), 5.22-5.29 (m), 5.88-6.18 (m), 6.87-6.96 (m), 7.50-7.52 (m), 7.57 (d), 7.64-7.80 (m), 8.19 (s), 8.25 (s), 8.34 (s), 8.58 (d), 8.64-8.68 (m), 9.44-9.46 (m); ¹⁹F-NMR spectrum (CDCl₃). δ [ppm]: -139.12, -124.78.

### Example 71 Synthesis of [11-(2-Ethylhexyl)-5-(2,4,6-trimethylbenzoyl)-11H-benzo[a]carbazole-8-yl}-[2-(2,2,3,3,-tetrafluoropropoxy)-phenyl]-methanone oxime O-thiophene-2-carboxylate

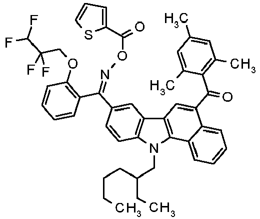

The corresponding oxime is prepared according to the procedure as disclosed in example 18 with 2,2,3,3-tetrafluoro-1-propanol instead of 2-methoxyethanol, and then the oxime ester is prepared according to the same procedure. The product is composed of an isomeric mixture, and the structure is confirmed by ¹H-NMR spectrum (CDCl₃). δ [ppm]: 0.81 (t), 0.85-0.92 (m), 1.19-1.41 (m), 2.16 (s), 2.20 (s), 2.30 (s), 2.39 (s), 4.27 (t), 4.71-4.79 (m), 5.32-5.62 (m), 6.86 (s), 6.95 (s), 7.04-7.06 (m), 7.20-7.30 (m), 7.46-7.62 (m), 7.70-7.77 (m), 8.24 (s), 8.31 (s), 8.39 (s), 8.52 (s), 8.64 (d), 9.51 (d); ¹⁹F-NMR spectrum (CDCl₃). δ [ppm]: -140.95, -126.32.

### Example 72 Synthesis of [5-(4,5-Diphenyl-1 H-imidazol-2-yl)-11-(2-ethylhexyl)-11 H-benzo-8-yl]-[2-(2,2,3,3-tetrafluoropropoxy)-phenyl]-methanone oxime O-acetate

### 72.a Synthesis of 11-(Ethylhexyl)-11H-benzo[a]carbazole-5-carbaldehyde

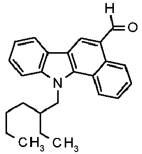

To 11H-benzo[a]carbazole (3.00 g, 9.12 mmol) in DMF (7.02 g, 96.1 mmol) is added phosphoryl chloride (14.0 g, 91.3 mmol) at 0°C. After stirring for 2 hours, the mixture is heated at 130°C, and it is stirred for 1 hour. The mixture is cooled at room temperature, and poured into ice-water. The crude product is extracted with CH₂Cl₂, and it is washed with water and brine. The organic layer is dried over MgSO₄. After concentration, the crude product is purified by column chromatography on silica gel eluting with CH₂Cl₂ to obtain an off-white solid (1.43g, 43%). The structure is confirmed by ¹H-NMR spectrum (CDCl₃). δ [ppm]: 0.80-0.90 (m), 1.21-1.46 (m), 2.25 (m), 4.68 (m), 7.40 (t), 7.52-7.60 (m), 7.66-7.74 (m), 8.19 (s), 8.61 (d), 8.64 (s), 9.65 (d), 10.39 (s).

### 72.b Synthesis of 11-(2-Ethylhexyl)-8-(2-fluorobenzoyl)-11 H-benzo[a]carbazole-5-carbaldehyde

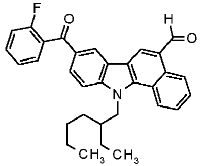

To 11-(2-ethylhexyl)-11H-benzo[a]carbazole-5-carbaldehyde (1.40 g, 3.91 mmol) in CH₂Cl₂ (20 ml) are added AlCl₃ (1.21 g, 9.07 mmol) and 2-fluorobenzoyl chloride (0.70g, 4.41 mmol) at 0°C. After stirring at room temperature for 17 hours, the reaction mixture is poured into ice-water. The crude product is extracted with CH₂Cl₂ and washed with water and brine. After drying over MgSO₄ and concentration, the crude product is purified by column chromatography on silica gel eluting with CH₂Cl₂ to obtain an off-white solid (1.76 g, 93%). The structure is confirmed by ¹H-NMR spectrum (CDCl₃). δ [ppm]: 0.82-0.91 (m), 1.17-1.43 (m), 2.24 (m), 4.69 (m), 7.25 (t), 7.33 (t), 7.57-7.77 (m), 8.12 (d), 8.57 (d), 8.60 (s), 8.64 (s), 9.63 (d), 10.38 (s); ¹⁹F-NMR spectrum (CDCl₃). δ [ppm]-111.97.

### 72.c Synthesis of 11-(2-Ethylhexyl)-8-[2-(2,2,3,3-tetrafluoropropoxy)-benzoyl]-11 H-benzo[a]carbazole-5-carbaldehyde

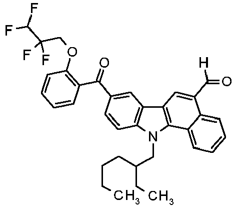

To 11-(2-ethylhexyl)-8-(2-fluorobenzoyl)-11H-benzo[a]carbazole-5-carbaldehyde (1.73 g, 3.60 mmol) in pyridine (18 ml) are added 2,2,3,3-tetrafluoropropanol (0.72 g, 5.48 mmol) and sodium hydroxide (0.24 g, 6.00mmol) at room temperature. The reaction mixture is heated at 70°C. After stirring for 3 hours, the reaction mixture is poured into ice-water. The crude product is extracted with CH₂Cl₂ and washed with water and brine. After drying over MgSO₄ and concentration, the product is reprecipitated from hexane and ethylacetate to obtain an off-white solid (1.91 g, 89%). The structure is confirmed by ¹H-NMR spectrum (CDCl₃). δ [ppm]: 0.83-0.89 (m), 1.19-1.51 (m), 2.26 (m), 4.32 (t), 4.72 (m), 5.29 (t), 7.03 (d), 7.26 (t), 7.56-7.61 (m), 7.69-7.78 (m), 8.07 (d), 8.58 (d), 8.60 (s), 8.61 (s), 10.39 (s) ; ¹⁹F-NMR spectrum (CDCl₃). δ [ppm] -140.92, -126.30.

### 72.d Synthesis of [5-(4,5-Diphenyl-1 H-imidazol-2-yl)-11-(2-ethylhexyl)-11 H-benzo[a]carbazol-8-yl]-[2-(2,2,3,3-tetrafluoropropoxy)-phenyl]-methanone

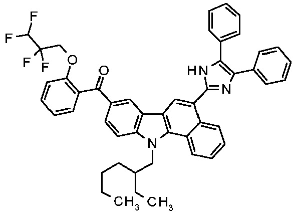

The mixture of 11-(2-ethylhexyl)-8-[2-(2,2,3,3-tetrafluoropropoxy)-benzoyl]-11H-benzo-[a]carbazole-5-carbaldehyde (1.00 g, 1.70 mmol), benzil (0.83 g, 1.82 mmol), and ammonium acetate (2.67 g, 34.6 mmol) in acetic acid (30 ml) is stirred for 3 hours at 130°C. After cooling at room temperature, the reaction mixture is poured into ice-water. The crude product is extracted with CH₂Cl₂ and washed with water and brine. After drying over MgSO₄ and concentration, the crude product is purified by column chromatography on silica gel eluting with CH₂Cl₂ to obtain yellow resin (1.25g, 94%). The structure is confirmed by ¹H-NMR spectrum (CDCl₃). δ [ppm]: 0.83-0.90 (m), 1.19-1.50 (m), 2.27 (m), 4.29 (m), 5.26 (t), 7.00 (d), 7.18 (t), 7.26-7.41 (br), 7.51-7.69 (m), 7.70-7.80 (br), 8.06 (d), 8.39 (s), 8.49 (s), 8.60 (d), 8.89 (d), 9.37 (br); ¹⁹F-NMR spectrum (CDCl₃). δ [ppm] -141.18, -126.49.

### 72.e Synthesis of [5-(4,5-Diphenyl-1H-imidazol-2-yl)-11-(2-ethylhexyl)-11H-benzo[a]-carbazol-8-yl]-[2-(2,2,3,3-tetrafluoropropoxy)-phenyl]-methanone oxime O-acetate

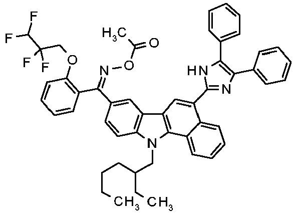

The mixture of [5-(4,5-diphenyl-1H-imidazol-2-yl)-11-(2-ethylhexyl)-11H-benzo[a]carb-azol-8-yl]-[2-(2,2,3,3-tetrafluoropropoxy)-phenyl]-methanone (1.00 g, 1.28 mmol) and hydroxyl ammonium chloride (0.18g, 2.63 mmol) in pyridine (7 ml) is stirred for 20 hours at 120°C. After cooling at room temperature, the reaction mixture is poured into ice-water. The crude product is extracted with CH₃COOC₂H₅ (AcOEt), and washed with water and brine. After drying over MgSO₄ and concentration, the obtained solid is dissolved in tetrahydrofurane (THF) (12 ml). To the solution are added triethylamine (0.17 g, 1.70 mmol) and acetyl chloride (0.13 g, 1.70 mmol) at room temperature. After stirring for 20 hours, the reaction mixture is poured into ice-water. The crude is extracted with AcOEt, and washed with water and brine. After drying over MgSO₄ and concentration, the crude is purified by column chromatography on silica gel eluting with a mixted solvent of n-hexane and AcOEt (2:1) to obtain yellow resin (340 mg, 33%). The product is composed of an isomeric mixture, and the structure is confirmed by ¹H-NMR spectrum (CDCl₃). δ [ppm]: 0.84-0.88 (m), 1.19-1.41 (m), 2.03 (s), 2.22 (m), 4.68 (m), 5.45 (m), 6.98 (d), 7.15-7.40 (m), 7.41-7.75 (m), 7.86 (d), 8.09 (s), 8.27 (s), 8.54 (d), 8.84 (d), 9.83 (br) ; ¹⁹F-NMR spectrum (CDCl₃). δ [ppm] -140.91, -126.30.

### Example 73 Synthesis of 1-[11-(2-Ethylhexyl)-5-(2,4,6-trimethylbenzoyl)-11H-benzo[a]carbazole-8-yl]-2-phenyl ethane-1,2-dione 2-oxime O-acetate

### 73.a Synthesis of 1-[11-(2-Ethylhexyl)-5-(2,4,6-trimethylbenzoyl)-11H-benzo[a]carbazol-8-yl]-2-phenyl ethanone

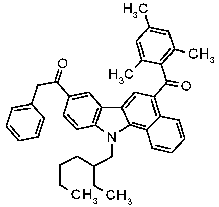

To 11 H-benzo[a]carbazole (1.06 g, 3.23 mmol) in CH₂Cl₂ (5 ml) are added AlCl₃ (0.510 g, 3.82 mmol) and 2,4,6-trimethylbenzoyl chloride (0.59 g, 3.24 mmol) at 0°C. After stirring at room temperature for 1 hour, AlCl₃ (0.51 g, 3.82 mmol) and phenylacetyl chloride (0.52 g, 3.39 mmol) are added at 0°C. The reaction mixture is stirred at room temperature for 2 hours, and then it is poured into ice-water. The crude product is extracted with CH₂Cl₂, and washed with water and brine. After drying over MgSO₄ and concentration, the product is reprecipitated from CH₂Cl₂ and n-hexane to obtain a white powder (1.53 g, 81%). The structure is confirmed by ¹H-NMR spectrum (CDCl₃). δ [ppm]: 0.79-0.91 (m), 1.18-1.50 (m), 2.19 (s), 2.28 (m), 2.41 (s), 4.38 (s), 4.74 (m), 6.97 (s), 7.22-7.36 (m), 7.56 (d), 7.71 (t), 7.77 (t), 8.14 (d), 8.35 (s), 8.62 (d), 8.63 (s), 9.58 (s).

### 73.b Synthesis of 1-[11-(2-Ehtylhexyl)-5-(2,4,6-trimethylbenzoyl)-11H-benzo[a]carb-azol-8-yl]-2-phenyl ethane-1,2-dione 2-oxime

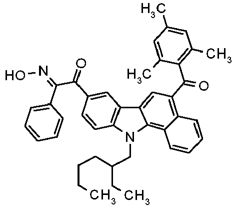

The mixture of 1-[11-(2-Ethylhexyl)-5-(2,4,6-trimethylbenzoyl)-11 H-benzo[a]carbazol-8-yl]-2-phenyl ethanone (1.20 g, 2.03 mmol) and isoamyl nitrite (0.50 g, 4.26 mmol) in THF (6 ml) is bubbled with HCl gas for 1 minute. After stirring at room temperature for 4 hours, the reaction mixture is poured into ice-water. The crude product is extracted with CH₂Cl₂, and washed with water and brine. After drying over MgSO₄ and concentration, the crude is purified by column chromatography on silica gel eluting with a mixted solvent of n-hexane and CH₂Cl₂ (1:1) to obtain an off-white resin (0.69 g, 50%). The structure is confirmed by ¹H-NMR spectrum (CDCl₃) δ [ppm]: 0.79-0.90 (m), 1.19-1.45 (m), 2.19 (s), 2.28 (m), 2.42 (s), 4.75 (m), 6.97 (s), 7.35-7.41 (m), 7.57 (d), 7.63 (d), 7.70-7.79 (m), 7.98 (d), 8.35 (s), 8.63 (d), 8.72 (s), 9.54 (d).

### 73.c Synthesis of 1-[11-(2-Ethylhexyl)-5-(2,4,6-trimethylbenzoyl)-11 H-benzo[a]carb-azol-8-yl]-2-phenyl ethane-1,2-dione 2-oxime O-acetate

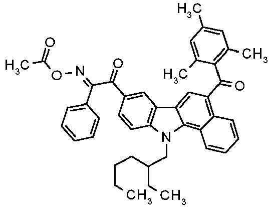

To 1-[11-(2-ethylhexyl)-5-(2,4,6-trimethylbenzoyl)-11H-benzo[a]carbazol-8-yl]-2-phenyl ethane-1,2-dione 2-oxime (0.40 g, 0.64 mmol) in THF (2 ml) are added triethylamine (94 mg, 0.90 mmol) and acetyl choride (77 mg, 0.90 mmol) at room temperature. The reaction mixture is stirred for 1 hour, and then it is poured into ice-water. The crude product is extracted with AcOEt, and washed with water and brine. After drying over MgSO₄ and concentration, the crude is purified by column chromatography on silica gel eluting with CH₂Cl₂ to obtain an off-white resin (324 mg, 76%). The structure is confirmed by ¹H-NMR spectrum (CDCl₃). δ [ppm]: 0.78-0.91 (m), 1.18-1.44 (m), 1.95 (s), 2.20 (s), 2.25 (m), 2.42 (s), 4.75 (m), 4.98 (s), 7.40 (t), 7.48 (d), 7.56 (d), 7.73-7.80 (m), 7.88 (d), 8.37 (s), 8.63 (d), 8.72 (s), 9.55 (d).

### Example 74 Synthesis of 1-[11-(2-Ethylhexyl)-11H-benzo[a]carbazole-5-yl]-2-phenyl ethane-1,2-dione 2-oxime O-acetate

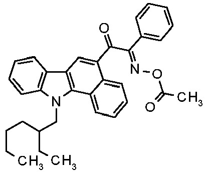

The compound of this example is prepared according to the procedure as disclosed in example 74.b and 74.c. with 1-[11-(2-ethylhexyl)-11 H-benzo[a]carbazol-5-yl]-2-phenyl ethanone prepared by reaction of 11 H-benzo[a]carbazole with phenylacetyl chloride. The product is composed of an isomeric mixture, and the structure is confirmed by ¹H-NMR spectrum (CDCl₃). δ [ppm]: 0.83 (t), 0.90 (t), 1.19-1.48 (m), 1.95 (s), 2.25-2.29 (m), 4.72-4.76 (m), 7.31 (t), 7.38-7.42 (m), 7.45-7.50 (m), 7.58 (d), 7.75 (td), 7.80 (td), 7.89 (dd), 8.05 (d), 8.67 (s), 8.69 (d), 9.86 (d).

### Example 75 Synthesis of [11-(2-Ethylhexyl)-8-(2,4,6-trimethylbenzoyl)-11H-benzo[a]carbazole-5-yl]-[4-(2,2,3,3-tetrafluoropropoxy)-phenyl]-methane oxime O-acetate

### 75.a Synthesis of [11-(2-Ethylhexyl)-11 H-benzo[a]carbazol-5-yl]-(4-fluorophenyl)-methanone

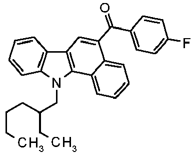

To 11 H-benzo[a]carbazole (8.24 g, 25.0 mmol) in CH₂Cl₂ (80 ml) are added AlCl₃ (3.33 g, 25.0 mmol) and 4-fluorobenzoyl chloride (4.04 g, 25.5 mmol) at 0°C. After stirring at room temperature for 4 hours, the reaction mixture is poured into ice-water. The crude product is extracted with CH₂Cl₂, and washed with water and brine. After drying over MgSO₄ and concentration, the crude product is purified by column chromatography on silica gel eluting with a mixed solvent of CH₂Cl₂ and n-hexane (1:9) to obtain a yellow solid (4.96 g, 44%). The structure is confirmed by ¹H-NMR spectrum (CDCl₃). δ [ppm]: 0.82-0.91 (m), 1.18-1.47 (m), 2.21-2.30 (m), 4.67-4.78 (m), 7.16 (t), 7.30 (t), 7.50 (t), 7.55-7.59 (m), 7.63-7.67 (m), 7.96-8.00 (m), 8.05 (d), 8.31 (s), 8.51 (d), 8.63 (d); ¹⁹F-NMR spectrum (CDCl₃). δ [ppm] -105.89.

### 75.b Synthesis of [11-(2-Ethylhexyl)-5-(4-fluorobenzoyl)-11 H-benzo[a]carbazol-8-yl]-(2,4,6-trimethylphenyl)-methanone

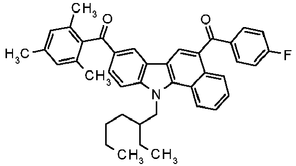

To [11-(2-ethylhexyl)-11H-benzo[a]carbazol-5-yl]-(4-fluorophenyl)-methanone (2.00 g, 4.43 mmol) in chlorobenzene (12 mL) are added AlCl₃ (1.24 g, 9.30 mmol) and 2,4,6-trimethylbenzoyl chloride (0.85 g, 4.65 mmol) at 0°C. After stirring for 2 hours at 65 °C, the reaction mixture is poured into ice-water. The crude product is extracted with CH₂Cl₂, and washed with water and brine. After drying over MgSO₄ and concentration, the crude product is purified by column chromatography on silica gel eluting with a mixed solvent of CH₂Cl₂ and n-hexane (4:1) to obtain a yellow solid (1.39 g, 52%). The structure is confirmed by ¹H-NMR spectrum (CDCl₃). δ [ppm]: 0.83 (t), 0.92 (t), 1.19-1.47 (m), 2.13 (s), 2.26-2.35 (m), 2.35 (s), 4.74-4.78 (m), 6.93 (s), 7.16 (t), 7.56-7.59 (m), 7.67 (t), 7.93-7.98 (m), 8.28 (s), 8.36 (d), 8.55 (br), 8.62 (d); ¹⁹F-NMR spectrum (CDCl₃). δ [ppm] -105.13.

### 75.c Synthesis of [11-(2-Ethylhexyl)-8-(2,4,6-trimethylbenzoyl)-11H-benzo[a]carbazole-5-yl]-[4-(2,2,3,3-tetrafluoropropoxy)-phenyl]-methane oxime

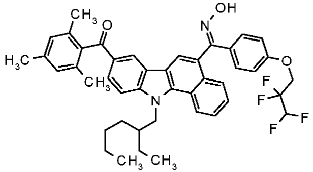

To [11-(2-ethylhexyl)-5-(4-fluorobenzoyl)-11H-benzo[a]carbazol-8-yl]-(2,4,6-trimethyl-phenyl)-methanone (1.29 g, 2.16 mmol) in pyridine (3 ml) are added 2,2,3,3-tetrafluoro-1-propanol (0.43 g, 3.24 mmol) and sodium hydroxide (0.13 g, 3.32 mmol). The mixture is stirred at 70 °C for 23 hours, and then it is stirred at 120 °C for 24 hours. After the reaction mixture is cooled at room temperature, it is poured into water, and then the crude product is extracted with AcOEt, and washed with water and brine. After drying over MgSO₄ and concentration, the crude product is purified by column chromatography on silica gel eluting with CH₂Cl₂ to obtain a white solid (0.80 g, 51%). The product is composed of an isomeric mixture, and the structure is confirmed by ¹H-NMR spectrum (CDCl₃) δ [ppm]: 0.79-0.99 (m), 1.24-1.52 (m), 2.05 (s), 2.13 (s), 2.35 (s), 2.37 (s), 4.31 (t), 4.73-4.77 (m), 5.90-6.16 (m), 6.83 (d), 6.87 (d), 6.92 (s), 6.94 (s), 7.50-7.73 (m), 7.88 (d), 8.00 (s), 8.11 (br), 8.19 (s), 8.41 (br), 8.55 (d), 8.62 (d); ¹⁹F-NMR spectrum (CDCl₃). δ [ppm] -125.04, -124.90.

### 75.d Synthesis of [11-(2-Ethylhexyl)-8-(2,4,6-trimethylbenzoyl)-11H-benzo[a]carbazole-5-yl]-[4-(2,2,3,3-tetrafluoropropoxy)-phenyl]-methane oxime O-acetate

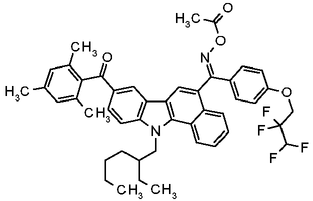

To [11-(2-ethylhexyl)-8-(2,4,6-trimethylbenzoyl)-11H-benzo[a]carbazole-5-yl]-[4-(2,2,3,-3-tetrafluoropropoxy)-phenyl]-methane oxime in N,N-dimethylacetamide (3 ml) are added triethylamine (0.33 g, 3.27 mmol) and acetyl chloride (0.26 g, 3.29 mmol) at 0 °C. After stirring at 0 °C for 2 hours, the reaction mixture is poured into ice-water. The crude product is extracted with AcOEt, and washed with water and brine. After drying over MgSO₄ and concentration, the crude product is purified by recrystallization from a mixed solvent of ethyl acetate and n-hexane to give a beige solid (0.29 g, 35%). The product is composed of an isomeric mixture, and the structure is confirmed by ¹H-NMR spectrum (CDCl₃) δ [ppm]: 0.81-0.98 (m), 1.22-1.49 (m), 1.83 (d), 2.14 (s), 2.15 (s), 2.27 (s), 2.36 (s), 4.34 (t), 4.77-4.79 (m), 5.90-6.17 (m), 6.87 (d), 6.93 (s), 6.94 (s), 7.49-7.69 (m), 7.81 (d), 7.95 (s), 7.99 (s), 8.10 (br), 8.26 (s), 8.46 (br), 8.57 (d), 8.63 (d); ¹⁹F-NMR spectrum (CDCl₃). δ [ppm] -139.16, -124.87.

### Example 76 Synthesis of [7-(2-ethyl-hexyl)-3-(2,4,6-trimethylbenzoyl)-7H-benzo[c]-carbazole-10-yl]-o-tolyl-methanone oxime O-acetate

### 76.a Synthesis of 1-(2-nitoro-phenyl)-naphthalene

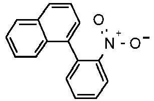

To o-bromobenzene (2.02 g, 10.0 mmol) in DMA (15 ml) are added 1-naphthal-eneboronic acid (1.73 g, 10.0 mmol) and tetrakis(triphenylphosphine)palladium (116 mg, 0.1 mmol). The mixture is heated at 110°C, and it is stirred overnight. After the reaction mixture is cooled at room temperature, it is poured into water. The crude product is extracted with AcOEt, and washed with water and brine. After drying over MgSO₄ and concentration, it is washed with n-hexane to give a beige solid (2.1 g, 84%). The structure is confirmed by ¹H-NMR spectrum (CDCl₃) δ [ppm]: 7.35 (dd), 7.38-7.44 (m), 7.46-7.54 (m), 7.61 (td), 7.70 (td), 7.90 (d), 8.07 (dd).

### 76.a Synthesis of 7H-Benzo[c]carbazole

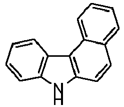

To 1-(2-nitoro-phenyl)-naphthalene (1.10 g, 4.41 mmol) in *o*-dichlorobenzene (6 ml) is added triphenylphosphine (2.89 g, 11.03 mmol). The mixture is heated at 180°C, and then it is stirred for 24 hours. After the reaction mixture is cooled at room temperature, the crude product is purified by column chromatography on silica gel eluting with a mixted solvent of n-hexane and CH₂Cl₂ (4:1) to obtain a beige solid (0.79 g, 82%). The structure is confirmed by ¹H-NMR spectrum (CDCl₃) δ [ppm]: 7.39 (td), 7.43-7.50 (m), 7.57 (dt), 7.62 (d), 7.71 (td), 7.86 (d), 8.00 (td), 8.40 (br), 8.57 (d), 8.77 (d).

### 76.b Synthesis of 7-(2-Ethylhexyl)-7H-benzo[c]carbazole

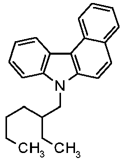

To 7 H-benzo[c]carbazole (0.78 g, 3.59 mmol) in dimethylformamide (DMF) (4 ml) is added sodium hydride (0.21 g, 5.21 mmol) at 0°C, and the mixture is stirred for 1 hour. To the reaction mixture is added 1-bromo-2-ethylhexane (1.04 g, 5.39 mmol). The mixture is stiired at room temperature overnight, and then at 0°C for 5 hours. After the reaction mixture is cooled at room temeratrue, it is poured into ice-water, and the crude product is extracted with AcOEt, and washed with water and brine. After drying over MgSO₄ and concentration, the desired product is obtained as a brown liquid (1.21 g, 99%). The structure is confirmed by ¹H-NMR spectrum (CDCl₃) δ [ppm]: 0.83-0.92 (m), 1.20-1.43 (m), 2.07-2.10 (m), 4.24-4.38 (m), 7.37 (td), 7.44-7.50 (m), 7.52-7.54 (m), 7.62 (d), 7.69 (td), 7.87 (d), 7.99 (d), 8.59 (d), 8.80 (d)

### 76.c Synthesis of [7-(2-ethyl-hexyl)-3-(2,4,6-trimethylbenzoyl)-7H-benzo[c]carbazole-10-yl]-o-tolyl-methanone

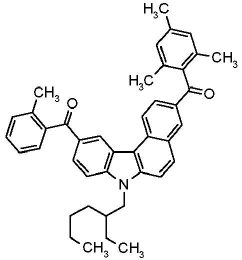

To 7-(2-ethylhexyl)-7H-benzo[c]carbazole (0.66 g, 2.00 mmol) in CH₂Cl₂ are added AlCl₃ (0.27 g, 2.06 mmol) and 2,4,6-trimethylbenzoyl chloride (0.37 g, 2.00 mmol) at 0°C, and then the mixture is stirred for 2 hours. To the reaction mixture are added AlCl₃ (0.29 g, 2.20 mmol) and *o*-toluoyl chloride (0.32 g, 2.06 mmol) at 0°C, and then the mixture is stirred overnight. The reaction mixture is poured into ice-water, and the crude product is extracted with CH₂Cl₂, and washed with water and brine. After drying over MgSO₄ and concentration, the crude product is purified by column chromatography on silica gel eluting with a mixted solvent of CH₂Cl₂ and n-hexane (4 :1) to obtain a white solid (0.25 g, 21%). The structure is confirmed by ¹H-NMR spectrum (CDCl₃) δ [ppm]: 0.84 (t), 0.93 (t), 1.22-1.45 (m), 2.04-2.09 (m), 2.18 (s), 2.38 (s), 2.39 (s), 4.32-4.34 (m), 6.97 (s), 7.30-7.37 (m), 7.42-7.47 (m), 7.51 (d), 7.68 (d), 7.88 (br), 7.96 (d), 8.21 (dd), 8.37 (d), 8.78 (br), 9.19 (br).

### 76.d Synthesis of [7-(2-ethyl-hexyl)-3-(2,4,6-trimethylbenzoyl)-7H-benzo[c]carbazole-10-yl]-o-tolyl-methanone oxime

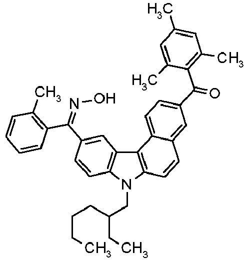

To [7-(2-ethyl-hexyl)-3-(2,4,6-trimethylbenzoyl)-7H-benzo[c]carbazole-10-yl]-o-tolyl-me-thanone (0.25 g, 0.42 mmol) in pyridine (2 ml) is added hydroxylammonium chloride (59 mg, 0.84 mmol). The mixture is heated at 130°C, and then it is stirred for 6 hours. After the reaction mixture is cooled to room temperature, it is poured into water, and then the crude product is extracted with CH₂Cl₂ and washed with water and brine. After drying over MgSO₄ and concentration, the desired product is obtained as a pale yellow solid (0.23 g, 90%). It is composed of an isomeric mixture, and the structure is confirmed by ¹H-NMR spectrum (CDCl₃) δ [ppm]: 0.84 (t), 0.92 (t), 1.23-1.40 (m), 2.05-2.07 (m), 2.10 (s), 2.18 (s), 2.20 (s), 2.40 (s), 4.32 (dd), 6.97 (s), 7.19 (dd), 7.34-7.38 (m), 7.42 (dd), 7.51 (dd), 7.62 (dd), 7.84 (d), 7.97 (d), 8.12 (dd), 8.61 (br), 9.06 (br).

### 76.e Synthesis of [7-(2-ethyl-hexyl)-3-(2,4,6-trimethylbenzoyl)-7H-benzo[c]carbazole-10-yl]-o-tolyl-methanone oxime O-acetate

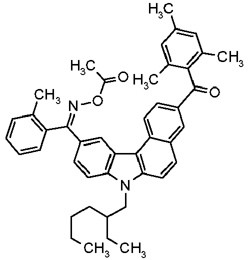

To [7-(2-ethyl-hexyl)-3-(2,4,6-trimethylbenzoyl)-7H-benzo[c]carbazole-10-yl]-*o*-tolyl-methanone oxime (0.23 g, 0.38 mmol) in AcOEt (5 ml) are added triethylamine (0.11 g, 1.13 mmol) and acetyl chloride (89 mg, 1.13 mmol) at 0°C, and then the mixture is stirred for 2.5 hours. The reaction mixture is poured into water, and the crude product is extracted with AcOEt, and washed with water and brine. After drying over MgSO₄ and concentration, the crude product is purified by column chromatography on silica gel eluting with a mixted solvent of CH₂Cl₂ and n-hexane (9:1) to obtain a white solid (0.11 g, 44%). It is composed of an isomeric mixture, and the structure is confirmed by ¹H-NMR spectrum (CDCl₃) δ [ppm]:0.84 (t), 0.92 (t), 1.21-1.44 (m), 2.05 (s), 2.09-2.11 (m), 2.18 (s), 2.20 (s), 2.23 (s), 2.25 (s), 2.38 (s), 2.40 (s), 4.30-4.32 (m), 6.96 (s), 6.97 (s), 7.18 (d), 7.23-7.25 (m), 7.28-7.38 (m), 7.40-7.53 (m), 7.62 (d), 7.66 (d), 7.84 (d), 7.93-7.97 (m), 8.13 (dd), 8.62 (br), 8.62 (br), 8.79 (br), 9.04 (br), 9.24 (br)

### Example 77 Synthesis of [11-(2-ethyl-hexyl)-5-nitro-11 H-benzo[a]carbazole-8-yl]-o-tolyl-methanone oxime O-acetate

### 77.a Synthesis of 11-(2-ethyl-hexyl)-5-nitoro-11H-benzo[a]carbazole

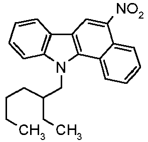

To 11-(2-Ethylhexyl)-11H-benzo[a]carbazole (10.09 g, 30.64 mmol) in acetic acid (100 ml) is added 70% nitric acid (2.85 g, 3.16 mmol) at 80 °C. After stirring for 1 hour, the reaction mixture is poured into ice-water. The crude is extracted with CH₂Cl₂, and washed with water and brine. After drying over MgSO₄ and concentration, the crude is purified by column chromatography on silica gel eluting with CH₂Cl₂ to obtain a yellow resin (10.89g, 94%) The structure is confirmed by ¹H-NMR spectrum (CDCl₃). δ [ppm]: 0.79-0.87 (m), 1.19-1.46 (m), 2.17 (m), 4.58 (m), 7.25-7.40-(m), 7.54-7.55 (m), 7.64-7.74 (m), 8.11 (d), 8.55 (d), 8.92 (d), 8.99 (s).

### 77.b Synthesis of [11-(2-ethyl-hexyl)-5-nitro-11 H-benzo[a]carbazole-8-yl]-o-tolyl-methanone

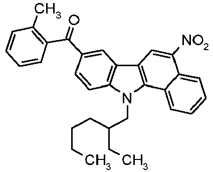

To 11-(2-ethyl-hexyl)-5-nitoro-11 H-benzo[a]carbazole (0.59 g, 1.58 mmol) in *o*-toluoyl chloride (7 ml) is added zinc oxide (45 mg, 0.55 mmol) at room temperature. The reaction mixture is heated at 100°C. After stirring for 5 min, the reaction mixture is poured into ice-water. The crude is extracted with CH₂Cl₂, and washed with water and brine. After drying over MgSO₄ and concentration, the crude is purified by column chromatography on silica gel eluting with a mixed solvent of n-hexane and CH₂Cl₂ (1:1) to obtain a yellow resin (0.37 g, 47%). The structure is confirmed by ¹H-NMR spectrum (CDCl₃). δ [ppm]: 0.79-0.99 (m), 1.19-1.50 (m), 2.25 (m), 2.38 (s), 4.79 (m), 7.33 (t), 7.37 (d) 7.42 (d), 7.47 (t), 7.67 (d), 7.75 (t), 7.78 (t), 8.19 (d), 8.52 (s), 8.66 (d), 8.89 (d), 8.99 (s).

### 77.c Synthesis of [11-(2-ethyl-hexyl)-5-nitro-11 H-benzo[a]carbazole-8-yl]-o-tolyl-methanone oxime

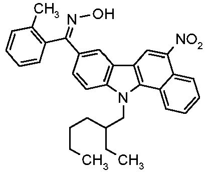

To [11-(2-ethyl-hexyl)-5-nitro-11H-benzo[a]carbazole-8-yl]-o-tolyl-methanone (0.36 g, 0.73 mmol) in pyridine (6 ml) is added hydroxylammonium chloride (0.10 g, 1.45 mmol) at 120°C. After stirring for 16 hours, the reaction mixture is poured into ice-water. The crude is extracted with AcOEt, and washed with water and brine. After drying over MgSO₄ and concentration, the crude is purified by column chromatography on silica gel eluting with CH₂Cl₂ to obtain a yellow resin (0.28 g, 76%). The structure is confirmed by ¹H-NMR spectrum (CDCl₃). δ [ppm]: 0.79-0.90 (m), 1.13-1.48(m), 2.21 (m), 2.29 (s), 4.69 (m), 7.25 (d), 7.37-7.47 (m), 7.55 (d), 7.67-7.75 (m), 7.90 (d), 8.04 (s), 8.60 (d), 8.89 (d), 8.92 (s).

### 77.d Synthesis of [11-(2-ethyl-hexyl)-5-nitro-11 H-benzo[a]carbazole-8-yl]-o-tolyl-methanone oxime O-acetate

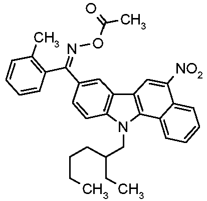

To [11-(2-ethyl-hexyl)-5-nitro-11H-benzo[a]carbazole-8-yl]-*o*-tolyl-methanone oxime (0.27 g, 0.53 mmol) in THF (4 ml) are added triethylamine (79 mg, 0.78 mmol) and acetyl chloride (66 mg, 0.84 mmol) at 0°C. After stirring for 2 hours, the reaction mixture is poured into ice-water. The crude is extracted with AcOEt, and washed with water and brine. After drying over MgSO₄ and concentration, the crude is purified by column chromatography on silica gel eluting with CH₂Cl₂ to obtain a yellow resin (0.27 g, 94%). The structure is confirmed by ¹H-NMR spectrum (CDCl₃). δ [ppm]: 0.79-0.99 (m), 1.15-1.48 (m), 2.08 (s), 2.22 (s), 2.23 (m), 4.74 (m), 7.19 (d), 7.36 (t), 7.39 (d), 7.45 (t), 7.59 (d), 7.70-7.79 (m), 8.08 (d), 8.12 (s), 8.63 (d), 8.89 (d), 8.94 (s).

### Example 78 Synthesis of [11-(2-ethyl-hexyl)-5-nitro-11 H-benzo[a]carbazole-8-yl]-[4-(2,2,3,3-tetrafluoropropoxy)-phenyl]-methanone oxime O-acetate

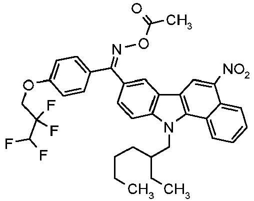

To [11-(2-ethyl-hexyl)-5-nitro-11H-benzo[a]carbazole-8-yl]-[4-(2,2,3,3-tetrafluoroprop-oxy)-phenyl]-methanone oxime (160 mg, 0.26 mmol) in DMA (4 ml) are added triethylamine (265 mg, 2.66 mmol) and acetyl chloride (208 mg, 2.57 mmol) at 0°C. After stirring for overnight, the reaction mixture is poured into ice-water. The crude is extracted with AcOEt, and washed with water and brine. After drying over MgSO₄ and concentration, the crude is purified by column chromatography on silica gel eluting with a mixed solvent of n-hexane and CH₂Cl₂ (1:3) to obtain a yellow resin (67 mg, 38%). It is composed of an isomeric mixture, and the structure is confirmed by ¹H-NMR spectrum (CDCl₃). δ [ppm]: 0.83 (t), 0.87-0.96 (m), 1.20-1.47 (m), 2.12 (s), 2.12 (s), 2.16 (s), 2.21-2.26 (m), 4.40 (t), 4.49 (t), 4.74-4.80 (t), 5.93-6.26 (m), 709 (d), 7.42-7.45 (m), 7.58-7.64 (m), 7.68-7.81 (m), 8.01 (dd), 8.11 (d), 8.15 (d), 8.64-8.70 (m), 8.90-8.94 (m), 8.98 (s), 9.01 (s).

### Application Examples

### Example A1: Preparation of Poly(benzylmethacrylate-co-methacrylic acid)

24 g of benzylmethacrylate, 6 g of methacrylic acid and 0.525 g of azobisisobutyronitrile (AIBN) are dissolved in 90 ml of propylene glycol 1-monomethyl ether 2-acetate (PGMEA). The resulting reaction mixture is placed in a preheated oil bath at 80°C. After stirring for 5 hours at 80°C under nitrogen, the resulting viscous solution is cooled to room temperature and used without further purification. The solid content is about 25%.

### Example A2: Sensitivity tests

A photocurable composition for a sensitivity test is prepared by mixing the following components:

| | |
|---|---|
| 200.0 parts by weight of | copolymer of benzylmethacrylate and methacrylic acid (benzylmethacrylate : methacrylic acid = 80 : 20 by weight) 25% propylene glycol 1-monomethyl ether 2-acetate (PGMEA) solution, prepared in above example A1 |
| 50.0 parts by weight of | dipentaerythritol hexaacrylate ((DPHA), provided by UCB-Chemicals), |
| 1.0 parts by weight of | photoinitiator, and |
| 150.0 parts by weight of | PGMEA |

All operations are carried out under yellow light. The compositions are applied to an aluminum plate using an electric applicator with a wire wound bar. The solvent is removed by heating at 80°C for 10 minutes in a convection oven. The thickness of the dry film is approximately 2 µm. A standardized test negative film with 21 steps of different optical density (Stouffer step wedge) is placed with an air gap of around 100 µm between the film and the resist. A glass filter (UV-35) is placed on the negative film. Exposure is carried out using a 250W super high pressure mercury lamp (USHIO, USH-250BY) at a distance of 15 cm. A total exposure dose measured by an optical power meter (ORC UV Light Measure Model UV-M02 with UV-35 detector) on the glass filter is 1000 mJ/cm². After exposure, the exposed film is developed with an alkaline solution (5% aqueous solution of DL-A4, Yokohama Yushi) for 120 sec. at 28°C by using a spray type developer (AD-1200, Takizawa Sangyo). The sensitivity of the initiator used is characterized by indicating the highest number of the step remained (i.e. polymerized) after developing. The higher the number of steps, the more sensitive is the initiator tested. The results are listed in table 3.

**Table 3:**

| photoinitiator of example | Number of reproduced steps |
|---|---|
| 1 | 18 |
| 2 | 17 |
| 3 | 18 |
| 4 | 16 |
| 5 | 18 |
| 6 | 18 |
| 7 | 19 |
| 8 | 17 |
| 9 | 18 |
| 10 | 17 |
| 11 | 18 |
| 12 | 18 |
| 13 | 19 |
| 14 | 18 |
| 15 | 18 |
| 16 | 18 |
| 17 | 18 |
| 18 | 17 |
| 19 | 16 |
| 20 | 16 |
| 21 | 16 |
| 22 | 16 |
| 23 | 17 |
| 24 | 17 |
| 25 | 17 |
| 26 | 17 |
| 27 | 17 |
| 28 | 18 |
| 29 | 17 |
| 30 | 19 |
| 31 | 18 |
| 32 | 18 |
| 33 | 18 |
| 34 | 18 |
| 35 | 19 |
| 36 | 19 |
| 37 | 17 |
| 38 | 18 |
| 39 | 18 |
| 40 | 17 |
| 41 | 17 |
| 43 | 18 |
| 44 | 18 |
| 45 | 17 |
| 46 | 17 |
| 47 | 17 |
| 48 | 18 |
| 49 | 17 |
| 50 | 19 |
| 51 | 19 |
| 52 | 19 |
| 53 | 19 |
| 55 | 17 |
| 56 | 18 |
| 57 | 17 |
| 60 | 17 |
| 61 | 18 |
| 62 | 19 |
| 63 | 17 |
| 64 | 17 |
| 65 | 17 |
| 66 | 18 |
| 68 | 17 |
| 69 | 17 |
| 70 | 17 |
| 76 | 18 |

## Claims

1. Compounds of the formula I 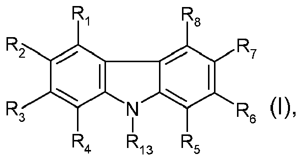 wherein
**R₁, R₂, R₃, R₄, R₅, R₆, R₇** and **R₈** independently of each other are hydrogen, C₁-C₂₀alkyl, 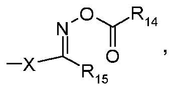 COR₁₆, OR₁₇, halogen, NO₂, or 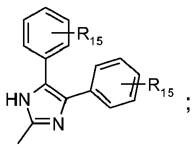 or R₁ and R₂, R₂ and R₃, R₃ and R₄, R₅ and R₆, R₆ and R₇, or R₇ and R₈ independently of each other are C₂-C₁₀alkenyl which is substituted by 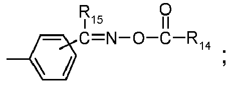 or R₁ and R₂, R₂ and R₃, R₃ and R₄, R₅ and R₆, R₆ and R₇, or R₇ and R₈ independently of each other together are -(CH₂)ₚ-Y-(CH₂)_{q}-;
or R₁ and R₂, R₂ and R₃, R₃ and R₄, R₅ and R₆, R₆ and R₇, or R₇ and R₈ independently
of each other together are 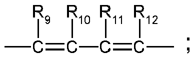 ***provided that*** at least one pair of R₁ and R₂, R₂ and R₃, R₃ and R₄, R₅ and R₆, R₆ and
R₇, or R₇ and R₈ is 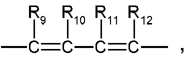 **R₉, R₁₀, R₁₁** and **R₁₂** independently of each other are hydrogen, C₁-C₂₀alkyl which is unsubstituted or substituted by one or more halogen, phenyl, CN, OH, SH, C₁-C₄-alkoxy, (CO)OH or by (CO)O(C₁-C₄alkyl);
or R₉, R₁₀, R₁₁, and R₁₂ independently of each other are unsubstituted phenyl or phenyl substituted by one or more C₁-C₆alkyl, halogen, CN, OR₁₇, SR₁₈ or by NR₁₉R₂₀;
or R₉, R₁₀, R₁₁, and R₁₂ independently of each other are halogen, CN, OR₁₇, SR₁₈, SOR₁₈, SO₂R₁₈ or NR₁₉R₂₀, wherein the substituents OR₁₇, SR₁₈ or NR₁₉R₂₀ optionally form 5- or 6-membered rings *via* the radicals R₁₇, R₁₈, R₁₉ and/or R₂₀ with one of the carbon atoms of the naphthyl ring;
or R₉, R₁₀, R₁₁ and R₁₂ independently of each other are 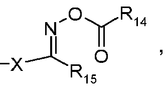 COR₁₆ or NO₂;
**Y** is O, S, NR₂₆ or a direct bond;
**p** is an integer 0,1, 2 or 3;
**q** is an integer 1, 2 or 3;
**X** is CO or a direct bond;
**R₁₃** is C₁-C₂₀alkyl which is unsubstituted or substituted by one or more halogen, R₁₇,
COOR₁₇, OR₁₇, SR₁₈, CONR₁₉R₂₀, NR₁₉R₂₀, PO(OCₖH₂ₖ₊₁)₂, or by 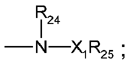 or R₁₃ is C₂-C₂₀alkyl which is interrupted by one or more O, S, SO, SO₂, NR₂₆ or CO, or is C₂-C₁₂alkenyl which is uninterrupted or is interrupted by one or more O, CO or NR₂₆,
wherein the interrupted C₂-C₂₀alkyl and the uninterrupted or interrupted C₂-C₁₂alkenyl are unsubstituted or are substituted by one or more halogen;
or R₁₃ is C₄-C₈cycloalkenyl, C₂-C₁₂alkinyl, or C₃-C₁₀cycloalkyl which is uninterrupted or is interrupted by one or more O, S, CO or NR₂₆;
or R₁₃ is phenyl or naphthyl each of which is unsubstituted or substituted by one or
more OR₁₇, SR₁₈, NR₁₉R₂₀, 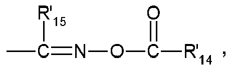 COR₁₆, CN, NO₂, halogen, C₁-C₂₀alkyl, C₁-C₄haloalkyl, C₂-C₂₀alkyl which is interrupted by one or more O, S, CO or NR₂₆, or each of which is substituted by C₃-C₁₀cycloalkyl or by C₃-C₁₀cycloalkyl which is interrupted by one or more O, S, CO, or NR₂₆;
**k** is an integer 1-10;
**R₁₄** is hydrogen, C₃-C₈cycloalkyl, C₂-C₅alkenyl, C₁-C₂₀alkoxy or C₁-C₂₀alkyl which is unsubstituted or substituted by one or more halogen, phenyl, C₁-C₂₀alkylphenyl or CN; or R₁₄ is phenyl, or naphthyl, each of which is unsubstituted or substituted by one or more C₁-C₆alkyl, C₁-C₄haloalkyl, halogen, CN, OR₁₇, SR₁₈ and/or NR₁₉R₂₀;
or R₁₄ is C₃-C₂₀heteroaryl, C₁-C₈alkoxy, benzyloxy or phenoxy, which benzyloxy and phenoxy are unsubstituted or substituted by one or more C₁-C₆alkyl, C₁-C₄haloalkyl and/or halogen;
**R₁₅** is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, PO(OCₖH₂ₖ₊₁)₂, SO-C₁-C₁₀alkyl, SO₂-C₁-C₁₀alkyl, by C₂-C₂₀alkyl which is interrupted by one or more O, S or NR₂₆, or each of which is substituted by C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂₀heteroaryloxycarbonyl, OR₁₇, SR₁₈ or NR₁₉R₂₀;
or R₁₅ is hydrogen, C₂-C₁₂alkenyl, C₃-C₈cycloalkyl which is uninterrupted or is interrupted by one or more O, CO or NR₂₆;
or R₁₅ is C₁-C₂₀alkyl which is unsubstituted or substituted by one or more halogen, OR₁₇, SR₁₈, C₃-C₈cycloalkyl, C₃-C₂₀heteroaryl, C₆-C₂₀aryloxycarbonyl, C₃-C₂₀hetero-aryloxycarbonyl, NR₁₉R₂₀, COOR₁₇, CONR₁₉R₂₀, PO(OCₖH₂ₖ₊₁)₂, 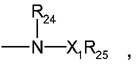 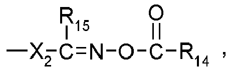 phenyl, or the C₁-C₂₀alkyl is substituted by phenyl which is substituted by halogen, C₁-C₂₀alkyl, C₁-C₄haloalkyl, OR₁₇, SR₁₈, or NR₁₉R₂₀;
or R₁₅ is C₂-C₂₀alkyl which is interrupted by one or more O, SO or SO₂, and which interrupted C₂-C₂₀alkyl is unsubstituted or substituted by one or more halogen, OR₁₇, COOR₁₇, CONR₁₉R₂₀, phenyl or by phenyl which is substituted by OR₁₇, SR₁₈ or NR₁₉R₂₀;
or R₁₅ is C₂-C₂₀alkanoyl or benzoyl which is unsubstituted or substituted by one or more C₁-C₆alkyl, halogen, phenyl, OR₁₇, SR₁₈ or NR₁₉R₂₀;
or R₁₅ is naphthoyl which is unsubstituted or is substituted by one or more OR₁₇ or is C₃-C₁₄heteroarylcarbonyl;
or R₁₅ is C₂-C₁₂alkoxycarbonyl which is uninterrupted or is interrupted by one or more O and which interrupted or uninterrupted C₂-C₁₂alkoxycarbonyl is unsubstituted or substituted by one or more hydroxyl groups;
or R₁₅ is phenoxycarbonyl which is unsubstituted or substituted by one or more C₁-C₆alkyl, halogen, C₁-C₄haloalkyl, phenyl, OR₁₇, SR₁₈ or NR₁₉R₂₀;
or R₁₅ is CN, CONR₁₉R₂₀, NO₂, C₁-C₄haloalkyl, S(O)ₘ-C₁-C₆alkyl; S(O)ₘ-phenyl which is unsubstituted or substituted by C₁-C₁₂alkyl or SO₂-C₁-C₆alkyl;
or R₁₅ is SO₂O-phenyl which is unsubstituted or substituted by C₁-C₁₂alkyl; or is diphenyl phosphinoyl or di-(C₁-C₄alkoxy)-phosphinoyl;
**m** is 1 or 2;
**R**'₁₄ has one of the meanings as given for R₁₄;
**R'₁₅** has one of the meanings as given for R₁₅;
**X₁** is O, S, SO or SO₂;
**X₂** is O, CO, S or a direct bond;
**R₁₆** is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, or by C₁-C₂₀alkyl which is interrupted by one or more O, S, or NR₂₆, or each of which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂₀heteroaryloxycarbonyl, OR₁₇, SR₁₈ or NR₁₉R₂₀;
or R₁₆ is hydrogen, C₁-C₂₀alkyl which is unsubstituted or substituted by one or more halogen, phenyl, OH, SH, CN, C₃-C₆alkenoxy, OCH₂CH₂CN, OCH₂CH₂(CO)O(C₁-C₄alkyl), O(CO)-(C₁-C₄alkyl), O(CO)-phenyl, (CO)OH, or (CO)O(C₁-C₄alkyl);
or R₁₆ is C₂-C₁₂alkyl which is interrupted by one or more O, S or NR₂₆;
or R₁₆ is (CH₂CH₂O)ₙ₊₁H, (CH₂CH₂O)ₙ(CO)-(C₁-C₈alkyl), C₂-C₁₂alkenyl or C₃-C₈cycloalkyl;
or R₁₆ is phenyl substituted by SR₁₈ wherein the radical R₁₈ denotes a direct bond to the phenyl or naphthyl ring of the carbazole moiety to which the COR₁₆ group is attached;
n is 1-20;
**R₁₇** is hydrogen, phenyl-C₁-C₃alkyl, C₁-C₂₀alkyl which is unsubstituted or substituted by one or more halogen, OH, SH, CN, C₃-C₆alkenoxy, OCH₂CH₂CN, OCH₂CH₂(CO)O(C₁-C₄alkyl), O(CO)-(C₁-C₄alkyl), O(CO)-(C₂-C₄)alkenyl, O(CO)-phenyl, (CO)OH, (CO)O(C₁-C₄alkyl), C₃-C₂₀cycloalkyl, SO₂-(C₁-C₄haloalkyl), O(C₁-C₄haloalkyl) or by C₃-C₂₀cycloalkyl which is interrupted by one or more O;
or R₁₇ is C₂-C₂₀alkyl which is interrupted by one or more O, S or NR₂₆;
or R₁₇ is (CH₂CH₂O)ₙ₊₁H, (CH₂CH₂O)ₙ(CO)-(C₁-C₈alkyl), C₁-C₈alkanoyl, C₂-C₁₂alkenyl, C₃-C₆alkenoyl, or C₃-C₂₀cycloalkyl which is uninterrupted or interrupted by one or more O, S, CO, or NR₂₆;
or R₁₇ is C₁-C₈alkyl-C₃-C₁₀cycloalkyl which is uninterrupted or interrupted by one or more O;
or R₁₇ is benzoyl which is unsubstituted or substituted by one or more C₁-C₆alkyl, halogen, OH or C₁-C₃alkoxy;
or R₁₇ is phenyl, naphthyl or C₃-C₂₀heteroaryl, each of which is unsubstituted or substituted by one or more halogen, OH, C₁-C₁₂alkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenyl-amino or 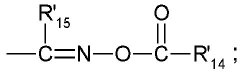 or R₁₇ forms a direct bond to one of the carbon atoms of the phenyl or naphthyl ring on
which the group 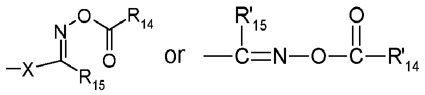 is located;
**R₁₈** is hydrogen, C₂-C₁₂alkenyl, C₃-C₂₀cycloalkyl, or phenyl-C₁-C₃alkyl, wherein the C₂-C₁₂alkenyl, C₃-C₂₀cycloalkyl, or phenyl-C₁-C₃alkyl is uninterrupted or interrupted by one or more O, S, CO, NR₂₆ or COOR₁₇;
or R₁₈ is C₁-C₂₀alkyl which is unsubstituted or is substituted by one or more OH, SH, CN, C₃-C₆alkenoxy, OCH₂CH₂CN, OCH₂CH₂(CO)O(C₁-C₄alkyl), O(CO)-(C₂-C₄)alkenyl, O(CO)-(C₁-C₄alkyl), O(CO)-phenyl or (CO)OR₁₇;
or R₁₈ is C₂-C₂₀alkyl which is interrupted by one or more O, S, CO, NR₂₆ or COOR₁₇;
or R₁₈ is (CH₂CH₂O)ₙH, (CH₂CH₂O)ₙ(CO)-(C₁-C₈alkyl), C₂-C₈alkanoyl or C₃-C₆alkenoyl; or R₁₈ is benzoyl which is unsubstituted or substituted by one or more C₁-C₆alkyl, halogen, OH, C₁-C₄alkoxy or C₁-C₄alkylsulfanyl;
or R₁₈ is phenyl, naphthyl or C₃-C₂₀heteroaryl, each of which is unsubstituted or substituted by one or more halogen, C₁-C₁₂alkyl, C₁-C₄haloalkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenylamino, (CO)O(C₁-C₈alkyl), (CO)-C₁-C₈alkyl, (CO)N(C₁-C₈alkyl)₂ or 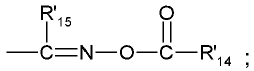 **R₁₉** and **R₂₀** independently of each other are hydrogen, C₁-C₂₀alkyl, C₂-C₄hydroxyalkyl, C₂-C₁₀alkoxyalkyl, C₂-C₅alkenyl, C₃-C₂₀cycloalkyl, phenyl-C₁-C₃alkyl, C₁-C₈alkanoyl, C₁-C₈alkanoyloxy, C₃-C₁₂alkenoyl SO₂-(C₁-C₄haloalkyl), or benzoyl;
or R₁₉ and R₂₀ are phenyl, naphthyl or C₃-C₂₀heteroaryl, each of which is unsubstituted or substituted by one or more halogen, C₁-C₄haloalkyl, C₁-C₂₀alkoxy, C₁-C₁₂alkyl, benzoyl or C₁-C₁₂alkoxy;
or R₁₉ and R₂₀ together with the N-atom to which they are attached form a 5- or 6-membered saturated or unsaturated ring which is uninterrupted or is interrupted by O, S or NR₁₇, and which 5- or 6-membered saturated or unsaturated ring is unsubstituted or substituted by one or more C₁-C₂₀alkyl, C₁-C₂₀alkoxy, =O, OR₁₇, SR₁₈, NR₂₁R₂₂,
(CO)R₂₃, NO₂, halogen, C₁-C₄-haloalkyl, CN, phenyl, 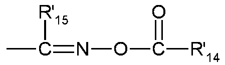 or by C₃-C₂₀cyclalkyl which is uninterrupted or is interrupted by one or more O, S, CO or NR₁₇;
or R₁₉ and R₂₀ together with the N-atom to which they are attached form a heteroaromatic ring system which is unsubstituted or substituted by one or more C₁-C₂₀alkyl, C₁-C₄haloalkyl, C₁-C₂₀alkoxy, =O, OR₁₇, SR₁₈, NR₂₁R₂₂, (CO)R₂₃, 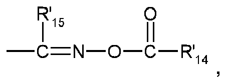 halogen, NO₂, CN, phenyl or by C₃-C₂₀cycloalkyl which is uninterrupted or is interrupted by one or more O, S, CO or NR₁₇;
R₂₁ and R₂₂ independently of each other are hydrogen, C₁-C₂₀alkyl, C₁-C₄haloalkyl, C₃-C₁₀cycloalkyl or phenyl;
or R₂₁ and R₂₂ together with N-atom to which they are attached form a 5- or 6-membered saturated or unsaturated ring, which is uninterrupted or is interrupted by O, S or NR₂₆, and which 5- or 6-membered saturated or unsaturated ring is not condensed or to which 5- or 6-membered saturated or unsaturated ring a benzene ring is condensed;
**R₂₃** is hydrogen, OH, C₁-C₂₀alkyl, C₁-C₄haloalkyl, C₂-C₂₀alkyl which is interrupted by one or more O, CO or NR₂₆, C₃-C₂₀cycloalkyl which is uninterrupted or is interrupted by O, S, CO or NR₂₆,
or R₂₃ is phenyl, naphthyl, phenyl-C₁-C₄alkyl, OR₁₇, SR₁₈ or NR₂₁R₂₂;
**R₂₄** is (CO)OR₁₇, CONR₁₉R₂₀, (CO)R₁₇; or R₂₄ has one of the meanings given for R₁₉ and R₂₀;
**R₂₅** is COOR₁₇, CONR₁₉R₂₀, (CO)R₁₇; or R₂₅ has one of the meanings given for R₁₇;
**R₂₆** is hydrogen, C₁-C₂₀alkyl, C₁-C₄haloalkyl, C₂-C₂₀alkyl which is interrupted by one or more O or CO, or is phenyl-C₁-C₄alkyl, C₃-C₈cycloalkyl which is uninterrupted or is interrupted by one or more O or CO, or is (CO)R₁₉ or is phenyl which is unsubstituted or substituted by one or more C₁-C₂₀alkyl, halogen, C₁-C₄haloalkyl, OR₁₇, SR₁₈, NR₁₉R₂₀ or 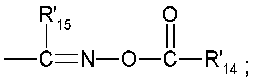 ***provided** that* at least one group 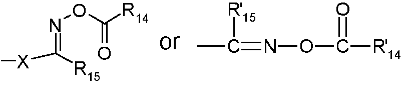 is present in the molecule.

2. Compounds of the formula I according to claim 1, wherein
**R₁, R₂**, **R₃**, **R₄**, **R₅**, **R₆**, **R₇** and **R₈** independently of each other are hydrogen, C₁-C₂₀alkyl, 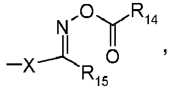 COR₁₆ or NO₂, or R₁ and R₂, R₂ and R₃, R₃ and R₄, R₅ and R₆, R₆ and R₇ or R₇ and R₈ independently of each other together are 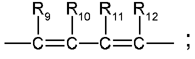 ***provided that*** at least one pair of R₁ and R₂, R₂ and R₃, R₃ and R₄, R₅ and R₆, R₆ and
R₇, or R₇ and R₈ is 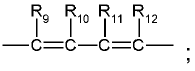 **X** is CO or a direct bond;
R₁₃ is C₁-C₂₀alkyl which is unsubstituted or substituted by one or more halogen, OR₁₇, SR₁₈, COOR₁₇, CONR₁₉R₂₀ or by PO(OCₖH₂ₖ₊₁)₂;
or R₁₃ is C₂-C₂₀alkyl which is interrupted by one or more O, S, NR₂₆ or CO;
or R₁₃ is phenyl or naphthyl both of which are unsubstituted or substituted by one or more 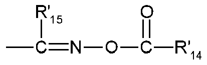 or COR₁₆;
R₁₄ is C₁-C₂₀alkyl, phenyl or C₁-C₈alkoxy;
R₁₅ is phenyl, naphthyl, C₃-C₂₀heteroaryl each of which is unsubstituted or substituted by one or more phenyl, halogen, C₁-C₄haloalkyl, OR₁₇, SR₁₈, or C₂-C₂₀alkyl which is interrupted by one or more O or S, or each of which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₄-C₂₀heteroaryloxycarbonyl, OR₁₇, SR₁₈, NR₁₉R₂₀ or PO(OCₖH₂ₖ₊₁)₂;
or R₁₅ is C₁-C₂₀alkyl which is unsubstituted or substituted by one or more OR₁₇, SR₁₈, C₃-C₈cycloalkyl, C₃-C₂₀heteroaryl, NR₁₉R₂₀, COOR₁₇, CONR₁₉R₂₀ or PO(OCₖH₂ₖ₊₁)₂; **R'₁₄** has one of the meanings as given for R₁₄;
**R'₁₅** has one of the meanings as given for R₁₅;
**R₁₆** is phenyl which is unsubstituted or substituted by one or more OR₁₇, SR₁₈, NR₁₉R₂₀, or by C₂-C₂₀alkyl which is interrupted by one or more O, S, or NR₂₆,
or R₁₆ is phenyl which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₄-C₂₀heteroaryloxycarbonyl, OR₁₇, SR₁₈ or NR₁₉R₂₀;
or R₁₆ is C₁-C₂₀alkyl which is unsubstituted or substituted by halogen, phenyl, OH, SH, CN, C₃-C₆alkenoxy, OCH₂CH₂(CO)O(C₁-C₄alkyl), O(CO)-(C₁-C₄alkyl), or (CO)O(C₁-C₄alkyl);
**R₁₇** is C₁-C₂₀alkyl which is unsubstituted or substituted by one or more halogen, OCH₂CH₂(CO)O(C₁-C₄alkyl), O(C₁-C₄alkyl), (CO)O(C₁-C₄alkyl), C₃-C₂₀cycloalkyl, or by C₃-C₂₀cycloalkyl which is interrupted by one or more O; or
R₁₇ is C₂-C₂₀alkyl which is interrupted by one or more O;
R₁₆ is methyl substituted by (CO)OR₁₇;
**R₁₉** and **R₂₀** independently of one another are hydrogen, phenyl, C₁-C₂₀alkyl, C₁-C₈alkanoyl or C₁-C₈alkanoyloxy;
or R₁₉ and R₂₀ together with the N-atom to which they are attached form a heteroaromatic ring system which is unsubstituded or substituted by 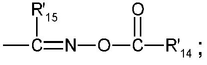 ***provided that*** at least one group 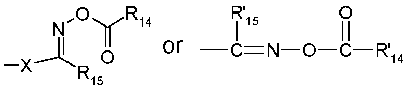 is present in the molecule.

3. Compounds of the formula I according to claim 1, wherein
**R₁, R₂**, **R₃**, **R₄**, **R₅**, **R₆**, **R₇** and **R₈** independently of each other are hydrogen,
or R₁ and R₂, R₃ and R₄, or R₅ and R₆, independently of each other together are 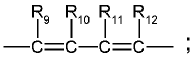 ***provided that*** at least one pair of R₁ and R₂, R₃ and R₄ or R₅ and R₆ is 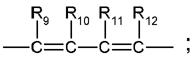 or R₂ is 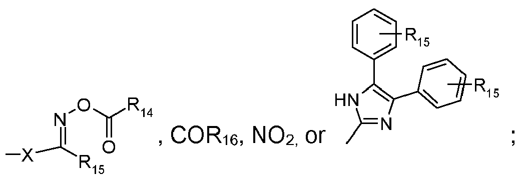 or R₇ is 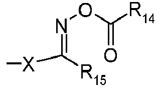 or COR₁₆;
**R₉**, **R₁₁** and **R₁₂** are hydrogen;
**R₁₀** is hydrogen, OR₁₇ or COR₁₆;
**X** is CO or a direct bond;
**R₁₃** is C₁-C₂₀alkyl which is unsubstituted or substituted by one or more halogen, R₁₇, OR₁₇, SR₁₈, or by PO(OCₖH₂ₖ₊₁)₂;
or R₁₃ is C₂-C₂₀alkyl which is interrupted by one or more O;
or R₁₃ is phenyl;
**k** is an integer 2;
**R₁₄** is C₁-C₂₀alkyl or thienyl;
**R₁₅** is phenyl or naphthyl each of which is unsubstituted or substituted by one or more OR₁₇ or C₁-C₂₀alkyl, or R₁₅ is thienyl, hydrogen, C₁-C₂₀alkyl which is unsubstituted or substituted by one or more OR₁₇, SR₁₈, C₃-C₈cycloalkyl, NR₁₉R₂₀ or by COOR₁₇;
or R₁₅ is C₂-C₂₀alkyl which is interrupted by SO₂;
**R₁₆** is phenyl or naphthyl each of which is unsubstituted or substituted by one or more OR₁₇, SR₁₈, NR₁₉R₂₀, or by C₁-C₂₀alkyl;
or R₁₆ is thienyl;
**R₁₇** is hydrogen, C₁-C₈alkanoyl, C₁-C₂₀alkyl which is unsubstituted or substituted by one or more halogen, O(CO)-(C₁-C₄alkyl), O(CO)-(C₂-C₄)alkenyl or by C₃-C₂₀cycloalkyl which is interrupted by one or more O;
or R₁₇ is C₂-C₂₀alkyl which is interrupted by one or more O;
**R₁₈** is C₃-C₂₀cycloalkyl, C₁-C₂₀alkyl which is unsubstituted or is substituted by one or more OH, O(CO)-(C₂-C₄)alkenyl or (CO)OR₁₇;
or R₁₈ is phenyl which is unsubstituted or substituted by one or more halogen;
**R₁₉** and **R₂₀** independently of each other are C₁-C₈alkanoyl or C₁-C₈alkanoyloxy;
or R₁₉ and R₂₀ together with the N-atom to which they are attached form a 5- or 6-membered saturated ring which is interrupted by O;
***provided that*** at least one group 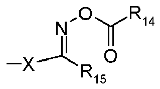 is present in the molecule.

4. A photopolymerizable composition comprising
(a) at least one ethylenically unsaturated photopolymerizable compound and
(b) as photoinitiator, at least one compound of the formula I as defined in claim 1.

5. A photopolymerizable composition according to claim **4,** wherein the component (a) is a resin obtained by the reaction of a saturated or unsaturated polybasic acid anhydride with a product of the reaction of an epoxy resin and an unsaturated monocarboxylic acid.

6. A photopolymerizable composition according to claim 4, additionally to the photoinitiator (b) comprising at least one further photoinitiator (c), and/or other additives (d).

7. A photopolymerizable composition according to claim 6 as further additive (d) comprising a pigment or a mixture of pigments.

8. A photopolymerizable composition according to claim 7 as further additive (d) comprising a dispersant or a mixture of dispersants.

9. A photopolymerizable composition according to anyone of claims **4-8,** comprising 0.05 to 25 % by weight of the photoinitiator (b), or the photoinitiators (b) and (c), based on the composition.

10. A photopolymerizable composition according to anyone of claims **4-9** as further additive (d) comprising a photosensitizer, in particular a compound selected from the group consisting of benzophenone, benzophenone derivatives, thioxanthone, thioxanthone derivatives, anthraquinone, anthraquinone derivatives, coumarin and coumarine derivatives.

11. A photopolymerizable composition according to anyone of claims **4-10** additionally comprising a binder polymer (e), in particular a copolymer of methacrylate and methacrylic acid.

12. A process for the preparation of a compound of the formula I according to claim 1 by reacting the corresponding oxime compound with an acyl halide of the formula II or an anhydride of the formula III 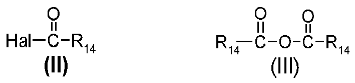 wherein **HaI** is a halogen, in particular Cl, and **R₁₄** is as defined in claim 1, in the presence of a base or a mixture of bases.

13. A process for the photopolymerization of compounds containing ethylenically unsaturated double bonds, which comprises irradiating a composition according to anyone of claims **4-11** with electromagnetic radiation in the range from 150 to 600 nm, or with electron beam or with X-rays.

14. A process according to claim 13 for producing pigmented and nonpigmented paints and varnishes, powder coatings, printing inks, printing plates, adhesives, pressure sensitive adhesives, dental compositions, gel coats, photoresists for electronics, electroplating resists, etch resists, both liquid and dry films, solder resists, resists to manufacture color filters for a variety of display applications, resists to generate structures in the manufacturing processes of plasma-display panels, electroluminescence displays and LCD, spacers for LCD, for holographic data storage (HDS), as composition for encapsulating electrical and electronic components, for producing magnetic recording materials, micromechanical parts, waveguides, optical switches, plating masks, etch masks, colour proofing systems, glass fibre cable coatings, screen printing stencils, for producing three-dimensional objects by means of stereolithography, as image recording material, for holographic recordings, microelectronic circuits, decolorizing materials, decolorizing materials for image recording materials, for image recording materials using microcapsules, as a photoresist material for a UV and visible laser direct imaging system, as a photoresist material used for forming dielectric layers in a sequential build-up layer of a printed circuit board.

15. Coated substrate which is coated on at least one surface with a composition according to claim **4.**

16. Process for the photographic production of relief images, in which a coated substrate according to claim 15 is subjected to imagewise exposure and then the unexposed portions are removed with a developer.

17. A color filter prepared by providing red, green and blue picture elements and a black matrix, all comprising a photosensitive resin and a pigment on a transparent substrate and providing a transparent electrode either on the surface of the substrate or on the surface of the color filter layer, wherein said photosensitive resin comprises a polyfunctional acrylate monomer, an organic polymer binder and a photopolymerization initiator of formula I as defined in claim **1.**

18. Use of a compound of the formula (I) as defined in claim 1 for the photopolymerization of a composition comprising at least one ethylenically unsaturated photopolymerizable compound.

19. Compounds of the formula (IA) 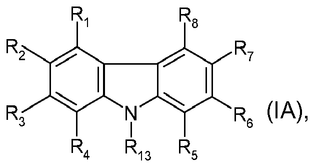 wherein
**R₁, R₂**, **R₃**, **R₄**, **R₅**, **R₆**, **R₇** and **R₈** independently of each other are hydrogen, C₁-C₂₀alkyl, 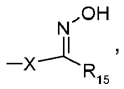 COR₁₆, OR₁₇, halogen, NO₂, or 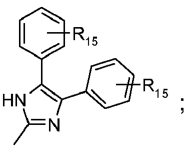 or R₁ and R₂, R₂ and R₃, R₃ and R₄, R₅ and R₆, R₆ and R₇, or R₇ and R₈ independently of each other are C₂-C₁₀alkenyl which is substituted by 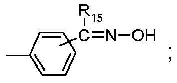 or R₁ and R₂, R₂ and R₃, R₃ and R₄, R₅ and R₆, R₆ and R₇, or R₇ and R₈ independently of each other together are -(CH₂)ₚ-Y-(CH₂)_{q}-;
or R₁ and R₂, R₂ and R₃, R₃ and R₄, R₅ and R₆, R₆ and R₇, or R₇ and R₈ independently of each other together are 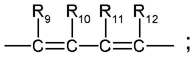 ***provided that*** at least one pair of R₁ and R₂, R₂ and R₃, R₃ and R₄, R₅ and R₆, R₆ and R₇, or R₇ and R₈ is 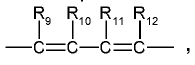 **R₉**, **R₁₀, R₁₁** and **R₁₂** independently of each other are hydrogen, C₁-C₂₀alkyl which is unsubstituted or substituted by one or more halogen, phenyl, CN, OH, SH, C₁-C₄-alkoxy, (CO)OH or by (CO)O(C₁-C₄alkyl);
or R₉, R₁₀, R₁₁, and R₁₂ independently of each other are unsubstituted phenyl or phenyl substituted by one or more C₁-C₆alkyl, halogen, CN, OR₁₇, SR₁₈ or by NR₁₉R₂₀;
or R₉, R₁₀, R₁₁, and R₁₂ independently of each other are halogen, CN, OR₁₇, SR₁₈, SOR₁₈, SO₂R₁₈ or NR₁₉R₂₀, wherein the substituents OR₁₇, SR₁₈ or NR₁₉R₂₀ optionally form 5- or 6-membered rings *via* the radicals R₁₇, R₁₈, R₁₉ and/or R₂₀ with one of the carbon atoms of the naphthyl ring;
or R₉, R₁₀, R₁₁ and R₁₂ independently of each other are 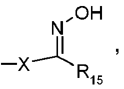 COR₁₆ or NO₂;
**Y** is O, S, NR₂₆ or a direct bond;
**p** is an integer 0,1, 2 or 3;
**q** is an integer 1, 2 or 3;
**X** is CO or a direct bond;
**R₁₃** is C₁-C₂₀alkyl which is unsubstituted or substituted by one or more halogen, R₁₇, COOR₁₇, OR₁₇, SR₁₈, CONR₁₉R₂₀, NR₁₉R₂₀, PO(OCₖH₂ₖ₊₁)₂, or by 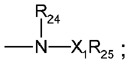 or R₁₃ is C₂-C₂₀alkyl which is interrupted by one or more O, S, SO, SO₂, NR₂₆ or CO, or is C₂-C₁₂alkenyl which is uninterrupted or interrupted by one or more O, CO or NR₂₆, wherein the interrupted C₂-C₂₀alkyl and the uninterrupted or interrupted C₂-C₁₂alkenyl are unsubstituted or are substituted by one or more halogen;
or R₁₃ is C₄-C₈cycloalkenyl, C₂-C₁₂alkinyl, or C₃-C₁₀cycloalkyl which is uninterrupted or interrupted by one or more O, S, CO or NR₂₆;
or R₁₃ is phenyl or naphthyl each of which is unsubstituted or substituted by one or
more OR₁₇, SR₁₈, NR₁₉R₂₀, 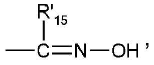 COR₁₆, CN, NO₂, halogen, C₁-C₂₀alkyl, C₁-C₄haloalkyl, C₂-C₂₀alkyl which is interrupted by one or more O, S, CO or NR₂₆, or each of which is substituted by C₃-C₁₀cycloalkyl or by C₃-C₁₀cycloalkyl which is interrupted by one or more O, S, CO, or NR₂₆;
**k** is an integer 1-10;
**R₁₅** is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, PO(OCₖH₂ₖ₊₁)₂, SO-C₁-C₁₀alkyl, SO₂-C₁-C₁₀alkyl, by C₂-C₂₀alkyl which is interrupted by one or more O, S or NR₂₆, or each of which is substituted by C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂oheteroaryloxycarbonyl, OR₁₇, SR₁₈ or NR₁₉R₂₀;
or R₁₅ is hydrogen, C₂-C₁₂alkenyl, C₃-C₈cycloalkyl which is uninterrupted or is interrupted by one or more O, CO or NR₂₆;
or R₁₅ is C₁-C₂₀alkyl which is unsubstituted or substituted by one or more halogen, OR₁₇, SR₁₈, C₃-C₈cycloalkyl, C₃-C₂₀heteroaryl, C₆-C₂₀aryloxycarbonyl, C₃-C₂₀hetero-aryloxycarbonyl, NR₁₉R₂₀, COOR₁₇, CONR₁₉R₂₀, PO(OCₖH₂ₖ₊₁)₂, 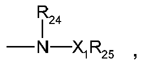 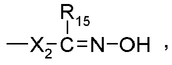 phenyl, or the C₁-C₂₀alkyl is substituted by phenyl which is substituted by halogen, C₁-C₂₀alkyl, C₁-C₄haloalkyl, OR₁₇, SR₁₈, or NR₁₉R₂₀;
or R₁₅ is C₂-C₂₀alkyl which is interrupted by one or more O, SO or SO₂, and which interrupted C₂-C₂₀alkyl is unsubstituted or substituted by one or more halogen, OR₁₇, COOR₁₇, CONR₁₉R₂₀, phenyl or by phenyl which is substituted by O R₁₇, SR₁₈ or NR₁₉R₂₀;
or R₁₅ is C₂-C₂₀alkanoyl or benzoyl which is unsubstituted or substituted by one or more C₁-C₆alkyl, halogen, phenyl, OR₁₇, SR₁₈ or NR₁₉R₂₀;
or R₁₅ is naphthoyl which is unsubstituted or is substituted by one or more OR₁₇ or is C₃-C₁₄heteroarylcarbonyl;
or R₁₅ is C₂-C₁₂alkoxycarbonyl which is uninterrupted or is interrupted by one or more O and which interrupted or uninterrupted C₂-C₁₂alkoxycarbonyl is unsubstituted or substituted by one or more hydroxyl groups;
or R₁₅ is phenoxycarbonyl which is unsubstituted or substituted by one or more C₁-C₆alkyl, halogen, C₁-C₄haloalkyl, phenyl, OR₁₇, SR₁₈ or NR₁₉R₂₀;
or R₁₅ is CN, CONR₁₉R₂₀, NO₂, C₁-C₄haloalkyl, S(O)ₘ-C₁-C₆alkyl; S(O)ₘ-phenyl which is unsubstituted or substituted by C₁-C₁₂alkyl or SO₂-C₁-C₆alkyl;
or R₁₅ is SO₂O-pheny which is unsubstituted or substituted by C₁-C₁₂alkyl; or is diphenyl phosphinoyl or di-(C₁-C₄alkoxy)-phosphinoyl;
m is 1 or 2;
**R'₁₅** has one of the meanings as given for R₁₅;
**X₁** is O, S, SO or SO₂;
**X₂** is O, CO, S or a direct bond;
**R₁₆** is C₆-C₂₀aryl or C₃-C₂₀heteroaryl each of which is unsubstituted or substituted by one or more phenyl, halogen, C₁-C₄haloalkyl, CN, NO₂, OR₁₇, SR₁₈, NR₁₉R₂₀, or by C₁-C₂₀alkyl which is interrupted by one or more O, S, or NR₂₆, or each of which is substituted by one or more C₁-C₂₀alkyl which is unsubstituted or substituted by one or more halogen, COOR₁₇, CONR₁₉R₂₀, phenyl, C₃-C₈cycloalkyl, C₃-C₂₀heteroaryl, C₆-C₂₀aryloxycarbonyl, C₃-C₂₀heteroaryloxycarbonyl, OR₁₇, SR₁₈ or NR₁₉R₂₀;
or R₁₆ is hydrogen, C₁-C₂₀alkyl which is unsubstituted or substituted by one or more halogen, phenyl, OH, SH, CN, C₃-C₆alkenoxy, OCH₂CH₂CN, OCH₂CH₂(CO)O(C₁-C₄alkyl), O(CO)-(C₁-C₄alkyl), O(CO)-phenyl, (CO)OH or (CO)O(C₁-C₄alkyl);
or R₁₆ is C₂-C₁₂alkyl which is interrupted by one or more O, S or NR₂₆;
or R₁₆ is (CH₂CH₂O)ₙ₊₁H, (CH₂CH₂O)ₙ(CO)-(C₁-C₈alkyl), C₂-C₁₂alkenyl or C₃-C₈cycloalkyl;
or R₁₆ is phenyl substituted by SR₁₈ wherein the radical R₁₈ denotes a direct bond to the phenyl or naphthyl ring of the carbazole moiety to which the COR₁₆ group is attached;
n is 1-20;
**R₁₇** is hydrogen, phenyl-C₁-C₃alkyl, C₁-C₂₀alkyl which is unsubstituted or substituted by one or more halogen, OH, SH, CN, C₃-C₆alkenoxy, OCH₂CH₂CN, OCH₂CH₂(CO)O(C₁-C₄alkyl), O(CO)-(C₁-C₄alkyl), O(CO)-(C₂-C₄)alkenyl, O(CO)-phenyl, (CO)OH, (CO)O(C₁-C₄alkyl), S0₂-(C₁-C₄haloalkyl), O(C₁-C₄haloalkyl), C₃-C₂₀cycloalkyl, or by C₃-C₂₀cycloalkyl which is interrupted by one or more O;
or R₁₇ is C₂-C₂₀alkyl which is interrupted by one or more O, S or NR₂₆;
or R₁₇ is (CH₂CH₂O)ₙ₋₁H, (CH₂CH₂O)ₙ(CO)-(C₁-C₈alkyl), C₁-C₈alkanoyl, C₂-C₁₂alkenyl, C₃-C₆alkenoyl, or C₃-C₂₀cycloalkyl which is uninterrupted or interrupted by one or more O, S, CO or NR₂₆;
or R₁₇ is C₁-C₈alkyl-C₃-C₁₀cycloalkyl which is uninterrupted or interrupted by one or more O;
or R₁₇ is benzoyl which is unsubstituted or substituted by one or more C₁-C₆alkyl, halogen, OH or C₁-C₃alkoxy;
or R₁₇ is phenyl, naphthyl or C₃-C₂₀heteroaryl, each of which is unsubstituted or substituted by one or more halogen, OH, C₁-C₁₂alkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenyl-amino or 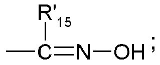 or R₁₇ forms a direct bond to one of the carbon atoms of the phenyl or naphthyl ring on
which the group 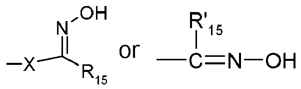 is located;
**R₁₈** is hydrogen, C₂-C₁₂alkenyl, C₃-C₂₀cycloalkyl, or phenyl-C₁-C₃alkyl, wherein the C₂-C₁₂alkenyl, C₃-C₂₀cycloalkyl, or phenyl-C₁-C₃alkyl is uninterrupted or interrupted by one or more O, S, CO, NR₂₆ or COOR₁₇;
or R₁₈ is C₁-C₂₀alkyl which is unsubstituted or is substituted by one or more OH, SH, CN, C₃-C₆alkenoxy, OCH₂CH₂CN, OCH₂CH₂(CO)O(C₁-C₄alkyl), O(CO)-(C₂-C₄)alkenyl, O(CO)-(C₁-C₄alkyl), O(CO)-phenyl or (CO)OR₁₇;
or R₁₈ is C₂-C₂₀alkyl which is interrupted by one or more O, S, CO, NR₂₆ or COOR₁₇;
or R₁₈ is (CH₂CH₂O)ₙH, (CH₂CH₂O)ₙ(CO)-(C₁-C₈alkyl), C₂-C₈alkanoyl or C₃-C₆alkenoyl; or R₁₈ is benzoyl which is unsubstituted or substituted by one or more C₁-C₆alkyl, halogen, OH, C₁-C₄alkoxy or C₁-C₄alkylsulfanyl;
or R₁₈ is phenyl, naphthyl or C₃-C₂₀heteroaryl, each of which is unsubstituted or substituted by one or more halogen, C₁-C₁₂alkyl, C₁-C₄haloalkyl, C₁-C₁₂alkoxy, CN, NO₂, phenyl-C₁-C₃alkyloxy, phenoxy, C₁-C₁₂alkylsulfanyl, phenylsulfanyl, N(C₁-C₁₂alkyl)₂, diphenylamino, (CO)O(C₁-C₈alkyl), (CO)-C₁-C₈alkyl, (CO)N(C₁-C₈alkyl)₂ or 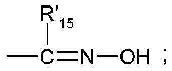 **R₁₉** and **R₂₀** independently of each other are hydrogen, C₁-C₂₀alkyl, C₂-C₄hydroxyalkyl, C₂-C₁₀alkoxyalkyl, C₂-C₅alkenyl, C₃-C₂₀cycloalkyl, phenyl-C₁-C₃alkyl, SO₂-(C₁-C₄haloalkyl), C₁-C₈alkanoyl, C₁-C₈alkanoyloxy, C₃-C₁₂alkenoyl or benzoyl;
or R₁₉ and R₂₀ are phenyl, naphthyl or C₃-C₂₀heteroaryl, each of which is unsubstituted or substituted by one or more halogen, C₁-C₄haloalkyl, C₁-C₂₀alkoxy, C₁-C₁₂alkyl, benzoyl or C₁-C₁₂alkoxy;
or R₁₉ and R₂₀ together with the N-atom to which they are attached form a 5- or 6-membered saturated or unsaturated ring which is uninterrupted or is interrupted by O, S or NR₁₇, and which 5- or 6-membered saturated or unsaturated ring is unsubstituted or substituted by one or more C₁-C₂₀alkyl, C₁-C₂₀alkoxy, =O, OR₁₇, SR₁₈, NR₂₁R₂₂,
(CO)R₂₃, NO₂, halogen, C₁-C₄-haloalkyl, CN, phenyl, 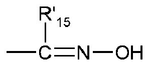 or by C₃-C₂₀cyclalkyl which is uninterrupted or is interrupted by one or more O, S, CO or NR₁₇; or R₁₉ and R₂₀ together with the N-atom to which they are attached form a heteroaromatic ring system which is unsubstituted or substituted by one or more C₁-C₂₀alkyl, C₁-C₄haloalkyl, C₁-C₂₀alkoxy, =O, OR₁₇, SR₁₈, NR₂₁R₂₂, (CO)R₂₃, 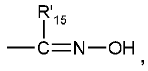 halogen, NO₂, CN, phenyl or by C₃-C₂₀cycloalkyl which is uninterrupted or is interrupted by one or more O, S, CO or NR₁₇;
R₂₁ and R₂₂ independently of each other are hydrogen, C₁-C₂₀alkyl, C₁-C₄haloalkyl, C₃-C₁₀cycloalkyl or phenyl;
or R₂₁ and R₂₂ together with N-atom to which they are attached form a 5- or 6-membered saturated or unsaturated ring, which is uninterrupted or is interrupted by O, S or NR₂₆, and which 5- or 6-membered saturated or unsaturated ring is not condensed or to which 5- or 6-membered saturated or unsaturated ring a benzene ring is condensed;
**R₂₃** is hydrogen, OH, C₁-C₂₀alkyl, C₁-C₄haloalkyl, C₂-C₂₀alkyl which is interrupted by one or more O, CO or NR₂₆, C₃-C₂₀cycloalkyl which is uninterrupted or is interrupted by O, S, CO or NR₂₆,
or R₂₃ is phenyl, naphthyl, phenyl-C₁-C₄alkyl, OR₁₇, SR₁₈ or NR₂₁R₂₂;
**R₂₄** is (CO)OR₁₇, CONR₁₉R₂₀, (CO)R₁₇; or R₂₄ has one of the meanings given for R₁₉ and R₂₀;
**R₂₅** is COOR₁₇, CONR₁₉R₂₀, (CO)R₁₇; or R₂₅ has one of the meanings given for R₁₇;
**R₂₆** is hydrogen, C₁-C₂₀alkyl, C₁-C₄haloalkyl, C₂-C₂₀alkyl which is interrupted by one or more O or CO, or is phenyl-C₁-C₄alkyl, C₃-C₈cycloalkyl which is uninterrupted or is interrupted by one or more O or CO, or is (CO)R₁₉ or is phenyl which is unsubstituted or substituted by one or more C₁-C₂₀alkyl, halogen, C₁-C₄haloalkyl, OR₁₇, SR₁₈, NR₁₉R₂₀ 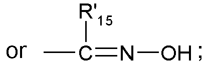 ***provided that* at least one group** 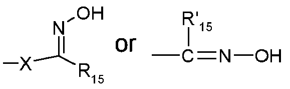 is present in the molecule.

## Patentansprüche

1. Verbindungen der Formel I 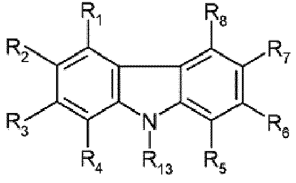 wobei
R₁, R₂, R₃, R₄, R₅, R₆, R₇ und R₈ unabhängig voneinander für Wasserstoff, C₁-C₂₀-Alkyl, 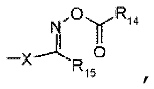 COR₁₆, OR₁₇, Halogen, NO₂ oder 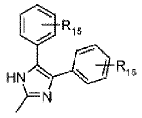 stehen;
oder R₁ und R₂, R₂ und R₃, R₃ und R₄, R₅ und R₆, R₆ und R₇ oder R₇ und R₈ unabhängig voneinander für C₂-C₁₀-Alkenyl, das durch 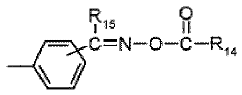 substituiert ist, stehen;
oder R₁ und R₂, R₂ und R₃, R₃ und R₄, R₅ und R₆, R₆ und R₇ oder R₇ und R₈ unabhängig voneinander für - (CH₂)ₚ-Y-(CH₂)_{q}- stehen;
oder R₁ und R₂, R₂ und R₃, R₃ und R₄, R₅ und R₆, R₆ und R₇ oder R₇ und R₈ unabhängig voneinander zusammen für 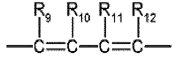 stehen;
mit der Maßgabe, dass mindestens ein Paar von R₁ und R₂, R₂ und R₃, R₃ und R₄, R₅ und R₆, R₆ und R₇
oder R₇ und R₈ für 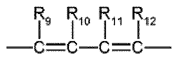 steht,
R₉, R₁₀, R₁₁ und R₁₂ unabhängig voneinander für Wasserstoff oder C₁-C₂₀-Alkyl, das unsubstituiert oder durch ein oder mehrere Halogen, Phenyl, CN, OH, SH, C₁-C₄-Alkoxy, (CO)OH oder durch (CO)O(C₁-C₄-Alkyl) substituiert ist, stehen;
oder R₉, R₁₀, R₁₁ und R₁₂ unabhängig voneinander für unsubstituiertes Phenyl oder Phenyl, das durch ein oder mehrere C₁-C₆-Alkyl, Halogen, CN, OR₁₇, SR₁₈ oder durch NR₁₉R₂₀ substituiert ist, stehen;
oder R₉, R₁₀, R₁₁ und R₁₂ unabhängig voneinander für Halogen, CN, OR₁₇, SR₁₈, SOR₁₈, SO₂R₁₈ oder NR₁₉R₂₀ stehen, wobei die Substituenten OR₁₇, SR₁₈ oder NR₁₉R₂₀ gegebenenfalls über die Reste R₁₇, R₁₈, R₁₉ und/oder R₂₀ mit einem der Kohlenstoffatome des Naphthylrings 5- oder 6-gliedrige Ringe bilden; oder R₉, R₁₀, R₁₁ und R₁₂ unabhängig voneinander für 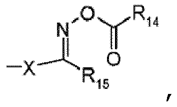 COR₁₆ oder NO₂ stehen;
Y für 0, S, NR₂₆ oder eine direkte Bindung steht;
p für eine ganze Zahl mit einem Wert von 0, 1, 2 oder 3 steht;
q für eine ganze Zahl mit einem Wert von 1, 2 oder 3 steht;
X für CO oder eine direkte Bindung steht;
R₁₃ für C₁-C₂₀-Alkyl, das unsubstituiert oder durch ein oder mehrere Halogen, R₁₇, COOR₁₇, OR₁₇, SR₁₈, CONR₁₉R₂₀, NR₁₉R₂₀, PO (OCₖH₂ₖ₊₁)₂ oder durch 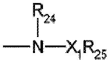 substituiert ist, steht;
oder R₁₃ für C₂-C₂₀-Alkyl, das durch ein oder mehrere 0, S, SO, SO₂, NR₂₆ oder CO unterbrochen ist, oder für C₂-C₁₂-Alkenyl, das ununterbrochen oder durch ein oder mehrere 0, CO oder NR₂₆ unterbrochen ist, steht,
wobei das unterbrochene C₂-C₂₀-Alkyl und das ununterbrochene oder unterbrochene C₂-C₁₂-Alkenyl unsubstituiert oder durch ein oder mehrere Halogen substituiert sind;
oder R₁₃ für C₄-C₈-Cycloalkenyl, C₂-C₁₂-Alkinyl oder C₃-C₁₀-Cycloalkyl, das ununterbrochen oder durch ein oder mehrere 0, S, CO oder NR₂₆ unterbrochen ist, steht;
oder R₁₃ für Phenyl oder Naphthyl steht, wobei jede dieser Gruppen unsubstituiert oder durch ein oder
mehrere OR₁₇, SR₁₈, NR₁₉R₂₀, 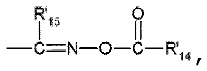 COR₁₆, CN, NO₂, Halogen, C₁-C₂₀-Alkyl, C₁-C₄-Halogenalkyl, C₂-C₂₀-Alkyl, das durch ein oder mehrere 0, S, CO oder NR₂₆ unterbrochen ist, substituiert ist oder jede dieser Gruppen durch C₃-C₁₀-Cycloalkyl oder C₃-C₁₀-Cycloalkyl, das durch ein oder mehrere 0, S, CO oder NR₂₆ unterbrochen ist, substituiert ist;
k für eine ganze Zahl mit einem Wert von 1-10 steht;
R₁₄ für Wasserstoff, C₃-C₈-Cycloalkyl, C₂-C₅-Alkenyl, C₁-C₂₀-Alkoxy oder C₁-C₂₀-Alkyl, das unsubstituiert oder durch ein oder mehrere Halogen, Phenyl, C₁-C₂₀-Alkylphenyl oder CN substituiert ist, steht;
oder R₁₄ für Phenyl oder Naphthyl steht, wobei jede dieser Gruppen unsubstituiert oder durch ein oder mehrere C₁-C₆-Alkyl, C₁-C₄-Halogenalkyl, Halogen, CN, OR₁₇, SR₁₈ und/oder NR₁₉R₂₀ substituiert ist; oder R₁₄ für C₃-C₂₀-Heteroaryl, C₁-C₈-Alkoxy, Benzyloxy oder Phenoxy steht, wobei Benzyloxy und Phenoxy unsubstituiert oder durch ein oder mehrere C₁-C₆-Alkyl, C₁-C₄-Halogenalkyl und/oder Halogen substituiert sind;
R₁₅ für C₆-C₂₀-Aryl oder C₃-C₂₀-Heteroaryl steht, wobei jede dieser Gruppen unsubstituiert oder durch ein oder mehrere Phenyl, Halogen, C₁-C₄-Halogenalkyl, CN, NO₂, OR₁₇, SR₁₈, NR₁₉R₂₀, PO(OCₖH₂ₖ₊₁)₂, SO-C₁-C₁₀-Alkyl, SO₂-C₁-C₁₀-Alkyl, durch C₂-C₂₀-Alkyl, das durch ein oder mehrere 0, S oder NR₂₆ unterbrochen ist, substituiert ist oder wobei jede dieser Gruppen durch C₁-C₂₀-Alkyl, das unsubstituiert oder durch ein oder mehrere Halogen, COOR₁₇, CONR₁₉R₂₀, Phenyl, C₃-C₈-Cycloalkyl, C₃-C₂₀-Heteroaryl, C₆-C₂₀-Aryloxycarbonyl, C₃-C₂₀-Heteroaryloxycarbonyl, OR₁₇, SR₁₈ oder NR₁₉R₂₀ substituiert ist, substituiert ist;
oder R₁₅ für Wasserstoff, C₂-C₁₂-Alkenyl, C₃-C₈-Cycloalkyl, das ununterbrochen oder durch ein oder mehrere 0, CO oder NR₂₆ unterbrochen ist, steht; oder R₁₅ für C₁-C₂₀-Alkyl, das unsubstituiert oder durch ein oder mehrere Halogen, OR₁₇, SR₁₈, C₃-C₈-Cycloalkyl, C₃-C₂₀-Heteroaryl, C₆-C₂₀-Aryloxy-carbonyl, C₃-C₂₀-Heteroaryloxycarbonyl, NR₁₉R₂₀, COOR₁₇, CONR₁₉R₂₀, PO (OCₖH₂ₖ₊₁)₂, 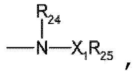 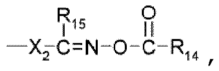 Phenyl substituiert ist, steht oder das C₁-C₂₀-Alkyl durch Phenyl, das durch Halogen, C₁-C₂₀-Alkyl, C₁-C₄-Halogenalkyl, OR₁₇, SR₁₈ oder NR₁₉R₂₀, substituiert ist, substituiert ist;
oder R₁₅ für C₂-C₂₀-Alkyl, das durch ein oder mehrere 0, SO oder SO₂ unterbrochen ist, steht, und wobei das unterbrochene C₂-C₂₀-Alkyl durch ein oder mehrere Halogen, OR₁₇, COOR₁₇, CONR₁₉R₂₀, Phenyl oder durch Phenyl, das durch OR₁₇, SR₁₈ oder NR₁₉R₂₀ substituiert ist, substituiert ist;
oder R₁₅ für C₂-C₂₀-Alkanoyl oder Benzoyl, das unsubstituiert oder durch ein oder mehrere C₁-C₆-Alkyl, Halogen, Phenyl, OR₁₇, SR₁₈ oder NR₁₉R₂₀ substituiert ist, steht;
oder R₁₅ für Naphthoyl, das unsubstituiert oder durch ein oder mehrere OR₁₇ substituiert ist, oder für C₃-C₁₄-Heteroarylcarbonyl steht;
oder R₁₅ für C₂-C₁₂-Alkoxycarbonyl, das ununterbrochen oder durch ein oder mehrere 0 unterbrochen ist, steht, wobei das unterbrochene oder ununterbrochene C₂-C₁₂-Alkoxycarbonyl unsubstituiert oder durch eine oder mehrere Hydroxylgruppen substituiert ist;
oder R₁₅ für Phenoxycarbonyl, das unsubstituiert oder durch ein oder mehrere C₁-C₆-Alkyl, Halogen, C₁-C₄-Halogenalkyl, Phenyl, OR₁₇, SR₁₈ oder NR₁₉R₂₀ substituiert ist, steht;
oder R₁₅ für CN, CONR₁₉R₂₀, NO₂, C₁-C₄-Halogenalkyl, S(O)ₘ-C₁-C₆-Alkyl oder S(O)ₘ-Phenyl, das unsubstituiert oder durch C₁-C₁₂-Alkyl oder SO₂-C₁-C₆-Alkyl substituiert ist, steht;
oder R₁₅ für SO₂O-Phenyl, das unsubstituiert oder durch C₁-C₁₂-Alkyl substituiert ist; oder für Di-phenylphosphinoyl oder Di(C₁-C₄-alkoxy)phosphinoyl steht;
m für 1 oder 2 steht;
R'₁₄ eine der für R₁₄ angegebenen Bedeutungen besitzt;
R'₁₅ eine der für R₁₅ angegebenen Bedeutungen besitzt;
X₁ für 0, S, SO oder SO₂ steht;
X₂ für 0, CO, S oder eine direkte Bindung steht;
R₁₆ für C₆-C₂₀-Aryl oder C₃-C₂₀-Heteroaryl steht, wobei jede dieser Gruppen unsubstituiert oder durch ein oder mehrere Phenyl, Halogen, C₁-C₄-Halogenalkyl, CN, NO₂, OR₁₇, SR₁₈, NR₁₉R₂₀ oder durch C₁-C₂₀-Alkyl, das durch ein oder mehrere 0, S oder NR₂₆ unterbrochen ist, substituiert ist oder wobei jede dieser Gruppen durch ein oder mehrere C₁-C₂₀-Alkyl, das unsubstituiert oder durch ein oder mehrere Halogen, COOR₁₇, CONR₁₉R₂₀, Phenyl, C₃-C₈-Cycloalkyl, C₃-C₂₀-Heteroaryl, C₆-C₂₀-Aryloxy-carbonyl, C₃-C₂₀-Heteroaryloxycarbonyl, OR₁₇, SR₁₈ oder NR₁₉R₂₀ substituiert ist, substituiert ist; oder R₁₆ für Wasserstoff oder C₁-C₂₀-Alkyl, das unsubstituiert oder durch ein oder mehrere Halogen, Phenyl, OH, SH, CN, C₃-C₆-Alkenoxy, OCH₂CH₂CN, OCH₂CH₂(CO)O(C₁-C₄-Alkyl)O(CO)-(C₁-C₄-Alkyl), O(CO)-Phenyl, (CO)OH oder (CO)O(C₁-C₄-Alkyl) substituiert ist, steht;
oder R₁₆ für C₂-C₁₂-Alkyl, das durch ein oder mehrere 0, S oder NR₂₆ unterbrochen ist, steht; oder R₁₆ für (CH₂CH₂O)ₙ₊₁H, (CH₂CH₂O)ₙ(CO)-(C₁-C₈-Alkyl), C₂-C₁₂-Alkenyl oder C₃-C₈-Cycloalkyl steht; oder R₁₆ für Phenyl, das durch SR₁₈ substituiert ist, steht, wobei der Rest R₁₈ eine direkte Bindung zum Phenyl- bzw. Naphthylring der Carbazolgruppierung, an die die COR₁₆-Gruppe gebunden ist, bedeutet;
n für 1-20 steht;
R₁₇ für Wasserstoff, Phenyl-C₁-C₃-alkyl oder C₁-C₂₀-Alkyl, das unsubstituiert oder durch ein oder mehrere Halogen, OH, SH, CN, C₃-C₆-Alkenoxy, OCH₂CH₂CN, OCH₂CH₂ (CO)0 (C₁-C₄-Alkyl), O(CO)-(C₁-C₄-Alkyl), O(CO)-(C₂-C₄-Alkenyl), O(CO)-Phenyl, (CO)OH, (CO)O(C₁-C₄-Alkyl), C₃-C₂₀-Cycloalkyl, SO₂-(C₁-C₄-Halogenalkyl), O(C₁-C₄-Halogenalkyl) oder durch C₃-C₂₀-Cycloalkyl, das durch ein oder mehrere 0 unterbrochen ist, substituiert ist, steht;
oder R₁₇ für C₂-C₂₀-Alkyl, das durch ein oder mehrere 0, S oder NR₂₆ unterbrochen ist, steht; oder R₁₇ für (CH₂CH₂O)ₙ₊₁H, (CH₂CH₂O)ₙ(CO)-(C₁-C₈-Alkyl), C₁-C₈-Alkanoyl, C₂-C₁₂-Alkenyl, C₃-C₆-Alkenoyl oder C₃-C₂₀-Cycloalkyl steht, das ununterbrochen oder durch ein oder mehrere 0, S, CO oder NR₂₆ unterbrochen ist;
oder R₁₇ für C₁-C₈-Alkyl-C₃-C₁₀-cykloalkyl steht, das ununterbrochen oder durch ein oder mehrere 0 unterbrochen ist;
oder R₁₇ für Benzoyl, das unsubstituiert oder durch ein oder mehrere C₁-C₆-Alkyl, Halogen, OH oder C₁-C₃-Alkoxy substituiert ist, steht;
oder R₁₇ für Phenyl, Naphthyl oder C₃-C₂₀-Heteroaryl steht, wobei jede dieser Gruppen unsubstituiert oder durch ein oder mehrere Halogen, OH, C₁-C₁₂-Alkyl, C₁-C₁₂-Alkoxy, CN, NO₂, Phenyl-C₁-C₃-alkyloxy, Phenoxy, C₁-C₁₂-Alkylsulfanyl, Phenylsulfanyl, N(C₁-C₁₂-Alkyl)₂, Diphenylamino oder 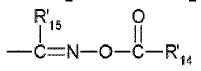 substituiert ist;
oder R₁₇ eine direkte Bindung zu einem der Kohlenstoffatome des Phenyl- bzw. Naphthylrings, an dem sich die Gruppe 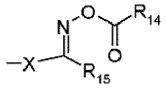 bzw. 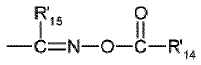 befindet, bildet;
R₁₈ für Wasserstoff, C₂-C₁₂-Alkenyl, C₃-C₂₀-Cycloalkyl oder Phenyl-C₁-C₃-alkyl steht, wobei das C₂-C₁₂-Alkenyl, C₃-C₂₀-Cycloalkyl oder Phenyl-C₁-C₃-alkyl ununterbrochen oder durch ein oder mehrere 0, S, CO, NR₂₆ oder COOR₁₇ unterbrochen ist;
oder R₁₈ für C₁-C₂₀-Alkyl, das unsubstituiert oder durch ein oder mehrere OH, SH, CN, C₃-C₆-Alkenoxy, OCH₂CH₂CN, OCH₂CH₂(CO)O(C₁-C₄-Alkyl), O(CO)-(C₂-C₄)-Alkenyl, O(CO)-(C₁-C₄-Alkyl), O(CO)-Phenyl oder (CO)OR₁₇ substituiert ist, steht;
oder R₁₈ für C₂-C₂₀-Alkyl, das durch ein oder mehrere 0, S, CO, NR₂₆ oder COOR₁₇ unterbrochen ist, steht;
oder R₁₈ für (CH₂CH₂O)ₙH, (CH₂CH₂O)ₙ(CO)-(C₁-C₈-Alkyl), C₂-C₈-Alkanoyl oder C₃-C₆-Alkenoyl steht; oder R₁₈ für Benzoyl, das unsubstituiert oder durch ein oder mehrere C₁-C₆-Alkyl, Halogen, OH, C₁-C₄-Alkoxy oder C₁-C₄-Alkylsulfanyl substituiert ist, steht;
oder R₁₈ für Phenyl, Naphthyl oder C₃-C₂₀-Heteroaryl steht, wobei jede dieser Gruppen unsubstituiert oder durch ein oder mehrere Halogen, C₁-C₁₂-Alkyl, C₁-C₄-Halogenalkyl, C₁-C₁₂-Alkoxy, CN, NO₂, Phenyl-C₁-C₃-alkyloxy, Phenoxy, C₁-C₁₂-Alkylsulfanyl, Phenylsulfanyl, N(C₁-C₁₂-Alkyl)₂, Diphenylamino, (CO)O(C₁-C₈-Alkyl), (CO)-C₁-C₈-Alkyl, (CO)N(C₁-C₈-Alkyl)₂ oder 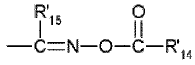 substituiert ist;
R₁₉ und R₂₀ unabhängig voneinander für Wasserstoff, C₁-C₂₀-Alkyl, C₂-C₄-Hydroxyalkyl, C₂-C₁₀-Alkoxyalkyl, C₂-C₅-Alkenyl, C₃-C₂₀-Cycloalkyl, Phenyl-C₁-C₃-alkyl, C₁-C₈-Alkanoyl, C₁-C₈-Alkanoyloxy, C₃-C₁₂-Alkenoyl, SO₂-(C₁-C₄-Halogenalkyl) oder Benzoyl stehen;
oder R₁₉ und R₂₀ für Phenyl, Naphthyl oder C₃-C₂₀-Heteroaryl stehen, wobei jede dieser Gruppen unsubstituiert oder durch ein oder mehrere Halogen, C₁-C₄-Halogenalkyl, C₁-C₂₀-Alkoxy, C₁-C₁₂-Alkyl, Benzoyl oder C₁-C₁₂-Alkoxy substituiert ist; oder R₁₉ und R₂₀ zusammen mit dem N-Atom, an das sie gebunden sind, einen 5- oder 6-gliedrigen gesättigten oder ungesättigten Ring bilden, der ununterbrochen oder durch 0, S oder NR₁₇ unterbrochen ist und unsubstituiert oder durch ein oder mehrere C₁-C₂₀-Alkyl, C₁-C₂₀-Alkoxy, =0, OR₁₇, SR₁₈, NR₂₁R₂₂, (CO)R₂₃, NO₂, Halogen, C₁-C₄-Halogenalkyl, CN, Phenyl, 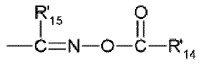 oder durch C₃-C₂₀-Cycloalkyl, das ununterbrochen oder durch ein oder mehrere 0, S, CO oder NR₁₇ unterbrochen ist, substituiert ist;
oder R₁₉ und R₂₀ zusammen mit dem N-Atom, an das sie gebunden sind, ein heteroaromatisches Ringsystem bilden, das unsubstituiert oder durch ein oder mehrere C₁-C₂₀-Alkyl, C₁-C₄-Halogenalkyl, C₁-C₂₀-Alkoxy, =O, OR₁₇, SR₁₈, NR₂₁R₂₂, (CO)R₂₃, 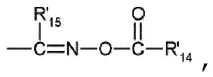 Halogen, NO₂, CN, Phenyl oder durch C₃-C₂₀-Cycloalkyl, das ununterbrochen oder durch ein oder mehrere 0, S, CO oder NR₁₇ unterbrochen ist, substituiert ist;
R₂₁ und R₂₂ unabhängig voneinander für Wasserstoff, C₁-C₂₀-Alkyl, C₁-C₄-Halogenalkyl, C₃-C₁₀-Cycloalkyl oder Phenyl stehen;
oder R₂₁ und R₂₂ zusammen mit dem N-Atom, an das sie gebunden sind, einen 5- oder 6-gliedrigen gesättigten oder ungesättigten Ring bilden, der ununterbrochen oder durch 0, S oder NR₂₆ unterbrochen ist und nicht kondensiert ist oder an den ein Benzolring kondensiert ist;
R₂₃ für Wasserstoff, OH, C₁-C₂₀-Alkyl, C₁-C₄-Halogenalkyl, C₂-C₂₀-Alkyl, das durch ein oder mehrere 0, CO oder NR₂₆ unterbrochen ist, oder C₃-C₂₀-Cycloalkyl, das ununterbrochen oder durch 0, S, CO oder NR₂₆ unterbrochen ist, steht;
oder R₂₃ für Phenyl, Naphthyl, Phenyl-C₁-C₄-alkyl, OR₁₇, SR₁₈ oder NR₂₁R₂₂ steht;
R₂₄ für (CO)OR₁₇, CONR₁₉R₂₀ oder (CO)R₁₇ steht oder eine der für R₁₉ und R₂₀ angegebenen Bedeutungen besitzt;
R₂₅ für COOR₁₇, CONR₁₉R₂₀ oder (CO)R₁₇ steht oder eine der für R₁₇ angegebenen Bedeutungen besitzt;
R₂₆ für Wasserstoff, C₁-C₂₀-Alkyl, C₁-C₄-Halogenalkyl, C₂-C₂₀-Alkyl, das durch ein oder mehrere 0 oder CO unterbrochen ist, oder für Phenyl-C₁-C₄-alkyl, C₃-C₈-Cycloalkyl, das ununterbrochen oder durch ein oder mehrere 0 oder CO unterbrochen ist, oder für (CO)R₁₉ oder für Phenyl, das unsubstituiert oder durch ein oder mehrere C₁-C₂₀-Alkyl, Halogen, C₁-C₄-Halogenalkyl, OR₁₇, SR₁₈, NR₁₉R₂₀ oder 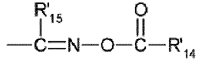 substituiert ist, steht;
mit der Maßgabe, dass in dem Molekül mindestens eine Gruppe 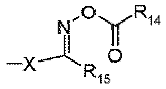 oder 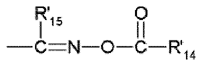 vorliegt.

2. Verbindungen der Formel I nach Anspruch 1, wobei R₁, R₂, R₃, R₄, R₅, R₆, R₇ und R₈ unabhängig voneinander für Wasserstoff, C₁-C₂₀-Alkyl, 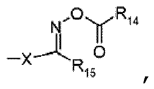 COR₁₆ oder NO₂ stehen;
oder R₁ und R₂, R₂ und R₃, R₃ und R₄, R₅ und R₆, R₆ und R₇ oder R₇ und R₈ unabhängig voneinander zusammen für 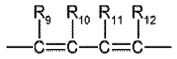 stehen;
mit der Maßgabe, dass mindestens ein Paar von R₁ und R₂, R₂ und R₃, R₃ und R₄, R₅ und R₆, R₆ und R₇ oder R₇ und R₈ für 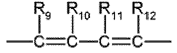 steht,
X für CO oder eine direkte Bindung steht;
R₁₃ für C₁-C₂₀-Alkyl, das unsubstituiert oder durch ein oder mehrere Halogen, OR₁₇, SR₁₈, COOR₁₇, CONR₁₉R₂₀ oder durch PO(OCₖH₂ₖ₊₁)₂ substituiert ist, steht;
oder R₁₃ für C₂-C₂₀-Alkyl, das durch ein oder mehrere 0, S, NR₂₆ oder CO unterbrochen ist, steht; oder R₁₃ für Phenyl oder Naphthyl steht, wobei diese beiden Gruppen unsubstituiert oder durch ein
oder mehrere 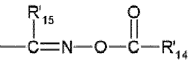 oder COR₁₆ substituiert sind;
R₁₄ für C₁-C₂₀-Alkyl, Phenyl oder C₁-C₈-Alkoxy steht; R₁₅ für Phenyl, Naphthyl oder C₃-C₂₀-Heteroaryl steht, wobei jede dieser Gruppen unsubstituiert oder durch ein oder mehrere Phenyl, Halogen, C₁-C₄-Halogenalkyl, OR₁₇, SR₁₈ oder C₂-C₂₀-Alkyl, das durch ein oder mehrere 0 oder S unterbrochen ist, substituiert ist oder wobei jede dieser Gruppen durch ein oder mehrere C₁-C₂₀-Alkyl, das unsubstituiert oder durch ein oder mehrere Halogen, COOR₁₇, CONR₁₉R₂₀, Phenyl, C₃-C₈-Cycloalkyl, C₃-C₂₀-Heteroaryl, C₆-C₂₀-Aryloxycarbonyl, C₄-C₂₀-Heteroaryloxycarbonyl, OR₁₇, SR₁₈, NR₁₉R₂₀ oder PO(OCₖH₂ₖ₊₁)₂ substituiert ist, substituiert ist; oder R₁₅ für C₁-C₂₀-Alkyl, das unsubstituiert oder durch ein oder mehrere OR₁₇, SR₁₈, C₃-C₈-Cycloalkyl, C₃-C₂₀-Heteroaryl, NR₁₉R₂₀, COOR₁₇, CONR₁₉R₂₀ oder PO(OCₖH₂ₖ₊₁)₂ substituiert ist, steht;
R'₁₄ eine der für R₁₄ angegebenen Bedeutungen besitzt;
R'₁₅ eine der für R₁₅ angegebenen Bedeutungen besitzt;
R₁₆ für Phenyl, das unsubstituiert oder durch ein oder mehrere OR₁₇, SR₁₈, NR₁₉R₂₀ oder durch C₂-C₂₀-Alkyl, das durch ein oder mehrere 0, S oder NR₂₆ unterbrochen ist, substituiert ist, steht;
oder R₁₆ für Phenyl, das durch ein oder mehrere C₁-C₂₀-Alkyl, das unsubstituiert oder durch ein oder mehrere Halogen, COOR₁₇, CONR₁₉R₂₀, Phenyl, C₃-C₈-Cycloalkyl, C₃-C₂₀-Heteroaryl, C₆-C₂₀-Aryloxy-carbonyl, C₄-C₂₀-Heteroaryloxycarbonyl, OR₁₇, SR₁₈ oder NR₁₉R₂₀ substituiert ist, substituiert ist, steht;
oder R₁₆ für C₁-C₂₀-Alkyl, das unsubstituiert oder durch Halogen, Phenyl, OH, SH, CN, C₃-C₆-Alkenoxy, OCH₂CH₂ (CO)0 (C₁-C₄-Alkyl), O(CO)-(C₁-C₄-Alkyl), oder (CO)O(C₁-C₄-Alkyl) substituiert ist, steht;
R₁₇ für C₁-C₂₀-Alkyl, das unsubstituiert oder durch ein oder mehrere Halogen, OCH₂CH₂(CO)O(C₁-C₄-Alkyl), O(C₁-C₄-Alkyl), (CO)O(C₁-C₄-Alkyl), C₃-C₂₀-Cycloalkyl oder durch C₃-C₂₀-Cycloalkyl, das durch ein oder mehrere 0 unterbrochen ist, substituiert ist, steht;
oder R₁₇ für C₂-C₂₀-Alkyl, das durch ein oder mehrere 0 unterbrochen ist, steht;
R₁₈ für Methyl, das durch (CO)OR₁₇ substituiert ist, steht;
R₁₉ und R₂₀ unabhängig voneinander für Wasserstoff, Phenyl, C₁-C₂₀-Alkyl, C₁-C₈-Alkanoyl oder C₁-C₈-Alkanoyloxy stehen;
oder R₁₉ und R₂₀ zusammen mit dem N-Atom, an das sie gebunden sind, ein heteroaromatisches Ringsystem bilden, das unsubstituiert oder durch 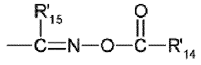 substituiert ist;
mit der Maßgabe, dass in dem Molekül mindestens eine Gruppe 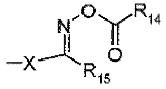 oder 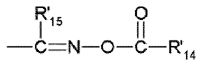 vorliegt.

3. Verbindungen der Formel I nach Anspruch 1, wobei R₁, R₂, R₃, R₄, R₅, R₆, R₇ und R₈ unabhängig voneinander für Wasserstoff, stehen;
oder R₁ und R₂, R₃ und R₄ oder R₅ und R₆ unabhängig voneinander zusammen für 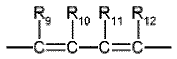 stehen;
mit der Maßgabe, dass mindestens ein Paar von R₁
und R₂, R₃ und R₄ oder R₅ und R₆ für 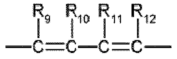 steht;
oder R₂ für 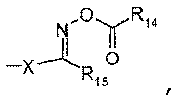 COR₁₆, NO₂ oder 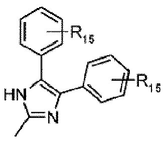 steht;
oder R₇ für 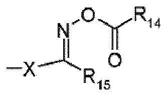 oder COR₁₆ steht;
R₉, R₁₁ und R₁₂ für Wasserstoff stehen;
R₁₀ für Wasserstoff, OR₁₇ oder COR₁₆ steht;
X für CO oder eine direkte Bindung steht;
R₁₃ für C₁-C₂₀-Alkyl, das unsubstituiert oder durch ein oder mehrere Halogen, R₁₇, OR₁₇, SR₁₈ oder durch PO(OCₖH₂ₖ₊₁)₂ substituiert ist, steht;
oder R₁₃ für C₂-C₂₀-Alkyl, das durch ein oder mehrere 0 unterbrochen ist, steht;
oder R₁₃ für Phenyl steht;
k für eine ganze Zahl mit einem Wert von 2 steht; R₁₄ für C₁-C₂₀-Alkyl oder Thienyl steht;
R₁₅ für Phenyl oder Naphthyl steht, wobei jede dieser Gruppen unsubstituiert oder durch ein oder mehrere OR₁₇ oder C₁-C₂₀-Alkyl substituiert ist, oder R₁₅ für Thienyl, Wasserstoff oder C₁-C₂₀-Alkyl, das unsubstituiert oder durch ein oder mehrere OR₁₇, SR₁₈, C₃-C₈-Cycloalkyl, NR₁₉R₂₀ oder durch COOR₁₇ substituiert ist, steht;
oder R₁₅ für C₂-C₂₀-Alkyl, das durch SO₂ unterbrochen ist, steht;
R₁₆ für Phenyl oder Naphthyl steht, wobei jede dieser Gruppen unsubstituiert oder durch ein oder mehrere OR₁₇, SR₁₈, NR₁₉R₂₀ oder durch C₁-C₂₀-Alkyl substituiert ist, steht;
oder R₁₆ für Thienyl steht;
R₁₇ für Wasserstoff, C₁-C₈-Alkanoyl, C₁-C₂₀-Alkyl, das unsubstituiert oder durch ein oder mehrere Halogen, O(CO)-(C₁-C₄-Alkyl), O(CO)-(C₂-C₄-Alkenyl) oder durch C₃-C₂₀-Cycloalkyl, das durch ein oder mehrere 0 unterbrochen ist, substituiert ist, steht;
oder R₁₇ für C₂-C₂₀-Alkyl, das durch ein oder mehrere 0 unterbrochen ist, steht;
R₁₈ für C₃-C₂₀-Cycloalkyl oder C₁-C₂₀-Alkyl, das unsubstituiert oder durch ein oder mehrere OH, O(CO)-(C₂-C₄-Alkenyl) oder (CO)OR₁₇ substituiert ist, steht;
oder R₁₈ für Phenyl, das unsubstituiert oder durch ein oder mehrere Halogen substituiert ist, steht; R₁₉ und R₂₀ unabhängig voneinander für C₁-C₈-Alkanoyl oder C₁-C₈-Alkanoyloxy stehen;
oder R₁₉ und R₂₀ zusammen mit dem N-Atom, an das sie gebunden sind, einen 5- oder 6-gliedrigen gesättigten Ring bilden, der durch 0 unterbrochen ist;
mit der Maßgabe, dass in dem Molekül mindestens eine Gruppe 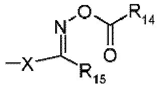 vorliegt.

4. Photopolymerisierbare Zusammensetzung, umfassend
(a) mindestens eine ethylenisch ungesättigte photopolymerisierbare Verbindung und
(b) als Photoinitiator mindestens eine Verbindung der Formel I gemäß Anspruch 1.

5. Photopolymerisierbare Zusammensetzung nach Anspruch 4, wobei es sich bei der Komponente (a) um ein durch Umsetzung eines Anhydrids einer gesättigten oder ungesättigten mehrbasigen Säure mit einem Produkt der Umsetzung eines Epoxidharzes und einer ungesättigten Monocarbonsäure erhaltenes Harz handelt.

6. Photopolymerisierbare Zusammensetzung nach Anspruch 4, umfassend zusätzlich zu dem Photoinitiator (b) mindestens einen weiteren Photoinitiator (c) und/oder weitere Zusatzstoffe (d).

7. Photopolymerisierbare Zusammensetzung nach Anspruch 6, umfassend als weiteren Zusatzstoff (d) ein Pigment oder eine Mischung von Pigmenten.

8. Photopolymerisierbare Zusammensetzung nach Anspruch 7, umfassend als weiteren Zusatzstoff (d) ein Dispergiermittel oder eine Mischung von Dispergiermitteln.

9. Photopolymerisierbare Zusammensetzung nach einem der Ansprüche 4-8, umfassend 0,05 bis 25 Gew.-% des Photoinitiators (b) bzw. der Photoinitiatoren (b) und (c), bezogen auf die Zusammensetzung.

10. Photopolymerisierbare Zusammensetzung nach einem der Ansprüche 4 bis 9, umfassend als weiteren Zusatzstoff (d) einen Photosensibilisator, insbesondere eine Verbindung aus der Gruppe bestehend aus Benzophenon, Benzophenonderivaten, Thioxanthon, Thioxanthonderivaten, Anthrachinon, Anthrachinonderivaten, Cumarin und Cumarinderivaten.

11. Photopolymerisierbare Zusammensetzung nach einem der Ansprüche 4-10, zusätzlich umfassend ein Bindemittelpolymer (e), insbesondere ein Copolymer von Methacrylat und Methacrylsäure.

12. Verfahren zur Herstellung einer Verbindung der Formel I nach Anspruch 1 durch Umsetzung der entsprechenden Oximverbindung mit einem Acylhalogenid der Formel II oder einem Anhydrid der Formel III 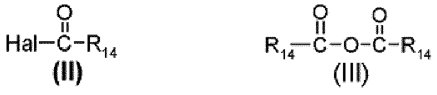 wobei Hal für ein Halogen, insbesondere Cl, steht und R₁₄ wie in Anspruch 1 definiert ist, in Gegenwart einer Base oder einer Mischung von Basen.

13. Verfahren zur Photopolymerisation von Verbindungen mit ethylenisch ungesättigten Doppelbindungen, bei dem man eine Zusammensetzung nach einem der Ansprüche 4-11 mit elektromagnetischer Strahlung im Bereich von 150 bis 600 nm oder mit Elektronenstrahlung oder mit Röntgenstrahlen bestrahlt.

14. Verfahren nach Anspruch 13 zur Herstellung von pigmentierten und nichtpigmentierten Farben und Lacken, Pulverbeschichtungen, Druckfarben, Druckplatten, Klebstoffen, Haftklebstoffen, Dentalzusammensetzungen, Gelcoats, Photoresists für die Elektronik, Galvanisierungsresists, Ätzresists, sowohl flüssigen als auch trockenen Filmen, Lötresists, Resists zur Herstellung von Farbfiltern für verschiedene Anzeigeanwendungen, Resists zur Erzeugung von Strukturen bei Herstellungsverfahren für Plasmaanzeigefelder, Elektrolumineszenzanzeigen und LCD, Abstandshalter für LCD, für die holographische Datenspeicherung (HDS), als Zusammensetzung zum Verkapseln von elektrischen und elektronischen Bauteilen, zur Herstellung von magnetischen Aufzeichnungsmaterialien, mikromechanischen Teilen, Wellenleitern, optischen Schaltern, Plattierungsmasken, Ätzmasken, Farbprüfsystemen, Überzügen für Glasfaserkabel, Siebdruckschablonen, zur Herstellung dreidimensionaler Objekte mittels Stereolithographie, als Bildaufzeichnungsmaterial, für holographische Aufzeichnungen, mikroelektronische Schaltkreise, Entfärbungsmaterialien, Entfärbungsmaterialien für Bildaufzeichnungsmaterialien, für Bildaufzeichnungsmaterialien unter Verwendung von Mikrokapseln, als Photoresistmaterial für ein UV- und VIS-Laser-Direct-Imaging-System, als Photoresistmaterial zur Bildung dielektrischer Schichten in einer sequentiellen Aufbauschicht einer Leiterplatte.

15. Beschichtetes Substrat, das auf mindestens einer Oberfläche mit einer Zusammensetzung nach Anspruch 4 beschichtet ist.

16. Verfahren zur photographischen Herstellung von Reliefbildern, bei dem man ein beschichtetes Substrat nach Anspruch 15 bildmäßig belichtet und dann die unbelichteten Teile mit einem Entwickler entfernt.

17. Farbfilter, hergestellt durch Bereitstellen von roten, grünen und blauen Bildelementen und einer schwarzen Matrix, die alle ein lichtempfindliches Harz und ein Pigment auf einem transparenten Substrat umfassen, und Bereitstellen einer transparenten Elektrode entweder auf der Oberfläche des Substrats oder auf der Oberfläche der Farbfilterschicht, wobei das lichtempfindliche Harz ein polyfunktionelles Acrylatmonomer, ein organisches Polymerbindemittel und einen Photopolymerisationsinitiator der Formel I gemäß Anspruch 1 umfasst.

18. Verwendung einer Verbindung der Formel (I) gemäß Anspruch 1 zur Photopolymerisation einer Zusammensetzung, die mindestens eine ethylenisch ungesättigte photopolymerisierbare Verbindung umfasst.

19. Verbindungen der Formel IA 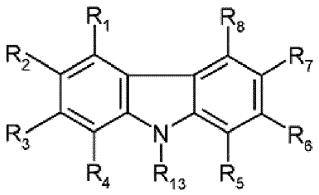 wobei
R₁, R₂, R₃, R₄, R₅, R₆, R₇ und R₈ unabhängig voneinander für Wasserstoff, C₁-C₂₀-Alkyl, 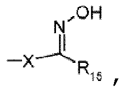 COR₁₆, OR₁₇, Halogen, NO₂ oder 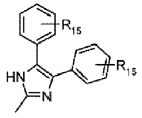 stehen;
oder R₁ und R₂, R₂ und R₃, R₃ und R₄, R₅ und R₆, R₆ und R₇ oder R₇ und R₈ unabhängig voneinander für C₂-C₁₀-Alkenyl, das durch 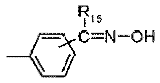 substituiert ist, stehen;
oder R₁ und R₂, R₂ und R₃, R₃ und R₄, R₅ und R₆, R₆ und R₇ oder R₇ und R₈ unabhängig voneinander zusammen für -(CH₂)ₚ-Y-(CH₂)_{q}- stehen;
oder R₁ und R₂, R₂ und R₃, R₃ und R₄, R₅ und R₆, R₆ und R₇ oder R₇ und R₈ unabhängig voneinander zusammen für 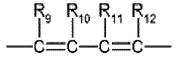 stehen; mit der Maßgabe, dass mindestens ein Paar von R₁ und R₂, R₂ und R₃, R₃ und R₄, R₅ und R₆, R₆ und R₇
oder R₇ und R₈ für 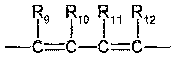 steht,
R₉, R₁₀, R₁₁ und R₁₂ unabhängig voneinander für Wasserstoff oder C₁-C₂₀-Alkyl, das unsubstituiert oder durch ein oder mehrere Halogen, Phenyl, CN, OH, SH, C₁-C₄-Alkoxy, (CO)OH oder durch (CO)O(C₁-C₄-Alkyl) substituiert ist, stehen;
oder R₉, R₁₀, R₁₁ und R₁₂ unabhängig voneinander für unsubstituiertes Phenyl oder Phenyl, das durch ein oder mehrere C₁-C₆-Alkyl, Halogen, CN, OR₁₇, SR₁₈ oder durch NR₁₉R₂₀ substituiert ist, stehen;
oder R₉, R₁₀, R₁₁ und R₁₂ unabhängig voneinander für Halogen, CN, OR₁₇, SR₁₈, SOR₁₈, SO₂R₁₈ oder NR₁₉R₂₀ stehen, wobei die Substituenten OR₁₇, SR₁₈ oder NR₁₉R₂₀ gegebenenfalls über die Reste R₁₇, R₁₈, R₁₉ und/oder R₂₀ mit einem der Kohlenstoffatome des Naphthylrings 5- oder 6-gliedrige Ringe bilden; oder R₉, R₁₀, R₁₁ und R₁₂ unabhängig voneinander für 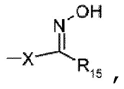 COR₁₆ oder NO₂ stehen;
Y für 0, S, NR₂₆ oder eine direkte Bindung steht;
p für eine ganze Zahl mit einem Wert von 0, 1, 2 oder 3 steht;
q für eine ganze Zahl mit einem Wert von 1, 2 oder 3 steht;
X für CO oder eine direkte Bindung steht;
R₁₃ für C₁-C₂₀-Alkyl, das unsubstituiert oder durch ein oder mehrere Halogen, R₁₇, COOR₁₇, OR₁₇, SR₁₈,
CONR₁₉R₂₀, NR₁₉R₂₀, PO (OCₖH₂ₖ₊₁)₂ oder durch 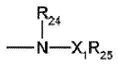 substituiert ist, steht;
oder R₁₃ für C₂-C₂₀-Alkyl, das durch ein oder mehrere 0, S, SO, SO₂, NR₂₆ oder CO unterbrochen ist, oder für C₂-C₁₂-Alkenyl, das ununterbrochen oder durch ein oder mehrere 0, CO oder NR₂₆ unterbrochen ist, steht,
wobei das unterbrochene C₂-C₂₀-Alkyl und das ununterbrochene oder unterbrochene C₂-C₁₂-Alkenyl unsubstituiert oder durch ein oder mehrere Halogen substituiert sind;
oder R₁₃ für C₄-C₈-Cycloalkenyl, C₂-C₁₂-Alkinyl oder C₃-C₁₀-Cycloalkyl, das ununterbrochen oder durch ein oder mehrere 0, S, CO oder NR₂₆ unterbrochen ist, steht;
oder R₁₃ für Phenyl oder Naphthyl steht, wobei jede dieser Gruppen unsubstituiert oder durch ein oder mehrere OR₁₇, SR₁₈, NR₁₉R₂₀, 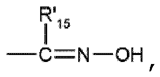 COR₁₆, CN, NO₂, Halogen, C₁-C₂₀-Alkyl, C₁-C₄-Halogenalkyl, C₂-C₂₀-Alkyl, das durch ein oder mehrere 0, S, CO oder NR₂₆ unterbrochen ist, substituiert ist oder jede dieser Gruppen durch C₃-C₁₀-Cycloalkyl oder C₃-C₁₀-Cycloalkyl, das durch ein oder mehrere 0, S, CO oder NR₂₆ unterbrochen ist, substituiert ist;
k für eine ganze Zahl mit einem Wert von 1-10 steht;
R₁₅ für C₆-C₂₀-Aryl oder C₃-C₂₀-Heteroaryl steht, wobei jede dieser Gruppen unsubstituiert oder durch ein oder mehrere Phenyl, Halogen, C₁-C₄-Halogenalkyl, CN, NO₂, OR₁₇, SR₁₈, NR₁₉R₂₀, PO(OCₖH₂ₖ₊₁)₂, SO-C₁-C₁₀-Alkyl, SO₂-C₁-C₁₀-Alkyl, durch C₂-C₂₀-Alkyl, das durch ein oder mehrere 0, S oder NR₂₆ unterbrochen ist, substituiert ist oder wobei jede dieser Gruppen durch C₁-C₂₀-Alkyl, das unsubstituiert oder durch ein oder mehrere Halogen, COOR₁₇, CONR₁₉R₂₀, Phenyl, C₃-C₈-Cycloalkyl, C₃-C₂₀-Heteroaryl, C₆-C₂₀-Aryloxycarbonyl, C₃-C₂₀-Heteroaryloxycarbonyl, OR₁₇, SR₁₈ oder NR₁₉R₂₀ substituiert ist, substituiert ist;
oder R₁₅ für Wasserstoff, C₂-C₁₂-Alkenyl, C₃-C₈-Cycloalkyl, das ununterbrochen oder durch ein oder mehrere 0, CO oder NR₂₆ unterbrochen ist, steht; oder R₁₅ für C₁-C₂₀-Alkyl, das unsubstituiert oder durch ein oder mehrere Halogen, OR₁₇, SR₁₈, C₃-C₈-Cycloalkyl, C₃-C₂₀-Heteroaryl, C₆-C₂₀-Aryloxy-carbonyl, C₃-C₂₀-Heteroaryloxycarbonyl, NR₁₉R₂₀, COOR₁₇, CONR₁₉R₂₀, PO(OCₖH₂ₖ₊₁)₂, 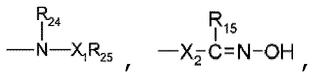 Phenyl substituiert ist, steht oder das C₁-C₂₀-Alkyl durch Phenyl, das durch Halogen, C₁-C₂₀-Alkyl, C₁-C₄-Halogenalkyl, OR₁₇, SR₁₈ oder NR₁₉R₂₀, substituiert ist, substituiert ist;
oder R₁₅ für C₂-C₂₀-Alkyl, das durch ein oder mehrere 0, SO oder SO₂ unterbrochen ist, steht,
wobei das unterbrochene C₂-C₂₀-Alkyl durch ein oder mehrere Halogen, OR₁₇, COOR₁₇, CONR₁₉R₂₀, Phenyl oder durch Phenyl, das durch OR₁₇, SR₁₈ oder NR₁₉R₂₀ substituiert ist, substituiert ist;
oder R₁₅ für C₂-C₂₀-Alkanoyl oder Benzoyl, das unsubstituiert oder durch ein oder mehrere C₁-C₆-Alkyl, Halogen, Phenyl, OR₁₇, SR₁₈ oder NR₁₉R₂₀ substituiert ist, steht;
oder R₁₅ für Naphthoyl, das unsubstituiert oder durch ein oder mehrere OR₁₇ substituiert ist, oder für C₃-C₁₄-Heteroarylcarbonyl steht;
oder R₁₅ für C₂-C₁₂-Alkoxycarbonyl, das ununterbrochen oder durch ein oder mehrere 0 unterbrochen ist, steht, wobei das unterbrochene oder ununterbrochene C₂-C₁₂-Alkoxycarbonyl unsubstituiert oder durch eine oder mehrere Hydroxylgruppen substituiert ist;
oder R₁₅ für Phenoxycarbonyl, das unsubstituiert oder durch ein oder mehrere C₁-C₆-Alkyl, Halogen, C₁-C₄-Halogenalkyl, Phenyl, OR₁₇, SR₁₈ oder NR₁₉R₂₀ substituiert ist, steht;
oder R₁₅ für CN, CONR₁₉R₂₀, NO₂, C₁-C₄-Halogenalkyl, S(O)ₘ-C₁-C₆-Alkyl oder S(O)ₘ-Phenyl, das unsubstituiert oder durch C₁-C₁₂-Alkyl oder SO₂-C₁-C₆-Alkyl substituiert ist, steht;
oder R₁₅ für SO₂O-Phenyl, das unsubstituiert oder durch C₁-C₁₂-Alkyl substituiert ist, steht; oder für Diphenylphosphinoyl oder Di(C₁-C₄-alkoxy)-phosphinoyl steht;
m für 1 oder 2 steht;
R'₁₅ eine der für R₁₅ angegebenen Bedeutungen besitzt;
X₁ für 0, S, SO oder SO₂ steht;
X₂ für 0, CO, S oder eine direkte Bindung steht; R₁₆ für C₆-C₂₀-Aryl oder C₃-C₂₀-Heteroaryl steht, wobei jede dieser Gruppen unsubstituiert oder durch ein oder mehrere Phenyl, Halogen, C₁-C₄-Halogenalkyl, CN, NO₂, OR₁₇, SR₁₈, NR₁₉R₂₀ oder durch C₁-C₂₀-Alkyl, das durch ein oder mehrere 0, S oder NR₂₆ unterbrochen ist, substituiert ist oder wobei jede dieser Gruppen durch ein oder mehrere C₁-C₂₀-Alkyl, das unsubstituiert oder durch ein oder mehrere Halogen, COOR₁₇, CONR₁₉R₂₀, Phenyl, C₃-C₈-Cycloalkyl, C₃-C₂₀-Heteroaryl, C₆-C₂₀-Aryloxy-carbonyl, C₃-C₂₀-Heteroaryloxycarbonyl, OR₁₇, SR₁₈ oder NR₁₉R₂₀ substituiert ist, substituiert ist; oder R₁₆ für Wasserstoff oder C₁-C₂₀-Alkyl, das unsubstituiert oder durch ein oder mehrere Halogen, Phenyl, OH, SH, CN, C₃-C₆-Alkenoxy, OCH₂CH₂CN, OCH₂CH₂ (CO)0(C₁-C₄-Alkyl), O(CO)-(C₁-C₄-Alkyl), O(CO)-Phenyl, (CO)OH oder (CO)O(C₁-C₄-Alkyl) substituiert ist, steht;
oder R₁₆ für C₂-C₁₂-Alkyl, das durch ein oder mehrere 0, S oder NR₂₆ unterbrochen ist, steht; oder R₁₆ für (CH₂CH₂O)ₙ₊₁H, (CH₂CH₂O)ₙ(CO)-(C₁-C₈-Alkyl), C₂-C₁₂-Alkenyl oder C₃-C₈-Cycloalkyl steht; oder R₁₆ für Phenyl, das durch SR₁₈ substituiert ist, steht, wobei der Rest R₁₈ eine direkte Bindung zum Phenyl- bzw. Naphthylring der Carbazolgruppierung, an die die COR₁₆-Gruppe gebunden ist, bedeutet;
n für 1-20 steht;
R₁₇ für Wasserstoff, Phenyl-C₁-C₃-alkyl oder C₁-C₂₀-Alkyl, das unsubstituiert oder durch ein oder mehrere Halogen, OH, SH, CN, C₃-C₆-Alkenoxy, OCH₂CH₂CN, OCH₂CH₂ (CO)0 (C₁-C₄-Alkyl), O(CO)-(C₁-C₄-Alkyl), O(CO)-(C₂-C₄-Alkenyl), O(CO)-Phenyl, (CO)OH, (CO)O(C₁-C₄-Alkyl), SO₂-(C₁-C₄-Halogenalkyl), 0 (C₁-C₄-Halogenalkyl), C₃-C₂₀-Cycloalkyl oder durch C₃-C₂₀-Cycloalkyl, das durch ein oder mehrere 0 unterbrochen ist, substituiert ist, steht;
oder R₁₇ für C₂-C₂₀-Alkyl, das durch ein oder mehrere 0, S oder NR₂₆ unterbrochen ist, steht; oder R₁₇ für (CH₂CH₂O)ₙ₊₁H, (CH₂CH₂O)ₙ(CO)-(C₁-C₈-Alkyl), C₁-C₈-Alkanoyl, C₂-C₁₂-Alkenyl, C₃-C₆-Alkenoyl oder C₃-C₂₀-Cycloalkyl steht, das ununterbrochen oder durch ein oder mehrere 0, S, CO oder NR₂₆ unterbrochen ist;
oder R₁₇ für C₁-C₈-Alkyl-C₃-C₁₀-cykloalkyl steht, das ununterbrochen oder durch ein oder mehrere 0 unterbrochen ist;
oder R₁₇ für Benzoyl, das unsubstituiert oder durch ein oder mehrere C₁-C₆-Alkyl, Halogen, OH oder C₁-C₃-Alkoxy substituiert ist, steht;
oder R₁₇ für Phenyl, Naphthyl oder C₃-C₂₀-Heteroaryl steht, wobei jede dieser Gruppen unsubstituiert oder durch ein oder mehrere Halogen, OH, C₁-C₁₂-Alkyl, C₁-C₁₂-Alkoxy, CN, NO₂, Phenyl-C₁-C₃-alkyloxy, Phenoxy, C₁-C₁₂-Alkylsulfanyl, Phenylsulfanyl, N(C₁-C₁₂-Alkyl)₂, Diphenylamino oder 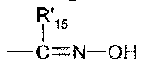 substituiert ist;
oder R₁₇ eine direkte Bindung zu einem der Kohlenstoffatome des Phenyl- bzw. Naphthylrings, an dem sich die Gruppe 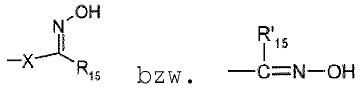 befindet, bildet;
R₁₈ für Wasserstoff, C₂-C₁₂-Alkenyl, C₃-C₂₀-Cycloalkyl oder Phenyl-C₁-C₃-alkyl steht, wobei das C₂-C₁₂-Alkenyl, C₃-C₂₀-Cycloalkyl oder Phenyl-C₁-C₃-alkyl ununterbrochen oder durch ein oder mehrere 0, S, CO, NR₂₆ oder COOR₁₇ unterbrochen ist;
oder R₁₈ für C₁-C₂₀-Alkyl, das unsubstituiert oder durch ein oder mehrere OH, SH, CN, C₃-C₆-Alkenoxy, OCH₂CH₂CN, OCH₂CH₂ (CO)0 (C₁-C₄-Alkyl), O(CO)-(C₂-C₄-Alkenyl), O(CO)-(C₁-C₄-Alkyl), O(CO)-Phenyl oder (CO)OR₁₇ substituiert ist, steht;
oder R₁₈ für C₂-C₂₀-Alkyl, das durch ein oder mehrere 0, S, CO, NR₂₆ oder COOR₁₇ unterbrochen ist, steht;
oder R₁₈ für (CH₂CH₂O)ₙH, (CH₂CH₂O)ₙ(CO)-(C₁-C₈-Alkyl), C₂-C₈-Alkanoyl oder C₃-C₆-Alkenoyl steht; oder R₁₈ für Benzoyl, das unsubstituiert oder durch ein oder mehrere C₁-C₆-Alkyl, Halogen, OH, C₁-C₄-Alkoxy oder C₁-C₄-Alkylsulfanyl substituiert ist, steht;
oder R₁₈ für Phenyl, Naphthyl oder C₃-C₂₀-Heteroaryl steht, wobei jede dieser Gruppen unsubstituiert oder durch ein oder mehrere Halogen, C₁-C₁₂-Alkyl, C₁-C₄-Halogenalkyl, C₁-C₁₂-Alkoxy, CN, NO₂, Phenyl-C₁-C₃-alkyloxy, Phenoxy, C₁-C₁₂-Alkylsulfanyl, Phenylsulfanyl, N(C₁-C₁₂-Alkyl)₂, Diphenylamino, (CO)O(C₁-C₈-Alkyl), (CO)-C₁-C₈-Alkyl, (CO)N(C₁-C₈-Alkyl)₂ oder 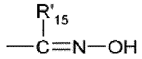 substituiert ist;
R₁₉ und R₂₀ unabhängig voneinander für Wasserstoff, C₁-C₂₀-Alkyl, C₂-C₄-Hydroxyalkyl, C₂-C₁₀-Alkoxyalkyl, C₂-C₅-Alkenyl, C₃-C₂₀-Cycloalkyl, Phenyl-C₁-C₃-alkyl, SO₂-(C₁-C₄-Halogenalkyl), C₁-C₈-Alkanoyl, C₁-C₈-Alkanoyloxy, C₃-C₁₂-Alkenoyl oder Benzoyl stehen; oder R₁₉ und R₂₀ für Phenyl, Naphthyl oder C₃-C₂₀-Heteroaryl stehen, wobei jede dieser Gruppen unsubstituiert oder durch ein oder mehrere Halogen, C₁-C₄-Halogenalkyl, C₁-C₂₀-Alkoxy, C₁-C₁₂-Alkyl, Benzoyl oder C₁-C₁₂-Alkoxy substituiert ist; oder R₁₉ und R₂₀ zusammen mit dem N-Atom, an das sie gebunden sind, einen 5- oder 6-gliedrigen gesättigten oder ungesättigten Ring bilden, der ununterbrochen oder durch 0, S oder NR₁₇ unterbrochen ist und unsubstituiert oder durch ein oder mehrere C₁-C₂₀-Alkyl, C₁-C₂₀-Alkoxy, =0, OR₁₇, SR₁₈, NR₂₁R₂₂, (CO)R₂₃, NO₂, Halogen, C₁-C₄-Halogenalkyl, CN, Phenyl, 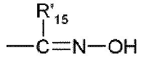 oder durch C₃-C₂₀-Cycloalkyl, das ununterbrochen oder durch ein oder mehrere 0, S, CO oder NR₁₇ unterbrochen ist, substituiert ist;
oder R₁₉ und R₂₀ zusammen mit dem N-Atom, an das sie gebunden sind, ein heteroaromatisches Ringsystem bilden, das unsubstituiert oder durch ein oder mehrere C₁-C₂₀-Alkyl, C₁-C₄-Halogenalkyl, C₁-C₂₀-Alkoxy, =0, OR₁₇, SR₁₈, NR₂₁R₂₂, (CO)R₂₃, 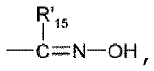 Halogen, NO₂, CN, Phenyl oder durch C₃-C₂₀-Cycloalkyl, das ununterbrochen oder durch ein oder mehrere 0, S, CO oder NR₁₇ unterbrochen ist, substituiert ist;
R₂₁ und R₂₂ unabhängig voneinander für Wasserstoff, C₁-C₂₀-Alkyl, C₁-C₄-Halogenalkyl, C₃-C₁₀-Cycloalkyl oder Phenyl stehen;
oder R₂₁ und R₂₂ zusammen mit dem N-Atom, an das sie gebunden sind, einen 5- oder 6-gliedrigen gesättigten oder ungesättigten Ring bilden, der durch 0, S oder NR₂₆ unterbrochen ist und nicht kondensiert ist oder an den ein Benzolring kondensiert ist;
R₂₃ für Wasserstoff, OH, C₁-C₂₀-Alkyl, C₁-C₄-Halogenalkyl, C₂-C₂₀-Alkyl, das durch ein oder mehrere 0, CO oder NR₂₆ unterbrochen ist, oder C₃-C₂₀-Cycloalkyl, das ununterbrochen oder durch 0, S, CO oder NR₂₆ unterbrochen ist, steht;
oder R₂₃ für Phenyl, Naphthyl, Phenyl-C₁-C₄-alkyl, OR₁₇, SR₁₈ oder NR₂₁R₂₂ steht;
R₂₄ für (CO)OR₁₇, CONR₁₉R₂₀ oder (CO)R₁₇ steht oder eine der für R₁₉ und R₂₀ angegebenen Bedeutungen besitzt;
R₂₅ für COOR₁₇, CONR₁₉R₂₀ oder (CO)R₁₇ steht oder eine der für R₁₇ angegebenen Bedeutungen besitzt;
R₂₆ für Wasserstoff, C₁-C₂₀-Alkyl, C₁-C₄-Halogen-alkyl, C₂-C₂₀-Alkyl, das durch ein oder mehrere 0 oder CO unterbrochen ist, oder für Phenyl-C₁-C₄-alkyl, C₃-C₈-Cycloalkyl, das ununterbrochen oder durch ein oder mehrere 0 oder CO unterbrochen ist, oder für (CO)R₁₉ oder für Phenyl, das unsubstituiert oder durch ein oder mehrere C₁-C₂₀-Alkyl, Halogen, C₁-C₄-Halogenalkyl, OR₁₇, SR₁₈, NR₁₉R₂₀ oder 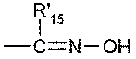 substituiert ist, steht;
mit der Maßgabe, dass in dem Molekül mindestens eine Gruppe 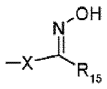 oder 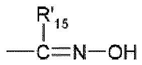 vorliegt.

## Revendications

1. Composés de formule I 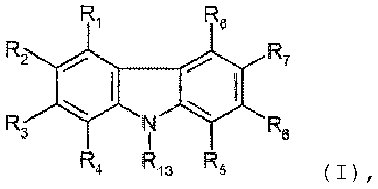 dans laquelle
**R₁, R₂, R₃, R₄, R₅, R₆, R₇** et **R₈,** indépendamment les uns des autres, représentent un hydrogène, un alkyle en C₁-C₂₀, 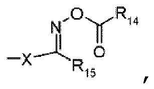 COR₁₆, OR₁₇, un halogène, NO₂, ou 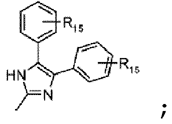 ou bien R₁ et R₂, R₂ et R₃, R₃ et R₄, R₅ et R₆, R₆ et R₇, ou R₇ et R₈, indépendamment les uns des autres, représentent un alcényle en C₂-C₁₀ qui est substitué par 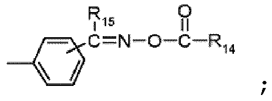 ou bien R₁ et R₂, R₂ et R₃, R₃ et R₄, R₅ et R₆, R₆ et R₇, ou R₇ et R₈, indépendamment les uns des autres, représentent ensemble -(CH₂)ₚ-Y-(CH2)_{q}- ;
ou bien R₁ et R₂, R₂ et R₃, R₃ et R₄, R₅ et R₆, R₆ et R₇, ou R₇ et R₈, indépendamment les uns des autres, représentent ensemble 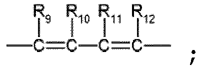 ***à condition qu*'**au moins une paire parmi R₁ et R₂, R₂ et R₃, R₃ et R₄, R₅ et R₆, R₆ et R₇, et R₇ et R₈ représente 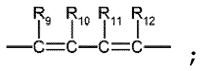 **R₉, R₁₀, R₁₁** et **R₁₂,** indépendamment les uns des autres, représentent un hydrogène, un alkyle en C₁-C₂₀ qui est non substitué ou substitué par un ou plusieurs groupes halogéno, phényle, CN, OH, SH, alcoxy en C₁-C₄, (CO)OH, ou par un groupe (CO)O(alkyle en C₁-C₄) ;
ou bien R₉, R₁₀, R₁₁ et R₁₂, indépendamment les uns des autres, représentent un phényle non substitué ou un phényle substitué par un ou plusieurs groupes alkyle en C₁-C₆, halogéno, CN, OR₁₇, SR₁₈, ou par NR₁₉R₂₀ ;
ou bien R₉, R₁₀, R₁₁ et R₁₂, indépendamment les uns des autres, représentent un halogène, CN, OR₁₇, SR₁₈, SOR₁₈, SO₂R₁₈ ou NR₁₉R₂₀, les substituants OR₁₇, SR₁₈ ou NR₁₉R₂₀ formant éventuellement des cycles à 5 ou 6 chaînons par le biais des radicaux R₁₇, R₁₈, R₁₉ et/ou R₂₀ avec l'un des atomes de carbone du cycle naphtyle ;
ou bien R₉, R₁₀, R₁₁ et R₁₂, indépendamment les uns des
autres, représentent 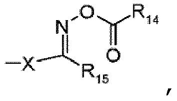 COR₁₆ ou NO₂ ;
**Y** représente 0, S, NR₂₆ ou une liaison directe ;
**p** représente l'entier 0, 1, 2 ou 3 ;
**q** représente l'entier 1, 2 ou 3 ;
**X** représente CO ou une liaison directe ;
**R₁₃** représente un alkyle en C₁-C₂₀ qui est non substitué ou substitué par un ou plusieurs groupes halogéno, R₁₇, COOR₁₇, OR₁₇, SR₁₈, CONR₁₉R₂₀, NR₁₉R₂₀, PO(OCₖH₂ₖ₊₁)₂, ou par 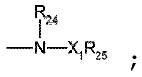 ou bien R₁₃ représente un alkyle en C₂-C₂₀ qui est interrompu par un ou plusieurs 0, S, SO, SO₂, NR₂₆ ou CO, ou représente un alcényle en C₂-C₁₂ qui est non interrompu ou est interrompu par un ou plusieurs 0, CO ou NR₂₆, l'alkyle en C₂-C₂₀ interrompu et l'alcényle en C₂-C₁₂ non interrompu ou interrompu étant non substitués ou étant substitués par un ou plusieurs halogènes ;
ou bien R₁₃ représente un cycloalcényle en C₄-C₈, un alcynyle en C₂-C₁₂, ou un cycloalkyle en C₃-C₁₀ qui est non interrompu ou est interrompu par un ou plusieurs 0, S, CO ou NR₂₆ ;
ou bien R₁₃ représente un phényle ou un naphtyle, chacun d'eux étant non substitué ou substitué par un ou
plusieurs groupes OR₁₇, SR₁₈, NR₁₉R₂₀, 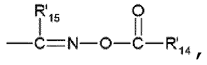 COR₁₆, CN, NO₂, halogéno, alkyle en C₁-C₂₀, halogénoalkyle en C₁-C₄, alkyle en C₂-C₂₀ qui est interrompu par un ou plusieurs 0, S, CO ou NR₂₆, ou chacun d'eux étant substitué par un cycloalkyle en C₃-C₁₀ ou par un cycloalkyle en C₃-C₁₀ qui est interrompu par un ou plusieurs 0, S, CO ou NR₂₆ ;
**k** représente un entier de 1 à 10 ;
**R₁₄** représente un hydrogène, un cycloalkyle en C₃-C₈, un alcényle en C₂-C₅, un alcoxy en C₁-C₂₀ ou un alkyle en C₁-C₂₀ qui est non substitué ou substitué par un ou plusieurs groupes halogéno, phényle, (alkyl en C₁-C₂₀)-phényle ou CN ;
ou bien R₁₄ représente un phényle ou un naphtyle, chacun d'eux étant non substitué ou substitué par un ou plusieurs groupes alkyle en C₁-C₆, halogénoalkyle en C₁-C₄, halogéno, CN, OR₁₇, SR₁₈ et/ou NR₁₉R₂₀ ;
ou bien R₁₄ représente un hétéroaryle en C₃-C₂₀, un alcoxy en C₁-C₈, un benzyloxy ou un phénoxy, lesquels benzyloxy et phénoxy sont non substitués ou substitués par un ou plusieurs groupes alkyle en C₁-C₆, halogénoalkyle en C₁-C₄ et/ou halogéno ;
**R₁₅** représente un aryle en C₆-C₂₀ ou un hétéroaryle en C₃-C₂₀, chacun d'eux étant non substitué ou substitué par un ou plusieurs groupes phényle, halogéno, halogénoalkyle en C₁-C₄, CN, NO₂, OR₁₇, SR₁₈, NR₁₉R₂₀, PO(OCₖH₂ₖ₊₁)₂, SO-(alkyle en C₁-C₁₀), SO₂-(alkyle en C₁-C₁₀), par un alkyle en C₂-C₂₀ qui est interrompu par un ou plusieurs 0, S ou NR₂₆, ou chacun d'eux étant substitué par un alkyle en C₁-C₂₀ qui est non substitué ou substitué par un ou plusieurs groupes halogéno, COOR₁₇, CONR₁₉R₂₀, phényle, cycloalkyle en C₃-C₈, hétéroaryle en C₃-C₂₀, aryloxy-carbonyle en C₆-C₂₀, hétéroaryloxycarbonyle en C₃-C₂₀, OR₁₇, SR₁₈ ou NR₁₉R₂₀ ;
ou bien R₁₅ représente un hydrogène, un alcényle en C₂-C₁₂, un cycloalkyle en C₃-C₈ qui est non interrompu ou est interrompu par un ou plusieurs 0, CO ou NR₂₆ ;
ou bien R₁₅ représente un alkyle en C₁-C₂₀ qui est non substitué ou substitué par un ou plusieurs groupes halogéno, OR₁₇, SR₁₈, cycloalkyle en C₃-C₈, hétéroaryle en C₃-C₂₀, aryloxycarbonyle en C₆-C₂₀, hétéroaryloxy-carbonyle en C₃-C₂₀, NR₁₉R₂₀, COOR₁₇, CONR₁₉R₂₀,
PO(OCₖH₂ₖ₊₁)₂, 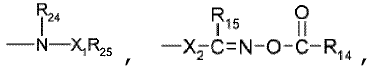 phényle, ou bien l'alkyle en C₁-C₂₀ est substitué par un phényle qui est substitué par un halogène, un alkyle en C₁-C₂₀, un halogénoalkyle en C₁-C₄, OR₁₇, SR₁₈ ou NR₁₉R₂₀ ;
ou bien R₁₅ représente un alkyle en C₂-C₂₀ qui est interrompu par un ou plusieurs 0, SO ou SO₂, et lequel alkyle en C₂-C₂₀ interrompu est non substitué ou substitué par un ou plusieurs groupes halogéno, OR₁₇, COOR₁₇, CONR₁₉R₂₀, phényle ou par un phényle qui est substitué par OR₁₇, SR₁₈ ou NR₁₉R₂₀ ;
ou bien R₁₅ représente un alcanoyle en C₂-C₂₀ ou un benzoyle qui est non substitué ou substitué par un ou plusieurs groupes alkyle en C₁-C₆, halogéno, phényle, OR₁₇, SR₁₈ ou NR₁₉R₂₀ ;
ou bien R₁₅ représente un naphtoyle qui est non substitué ou est substitué par un ou plusieurs OR₁₇ ou représente un hétéroarylcarbonyle en C₃-C₁₄ ;
ou bien R₁₅ représente un alcoxycarbonyle en C₂-C₁₂ qui est non interrompu ou est interrompu par un ou plusieurs 0 et lequel alcoxycarbonyle en C₂-C₁₂ interrompu ou non interrompu est non substitué ou substitué par un ou plusieurs groupes hydroxyle ;
ou bien R₁₅ représente un phénoxycarbonyle qui est non substitué ou substitué par un ou plusieurs groupes alkyle en C₁-C₆, halogéno, halogénoalkyle en C₁-C₄, phényle, OR₁₇, SR₁₈ ou NR₁₉R₂₀ ;
ou bien R₁₅ représente un groupe CN, CONR₁₉R₂₀, NO₂, halogénoalkyle en C₁-C₄, S(O)ₘ-(alkyle en C₁-C₆) ; S(O)ₘ-phényle qui est non substitué ou substitué par un groupe alkyle en C₁-C₁₂ ou SO₂-(alkyle en C₁-C₆) ;
ou bien R₁₅ représente un groupe SO₂O-phényle qui est non substitué ou substitué par un alkyle en C₁-C₁₂ ; ou représente un groupe diphényl-phosphinoyle ou di(alcoxy en C₁-C₄)-phosphinoyle ;
**m** vaut 1 ou 2 ;
**R'₁₄** a l'une des significations telles que données pour R₁₄ ;
**R'₁₅** a l'une des significations telles que données pour R₁₅ ;
**X₁** représente 0, S, SO ou SO₂ ;
**X₂** représente 0, CO, S ou une liaison directe ;
**R₁₆** représente un aryle en C₆-C₂₀ ou un hétéroaryle en C₃-C₂₀, chacun d'eux étant non substitué ou substitué par un ou plusieurs groupes phényle, halogéno, halogénoalkyle en C₁-C₄, CN, NO₂, OR₁₇, SR₁₈, NR₁₉R₂₀, ou par un alkyle en C₁-C₂₀ qui est interrompu par un ou plusieurs 0, S ou NR₂₆, ou chacun d'eux étant substitué par un ou plusieurs groupes alkyle en C₁-C₂₀ qui sont non substitués ou substitués par un ou plusieurs groupes halogéno, COOR₁₇, CONR₁₉R₂₀, phényle, cycloalkyle en C₃-C₈, hétéroaryle en C₃-C₂₀, aryloxycarbonyle en C₆-C₂₀, hétéroaryloxycarbonyle en C₃-C₂₀, OR₁₇, SR₁₈ ou NR₁₉R₂₀ ;
ou bien R₁₆ représente un hydrogène, un alkyle en C₁-C₂₀ qui est non substitué ou substitué par un ou plusieurs groupes halogéno, phényle, OH, SH, CN, alcénoxy en C₃-C₆, OCH₂CH₂CN, OCH₂CH₂(CO)O(alkyle en C₁-C₄), O(CO)-(alkyle en C₁-C₄), O(CO)-phényle, (CO)OH, ou (CO)O(alkyle en C₁-C₄) ; ou bien R₁₆ représente un alkyle en C₂-C₁₂ qui est interrompu par un ou plusieurs 0, S ou NR₂₆ ;
ou bien R₁₆ représente (CH₂CH₂O)ₙ₊₁H, un groupe (CH₂CH₂O)ₙ(CO)-(alkyle en C₁-C₈), un alcényle en C₂-C₁₂ ou un cycloalkyle en C₃-C₈ ;
ou bien R₁₆ représente un phényle substitué par SR₁₈, le radical R₁₈ désignant une liaison directe vers le cycle phényle ou naphtyle de la fraction carbazole à laquelle le groupe COR₁₆ est attaché ;
**n** vaut 1-20 ;
**R₁₇** représente un hydrogène, un groupe phényl-(alkyle en C₁-C₃), un alkyle en C₁-C₂₀ qui est non substitué ou substitué par un ou plusieurs groupes halogéno, OH, SH, CN, alcénoxy en C₃-C₆, OCH₂CH₂CN, OCH₂CH₂ (CO)0 (alkyle en C₁-C₄), O(CO)-(alkyle en C₁-C₄), O(CO)-(alcényle en C₂-C₄), O(CO)-phényle, (CO)OH, (CO)O(alkyle en C₁-C₄), cycloalkyle en C₃-C₂₀, SO₂-(halogénoalkyle en C₁-C₄), 0(halogénoalkyle en C₁-C₄), ou par un cycloalkyle en C₃-C₂₀ qui est interrompu par un ou plusieurs 0 ;
ou bien R₁₇ représente un alkyle en C₂-C₂₀ qui est interrompu par un ou plusieurs 0, S ou NR₂₆ ;
ou bien R₁₇ représente (CH₂CH₂O) ₙ₊₁H, un groupe (CH₂CH₂O)ₙ(CO)-(alkyle en C₁-C₈), un alcanoyle en C₁-C₈, un alcényle en C₂-C₁₂, un alcénoyle en C₃-C₆, ou un cycloalkyle en C₃-C₂₀ qui est non interrompu ou interrompu par un ou plusieurs 0, S, CO ou NR₂₆ ;
ou bien R₁₇ représente un groupe (alkyl en C₁-C₈)-(cycloalkyle en C₃-C₁₀) qui est non interrompu ou interrompu par un ou plusieurs 0 ;
ou bien R₁₇ représente un benzoyle qui est non substitué ou substitué par un ou plusieurs groupes alkyle en C₁-C₆, halogéno, OH ou alcoxy en C₁-C₃ ;
ou bien R₁₇ représente un phényle, un naphtyle ou un hétéroaryle en C₃-C₂₀, chacun d'eux étant non substitué ou substitué par un ou plusieurs groupes halogéno, OH, alkyle en C₁-C₁₂, alcoxy en C₁-C₁₂, CN, NO₂, phényl-(alkyloxy en C₁-C₃), phénoxy, (alkyl en C₁-C₁₂)sulfanyle, phénylsulfanyle, N(alkyle en C₁-C₁₂)₂, diphénylamino ou 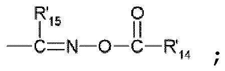 ou bien R₁₇ forme une liaison directe vers l'un des atomes de carbone du cycle phényle ou naphtyle sur lequel le groupe 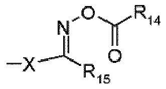 ou 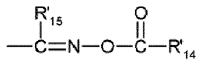 est situé ;
**R₁₈** représente un hydrogène, un alcényle en C₂-C₁₂, un cycloalkyle en C₃-C₂₀, ou un groupe phényl-(alkyle en C₁-C₃), l'alcényle en C₂-C₁₂, le cycloalkyle en C₃-C₂₀ ou le groupe phényl-(alkyle en C₁-C₃) étant non interrompu ou interrompu par un ou plusieurs 0, S, CO, NR₂₆ ou COOR₁₇ ; ou bien R₁₈ représente un alkyle en C₁-C₂₀ qui est non substitué ou est substitué par un ou plusieurs groupes OH, SH, CN, alcénoxy en C₃-C₆, OCH₂CH₂CN, OCH₂CH₂(CO)O(alkyle en C₁-C₄), O(CO)-(alcényle en C₂-C₄), O(CO)-(alkyle en C₁-C₄), O(CO)-phényle ou (CO)OR₁₇ ;
ou bien R₁₈ représente un alkyle en C₂-C₂₀ qui est interrompu par un ou plusieurs 0, S, CO, NR₂₆ ou COOR₁₇ ; ou bien R₁₈ représente (CH₂CH₂O)ₙH, un groupe (CH₂CH₂O)ₙ(CO)-(alkyle en C₁-C₈), un alcanoyle en C₂-C₈ ou un alcénoyle en C₃-C₆ ;
ou bien R₁₈ représente un benzoyle qui est non substitué ou substitué par un ou plusieurs groupes alkyle en C₁-C₆, halogéno, OH, alcoxy en C₁-C₄ ou (alkyl en C₁-C₄)sulfanyle ;
ou bien R₁₈ représente un phényle, un naphtyle ou un hétéroaryle en C₃-C₂₀, chacun d'eux étant non substitué ou substitué par un ou plusieurs groupes halogéno, alkyle en C₁-C₁₂, halogénoalkyle en C₁-C₄, alcoxy en C₁-C₁₂, CN, NO₂, phényl-(alkyloxy en C₁-C₃), phénoxy, (alkyl en C₁-C₁₂)sulfanyle, phénylsulfanyle, N(alkyle en C₁-C₁₂)₂, diphénylamino, (CO)O(alkyle en C₁-C₈), (CO)-(alkyle en C₁-C₈), (CO)N(alkyle en C₁-C₈)₂ ou 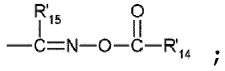 **R₁₉** et **R₂₀**, indépendamment l'un de l'autre, représentent un hydrogène, un alkyle en C₁-C₂₀, un hydroxyalkyle en C₂-C₄, un alcoxyalkyle en C₂-C₁₀, un alcényle en C₂-C₅, un cycloalkyle en C₃-C₂₀, un groupe phényl-(alkyle en C₁-C₃), un alcanoyle en C₁-C₈, un alcanoyloxy en C₁-C₈, un alcénoyle en C₃-C₁₂, un groupe SO₂-(halogénoalkyle en C₁-C₄), ou un benzoyle ;
ou bien R₁₉ et R₂₀ représentent un phényle, un naphtyle ou un hétéroaryle en C₃-C₂₀, chacun d'eux étant non substitué ou substitué par un ou plusieurs groupes halogéno, halogénoalkyle en C₁-C₄, alcoxy en C₁-C₂₀, alkyle en C₁-C₁₂, benzoyle ou alcoxy en C₁-C₁₂ ;
ou bien R₁₉ et **R₂₀**, conjointement avec l'atome N auquel ils sont attachés, forment un cycle saturé ou insaturé à 5 ou 6 chaînons qui est non interrompu ou est interrompu par 0, S ou NR₁₇, et lequel cycle saturé ou insaturé à 5 ou 6 chaînons est non substitué ou substitué par un ou plusieurs groupes alkyle en C₁-C₂₀, alcoxy en C₁-C₂₀, =0, OR₁₇, SR₁₈, NR₂₁R₂₂, (CO)R₂₃, NO₂, halogéno, halogénoalkyle en C₁-C₄, CN, phényle, 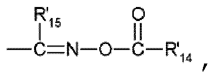 ou par un cycloalkyle en C₃-C₂₀ qui est non interrompu ou est interrompu par un ou plusieurs 0, S, CO ou NR₁₇ ;
ou bien R₁₉ et **R₂₀**, conjointement avec l'atome N auquel ils sont attachés, forment un système cyclique hétéroaromatique qui est non substitué ou substitué par un ou plusieurs groupes alkyle en C₁-C₂₀, halogénoalkyle en C₁-C₄, alcoxy en C₁-C₂₀, =0, OR₁₇, SR₁₈, NR₂₁R₂₂, (CO)R₂₃, 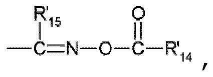 halogéno, NO₂, CN, phényle, ou par un cycloalkyle en C₃-C₂₀ qui est non interrompu ou est interrompu par un ou plusieurs 0, S, CO ou NR₁₇ ;
**R₂₁** et **R₂₂**, indépendamment l'un de l'autre, représentent un hydrogène, un alkyle en C₁-C₂₀, un halogénoalkyle en C₁-C₄, un cycloalkyle en C₃-C₁₀ ou un phényle ;
ou bien R₂₁ et R₂₂, conjointement avec l'atome N auquel ils sont attachés, forment un cycle saturé ou insaturé à 5 ou 6 chaînons qui est non interrompu ou est interrompu par 0, S ou NR₂₆, et lequel cycle saturé ou insaturé à 5 ou 6 chaînons n'est pas condensé ou auquel cycle saturé ou insaturé à 5 ou 6 chaînons est condensé un cycle benzène ;
**R₂₃** représente un hydrogène, OH, un alkyle en C₁-C₂₀, un halogénoalkyle en C₁-C₄, un alkyle en C₂-C₂₀ qui est interrompu par un ou plusieurs 0, CO ou NR₂₆, un cycloalkyle en C₃-C₂₀ qui est non interrompu ou est interrompu par 0, S, CO ou NR₂₆ ;
ou bien R₂₃ représente un phényle, un naphtyle, un groupe phényl-(alkyle en C₁-C₄), OR₁₇, SR₁₈ ou NR₂₁R₂₂ ; **R₂₄** représente (CO)OR₁₇, CONR₁₉R₂₀, (CO)R₁₇ ; ou bien R₂₄ a l'une des significations données pour R₁₉ et R₂₀ ;
**R₂₅** représente COOR₁₇, CONR₁₉R₂₀, (CO)R₁₇ ; ou bien R₂₅ a l'une des significations données pour R₁₇ ;
**R₂₆** représente un hydrogène, un alkyle en C₁-C₂₀, un halogénoalkyle en C₁-C₄, un alkyle en C₂-C₂₀ qui est interrompu par un ou plusieurs 0 ou CO, ou représente un groupe phényl-(alkyle en C₁-C₄), un cycloalkyle en C₃-C₈ qui est non interrompu ou est interrompu par un ou plusieurs 0 ou CO, ou représente (CO)R₁₉, ou représente un phényle qui est non substitué ou substitué par un ou plusieurs groupes alkyle en C₁-C₂₀, halogéno, halogénoalkyle en C₁-C₄, OR₁₇, SR₁₈, NR₁₉R₂₀ ou 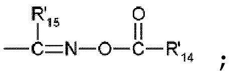 ***à condition qu*'**au moins un groupe 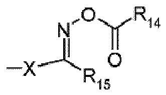 ou 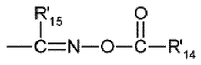 soit présent dans la molécule.

2. Composés de formule I selon la revendication 1, dans lesquels
**R₁, R₂, R₃, R₄, R₅, R₆, R₇** et **R₈,** indépendamment les uns des autres, représentent un hydrogène, un alkyle en C₁-C₂₀, 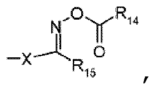 COR₁₆ ou NO₂ ;
ou bien R₁ et R₂, R₂ et R₃, R₃ et R₄, R₅ et R₆, R₆ et R₇, ou R₇ et R₈, indépendamment les uns des autres, représentent ensemble 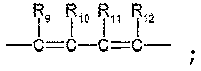 ***à condition qu'***au moins une paire parmi R₁ et R₂, R₂ et R₃, R₃ et R₄, R₅ et R₆, R₆ et R₇, et R₇ et R₈ représente 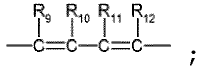 **X** représente CO ou une liaison directe ;
**R₁₃** représente un alkyle en C₁-C₂₀ qui est non substitué ou substitué par un ou plusieurs groupes halogéno, OR₁₇, SR₁₈, COOR₁₇, CONR₁₉R₂₀, ou par PO(OCₖH₂ₖ₊₁)₂ ;
ou bien R₁₃ représente un alkyle en C₂-C₂₀ qui est interrompu par un ou plusieurs 0, S, NR₂₆ ou CO ;
ou bien R₁₃ représente un phényle ou un naphtyle, chacun d'eux étant non substitué ou substitué par un ou
plusieurs 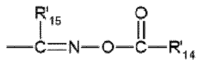 ou COR₁₆ ;
**R₁₄** représente un alkyle en C₁-C₂₀, un phényle ou un alcoxy en C₁-C₈ ;
**R₁₅** représente un phényle, un naphtyle, un hétéroaryle en C₃-C₂₀, chacun d'eux étant non substitué ou substitué par un ou plusieurs groupes phényle, halogéno, halogéno-alkyle en C₁-C₄, OR₁₇, SR₁₈, ou alkyle en C₂-C₂₀ qui est interrompu par un ou plusieurs 0 ou S, ou chacun d'eux étant substitué par un ou plusieurs groupes alkyle en C₁-C₂₀ qui sont non substitués ou substitués par un ou plusieurs groupes halogéno, COOR₁₇, CONR₁₉R₂₀, phényle, cycloalkyle en C₃-C₈, hétéroaryle en C₃-C₂₀, aryloxy-carbonyle en C₆-C₂₀, hétéroaryloxycarbonyle en C₄-C₂₀, OR₁₇, SR₁₈, NR₁₉R₂₀ ou PO(OCₖH₂ₖ₊₁)₂ ;
ou bien R₁₅ représente un alkyle en C₁-C₂₀ qui est non substitué ou substitué par un ou plusieurs groupes OR₁₇, SR₁₈, cycloalkyle en C₃-C₈, hétéroaryle en C₃-C₂₀, NR₁₉R₂₀, COOR₁₇, CONR₁₉R₂₀ ou PO(OCₖH₂ₖ₊₁)₂ ;
**R'₁₄** a l'une des significations telles que données pour R₁₄ ;
**R'₁₅** a l'une des significations telles que données pour R₁₅ ;
**R₁₆** représente un phényle qui est non substitué ou substitué par un ou plusieurs OR₁₇, SR₁₈, NR₁₉R₂₀, ou par un alkyle en C₂-C₂₀ qui est interrompu par un ou plusieurs 0, S ou NR₂₆ ;
ou bien R₁₆ représente un phényle qui est substitué par un ou plusieurs groupes alkyle en C₁-C₂₀ qui sont non substitués ou substitués par un ou plusieurs groupes halogéno, COOR₁₇, CONR₁₉R₂₀, phényle, cycloalkyle en C₃-C₈, hétéroaryle en C₃-C₂₀, aryloxycarbonyle en C₆-C₂₀, hétéroaryloxycarbonyle en C₄-C₂₀, OR₁₇, SR₁₈ ou NR₁₉R₂₀ ; ou bien R₁₆ représente un alkyle en C₁-C₂₀ qui est non substitué ou substitué par un groupe halogéno, phényle, OH, SH, CN, alcénoxy en C₃-C₆, OCH₂CH₂(CO)O(alkyle en C₁-C₄), O(CO)-(alkyle en C₁-C₄), ou (CO)O(alkyle en C₁-C₄) ;
**R₁₇** représente un alkyle en C₁-C₂₀ qui est non substitué ou substitué par un ou plusieurs groupes halogéno, OCH₂CH₂(CO)O(alkyle en C₁-C₄), 0 (alkyle en C₁-C₄), (CO)O(alkyle en C₁-C₄), cycloalkyle en C₃-C₂₀, ou par un cycloalkyle en C₃-C₂₀ qui est interrompu par un ou plusieurs 0 ;
ou bien R₁₇ représente un alkyle en C₂-C₂₀ qui est interrompu par un ou plusieurs 0 ;
**R₁₈** représente un méthyle substitué par (CO)OR₁₇ ;
**R₁₉** et **R₂₀,** indépendamment l'un de l'autre, représentent un hydrogène, un phényle, un alkyle en C₁-C₂₀, un alcanoyle en C₁-C₈ ou un alcanoyloxy en C₁-C₈ ;
ou bien R₁₉ et R₂₀, conjointement avec l'atome N auquel ils sont attachés, forment un système cyclique hétéro-aromatique qui est non substitué ou substitué par 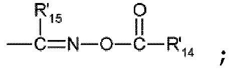 ***à condition qu'***au moins un groupe 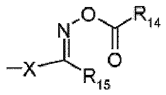 ou 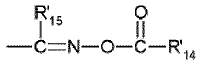 soit présent dans la molécule.

3. Composés de formule I selon la revendication 1, dans lesquels
**R₁, R₂, R₃, R₄, R₅, R₆, R₇** et **R₈,** indépendamment les uns des autres, représentent un hydrogène ;
ou bien R₁ et R₂, R₃ et R₄, ou R₅ et R₆, indépendamment les uns des autres, représentent 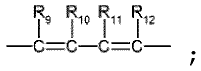 ***à condition qu'***au moins une paire parmi R₁ et R₂, R₃ et R₄, et R₅ et R₆ représente 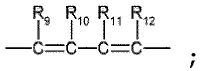 ou bien R₂ représente 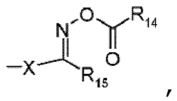 COR₁₆, NO₂, ou 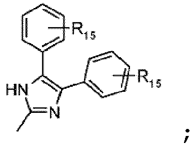 ou bien R₇ représente 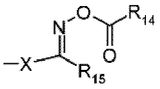 ou COR₁₆ ;
**R₉, R₁₁** et **R₁₂** représentent un hydrogène ;
**R₁₀** représente un hydrogène, OR₁₇ ou COR₁₆ ;
**X** représente CO ou une liaison directe ;
**R₁₃** représente un alkyle en C₁-C₂₀ qui est non substitué ou substitué par un ou plusieurs groupes halogéno, R₁₇, OR₁₇, SR₁₈, ou par PO(OCₖH₂ₖ₊₁)₂ ;
ou bien R₁₃ représente un alkyle en C₂-C₂₀ qui est interrompu par un ou plusieurs O ;
ou bien R₁₃ représente un phényle ;
k représente l'entier 2 ;
**R₁₄** représente un alkyle en C₁-C₂₀ ou un thiényle ;
**R₁₅** représente un phényle ou un naphtyle, chacun d'eux étant non substitué ou substitué par un ou plusieurs groupes OR₁₇ ou alkyle en C₁-C₂₀ ;
ou bien R₁₅ représente un thiényle, un hydrogène, un alkyle en C₁-C₂₀ qui est non substitué ou substitué par un ou plusieurs groupes OR₁₇, SR₁₈, cycloalkyle en C₃-C₈, NR₁₉R₂₀, ou par COOR₁₇ ;
ou bien R₁₅ représente un alkyle en C₂-C₂₀ qui est interrompu par SO₂ ;
**R₁₆** représente un phényle ou un naphtyle, chacun d'eux étant non substitué ou substitué par un ou plusieurs OR₁₇, SR₁₈, NR₁₉R₂₀, ou par un alkyle en C₁-C₂₀ ;
ou bien R₁₆ représente un thiényle ;
**R₁₇** représente un hydrogène, un alcanoyle en C₁-C₈, un alkyle en C₁-C₂₀ qui est non substitué ou substitué par un ou plusieurs groupes halogéno, O(CO)-(alkyle en C₁-C₄), O(CO)-(alcényle en C₂-C₄), ou par un cycloalkyle en C₃-C₂₀ qui est interrompu par un ou plusieurs 0 ;
ou bien R₁₇ représente un alkyle en C₂-C₂₀ qui est interrompu par un ou plusieurs 0 ;
**R₁₈** représente un cycloalkyle en C₃-C₂₀, un alkyle en C₁-C₂₀ qui est non substitué ou substitué par un ou plusieurs groupes OH, O(CO)-(alcényle en C₂-C₄) ou (CO)OR₁₇ ;
ou bien R₁₃ représente un phényle qui est non substitué ou substitué par un ou plusieurs halogènes ;
**R₁₉** et **R₂₀,** indépendamment l'un de l'autre, représentent un alcanoyle en C₁-C₈ ou un alcanoyloxy en C₁-C₈ ;
ou bien R₁₉ et R₂₀, conjointement avec l'atome N auquel ils sont attachés, forment un cycle saturé à 5 ou 6 chaînons qui est interrompu par 0 ;
***à condition qu'***au moins un groupe 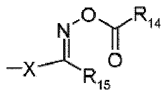 soit présent dans la molécule.

4. Composition photopolymérisable comprenant
(a) au moins un composé photopolymérisable éthyléniquement insaturé et
(b) comme photoinitiateur, au moins un composé de formule I tel que défini dans la revendication 1.

5. Composition photopolymérisable selon la revendication 4, dans laquelle le composant (a) est une résine obtenue par la réaction d'un anhydride d'acide polybasique saturé ou insaturé avec un produit de la réaction d'une résine époxyde et d'un acide monocarboxylique insaturé.

6. Composition photopolymérisable selon la revendication 4 comprenant, en plus du photoinitiateur (b), au moins un autre photoinitiateur (c), et/ou d'autres additifs (d).

7. Composition photopolymérisable selon la revendication 6 comprenant comme autre additif (d) un pigment ou un mélange de pigments.

8. Composition photopolymérisable selon la revendication 7 comprenant comme autre additif (d) un dispersant ou un mélange de dispersants.

9. Composition photopolymérisable selon l'une quelconque des revendications 4 à 8, comprenant 0,05 à 25 % en poids du photoinitiateur (b), ou des photoinitiateurs (b) et (c), rapporté à la composition.

10. Composition photopolymérisable selon l'une quelconque des revendications 4 à 9 comprenant comme autre additif (d) un photosensibilisateur, en particulier un composé choisi dans le groupe constitué par la benzophénone, les dérivés de benzophénone, la thioxanthone, les dérivés de thioxanthone, l'anthraquinone, les dérivés d'anthraquinone, la coumarine et les dérivés de coumarine.

11. Composition photopolymérisable selon l'une quelconque des revendications 4 à 10 comprenant également un polymère liant (e), en particulier un copolymère de méthacrylate et d'acide méthacrylique.

12. Procédé de préparation d'un composé de formule I selon la revendication 1 par réaction du composé oxime correspondant avec un halogénure d'acyle de formule II ou un anhydride de formule III 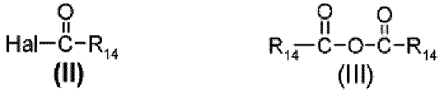 dans lesquelles **Hal** représente un halogène, en particulier Cl, et **R₁₄** est tel que défini dans la revendication 1, en présence d'une base ou d'un mélange de bases.

13. Procédé de photopolymérisation de composés contenant des doubles liaisons éthyléniquement insaturées, qui comprend l'irradiation d'une composition selon l'une quelconque des revendications 4 à 11 avec un rayonnement électromagnétique dans la gamme de 150 à 600 nm, ou avec un faisceau d'électrons ou avec des rayons X.

14. Procédé selon la revendication 13 pour produire des peintures et vernis pigmentés et non pigmentés, des revêtements en poudre, des encres d'impression, des plaques d'impression, des adhésifs, des adhésifs sensibles à la pression, des compositions dentaires, des enduits gélifiés, des résines photosensibles pour l'électronique, des réserves de galvanoplastie, des réserves de gravure, des films à la fois liquides et secs, des épargnes de soudure, des réserves pour fabriquer des filtres colorés pour diverses applications d'affichage, des réserves pour générer des structures dans les procédés de fabrication des écrans à plasma, des écrans électroluminescents et des écrans LCD, des entretoises pour écrans LCD, pour le stockage holographique de données (HDS), comme composition pour encapsuler des composants électriques et électroniques, pour produire des matériaux d'enregistrement magnétique, des pièces micromécaniques, des guides d'ondes, des commutateurs optiques, des masques de placage, des masques de gravure, des systèmes d'épreuvage couleur, des gaines de câbles de fibres de verre, des pochoirs de sérigraphie, pour produire des objets tridimensionnels par stéréolithographie, comme matériau d'enregistrement d'images, pour des enregistrements holographiques, des circuits microélectroniques, des matériaux de décoloration, des matériaux de décoloration pour matériaux d'enregistrement d'images, pour des matériaux d'enregistrement d'images utilisant des microcapsules, comme résine photosensible pour un système d'imagerie directe par laser UV et visible, comme résine photosensible utilisée pour former des couches diélectriques dans une couche élaborée de façon séquentielle d'une carte de circuit imprimé.

15. Substrat revêtu qui est recouvert sur au moins une surface d'une composition selon la revendication 4.

16. Procédé de production photographique d'images en relief, dans lequel un substrat revêtu selon la revendication 15 est soumis à une exposition en forme d'image puis les parties non exposées sont retirées avec un révélateur.

17. Filtré coloré préparé en déposant des éléments d'image rouges, verts et bleus et une matrice noire, comprenant tous une résine photosensible et un pigment, sur un substrat transparent, et en déposant une électrode transparente sur la surface du substrat ou sur la surface de la couche de filtre coloré, ladite résine photosensible comprenant un monomère d'acrylate polyfonctionnel, un liant polymère organique et un photoinitiateur de polymérisation de formule I tel que défini dans la revendication 1.

18. Utilisation d'un composé de formule (I) tel que défini dans la revendication 1 pour la photopolymérisation d'une composition comprenant au moins un composé photopolymérisable éthyléniquement insaturé.

19. Composés de formule (IA) 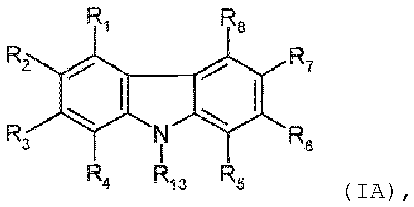 dans laquelle
**R₁, R₂**, **R₃, R₄, R₅, R₆, R₇** et **R₈,** indépendamment les uns des autres, représentent un hydrogène, un alkyle en C₁-C₂₀, 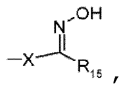 COR₁₆, OR₁₇, un halogène, NO₂, ou 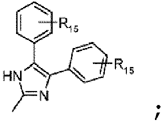 ou bien R₁ et R₂, R₂ et R₃, R₃ et R₄, R₅ et R₆, R₆ et R₇, ou R₇ et R₈, indépendamment les uns des autres, représentent un alcényle en C₂-C₁₀ qui est substitué par 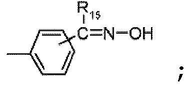 ou bien R₁ et R₂, R₂ et R₃, R₃ et R₄, R₅ et R₆, R₆ et R₇, ou R₇ et R₈, indépendamment les uns des autres, représentent ensemble -(CH₂)ₚ-Y-(CH₂)_{q}- ;
ou bien R₁ et R₂, R₂ et R₃, R₃ et R₄, R₅ et R₆, R₆ et R₇, ou R₇ et R₈, indépendamment les uns des autres,
représentent ensemble 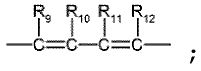 ***à condition qu'***au moins une paire parmi R₁ et R₂, R₂ et R₃, R₃ et R₄, R₅ et R₆, R₆ et R₇, et R₇ et R₈ représente 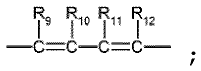 **R₉, R₁₀, R₁₁** et **R₁₂,** indépendamment les uns des autres, représentent un hydrogène, un alkyle en C₁-C₂₀ qui est non substitué ou substitué par un ou plusieurs groupes halogéno, phényle, CN, OH, SH, alcoxy en C₁-C₄, (CO)OH, ou par un groupe (CO)O(alkyle en C₁-C₄) ;
ou bien R₉, R₁₀, R₁₁ et R₁₂, indépendamment les uns des autres, représentent un phényle non substitué ou un phényle substitué par un ou plusieurs groupes alkyle en C₁-C₆, halogéno, CN, OR₁₇, SR₁₈, ou par NR₁₉R₂₀ ;
ou bien R₉, R₁₀, R₁₁ et R₁₂, indépendamment les uns des autres, représentent un halogène, CN, OR₁₇, SR₁₈, SOR₁₈, SO₂R₁₈ ou NR₁₉R₂₀, les substituants OR₁₇, SR₁₈ ou NR₁₉R₂₀ formant éventuellement des cycles à 5 ou 6 chaînons par le biais des radicaux R₁₇, R₁₈, R₁₉ et/ou R₂₀ avec l'un des atomes de carbone du cycle naphtyle ;
ou bien R₉, R₁₀, R₁₁ et R₁₂, indépendamment les uns des
autres, représentent 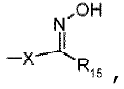 COR₁₆ ou NO₂ ;
**Y** représente 0, S, NR₂₆ ou une liaison directe ;
p représente l'entier 0, 1, 2 ou 3 ;
q représente l'entier 1, 2 ou 3 ;
**X** représente CO ou une liaison directe ;
**R₁₃** représente un alkyle en C₁-C₂₀ qui est non substitué ou substitué par un ou plusieurs groupes halogéno, R₁₇, COOR₁₇, OR₁₇, SR₁₈, CONR₁₉R₂₀, NR₁₉R₂₀, PO(OCₖH₂ₖ₊₁)₂, ou par 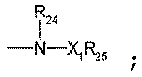 ou bien R₁₃ représente un alkyle en C₂-C₂₀ qui est interrompu par un ou plusieurs 0, S, SO, SO₂, NR₂₆ ou CO, ou représente un alcényle en C₂-C₁₂ qui est non interrompu ou interrompu par un ou plusieurs 0, CO ou NR₂₆, l'alkyle en C₂-C₂₀ interrompu et l'alcényle en C₂-C₁₂ non interrompu ou interrompu étant non substitués ou étant substitués par un ou plusieurs halogènes ;
ou bien R₁₃ représente un cycloalcényle en C₄-C₈, un alcynyle en C₂-C₁₂, ou un cycloalkyle en C₃-C₁₀ qui est non interrompu ou interrompu par un ou plusieurs 0, S, CO ou NR₂₆ ;
ou bien R₁₃ représente un phényle ou un naphtyle, chacun d'eux étant non substitué ou substitué par un ou
plusieurs groupes OR₁₇, SR₁₈, NR₁₉R₂₀, 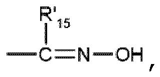 COR₁₆, CN, NO₂, halogéno, alkyle en C₁-C₂₀, halogénoalkyle en C₁-C₄, alkyle en C₂-C₂₀ qui est interrompu par un ou plusieurs 0, S, CO ou NR₂₆, ou chacun d'eux étant substitué par un cycloalkyle en C₃-C₁₀ ou par un cycloalkyle en C₃-C₁₀ qui est interrompu par un ou plusieurs 0, S, CO ou NR₂₆ ;
**k** représente un entier de 1 à 10 ;
**R₁₅** représente un aryle en C₆-C₂₀ ou un hétéroaryle en C₃-C₂₀, chacun d'eux étant non substitué ou substitué par un ou plusieurs groupes phényle, halogéno, halogénoalkyle en C₁-C₄, CN, NO₂, OR₁₇, SR₁₈, NR₁₉R₂₀, PO(OCₖH₂ₖ₊₁)₂, SO-(alkyle en C₁-C₁₀), SO₂-(alkyle en C₁-C₁₀), par un alkyle en C₂-C₂₀ qui est interrompu par un ou plusieurs 0, S ou NR₂₆, ou chacun d'eux étant substitué par un alkyle en C₁-C₂₀ qui est non substitué ou substitué par un ou plusieurs groupes halogéno, COOR₁₇, CONR₁₉R₂₀, phényle, cycloalkyle en C₃-C₈, hétéroaryle en C₃-C₂₀, aryloxy-carbonyle en C₆-C₂₀, hétéroaryloxycarbonyle en C₃-C₂₀, OR₁₇, SR₁₈ ou NR₁₉R₂₀ ;
ou bien R₁₅ représente un hydrogène, un alcényle en C₂-C₁₂, un cycloalkyle en C₃-C₈ qui est non interrompu ou est interrompu par un ou plusieurs 0, CO ou NR₂₆ ;
ou bien R₁₅ représente un alkyle en C₁-C₂₀ qui est non substitué ou substitué par un ou plusieurs groupes halogéno, OR₁₇, SR₁₈, cycloalkyle en C₃-C₈, hétéroaryle en C₃-C₂₀, aryloxycarbonyle en C₆-C₂₀, hétéroaryloxy-carbonyle en C₃-C₂₀, NR₁₉R₂₀, COOR₁₇, CONR₁₉R₂₀,
PO(OCₖH₂ₖ₊₁)₂, 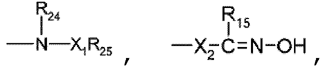 phényle, ou bien l'alkyle en C₁-C₂₀ est substitué par un phényle qui est substitué par un halogène, un alkyle en C₁-C₂₀, un halogénoalkyle en C₁-C₄, OR₁₇, SR₁₈ ou NR₁₉R₂₀ ;
ou bien R₁₅ représente un alkyle en C₂-C₂₀ qui est interrompu par un ou plusieurs 0, SO ou SO₂, et lequel alkyle en C₂-C₂₀ interrompu est non substitué ou substitué par un ou plusieurs groupes halogéno, OR₁₇, COOR₁₇, CONR₁₉R₂₀, phényle ou par un phényle qui est substitué par OR₁₇, SR₁₈ ou NR₁₉R₂₀ ;
ou bien R₁₅ représente un alcanoyle en C₂-C₂₀ ou un benzoyle qui est non substitué ou substitué par un ou plusieurs groupes alkyle en C₁-C₆, halogéno, phényle, OR₁₇, SR₁₈ ou NR₁₉R₂₀ ;
ou bien R₁₅ représente un naphtoyle qui est non substitué ou substitué par un ou plusieurs OR₁₇ ou représente un hétéroarylcarbonyle en C₃-C₁₄ ;
ou bien R₁₅ représente un alcoxycarbonyle en C₂-C₁₂ qui est non interrompu ou interrompu par un ou plusieurs 0 et lequel alcoxycarbonyle en C₂-C₁₂ non interrompu ou interrompu est non substitué ou substitué par un ou plusieurs groupes hydroxyle ;
ou bien R₁₅ représente un phénoxycarbonyle qui est non substitué ou substitué par un ou plusieurs groupes alkyle en C₁-C₆, halogéno, halogénoalkyle en C₁-C₄, phényle, OR₁₇, SR₁₈ ou NR₁₉R₂₀ ;
ou bien R₁₅ représente un groupe CN, CONR₁₉R₂₀, NO₂, halogénoalkyle en C₁-C₄, S(O)ₘ-(alkyle en C₁-C₆) ; S(O)ₘ-phényle qui est non substitué ou substitué par un groupe alkyle en C₁-C₁₂ ou SO₂-(alkyle en C₁-C₆) ;
ou bien R₁₅ représente un groupe SO₂O-phényle qui est non substitué ou substitué par un alkyle en C₁-C₁₂ ; ou représente un groupe diphényl-phosphinoyle ou di(alcoxy en C₁-C₄)-phosphinoyle ;
**m** vaut 1 ou 2 ;
**R'₁₅** a l'une des significations telles que données pour R₁₅ ;
**X₁** représente 0, S, SO ou SO₂ ;
**X₂** représente 0, CO, S ou une liaison directe ;
**R₁₆** représente un aryle en C₆-C₂₀ ou un hétéroaryle en C₃-C₂₀, chacun d'eux étant non substitué ou substitué par un ou plusieurs groupes phényle, halogéno, halogénoalkyle en C₁-C₄, CN, NO₂, OR₁₇, SR₁₈, NR₁₉R₂₀, ou par un alkyle en C₁-C₂₀ qui est interrompu par un ou plusieurs 0, S ou NR₂₆, ou chacun d'eux étant substitué par un ou plusieurs groupes alkyle en C₁-C₂₀ qui sont non substitués ou substitués par un ou plusieurs groupes halogéno, COOR₁₇, CONR₁₉R₂₀, phényle, cycloalkyle en C₃-C₈, hétéroaryle en C₃-C₂₀, aryloxycarbonyle en C₆-C₂₀, hétéroaryloxycarbonyle en C₃-C₂₀, OR₁₇, SR₁₈ ou NR₁₉R₂₀ ;
ou bien R₁₆ représente un hydrogène, un alkyle en C₁-C₂₀ qui est non substitué ou substitué par un ou plusieurs groupes halogéno, phényle, OH, SH, CN, alcénoxy en C₃-C₆, OCH₂CH₂CN, OCH₂CH₂(CO)O(alkyle en C₁-C₄), O(CO)-(alkyle en C₁-C₄), O(CO)-phényle, (CO)OH, ou (CO)O(alkyle en C₁-C₄) ; ou bien R₁₆ représente un alkyle en C₂-C₁₂ qui est interrompu par un ou plusieurs 0, S ou NR₂₆ ;
ou bien R₁₆ représente (CH₂CH₂O)ₙ₊₁H, un groupe (CH₂CH₂O)ₙ(CO)-(alkyle en C₁-C₈)), un alcényle en C₂-C₁₂ ou un cycloalkyle en C₃-C₈ ;
ou bien R₁₆ représente un phényle substitué par SR₁₈, le radical R₁₈ désignant une liaison directe vers le cycle phényle ou naphtyle de la fraction carbazole à laquelle le groupe COR₁₆ est attaché ;
**n** vaut 1-20 ;
**R₁₇** représente un hydrogène, un groupe phényl-(alkyle en C₁-C₃), un alkyle en C₁-C₂₀ qui est non substitué ou substitué par un ou plusieurs groupes halogéno, OH, SH, CN, alcénoxy en C₃-C₆, OCH₂CH₂CN, OCH₂CH₂ (CO)0 (alkyle en C₁-C₄), O(CO)-(alkyle en C₁-C₄), O(CO)-(alcényle en C₂-C₄), O(CO)-phényle, (CO)OH, (CO)O(alkyle en C₁-C₄), SO₂-(halogénoalkyle en C₁-C₄), O(halogénoalkyle en C₁-C₄), cycloalkyle en C₃-C₂₀, ou par un cycloalkyle en C₃-C₂₀ qui est interrompu par un ou plusieurs 0 ;
ou bien R₁₇ représente un alkyle en C₂-C₂₀ qui est interrompu par un ou plusieurs 0, S ou NR₂₆ ;
ou bien R₁₇ représente (CH₂CH₂O)ₙ₊₁H, un groupe (CH₂CH₂O)ₙ(CO)-(alkyle en C₁-C₈), un alcanoyle en C₁-C₈, un alcényle en C₂-C₁₂, un alcénoyle en C₃-C₆, ou un cycloalkyle en C₃-C₂₀ qui est non interrompu ou interrompu par un ou plusieurs 0, S, CO ou NR₂₆ ;
ou bien R₁₇ représente un groupe (alkyl en C₁-C₈)-(cycloalkyle en C₃-C₁₀) qui est non interrompu ou interrompu par un ou plusieurs 0 ;
ou bien R₁₇ représente un benzoyle qui est non substitué ou substitué par un ou plusieurs groupes alkyle en C₁-C₆, halogéno, OH ou alcoxy en C₁-C₃ ;
ou bien R₁₇ représente un phényle, un naphtyle ou un hétéroaryle en C₃-C₂₀, chacun d'eux étant non substitué ou substitué par un ou plusieurs groupes halogéno, OH, alkyle en C₁-C₁₂, alcoxy en C₁-C₁₂, CN, NO₂, phényl-(alkyloxy en C₁-C₃), phénoxy, (alkyl en C₁-C₁₂)sulfanyle, phénylsulfanyle, N(alkyle en C₁-C₁₂)₂, diphénylamino ou 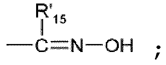 ou bien R₁₇ forme une liaison directe vers l'un des atomes de carbone du cycle phényle ou naphtyle sur
lequel le groupe 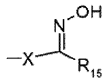 ou 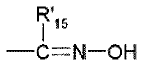 est situé ;
**R₁₈** représente un hydrogène, un alcényle en C₂-C₁₂, un cycloalkyle en C₃-C₂₀, ou un groupe phényl-(alkyle en C₁-C₃), l'alcényle en C₂-C₁₂, le cycloalkyle en C₃-C₂₀ ou le groupe phényl-(alkyle en C₁-C₃) étant non interrompu ou interrompu par un ou plusieurs 0, S, CO, NR₂₆ ou COOR₁₇ ; ou bien R₁₈ représente un alkyle en C₁-C₂₀ qui est non substitué ou est substitué par un ou plusieurs groupes OH, SH, CN, alcénoxy en C₃-C₆, OCH₂CH₂CN, OCH₂CH₂(CO)O(alkyle en C₁-C₄), O(CO)-(alcényle en C₂-C₄), O(CO)-(alkyle en C₁-C₄), O(CO)-phényle ou (CO)OR₁₇ ;
ou bien R₁₈ représente un alkyle en C₂-C₂₀ qui est interrompu par un ou plusieurs 0, S, CO, NR₂₆ ou COOR₁₇ ;
ou bien R₁₈ représente (CH₂CH₂O)ₙH, un groupe (CH₂CH₂O)ₙ(CO)-(alkyle en C₁-C₈), un alcanoyle en C₂-C₈ ou un alcénoyle en C₃-C₆ ;
ou bien R₁₈ représente un benzoyle qui est non substitué ou substitué par un ou plusieurs groupes alkyle en C₁-C₆, halogéno, OH, alcoxy en C₁-C₄ ou (alkyl en C₁-C₄)sulfanyle ;
ou bien R₁₅ représente un phényle, un naphtyle ou un hétéroaryle en C₃-C₂₀, chacun d'eux étant non substitué ou substitué par un ou plusieurs groupes halogéno, alkyle en C₁-C₁₂, halogénoalkyle en C₁-C₄, alcoxy en C₁-C₁₂, CN, NO₂, phényl-(alkyloxy en C₁-C₃), phénoxy, (alkyl en C₁-C₁₂)sulfanyle, phénylsulfanyle, N(alkyle en C₁-C₁₂)₂, diphénylamino, (CO)O(alkyle en C₁-C₈), (CO)-(alkyle en C₁-C₈), (CO)N(alkyle en C₁-C₈)₂ ou 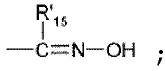 **R₁₉** et **R₂₀,** indépendamment l'un de l'autre, représentent un hydrogène, un alkyle en C₁-C₂₀, un hydroxyalkyle en C₂-C₄, un alcoxyalkyle en C₂-C₁₀, un alcényle en C₂-C₅, un cycloalkyle en C₃-C₂₀, un groupe phényl-(alkyle en C₁-C₃), un groupe SO₂-(halogénoalkyle en C₁-C₄), un alcanoyle en C₁-C₈, un alcanoyloxy en C₁-C₈, un alcénoyle en C₃-C₁₂ ou un benzoyle ;
ou bien R₁₉ et R₂₀ représentent un phényle, un naphtyle ou un hétéroaryle en C₃-C₂₀, chacun d'eux étant non substitué ou substitué par un ou plusieurs groupes halogéno, halogénoalkyle en C₁-C₄, alcoxy en C₁-C₂₀, alkyle en C₁-C₁₂, benzoyle ou alcoxy en C₁-C₁₂ ;
ou bien R₁₉ et R₂₀, conjointement avec l'atome N auquel ils sont attachés, forment un cycle saturé ou insaturé à 5 ou 6 chaînons qui est non interrompu ou est interrompu par 0, S ou NR₁₇, et lequel cycle saturé ou insaturé à 5 ou 6 chaînons est non substitué ou substitué par un ou plusieurs groupes alkyle en C₁-C₂₀, alcoxy en C₁-C₂₀, =0, OR₁₇, SR₁₈, NR₂₁R₂₂, (CO)R₂₃, NO₂, halogéno, halogénoalkyle en C₁-C₄, CN, phényle, 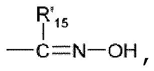 ou par un cycloalkyle en C₃-C₂₀ qui est non interrompu ou est interrompu par un ou plusieurs 0, S, CO ou NR₁₇ ;
ou bien R₁₉ et R₂₀, conjointement avec l'atome N auquel ils sont attachés, forment un système cyclique hétéro-aromatique qui est non substitué ou substitué par un ou plusieurs groupes alkyle en C₁-C₂₀, halogénoalkyle en C₁-C₄, alcoxy en C₁-C₂₀, =0, OR₁₇, SR₁₈, NR₂₁R₂₂, (CO)R₂₃, 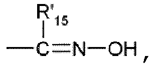 halogéno, NO₂, CN, phényle, ou par un cycloalkyle en C₃-C₂₀ qui est non interrompu ou est interrompu par un ou plusieurs 0, S, CO ou NR₁₇;
**R₂₁** et **R₂₂,** indépendamment l'un de l'autre, représentent un hydrogène, un alkyle en C₁-C₂₀, un halogénoalkyle en C₁-C₄, un cycloalkyle en C₃-C₁₀ ou un phényle ;
ou bien R₂₁ et R₂₂, conjointement avec l'atome N auquel ils sont attachés, forment un cycle saturé ou insaturé à 5 ou 6 chaînons qui est non interrompu ou est interrompu par 0, S ou NR₂₆, et lequel cycle saturé ou insaturé à 5 ou 6 chaînons n'est pas condensé ou auquel cycle saturé ou insaturé à 5 ou 6 chaînons est condensé un cycle benzène ;
**R₂₃** représente un hydrogène, OH, un alkyle en C₁-C₂₀, un halogénoalkyle en C₁-C₄, un alkyle en C₂-C₂₀ qui est interrompu par un ou plusieurs 0, CO ou NR₂₆, un cycloalkyle en C₃-C₂₀ qui est non interrompu ou est interrompu par 0, S, CO ou NR₂₆ ;
ou bien R₂₃ représente un phényle, un naphtyle, un groupe phényl-(alkyle en C₁-C₄), OR₁₇, SR₁₈ ou NR₂₁R₂₂ ; **R₂₄** représente (CO)OR_{17,} CONR₁₉R₂₀, (CO)R₁₇ ; ou bien R₂₄ a l'une des significations données pour R₁₉ et R₂₀ ;
**R₂₅** représente COOR₁₇, CONR₁₉R₂₀, (CO)R₁₇ ; ou bien R₂₅ a l'une des significations données pour R₁₇ ;
**R₂₆** représente un hydrogène, un alkyle en C₁-C₂₀, un halogénoalkyle en C₁-C₄, un alkyle en C₂-C₂₀ qui est interrompu par un ou plusieurs 0 ou CO, ou représente un groupe phényl-(alkyle en C₁-C₄), un cycloalkyle en C₃-C₈ qui est non interrompu ou est interrompu par un ou plusieurs 0 ou CO, ou représente (CO)R₁₉, ou représente un phényle qui est non substitué ou substitué par un ou plusieurs groupes alkyle en C₁-C₂₀, halogéno, halogéno-alkyle en C₁-C₄, OR₁₇, SR₁₈, NR₁₉R₂₀ ou 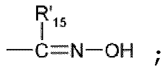 *à condition **qu'***au moins un groupe 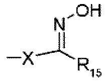 ou 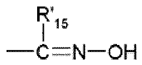 soit présent dans la molécule.
